(19) Europäisches Patentamt
European Patent Office
Office européen des brevets

(11) EP 0 472 207 B1

(12) EUROPEAN PATENT SPECIFICATION

(45) Date of publication and mention of the grant of the patent:
**13.10.1999 Bulletin 1999/41**

(51) Int Cl.[6]: **C12N 15/62**, G01N 33/576 // C12N15/40, C07K2/00

(21) Application number: **91114161.2**

(22) Date of filing: **23.08.1991**

# (54) Hepatitis C assay utilizing recombinant antigens

Nachweis von Hepatits C mit Verwendung rekombinanter Antigene

Essai d'hépatite C utilisant des antigènes recombinants

(84) Designated Contracting States:
**AT BE CH DE DK ES FR GB GR IT LI NL SE**

(30) Priority: **24.08.1990 US 572822**
**07.11.1990 US 614069**

(43) Date of publication of application:
**26.02.1992 Bulletin 1992/09**

(73) Proprietor: **ABBOTT LABORATORIES**
**Abbott Park, Illinois 60064-3500 (US)**

(72) Inventors:
- **Devare, Sushil G.**
  **Northbrook, Illinois 60062 (US)**
- **Desai, Suresh M.**
  **Libertyville, Illinois 60048 (US)**
- **Casey, James M.**
  **Waukegan, IL 60085 (US)**
- **Dawson, George J.**
  **Libertyville, IL 60048 (US)**
- **Lesniewski, Richard R.**
  **Kenosha, WI 53142 (US)**
- **Dailey, Stephen H.**
  **Vernon Hills, IL 60061 (US)**
- **Gutierrez, Robin A.**
  **Gurnee, IL 60031 (US)**
- **Stewart, James Lawrence**
  **Gurnee, IL 60031 (US)**

(74) Representative: **Modiano, Guido, Dr.-Ing. et al**
**Modiano & Associati SpA**
**Via Meravigli, 16**
**20123 Milano (IT)**

(56) References cited:

| | |
|---|---|
| EP-A- 0 253 193 | EP-A- 0 318 216 |
| EP-A- 0 331 961 | EP-A- 0 353 590 |
| EP-A- 0 388 232 | EP-A- 0 442 394 |
| EP-A- 0 445 423 | EP-A- 0 450 931 |
| EP-A- 0 468 527 | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 472 207 B1

## Description

This invention relates generally to an assay for identifying the presence in a sample of an antibody which is immunologically reactive with a hepatitis C virus antigen and specifically to an assay for detecting a complex of an antibody and recombinant antigens representing distinct regions of the HCV genome. Recombinant antigens derived from the molecular cloning and expression in a heterologous expression system of the synthetic DNA sequences representing distinct antigenic regions of the HCV genome can be used as reagents for the detection of antibodies and antigen in body fluids from individuals exposed to hepatitis C virus (HCV).

## BACKGROUND

Acute viral hepatitis is clinically diagnosed by a well-defined set of patient symptoms, including jaundice, hepatic tenderness, and an increase in the serum levels of alanine aminotransferase (ALT) and aspartate aminotransferase. Additional serologic immunoassays are generally performed to diagnose the specific type of viral causative agent. Historically, patients presenting clinical hepatitis symptoms and not otherwise infected by hepatitis A, hepatitis B, Epstein-Barr or cytomegalovirus were clinically diagnosed as having non-A non-B hepatitis (NANBH) by default. The disease may result in chronic liver damage.

Each of the well-known, immunologically characterized hepatitis-inducing viruses, hepatitis A virus (HAV), hepatitis B virus (HBV), and hepatitis D virus (HDV) belongs to a separate family of viruses and has a distinctive viral organization, protein structure, and mode of replication.

Attempts to identify the NANBH virus by virtue of genomic similarity to one of the known hepatitis viruses have failed, suggesting that NANBH has a distinct organization and structure. [Fowler, et al., J. Med. Virol., 12:205-213 (1983) and Weiner, et al., J. Med. Virol., 21: 239-247 (1987)].

Progress in developing assays to detect antibodies specific for NANBH has been particularly hampered by difficulties in correctly identifying antigens associated with NANBH. See, for example, Wands, J., et al., U.S. Patent 4,870,076, Wands, et al., Proc. Nat'l. Acad. Sci., 83:6608-6612 (1986), Ohori, et al., J. Med. Virol., 12:161-178 (1983), Bradley, et al., Proc. Nat'l. Acad. Sci., 84:6277-6281, (1987), Akatsuka, T., et al., J. Med. Virol, 20:43-56 (1986), Seto, B., et al., U.S. Patent Application Number 07/234,641 (available from U. S. Department of Commerce National Technical Information Service, Springfield, Virginia, No. 89138168), Takahashi, K., et al., European Patent Application No. 0 293 274, published November 30, 1988, and Seelig, R., et al., in PCT Application PCT/EP88/00123.

Recently, another hepatitis-inducing virus has been unequivocally identified as hepatitis C virus (HCV) by Houghton, M., et al., European Patent Application publication number 0 318 216, May 31, 1989. Related papers describing this virus include Kuo, G., et al., Science, 244:359-361 (1989) and Choo, Q., et. al, Science, 244:362-364 (1989). Houghton, M., Et al. reported isolating cDNA sequences from HCV which encode antigens which react immunologically with antibodies present in patients infected with NANBH, thus establishing that HCV is one of the viral agents causing NANBH. The cDNA sequences associated with HCV were isolated from a cDNA library prepared from the RNA obtained from pooled serum from a chimpanzee with chronic HCV infection. The cDNA library contained cDNA sequences of approximate mean size of about 200 base pairs. The cDNA library was screened for encoded epitopes expressed in clones that could bind to antibodies in sera from patients who had previously experienced NANBH.

In the European Patent Application, Houghton, M., et al. also described the preparation of several superoxide dismutase fusion polypeptides (SOD) and the use of these SOD fusion polypeptides to develop an HCV screening assay. The most complex SOD fusion polypeptide described in the European Patent Application, designated c100-3, was described as containing 154 amino acids of human SOD at the aminoterminus, 5 amino acid residues derived from the expression of a synthetic DNA adapter containing a restriction site, EcoRI, 363 amino acids derived from the expression of a cloned HCV cDNA fragment, and 5 carboxyl terminal amino acids derived from an MS2 cloning vector nucleotide sequence. The DNA sequence encoding this polypeptide was transformed into yeast cells using a plasmid. The transformed cells were cultured and expressed a 54,000 molecular weight polypeptide which was purified to about 80% purity by differential extraction.

Other SOD fusion polypeptides designated SOD-NANB$_{5-1-1}$ and SOD-NANB$_{81}$ were expressed in recombinant bacteria. The E.coli fusion polypeptides were purified by differential extraction and by chromatography using anion and cation exchange columns. The purification procedures were able to produce SOD-NANB$_{5-1-1}$ as about 80% pure and SOD-NAN38, as about 50% pure.

The recombinant SOD fusion polypeptides described by Houghton, M., et al. were coated on microtiter wells or polystyrene beads and used to assay serum samples. Briefly, coated microtiter wells were incubated with a sample in a diluent. After incubation, the microtiter wells were washed and then developed using either a radioactively labelled sheep antihuman antibody or a mouse antihuman IgG-HRP (horseradish peroxidase) conjugate. These assays were used to detect both post acute phase and chronic phase HCV infection.

Due to the preparative methods, assay specificity required adding yeast or E.coli extracts to the samples in order to prevent undesired immunological reactions with any yeast or E.coli antibodies present in samples.

Ortho Diagnostic Systems Inc. have developed a immunoenzyme assay to detect antibodies to HCV antigens. The Ortho assay procedure is a three-stage test for serum/plasma carried out in a microwell coated with the recombinant yeast/hepatitis C virus SOD fusion polypeptide c100-3.

In the first stage, a test specimen is diluted directly in the test well and incubated for a specified length of time. If antibodies to HCV antigens are present in the specimen, antigen-antibody complexes will be formed on the microwell surface. If no antibodies are present, complexes will not be formed and the unbound serum or plasma proteins will be removed in a washing step.

In the second stage, anti-human IgG murine monoclonal antibody horseradish peroxidase conjugate is added to the microwell. The conjugate binds specifically to the antibody portion of the antigen-antibody complexes. If antigen-antibody complexes are not present, the unbound conjugate will also be removed by a washing step.

In the third stage, an enzyme detection system composed of o-phenylenediamine 2HCl (OPD) and hydrogen peroxide is added to the test well. If bound conjugate is present, the OPD will be oxidized, resulting in a colored end product. After formation of the colored end product, dilute sulfuric acid is added to the microwell to stop the color-forming detection reaction.

The intensity of the colored end product is measured with a microwell reader. The assay may be used to screen patient serum and plasma.

It is established that HCV may be transmitted by contaminated blood and blood products. In transfused patients, as many as 10% will suffer from post-transfusion hepatitis. Of these, approximately 90% are the result of infections diagnosed as HCV. The prevention of transmission of HCV by blood and blood products requires reliable, sensitive and specific diagnosis and prognostic tools to identify HCV carriers as well as contaminated blood and blood products. Thus, there exists a need for an HCV assay which uses reliable and efficient reagents and methods to accurately detect the presence of HCV antibodies in samples.

## BRIEF SUMMARY

The present invention provides an improved assay for detecting the presence of an antibody to an HCV antigen in a sample by contacting the sample with at least one recombinant protein representing a distinct antigenic region of the HCV genome.

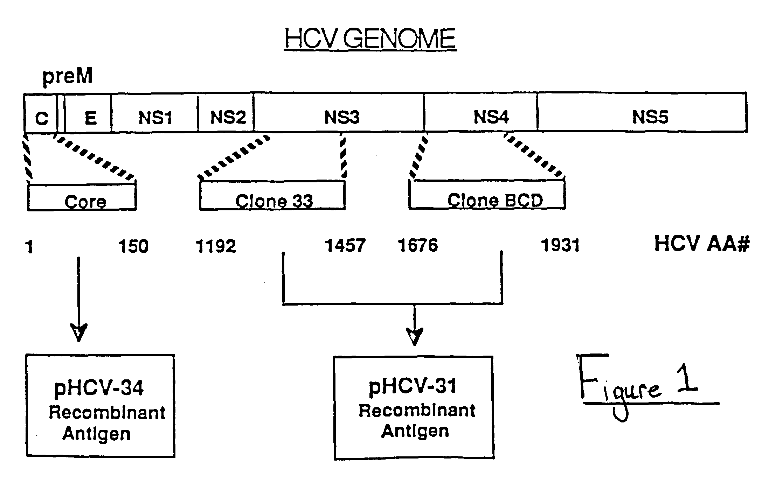

Recombinant antigens which are derived from the molecular cloning and expression of synthetic DNA sequences in heterologous hosts are provided. Briefly, synthetic DNA sequences which encode the desired proteins representing distinct antigenic regions of the HCV genome are optimized for expression in E.coli by specific codon selection. Specifically, two recombinant proteins representing three distinct antigenic regions of the HCV genome, including immunogenic regions of the c100-3 antigen and two additional non-overlapping regions upstream from the c100-3 region are described. Both proteins are expressed as chimeric fusions with E. coli CMP-KDO synthetase (CKS) gene. The first protein, expressed by plasmid pHCV-34 represents amino acids 1-150 of the HCV sequence and, based on analogy to the genomic organization of other flaviviruses, has been named HCV CKS-Core. Note that the term pHCV-34 will also refer to the fusion protein itself and that pHCV-34' will be the designation for a polypeptide representing the core region from about amino acids 1-150 of the HCV sequence prepared using other recombinant or synthetic methodologies. Other recombinant methodologies would include the preparation of pHCV-34', utilizing different expression systems. The methodology for the preparation of synthetic peptides of HCV is described in U.S. Serial No. 456,162, filed December 22, 1989, which enjoys common ownership and is incorporated herein by reference. The other protein is expressed by plasmid pHCV-31 and is composed of two non-contiguous coding regions located in the putative non-structural regions of HCV designated NS-3 and NS-4. The first of the two regions represents amino acids 1192-1457 of the HCV sequence (known as Clone 33) and is expressed by the plasmid pHCV-29. The fusion protein itself will also be referred to as pHCV-29 and pHCV-29' shall be the designation for a polypeptide from the NS-3 region representing from about amino acids 1192-1457 of the HCV sequence prepared using other recombinant or synthetic methodologies. The second region represents amino acids 1676-1931 of the HCV sequence and is expressed by the plasmid pHCV-23. The fusion protein will be referred to as pHCV-23 and pHCV-23' shall be the designation for a polypeptide from the NS4 region representing from about amino acids 1676-1931 of the HCV sequence prepared using other recombinant or synthetic methodologies. It has been designated Clone BCD based on the strategy used in its construction. Clone BCD represents the carboxyl-terminal 256 amino acids of c100-3: the amino terminal 108 amino acids of c100-3 are not represented in Clone BCD. The recombinant antigen produced by pHCV-31 is designated CKS-33c-BCD. The fusion protein is also designated by pHCV-31 and pHCV-31' refers to the polypeptide composed of two noncontiguous coding regions located in the putative nonstructural regions of HCV designated NS-3 and NS-4, representing from about amino acids 1192-1457 and from about 1676-1931 of the HCV sequence prepared using different recombinator synthetic methodologies. Figure 1 illustrates the position of the three HCV regions within the HCV genome. These antigens are used in the inventive immunoassays to detect the presence of HCV antibodies in samples.

One assay format according to the invention provides a screening assay for identifying the presence of an antibody that is immunologically reactive with an HCV antigen. Briefly, a fluid sample is incubated with a solid support containing the two commonly bound recombinant proteins HCV pHCV-34 and pHCV-31. Finally, the antibody-antigen complex is detected. In a modification of the screening assay the solid support additionally contains recombinant polypeptide c100-3.

Another assay format provides a confirmatory assay for unequivocally identifying the presence of an antibody that is immunologically reactive with an HCV antigen. The confirmatory assay includes synthetic peptides or recombinant antigens representing major epitopes contained within the three distinct regions of the HCV genome, which are the same regions represented by the two recombinant proteins described in the screening assay. These regions include NS4 (the c100-3 region) represented by pHCV-23, NS3 (the 33c region) represented by pHCV-29, and together with pHCV-23 (the c100-3 region) represented by pHCV-31, and a region near the 5' end of the HCV genome believed to be the core structural protein of HCV (pHCV-34). Recombinant proteins used in the confirmatory assay should have a heterologous source of antigen to that used in the primary screening assay (i.e. should not be an E.coli-derived recombinant antigen nor a recombinant antigen composed in part, of CKS sequences). Briefly, specimens repeatedly reactive in the primary screening assay are retested in the confirmatory assay. Aliquots containing identical amounts of specimen are contacted with a synthetic peptide or recombinant antigen individually coated onto a solid support. Finally, the antibody-antigen complex is detected. Seroreactivity for epitopes within the c100-3 region of the HCV genome are confirmed by use of the synthetic peptides sp67 and sp65. The synthetic peptide sp117 can also be used to confirm seroreactivity within the c100-3 region. Seroreactivity for HCV epitopes within the putative core region of HCV are confirmed by the use of the synthetic peptide sp75. In order to confirm seroreactivity for HCV epitopes within the 33c region of HCV, a recombinant antigen is expressed as a chimeric protein with superoxide dismutase (SOD) in yeast. The synthetic peptide sp65 (representing amino acids p1866-1930 of the HCV sequence), sp67 (representing amino acids p1684-1750), sp75 (representing amino acids p1-75), and sp117 (representing amino acids p1689-1805) are described in U. S. Serial No. 456,162 entitled "Hepatitis C Assay", filed December 22, 1989, which enjoys common ownership and is incorporated herein by reference.

Another assay format provides a competition assay or neutralization assay directed to the confirmation that positive results are not false by identifying the presence of an antibody that is immunologically reactive with an HCV antigen in a fluid sample where the sample is used to prepare first and second immunologically equivalent aliquots. The first aliquot is contacted with solid support containing a bound polypeptide which contains at least one epitope of an HCV antigen under conditions suitable for complexing with the antibody to form a detectable antibody-polypeptide complex and the second aliquot is first contacted with the same solid support containing bound polypeptide. The preferred recombinant polypeptide is derived from pHCV-23.

Another assay format provides an immunodot assay for identifying the presence of an antibody that is immunologically reactive with an HCV antigen by concurrently contacting a sample with recombinant polypeptides each containing distinct epitopes of an HCV antigen under conditions suitable for complexing the antibody with at least one of the polypeptides and detecting the antibodypolypeptide complex by reacting the complex with color-producing reagents. The preferred recombinant polypeptides employed include those recombinant polypeptides derived from pHCV-23, pHCV-29, pHCV-31, pHCV-34, as well as c100-3 expressed as a chimeric protein with superoxide dismutase (SOD) in yeast.

In all of the assays, the sample is preferably diluted before contacting the polypeptide absorbed on a solid support. Samples may be obtained from different biological samples such as whole blood, serum, plasma, cerebral spinal fluid, and lymphocyte or cell culture supernatants. Solid support materials may include cellulose materials, such as paper and nitrocellulose, natural and synthetic polymeric materials, such as polyacrylamide, polystyrene, and cotton, porous gels such as silica gel, agarose, dextran and gelatin, and inorganic materials such as deactivated alumina, magnesium sulfate and glass. Suitable solid support materials may be used in assays in a variety of well known physical configurations, including microtiter wells, test tubes, beads, strips, membranes, and microparticles. A preferred solid support for a non-immunodot assay is a polystyrene bead. A preferred solid support for an immnuodot assay is nitrocellulose.

Suitable methods and reagents for dectecting an antibody-antigen complex in an essay of the present invention are commercially available or known in the relevant art. Representative methods may employ detection reagents such as enzymatic, radioisotopic, fluorescent, luminescent, or chemiluminescent reagents. These reagents may be used to prepare hapten-labelled antihapten detection systems according to known procedures, for example, a biotin-labelled antibiotin system may be used to detect an antibody-antigen complex.

The present invention also encompasses assay kits including polypeptides which contain at least one epitope of an HCV antigen bound to a solid support as well as needed sample preparation reagents, wash reagents, detection reagents and signal producing reagents.

Other aspects and advantages of the invention will be apparent to those skilled in the art upon consideration of the following detailed description which provides illustrations of the invention in its presently preferred embodiments.

E.coli strains containing plasmids useful for constructs of the invention have been deposited at the American Type Culture Collection, Rockville, Maryland on August 10, 1990, under the accession Nos. ATCC 68380 (pHCV-23), ATCC 68381 (pHCV-29), ATCC 68382 (pHCV-31), ATCC 68383 (pHCV-34) and on November 6, 1990 for E. coli strains containing plasmids useful for constructs under the accession Nos. ATCC 68458 (pHCV-50), 68459 (pHCV-57), 68460 (pHCV-103), 68461 (pHCV-102), 68462 (pHCV-51), 68463 (pHCV-105), 68464 (pHCV-107), 68465 (pHCV-104), 68466 (pHCV-45), 68467 (pHCV-48), 68468 (pHCV-49), 68469 (pHCV-58), 68470 (pHCV-101).

## DESCRIPTION OF DRAWINGS

FIGURE 1 illustrates the HCV genome.

FIGURE 2 illustrates the use of recombinant polypeptides to identify the presence of antibodies in a chimpanzee inoculated with HCV.

FIGURE 3 illustrates the sensitivity and specificity increase in using the screening assay using pHCV-34 and pHCV-31 antigens.

FIGURE 4 illustrates the construction of plasmid pHCV-34.

FIGURE 5 illustrates the complete DNA and amino acid sequence of pHCV-34.

FIGURE 6 illustrates fusion protein pHCV-34.

FIGURE 7 illustrates the expression of pHCV-34 proteins in E.coli.

FIGURE 8 illustrates the construction of plasmid pHCV-23.

FIGURE 9 illustrates the construction of plasmid pHCV-29.

FIGURE 10 illustrates the construction of plasmid pHCV-31.

FIGURE 11 illustrates the complete DNA and amino acid sequence of pHCV-31.

FIGURE 12 illustrates the fusion protein pHCV-31.

FIGURE 13 illustrates the expression of pHCV-29 in E.coli.

FIGURE 14 illustrates the expression of pHCV-23 in E.coli.

FIGURE 15 illustrates the expression of pHCV-31 in E.coli.

FIGURE 16 illustrates the increased sensitivity using the screening assay utilizing the pHCV-34.

FIGURE 17 illustrates the increased specificity with the screening assay utilizing pHCV-34 and pHCV-31.

FIGURE 18 illustrates the results in hemodialysis patients using the screening and confirmatory assays.

FIGURE 19 illustrates earlier detection of HCV in a hemodialysis patient using the screening assay.

FIGURE 20 illustrates the results of the screening assay utilizing pHCV-34 and pHCV-31 on samples from individuals with acute NANBH.

FIGURE 21 illustrates the results of the confirmatory assay of the same population group as in Figure 20.

FIGURE 22 illustrates the results of the screening and confirmatory assays on individuals infected with chronic NANBH.

FIGURE 23 illustrates preferred buffers, pH conditions, and spotting concentrations for the HCV immunodot assay.

FIGURE 24 illustrates the results of the HCV immunodot assay.

FIGURE 25 illustrates the fusion protein pHCV-45.

FIGURE 26 illustrates the DNA and amino acid sequence of the recombinant antigen expressed by pHCV-45.

FIGURE 27 illustrates the expression of pHCV-45 in E.coli.

FIGURE 28 illustrates the fusion protein pHCV-48.

FIGURE 29 illustrates the DNA and amino acid sequence of the recombinant antigen expressed by pHCV-48.

FIGURE 30 illustrates the expression of pHCV-48 in E.coli.

FIGURE 31 illustrates the fusion protein pHCV-51.

FIGURE 32 illustrates the DNA and amino acid sequence of the recombinant antigen expressed by pHCV-51.

FIGURE 33 illustrates the expression of pHCV-51 in E.coli.

FIGURE 34 illustrates the fusion protein pHCV-50.

FIGURE 35 illustrates the DNA and amino acid sequence of the recombinant antigen expressed by pHCV-50.

FIGURE 36 illustrates the expression of pHCV-50 in E.coli.

FIGURE 37 illustrates the fusion protein pHCV-49.

FIGURE 38 illustrates the DNA and amino acid sequence of the recombinant antigen expressed by pHCV-49.

FIGURE 39 illustrates the expression of pHCV-49 in E.coli.

FIGURE 40 illustrates an immunoblot of pHCV-23, pHCV-45, pHCV-48, pHCV-51, pHCV-50 and pHCV-49.

FIGURE 41 illustrates the fusion proteins pHCV-24, pHCV-57, pHCV-58.

FIGURE 42 illustrates the DNA and amino acid sequence of the recombinant antigen expressed by pHCV-57.

FIGURE 43 illustrates the DNA and amino acid sequence of the recombinant antigen expressed by pHCV-58.

FIGURE 44 illustrates the expression of pHCV-24, pHCV-57, and pHCV-58 in E.coli.

FIGURE 45 illustrates the fusion protein pHCV-105.

FIGURE 46 illustrates the DNA and amino acid sequence of the recombinant antigen expressed by pHCV-105.

FIGURE 47 illustrates the expression of pHCV-105 in E.coli.

FIGURE 48 illustrates the fusion protein pHCV-103.

FIGURE 49 illustrates the DNA and amino acid sequence of the recombinant antigen expressed by pHCV-103.

FIGURE 50 illustrates the fusion protein pHCV-101.

FIGURE 51 illustrates the DNA and amino acid sequence of the recombinant antigen expressed by pHCV-101.

FIGURE 52 illustrates the fusion protein pHCV-102.

FIGURE 53 illustrates the DNA and amino acid sequence of the recombinant antigen expressed by pHCV-102.

FIGURE 54 illustrates the expression of pHCV-102 in E.coli.

FIGURE 55 illustrates the fusion protein pHCV-107.

FIGURE 56 illustrates the DNA and amino acid sequence of the recombinant antigen expressed by pHCV-107.

FIGURE 57 illustrates the fusion protein pHCV-104.

FIGURE 58 illustrates the DNA and amino acid sequence of the recombinant antigen expressed by pHCV-104.

## DETAILED DESCRIPTION

The present invention is directed to an assay to detect an antibody to an HCV antigen in a sample. Human serum or plasma is preferably diluted in a sample diluent and incubated with a polystyrene bead coated with a recombinant polypeptide that represents a distinct antigenic region of the HCV genome. If antibodies are present in the sample they will form a complex with the antigenic polypeptide and become affixed to the polystyrene bead. After the complex has formed, unbound materials and reagents are removed by washing the bead and the bead-antigen-antibody complex is reacted with a solution containing horseradish peroxidase labeled goat antibodies directed against human antibodies. This peroxidase enzyme then binds to the antigen-antibody complex already fixed to the bead. In a final reaction the horseradish peroxidase is contacted with o-phenylenediamine and hydrogen peroxide which results in a yellow-orange color. The intensity of the color is proportional to the amount of antibody which initially binds to the antigen fixed to the bead.

The preferred recombinant polypeptides having HCV antigenic epitopes were selected from portions of the HCV genome which encoded polypeptides which possessed amino acid sequences similar to other known immunologically reactive agents and which were identified as having some immunological reactivity. (The immunological reactivity of a polypeptide was initially identified by reacting the cellular extract of E.coli clones which had been transformed with cDNA fragments of the HCV genome with HCV infected serum. Polypeptides expressed by clone containing the incorporated cDNA were immunologically reactive with serum known to contain antibody to HCV antigens.) An analysis of a given amino acid sequence, however, only provides rough guides to predicting immunological reactivity. There is no invariably predictable way to ensure immunological activity short of preparing a given amino acid sequence and testing the suspected sequence in an assay.

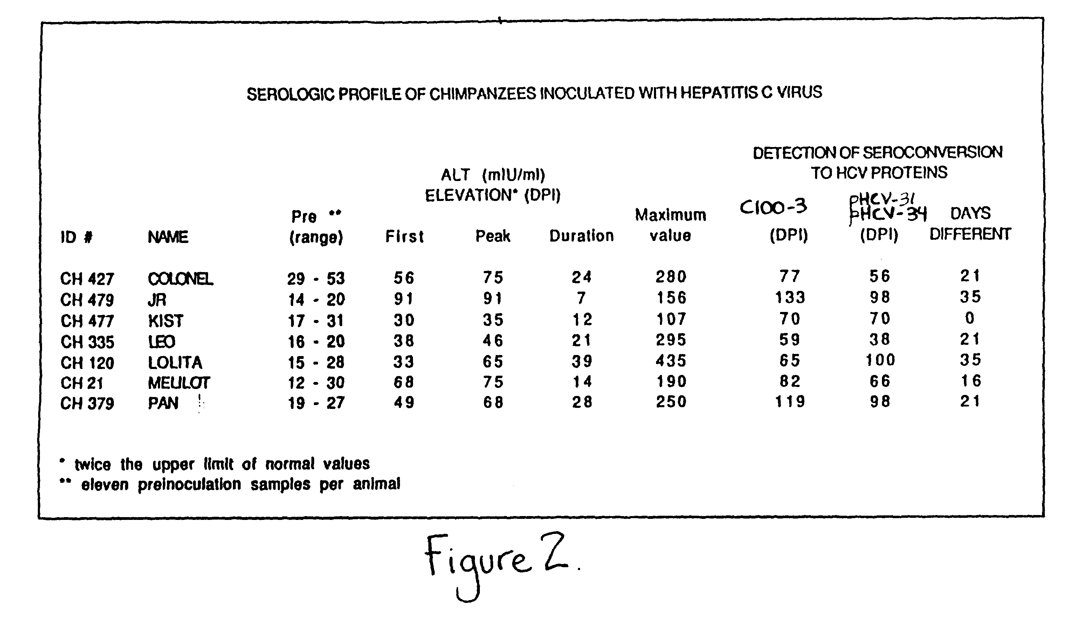

The use of recombinant polypeptides representing distinct antigenic regions of the HCV genome to detect the presence of an antibody to an HCV antigen is illustrated in Figure 2. The course of HCV infection in the chimpanzee, Pan, was followed with one assay using recombinant c100-3 polypeptide and with another improved assay, using the two recombinant antigens CKS-Core (pHCV-34) and pHCV-33c-BCD (pHCV-31) expressed by the plasmids pHCV-34 and pHCV-31, respectively. The assay utilizing the recombinant pHCV-34 and pHCV-31 proteins detected plasma antibody three weeks prior to detection of antibody by the assay using c100-3.

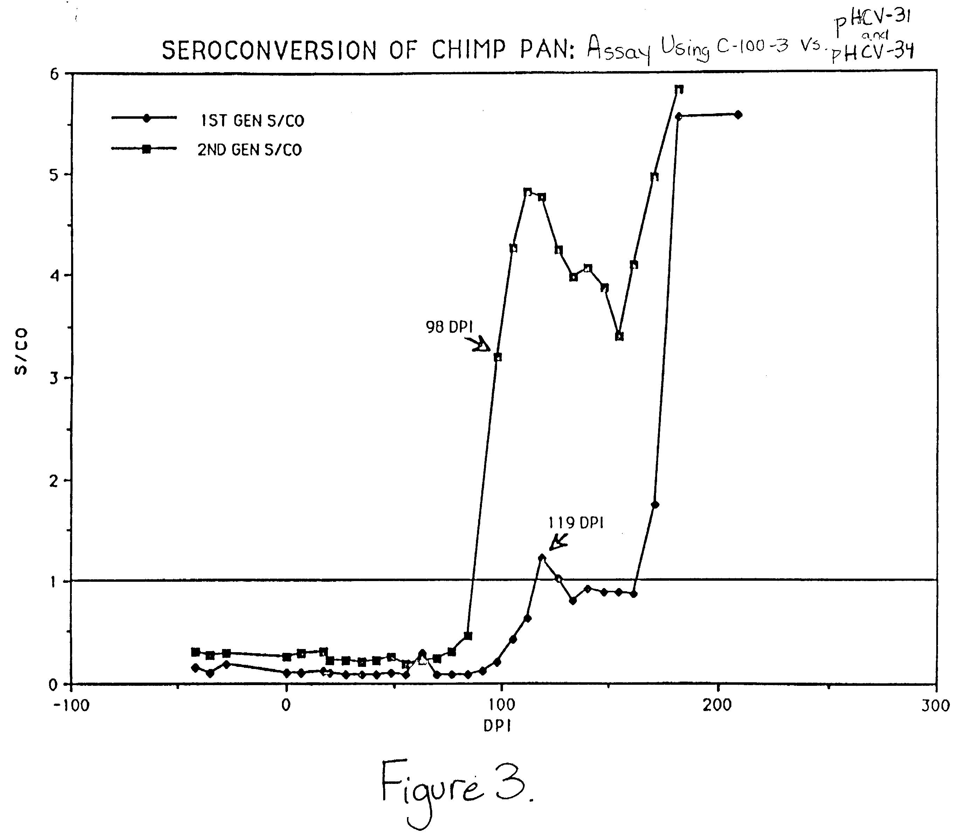

A summary of the results of a study which followed the course of HCV infection in Pan and six other chimpanzees using the two assays described above is summarized in Figure 3. Both assays gave negative results before inoculation and both assays detected the presence of antibodies after the animal had been infected with HCV. However, in the comparison of the two assays, the improved screening assay using pHCV-34 and pHCV-31 detected seroconversion to HCV antigens at an earlier or equivalent bleed date in six of the seven chimpanzees. Data from these chimpanzee studies clearly demonstrate that overall detection of HCV antibodies is greatly increased with the assay utilizing the pHCV-34 and pHCV-31 proteins. This test is sufficiently sensitive to detect seroconversion during the acute phase of this disease, as defined as an elevation in ALT levels, in most animals. Equally important is the high degree of specificity of the test as no pre-inoculation specimens were reactive.

The polypeptides useful in the practice of this invention are produced using recombinant technologies. The DNA sequences which encode the desired polypeptides are preferably assembled from fragments of the total desired sequence. Synthetic DNA fragments of the HCV genome can be synthesized based on their corresponding amino acid sequences. Once the amino acid sequence is chosen, this is then reverse translated to determine the complementary DNA sequence using codons optimized to facilitate expression in the chosen system. The fragments are generally prepared using well known automated processes and apparatus. After the complete sequence has been prepared the desired sequence is incorporated into an expression vector which is transformed into a host cell. The DNA sequence is then expressed by the host cell to give the desired polypeptide which is harvested from the host cell or from the medium in which the host cell is cultured. When smaller peptides are to be made using recombinant technologies it may be advantageous to prepare a single DNA sequence which encodes several copies of the desired polypeptide in a connected chain. The long chain is then isolated and the chain is cleaved into the shorter, desired sequences.

The methodology of polymerase chain reaction (PCR) may also be employed to develop PCR amplified genes from any portion of the HCV genome, which in turn may then be cloned and expressed in a manner similar to the synthetic genes.

Vector systems which can be used include plant, bacterial, yeast, insect, and mammalian expression systems. It is preferred that the codons are optimized for expression in the system used.

A preferred expression system utilizes a carrier gene for a fusion system where the recombinant HCV proteins are expressed as a fusion protein of an E.coli enzyme, CKS (CTP:CMP-3-deoxy-manno-octulosonate cytidylyl transferase or CMP-KDO synthetase). The CKS method of protein synthesis is disclosed in U. S. Patent Applications Serial Nos. 167,067 and 276,263 filed March 11, 1988 and November 23, 1988, respectively, by Bolling (EPO 891029282) which enjoy common ownership and are incorporated herein by reference.

Other expression systems may be utilized including the lambda PL vector system whose features include a strong lambda pL promoter, a strong three-frame translation terminator rrnBtl, and translation starting at an ATG codon.

In the present invention, the amino acid sequences encoding for the recombinant HCV antigens of interest were reverse translated using codons optimized to facilitate high level expression in E.coli. Individual oligonucleotides were synthesized by the method of oligonucleotide directed double-stranded break repair disclosed in U.S. Patent Application Serial No. 883,242, filed July 8, 1986 by Mandecki (EPO 87109357.1) which enjoys common ownership and is incorporated herein by reference. Alternatively, the individual oligonucleotides may be synthesized on the Applied Biosystem 380A DNA synthesizer using methods and reagents recommended by the manufacturer. The DNA sequences of the individual oligonucleotides were confirmed using the Sanger dideoxy chain termination method (Sanger et al., J. Mole. Biol., 162:729 (1982)). These individual gene fragments were then annealed and ligated together and cloned as EcoRI-BamHI subfragments in the CKS fusion vector pJO200. After subsequent DNA sequence confirmation by the Sanger dideoxy chain termination method, the subfragments were digested with appropriate restriction enzymes, gel purified, ligated and cloned again as an EcoRI-BamHI fragment in the CKS fusion vector pJO2OO. The resulting clones were mapped to identify a hybrid gene consisting of the EcoRI-BamHI HCV fragment inserted at the 3' end of the CKS (CMP-KDO synthetase) gene. The resultant fusion proteins, under control of the lac promoter, consist of 239 amino acids of the CKS protein fused to the various regions of HCV.

The synthesis, cloning, and characterization of the recombinant polypeptides as well as the preferred formats for assays using these polypeptides are provided in the following examples. Examples 1 and 2 describe the synthesis and cloning of CKS-Core and CKS-33-BCD, respectively. Example 3 describes a screening assay. Example 4 describes a confirmatory assay. Example 5 describes a competition assay. Example 6 describes an immunodot assay.

REAGENTS AND ENZYMES

Media such as Luria-Bertani (LB) and Superbroth II (Dri Form) were obtained from Gibco Laboratories Life Technologies, Inc., Madison Wisconsin. Restriction enzymes, Klenow fragment of DNA polymerase I, T4 DNA ligase, T4 polynucleotide kinase, nucleic acid molecular weight standards, M13 sequencing system, X-gal (5-bromo-4-chloro-3-indonyl-β-D-galactoside), IPTG (isopropyl-β-D-thiogalactoside), glycerol, Dithiothreitol, 4-chloro-1-naphthol were purchased from Boehringer Mannheim Biochemicals, Indianapolis, Indiana; or New England Biolabs, Inc., Beverly, Massachusetts; or Bethesda Research Laboratories Life Technologies, Inc., Gaithersburg, Maryland. Prestained protein molecular weight standards, acrylamide (crystallized, electrophoretic grade 99%); N-N'-Methylene-bis-acrylamide (BIS); N,N,N',N',-Tetramethylethylenediamine (TEMED) and sodium dodecylsulfate (SDS) were purchased from BioRad Laboratories, Richmond, California. Lysozyme and ampicillin were obtained from Sigma Chemical Co., St. Louis, Missouri. Horseradish peroxidase (HRPO) labeled secondary antibodies were obtained from Kirkegaard & Perry Laboratories, Inc., Gaithersburg, Maryland. Seaplaque® agarose (low melting agarose) was purchased from FMC Bioproducts, Rockland, Maine.

T50E10 contained 50mM Tris, pH 8.0, lOmM EDTA; 1X TG contained lOOmM Tris, pH 7.5 and 10% glycerol; 2X SDS/PAGE loading buffer consisted of 15% glycerol, 5% SDS, lOOmM Tris base, 1M β-mercaptoethanol and 0.8% Bromophenol blue dye; TBS container 50 mM Tris, pH 8.0, and 150 mM sodium chloride; Blocking solution consisted of 5% Carnation nonfat dry milk in TBS.

HOST CELL CULTURES, DNA SOURCES AND VECTORS

E.coli JM103 cells, pUC8, pUC18, pUC19 and M13 cloning vectors were purchased from Pharmacia LKB Biotechnology, Inc., Piscataway, New Jersey; Competent Epicurean™ coli stains XL1-Blue and JM109 were purchased from Stratagene Cloning Systems, LaJolla, California. RR1 cells were obtained from Coli Genetic Stock Center, Yale University, New Haven, Connecticut; and E.coli CAG456 cells from Dr. Carol Gross, University of Wisconsin, Madison, Wisconsin. Vector pRK248.clts was obtained from Dr. Donald R. Helinski, University of California, San Diego, California.

GENERAL METHODS

All restriction enzyme digestion were performed according to suppliers' instructions. At least 5 units of enzyme were used per microgram of DNA, and sufficient incubation was allowed to complete digestion of DNA. Standard procedures were used for minicell lysate DNA preparation, phenol-chloroform extraction, ethanol precipitation of DNA, restriction analysis of DNA on agarose, and low melting agarose gel purification of DNA fragments (Maniatis et al., Molecular Cloning. A Laboratory Manual [New York: Cold Spring Harbor, 1982]). Plasmid isolations from E.coli strains used the alkali lysis procedure and cesium chloride-ethidium bromide density gradient method (Maniatis et al., supra). Standard buffers were used for T4 DNA ligase and T4 polynucleotide kinase (Maniatis et al., supra).

EXAMPLE 1. CKS-CORE

A. Construction of the Plasmid pJ0200

The cloning vector pJO200 allows the fusion of recombinant proteins to the CKS protein. The plasmid consists of the plasmid pBR322 with a modified lac promoter fused to a KdsB gene fragment (encoding the first 239 of the entire 248 amino acids of the E.coli CMP-KDO synthetase of CKS protein), and a synthetic linker fused to the end of the KdsB gene fragment. The cloning vector pJO200 is a modification of vector pTB210. The synthetic linker includes: multiple restriction sites for insertion of genes; translational stop signals, and the trpA rho-independent transcriptional terminator. The CKS method of protein synthesis as well as CKS vectors including pTB210 are disclosed in U.S. Patent Application Serial Nos. 167,067 and 276,263, filed March 11, 1988 and November 23, 1988, respectively, by Bolling (EPO 891029282) which enjoy common ownership, and are herein incorporated by reference.

B. Preparation of HCV CKS-Core Expression Vector

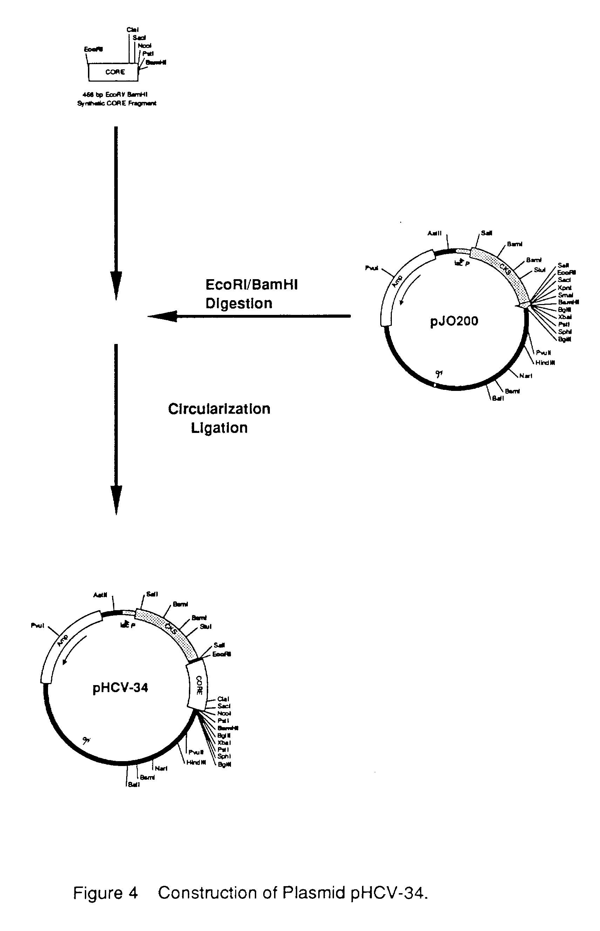

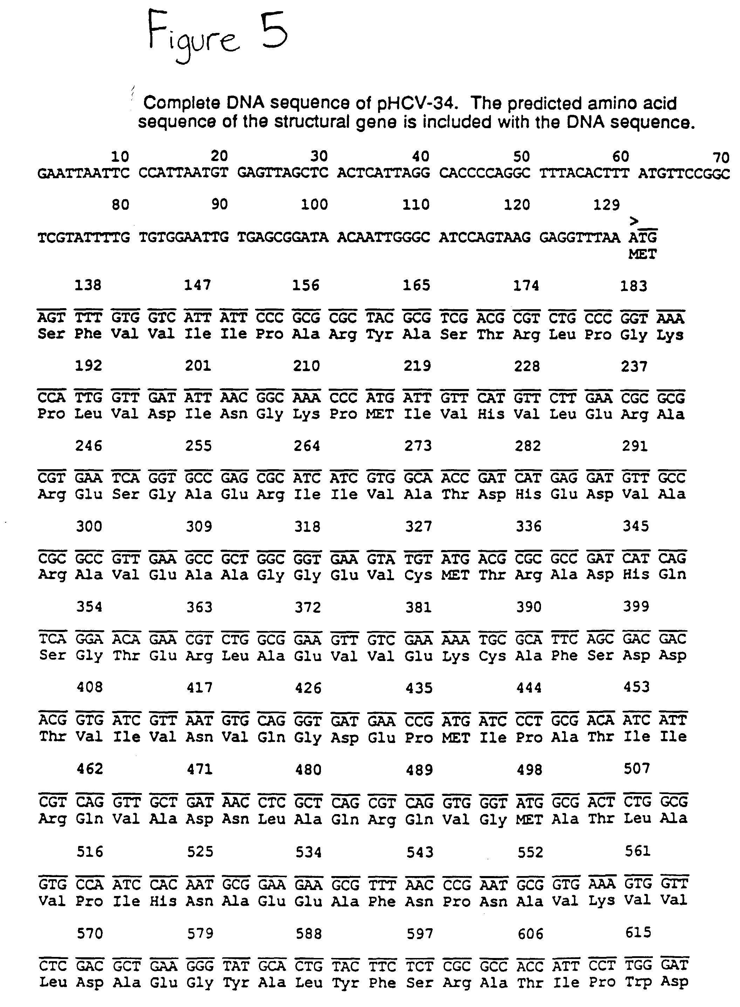

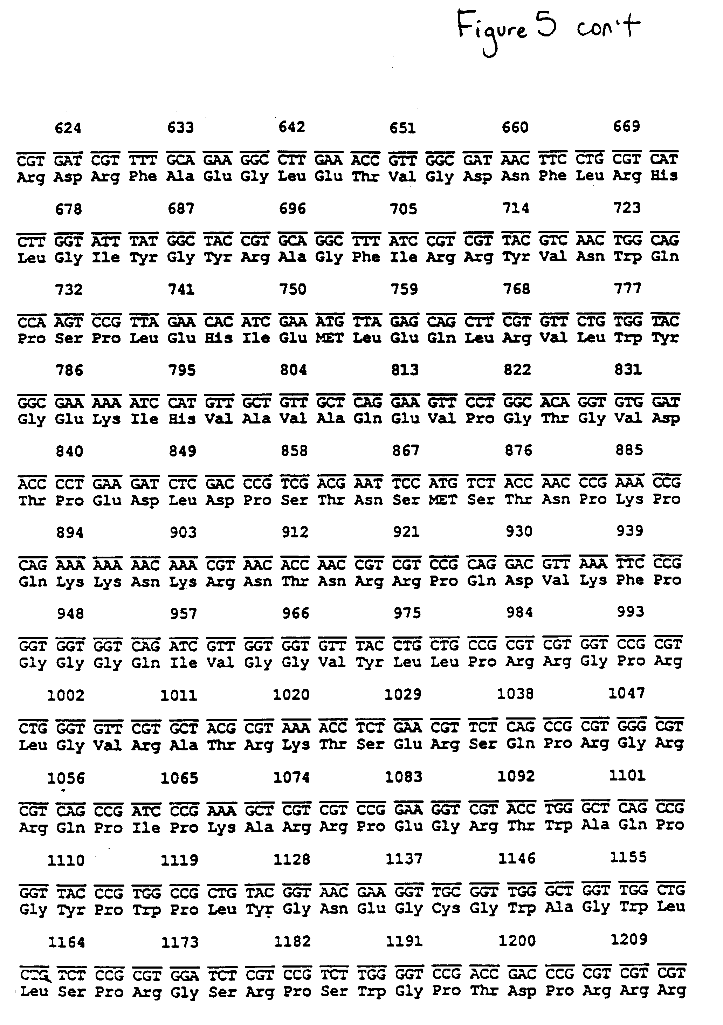

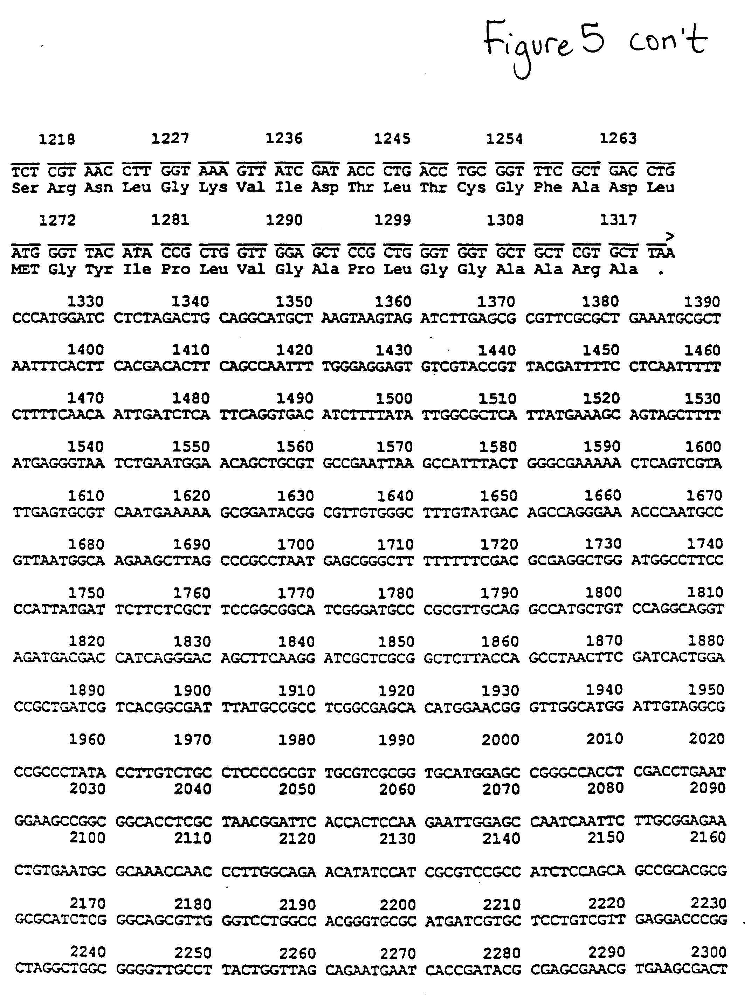

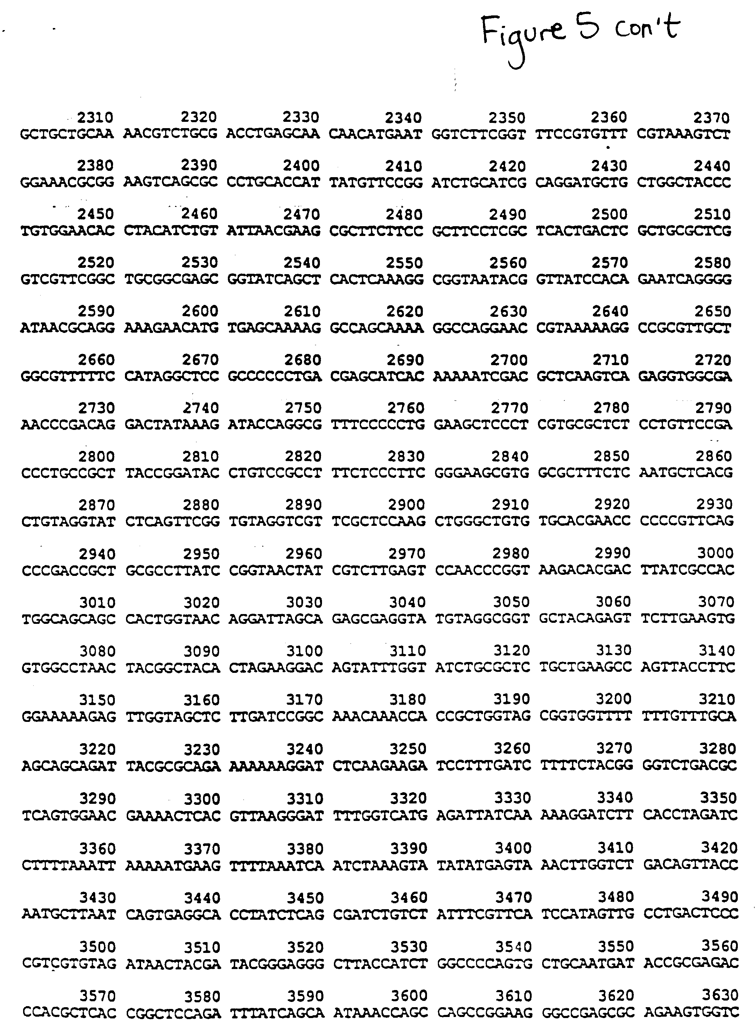

Six individual nucleotides representing amino acids 1-150 of the HCV genome were ligated together and cloned as a 466 base pair EcoRI-BamHI fragment into the CKS fusion vector pJO200 as presented in Figure 4. The complete DNA sequence of this plasmid, designated pHCV-34, and the entire amino acid sequence of the pHCV-34 recombinant antigen produced is presented in Figure 5. The resultant fusion protein HCV CKS-Core, consists of 239 amino acids of CKS, seven amino acids contributed by linker DNA sequences, and the first 150 amino acids of HCV as illustrated in Figure 6.

The pHCV-34 plasmid and the CKS plasmid pTB210 were transformed into E.coli K-12 strain xL-1 (recAl, endAl, gyrA96, thi-1, hsdRl7, supE44, relAl, lac/F', proAB, laclqZDM15, TN10) cells made competent by the calcium chloride method. In these constructions the expression of the CKS fusion proteins was under the control of the lac promoter and was induced by the addition of IPTG. These plasmids replicated as independent elements, were nonmobilizable and were maintained at approximately 10-30 copies per cell.

C. Characterization of Recombinant HCV-Core

In order to establish that clone pHCV-34 expressed the unique HCV-CKS Core protein, the pHCV-34/XL-1 culture was grown overnight at 37°C in growth media consisting of yeast extract, trytone, phosphate salts, glucose, and ampicillin. When the culture reached an OD600 of 1.0, IPTG was added to a final concentration of 1mM to induce expression. Samples (1.5 ml) were removed at 1 hour intervals, and cells were pelleted and resuspended to an OD600 of 1.0 in 2X SDS/PAGE loading buffer. Aliquots (15ul) of the prepared samples were separated on duplicate 12.5% SDS/PAGE gels.

One gel was fixed in a solution of 50% methanol and 10% acetic acid for 20 minutes at room temperature, and then stained with 0.25% Coomassie blue dye in a solution of 50% methanol and 10% acetic acid for 30 minutes. Destaining was carried out using a solution of 10% methanol and 7% acetic acid for 3-4 hours, or until a clear background was obtained.

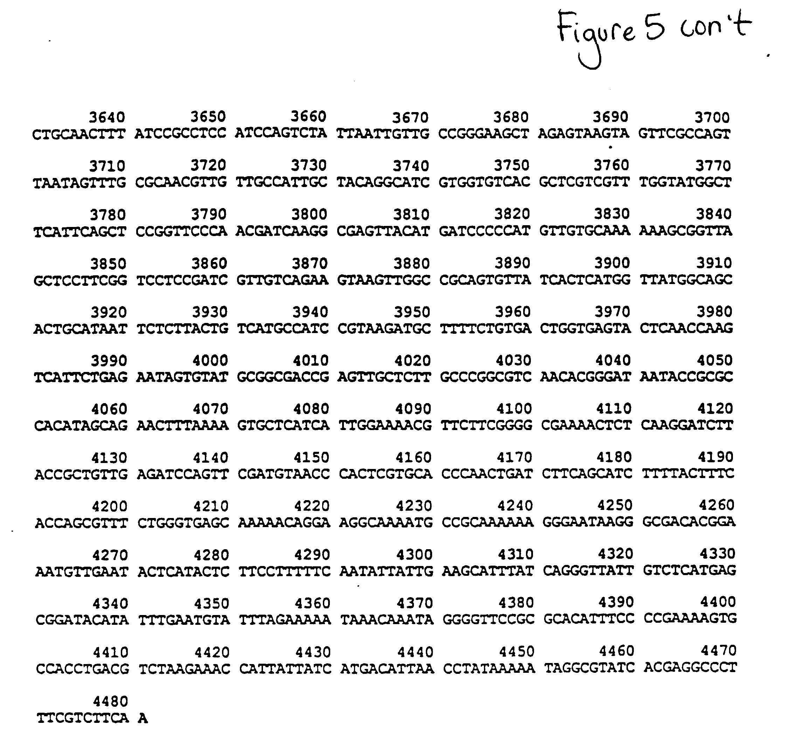

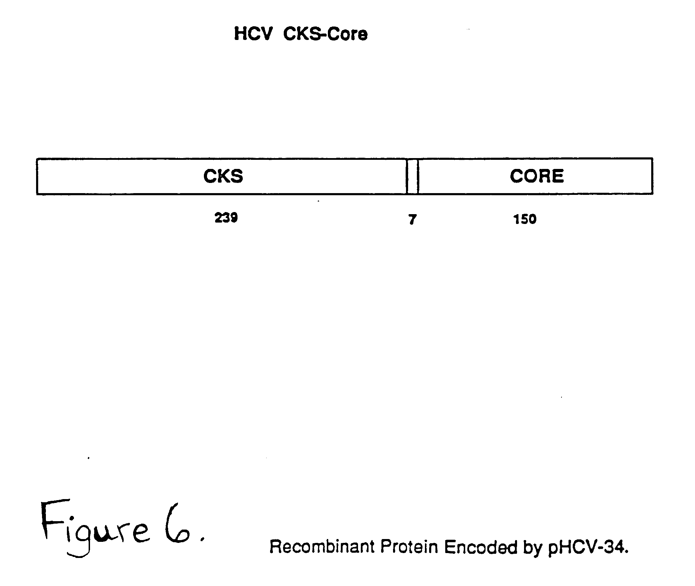

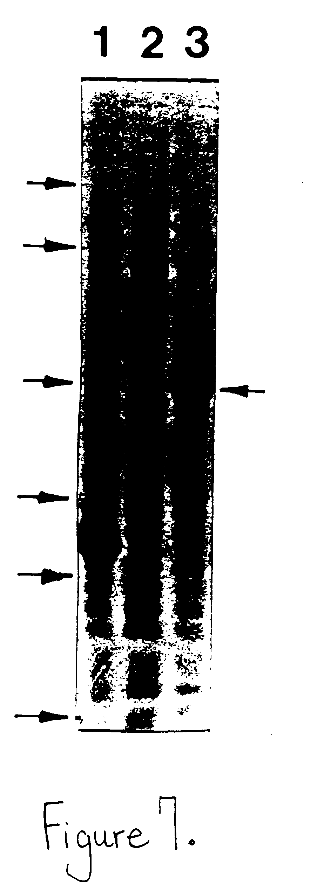

Figure 7 presents the expression of pHCV-34 proteins in E.coli. Molecular weight standards were run in Lane M. Lane 1 contains the plasmid pJ0200-the CKS vector without the HCV sequence. The arrows on the left indicate the mobilities of the molecular weight markers from top to bottom: 110,000; 84,000; 47,000; 33,000; 24,000; and 16,000 daltons. The arrows on the right indicate the mobilities of the recombinant HCV proteins. Lane 2 contains the E.coli lysate containing pHCV-34 expressing CKS-Core (amino acids 1 to 150) prior to induction; and Lane 3 after 3 hours of induction. The results show that the recombinant protein pHCV-34 has an apparent mobility corresponding to a molecular size of 48,000 daltons. This compares acceptably with the predicted molecular mass of 43,750 daltons.

Proteins from the second 12.5% SDS/PAGE gel were electrophoretically transferred to nitrocellulose for immunoblotting. The nitrocellulose sheet containing the transferred proteins was incubated with Blocking Solution for one hour and incubated overnight at 4°C with HCV patients' sera diluted in TBS containing E.coli K-12 strain XL-1 lysate. The nitrocellulose sheet was washed three times in TBS, then incubated with HRPO-labeled goat anti-human IgG, diluted in TBS containing 10% fetal calf sera. The nitrocellulose was washed three times with TBS and the color was developed in TBS containing 2 mg/ml 4-chloro-1-napthol, 0.02% hydrogen peroxide and 17% methanol. Clone HCV-34 demonstrated a strong immunoreactive band at 48,000 daltons with the HCV patients' sera. Thus, the major protein in the Coomassie stained protein gel was immunoreactive. Normal human serum did not react with any component of pHCV-34.

## EXAMPLE 2. HCV CKS-33C-BCD

### A. Preparation of HCV CKS-33c-BCD Expression Vector

The construction of this recombinant clone expressing the HCV CKS-33-BCD antigen was carried out in three steps described below. First, a clone expressing the HCV CKS-BCD antigen was constructed, designated pHCV-23. Second, a clone expressing the HCV CKS-33 antigen was constructed, designated pHCV-29. Lastly, the HCV BCD region was excised from pHCV-23 and inserted into pHCV-29 to construct a clone expressing the HCV CKS-33-BCD antigen, designated pHCV-31.

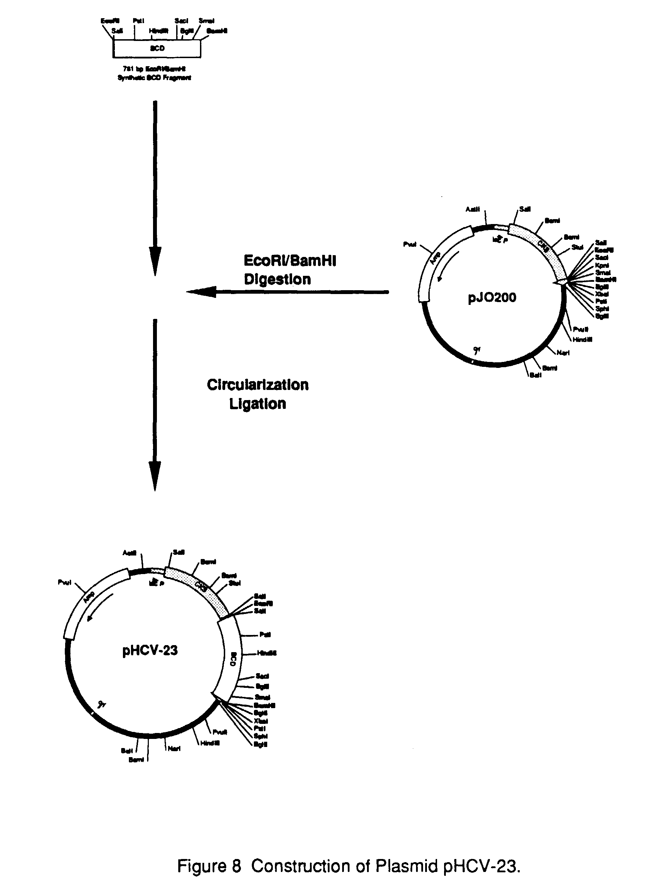

To construct the plasmid pHCV-23, thirteen individual oligonucleotides representing amino acids 1676-1931 of the HCV genome were ligated together and cloned as three separate EcoRI-BamHI subfragments into the CKS fusion vector pJO200. After subsequent DNA sequence confirmation, the three subfragments, designated B, C, and D respectively, were digested with the appropriate restriction enzymes, gel purified, ligated together, and cloned as a 781 base pair EcoRI-BamHI fragment in the CKS fusion vector pJO200, as illustrated in Figure 8. The resulting plasmid, designated pHCV-23, expresses the HCV CKS-BCD antigen under control of the lac promoter. The HCV CKS-BCD antigen consists of 239 amino acids of CKS, seven amino acids contributed by linker DNA sequences, 256 amino acids from the HCV NS4 region (amino acids 1676-1931, and 10 additional amino acids contributed by linker DNA sequences.

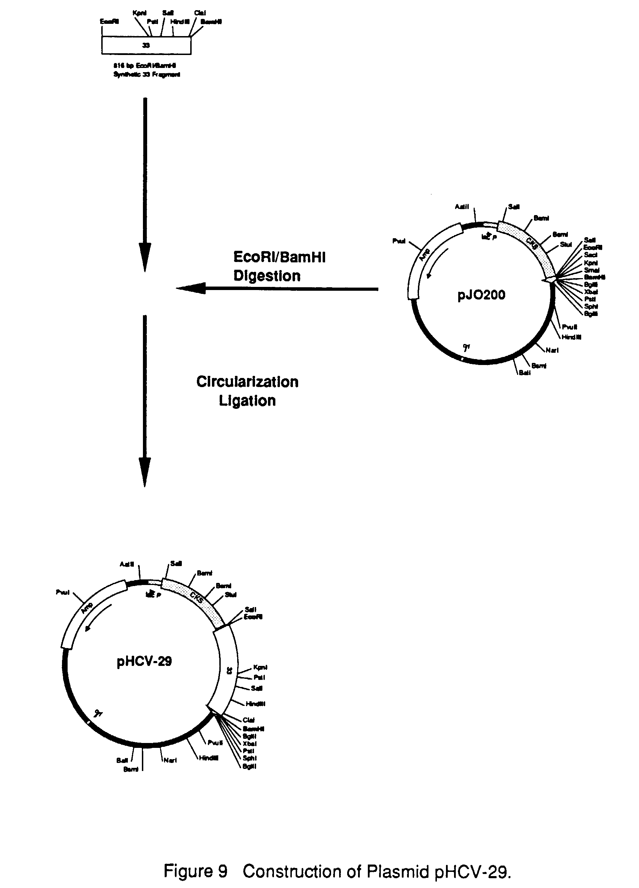

To construct the plasmid pHCV-29 twelve individual oligonucleotides representing amino acids 1192-1457 of the HCV genome were ligated together and cloned as two separate EcoRI-BamHI subfragments in the CKS fusion vector pJO200. After subsequent DNA sequence confirmation, the two subfragments were digested with the appropriate restriction enzymes, gel purified, ligated together and cloned again as an 816 base pair EcoRI-BamHI fragment in the CKS fusion vector pJO200, as illustrated in Figure 9. The resulting plasmid, designated pHCV-29, expresses the CKS-33 antigen under control of the lac promoter. The HCV CKS-33 antigen consists of 239 amino acids of CKS, eight amino acids contributed by linker DNA sequences, and 266 amino acids from the HCV NS3 region (amino acids 1192-1457).

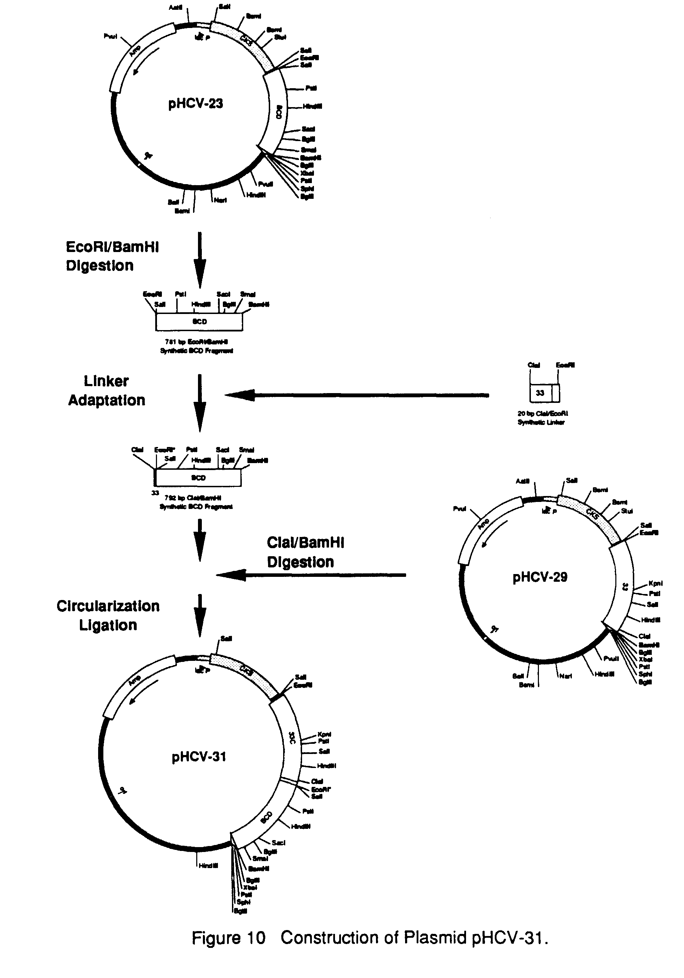

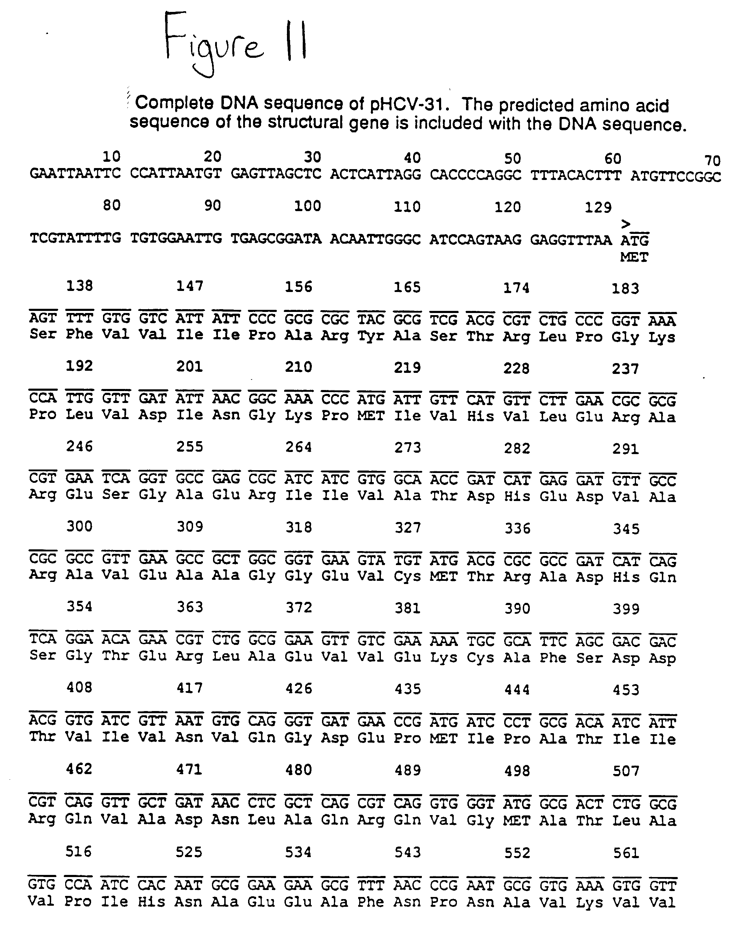

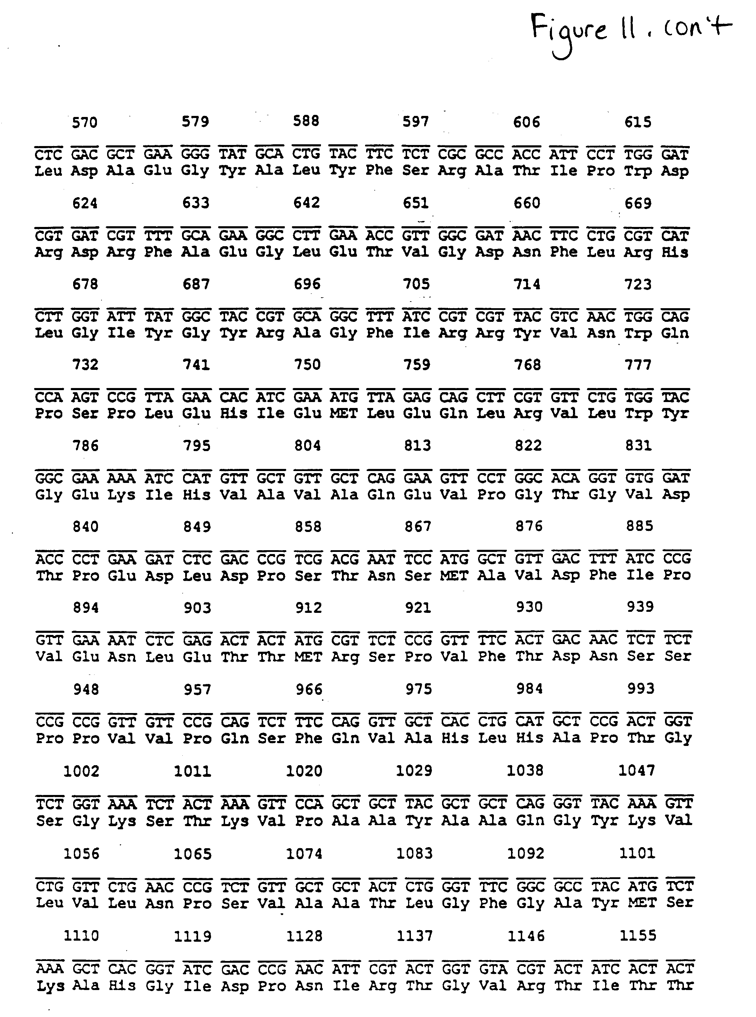

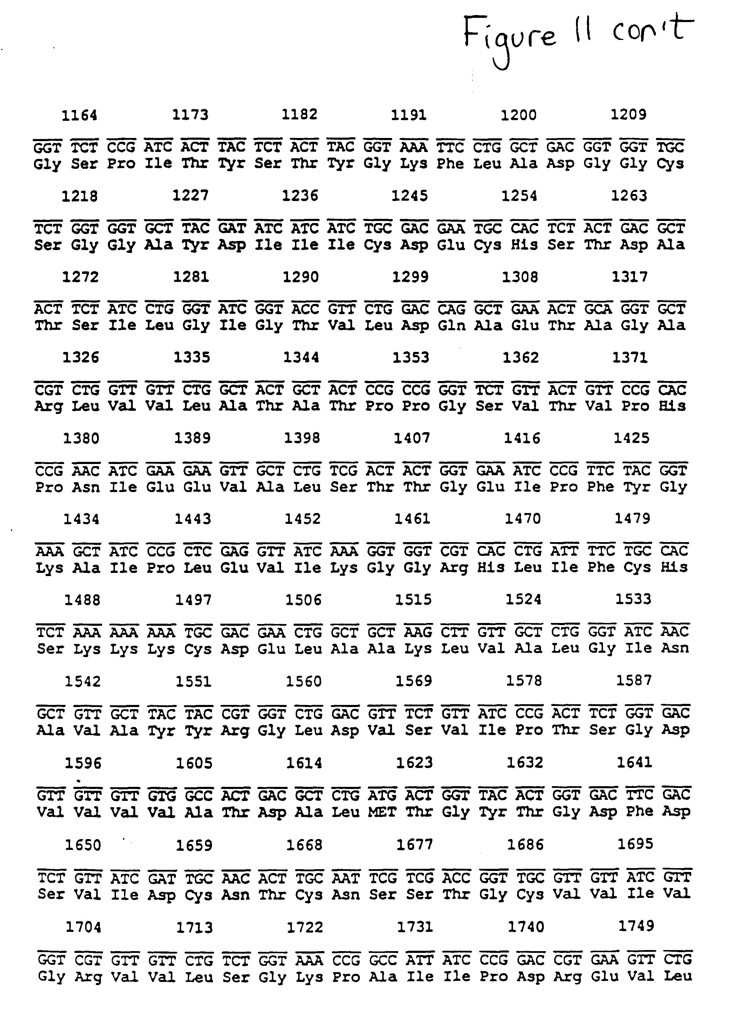

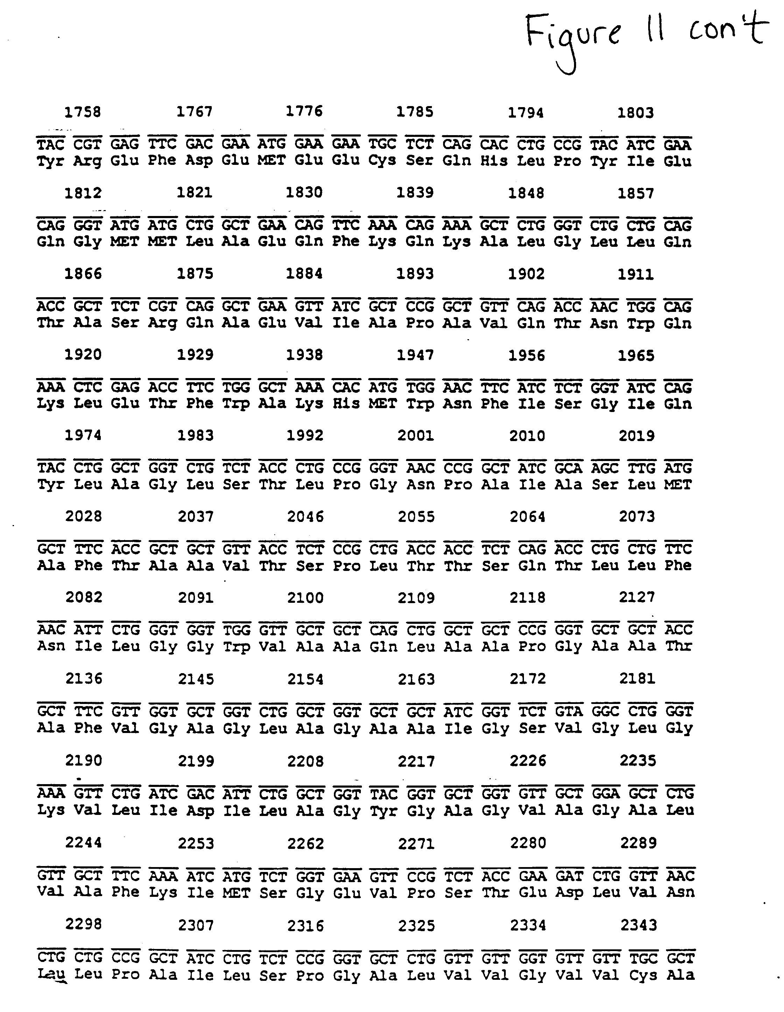

To construct the plasmid pHCV-31, the 781 base pair EcoRI-BamHI fragment from pHCV-23 representing the HCV-BCD region was linker-adapted to produce a Cla1-BamH1 fragment which was then gel purified and ligated into pHCV-29 at the Cla1-BamH1 sites as illustrated in Figure 10. The resulting plasmid, designated pHCV-31, expresses the pHCV-31 antigen under control of the lac promoter. The complete DNA sequence of pHCV-31 and the entire amino acid sequence of the HCV CKS-33-BCD recombinant antigen produced is presented in Figure 11. The HCV CKS-33-BCD antigen consists of 239 amino acids of CKS, eight amino acids contributed by linker DNA sequences, 266 amino acids of the HCV NS3 region (amino acids 1192-1457), 2 amino acids contributed by linker DNA sequences, 256 amino acids of the HCV NS4 region (amino acids 1676-1931), and 10 additional amino acids contributed by linker DNA sequences. Figure 12 presents a schematic representation of the pHCV-31 antigen.

The pHCV-31 plasmid was transformed into E. coli K-12 strain XL-1 in a manner similar to the pHCV-34 and CKS-pTB210 plasmids of Example 1.

### B. Characterization of Recombinant HCV CKS-33-BCD

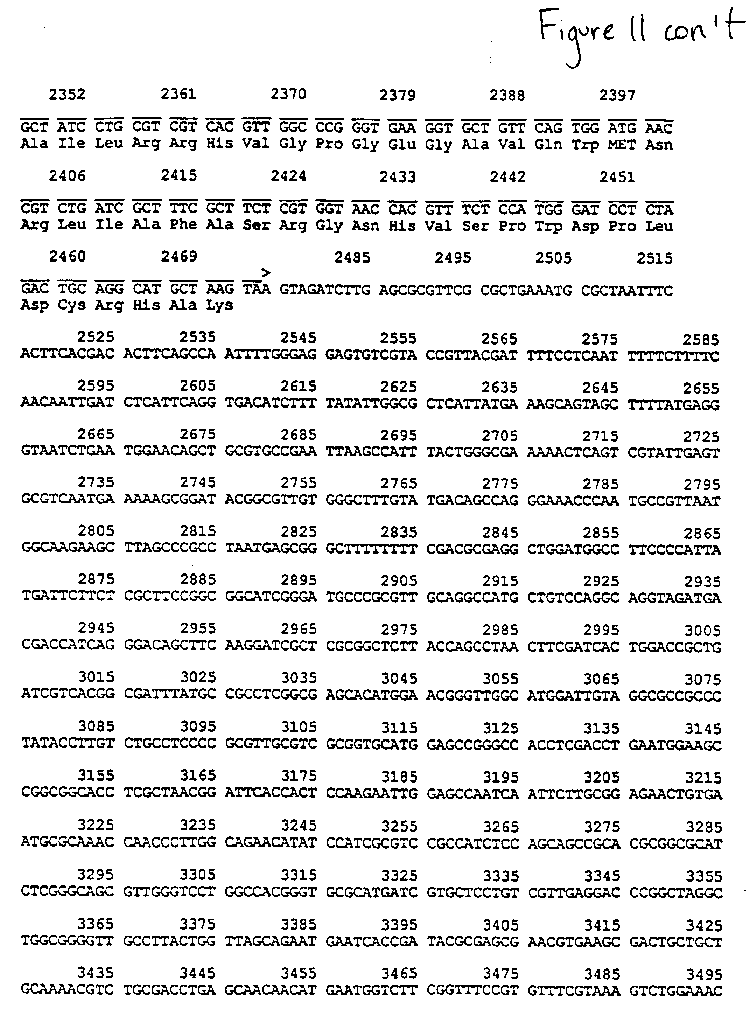

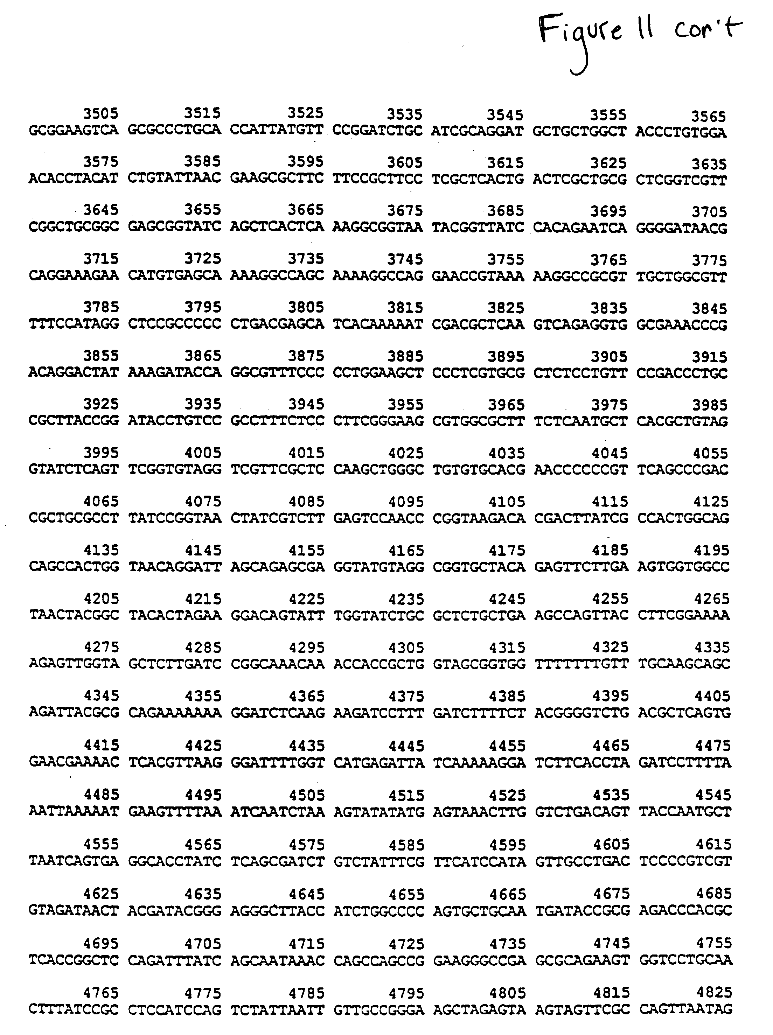

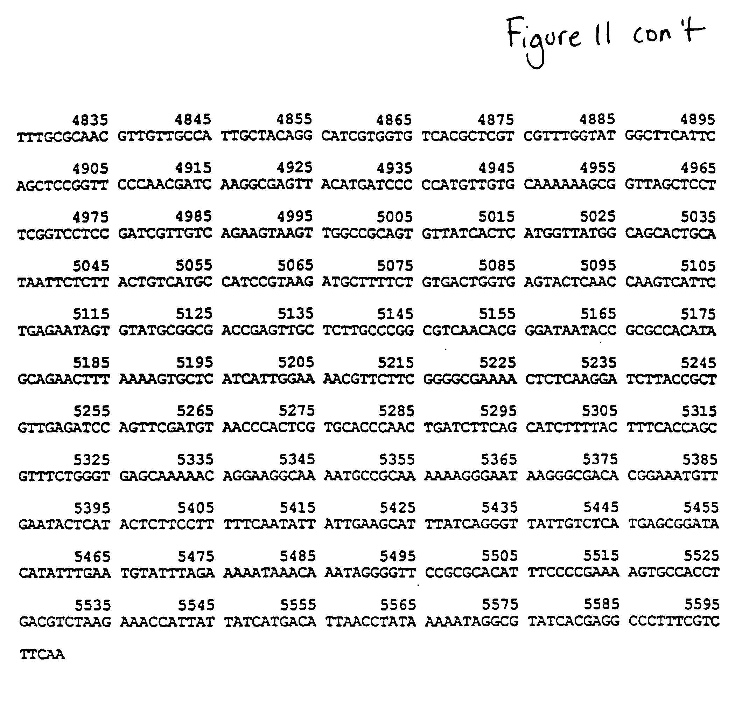

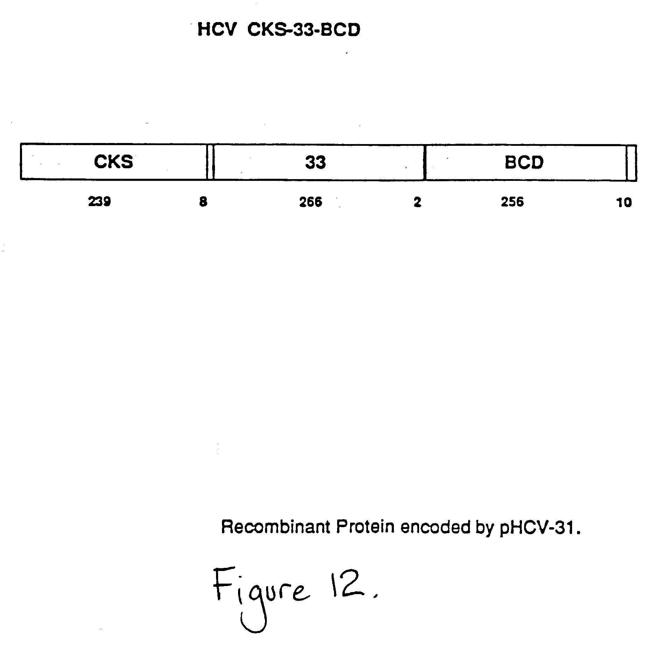

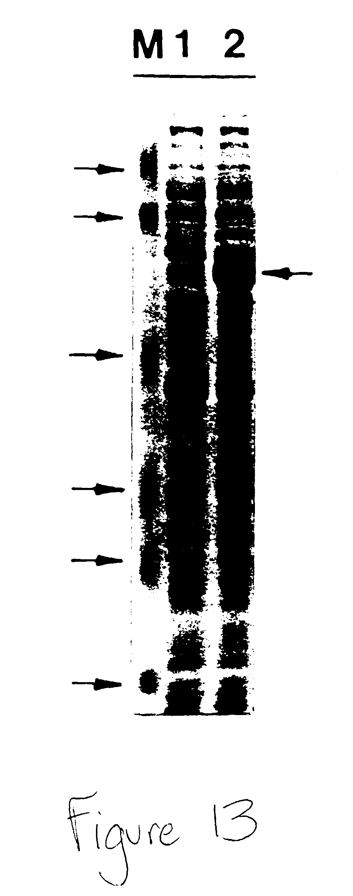

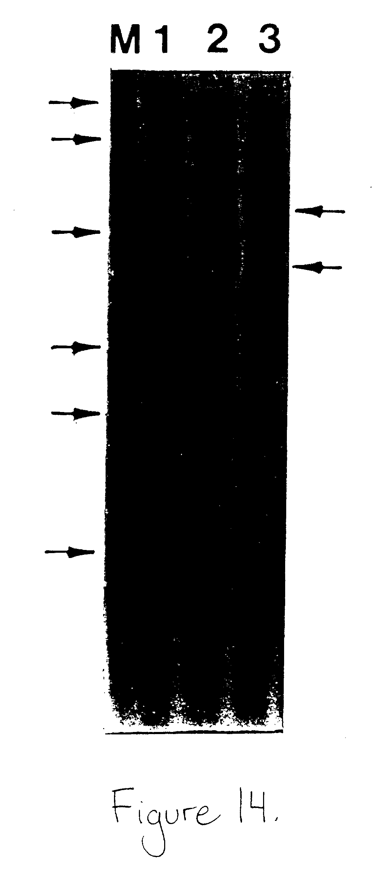

Characterization of pHCV CKS-33-BCD was carried out in a manner similar to pHCV CKS-Core of Example 1. pHCV-23, pHCV SDS/PAGE gels were run for E.coli lysates containing the plasmids pHCV-29 (Figure 13), pHCV-23 (Figure 14), and pHCV-31 (Figure 15) expressing the recombinant fusion proteins CKS-33c, CKS-BCD, and CKS-33-BCD, respectively. For all three figures, molecular weight standards were run in Lane M, with the arrows on the left indicating mobilities of the molecular weight markers the from top to bottom: 110,000; 84,000; 47,000; 33,000; 24,000; and 16,000 daltons. In Figure 13, Lane 1 contained the E.coli lysate containing pHCV-29 expressing HCV CKS-33c (amino acids 1192 to 1457) prior to induction and lane 2 after 4 hours induction. These results show that the recombinant pHCV-29 fusion protein has an apparent mobility corresponding to a molecular size of 60,000 daltons. This compares acceptably to the predicted molecular mass of 54,911.

In Figure 14, Lane 1 contained the E.coli lysate containing pJO200- the CKS vector without the HCV sequence. Lane 2, contained pHCV-20 expressing the HCV CKS-B (amino acids 1676 to 1790). Lane 3, contained the fusion protein pHCV-23 (amino acids 1676-1931). These results show that the recombinant pHCV-23 fusion protein has an apparent mobility corresponding to a molecular size of 55,000 daltons. This compares acceptably to the predicted molecular mass of 55,070 daltons.

In Figure 15, Lane 1 contained the E.coli lysate containing pJO200 the CKS vector without the HCV sequences. Lane 2 contained pHCV-31 expressing the CKS-33c-BCD fusion protein (amino acids 1192 to 1447 and 1676 to 1931) prior to induction and lane 3 after 2 hours induction. These results show that the recombinant pHCV-31 (CKS-33c-BCD) fusion protein has an apparent mobility corresponding to a molecular size of 90,000 daltons. This compares acceptably to the predicted molecular mass of 82,995 daltons.

An immunoblot was also run on one of the SDS/PAGE gels derived from the pHCV-31/X1-1 culture. Human serum from an HCV exposed individual reacted strongly with the major pHCV-31 band at 90,000 daltons. Normal human serum did not react with any component of the pHCV-31 (CKS-33-BCD) preparations.

EXAMPLE 3. SCREENING ASSAY

The use of recombinant polypeptides which contain epitopes within c100-3 as well as epitopes from other antigenic regions from the HCV genome, provide immunological assays which have increased sensitivity and may be more specific than HCV immunological assays using epitopes within c100-3 alone.

In the presently preferred screening assay, the procedure uses two E.coli expressed recombinant proteins, CKS-Core (pHCV-34) and CKS-33-BCD (pHCV-31), representing three distinct regions of the HCV genome. These recombinant polypeptides were prepared following procedures described above. In the screening assay, both recombinant antigens are coated onto the same polystyrene bead. In a modification of the screening assay the polystyrene bead may also be coated with the SOD-fusion polypeptide c100-3.

The polystyrene beads are first washed with distilled water and propanol and then incubated with a solution containing recombinant pHCV-31 diluted to 0.5 to 2.0 ug/ml and pHCV-34 diluted to 0.1 to 0.5 ug/ml in 0.1 M $NaH_2PO_4 \cdot H_20$ with 0.4M NaC1 and 0.0022% Triton X-100, pH 6.5. The beads are incubated in the antigen solution for 2 hours (plus or minus 10 minutes) at 38-42°C, washed in PBS and soaked in 0.1% (w/v) Triton X-100 in PBS for 60 minutes at 38-42°C. The beads are then washed two times in phosphate buffered saline (PBS), overcoated with a solution of 5.0% (w/v) bovine serum albumin (BSA) in PBS for 60 minutes at 38-42°C and washed one time in PBS. Finally, the beads are overcoated with 5% (w/v) sucrose in PBS, and dried under nitrogen or air.

The polystyrene beads coated with pHCV-31 and pHCV-34 are used in an antibody capture format. Ten microliters of sample are added to the wells of the reaction tray along with 400 ul of a sample diluent and the recombinant coated bead. The sample diluent consists of 10% (v/v) bovine serum and 20% (v/v) goat serum in 20 mM Tris phosphate buffer containing 0.15% (v/v) Triton X-100, 1%(w/v) BSA, 1% E.coli lysate and 500 ug/ml or less CKS lysate. When the recombinant yeast c100-3 polypeptide is used, antibodies to yeast antigens which may be present in a sample are reacted with yeast extracts which are added to the sample diluent (typically about 200 ug/ml). The addition of yeast extracts to the sample diluent is used to prevent false positive results. The final material is sterile filtered and filled in plastic bottles, and preserved with 0.1% sodium azide.

After one hour of incubation at 40°C, the beads are washed and 200 ul of conjugate is added to the wells of the reaction tray.

The preferred conjugate is goat anti-human IgG horseradish peroxidase conjugate. Concentrated conjugate is titered to determine a working concentration. A twenty-fold concentrate of the working conjugate solution is then prepared by diluting the concentrate in diluent. The 20X concentrate is sterile filtered and stored in plastic bottles.

The conjugate diluent includes 10% (v/v) bovine serum, 10% (v/v) goat serum and 0.15% Triton-X100 in 20 mM Tris buffer, pH 7.5 with 0.01% gentamicin sulfate, 0.01% thimerosal and red dye. The conjugate is sterile filtered and filled in plastic bottles.

Anti-HCV positive control is prepared from plasma units positive for antibodies to HCV. The pool of units used includes plasma with antibodies reactive to pHCV-31 and pHCV-34. The units are recalcified and heat inactivated at 59-61°C for 12 hours with constant stirring. The pool is aliquoted and stored at -20°C or at 2-8°C. For each lot of positive control, the stock solution is diluted with negative control containing 0.1% sodium azide as a preservative. The final material is sterile filtered and filled in plastic bottles.

Anti-HCV negative control is prepared from recalcified human plasma, negative for antibodies to pHCV-31 and pHCV-34 proteins of HCV. The plasma is also negative for antibodies to human immunodeficiency virus (HIV) and negative for hepatitis B surface antigen (HBsAg). The units are pooled, and 0.1% sodium azide is added as a preservative. The final material is sterile filtered and filled in plastic bottles.

After one hour of incubation with the conjugate at 40°C, the beads are washed, exposed to the OPD substrate for thirty minutes at room temperature and the reaction terminated by the addition of 1 N $H_2SO_4$. The absorbance is read at 492 nm.

In order to maintain acceptable specificity, the cutoff for the assay should be at least 5-7 standard deviations above the absorbance value of the normal population mean. In addition, it has generally been observed that acceptable specificity is obtained when the population mean runs at a sample to cutoff (S/CO) value of 0.25 or less. Consistent with these criteria, a "preclinical" cutoff for the screening assay was selected which clearly separated most of the presumed "true negative" from "true positive" specimens. The cutoff value was calculated as the sum of the positive control mean absorbance value multiplied by 0.25 and the negative control mean absorbance value. The cutoff may be expressed algebraically as:

$$\text{Cutoff value}=0.25\ PCx + NCx.$$

Testing may be performed by two methods which differ primarily in the degree of automation and the mechanism for reading the resulting color development in the assay. One method is referred to as the manual or Quantumt$_{tm}$ method because Quantum or Quantumatic is used to read absorbance at 492 nm. It is also called the manual method because sample pipetting, washing and reagent additions are generally done manually by the technician, using appropriately calibrated pipettes, dispensers and wash instruments. The second method is referred to as the PPC method and utilizes the automated Abbott Commander$^{R}$ system. This system employs a pipetting device referred to as the Sample Management Center (SMC) and a wash/dispense/read device referred to as the Parallel Processing Center (PPC) disclosed in the Abbott Disclosure No. 17256 entitled "Simultaneous Assay for Detecting One Or More Analytes" the inventor of which is William E. Brown, III. The optical reader used in the PPC has dual wavelength capabilities that can measure differential absorbencies (peak band and side band) from the sample wells. These readings are converted into results by the processor's Control Center.

Screening Assay Performance

1. Serum/Plasma From Inoculated Chimpanzees

As previously described, Table I summarizes the results of a study which followed the course of HCV infection in seven chimpanzees using a screening assay which utilized the c100-3 polypeptide, and the screening assay which utilized pHCV-31 and pHCV-34. Both assays gave negative results before inoculation and both assays detected the presence of antibodies after the animal had been infected with HCV. However, in the comparison of the two assays, the assay utilizing pHCV-31 and pHCV-34 detected seroconversion to HCV antigens at an earlier or equivalent bleed date in six of the seven chimpanzees. Data from these chimpanzee studies clearly demonstrate that overall detection of HCV antibodies is greatly increased with the assay utilizing the pHCV-31 and pHCV-34 proteins. This test is sufficiently sensitive to detect seroconversion during the acute phase of this disease, as defined as an elevation in ALT levels, in most animals. Equally important is the high degree of specificity of the test as no pre-inoculation specimens were reactive.

2. Non-A, Non-B Panel II (H. Alter, NIH)

A panel of highly pedigreed human sera from Dr. H. Alter, NIH, Bethesda, MD., containing infectious HCV sera, negative sera and other disease controls were tested. A total of 44 specimens were present in the panel.

Six of seven sera which were "proven infectious" in chimpanzees were positive in both the screening assay using c100-3 as well as in the screening assay utilizing the recombinant proteins pHCV-31 and pHCV-34. These six reactive specimens were obtained from individuals with chronic hepatitis. All six of the reactive specimens were confirmed positive using synthetic peptide sp67. One specimen obtained during the acute phase of NANB post-transfusion hepatitis was non-reactive in both screening assays.

In the group labeled "probable infectious" were three samples taken from the same post transfusion hepatitis patient. The first two acute phase samples were negative in both assays, but the third sample was reactive in both assay. The disease control samples and pedigreed negative controls were uniformly negative.

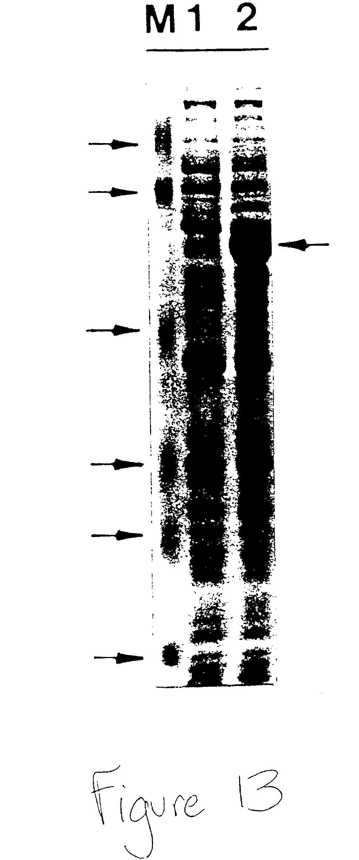

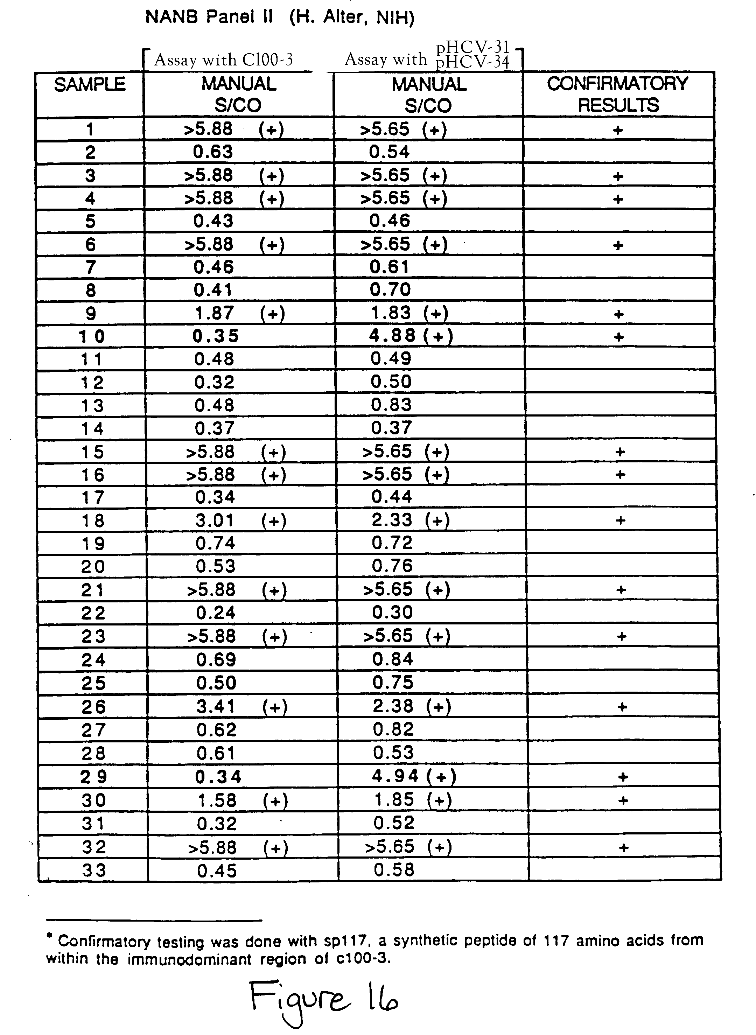

All sixteen specimens detected as positive by both screening assays were confirmed by the spll7 confirmatory assay (Figure 16). In addition, specimens 10 and 29 were newly detected in the screening assay utilizing the recombinant pHCV-31 and pHCV-34 antigens and were reactive by the sp75 confirmatory assay. Specimen 39 was initially reactive in the screening test utilizing pHCV-34 and pHCV-31, but upon retesting was negative and could not be confirmed by the confirmatory assays.

In summary, both screening tests identified 6 of 6 chronic NANBH carriers and 1 of 4 acute NANBH samples. Paired specimens from an implicated donor were non-reactive in the screening test utilizing c100-3 but were reactive in the screening test with pHCV-31 and pHCV-34. Thus, the screening test utilizing the recombinant antigens pHCV-31 and pHCV-34 appears to be more sensitive than the screening assay utilizing c100-3. None of the disease control specimens or pedigreed negative control specimens were reactive in either screening assay.

3. CBER Reference Panel

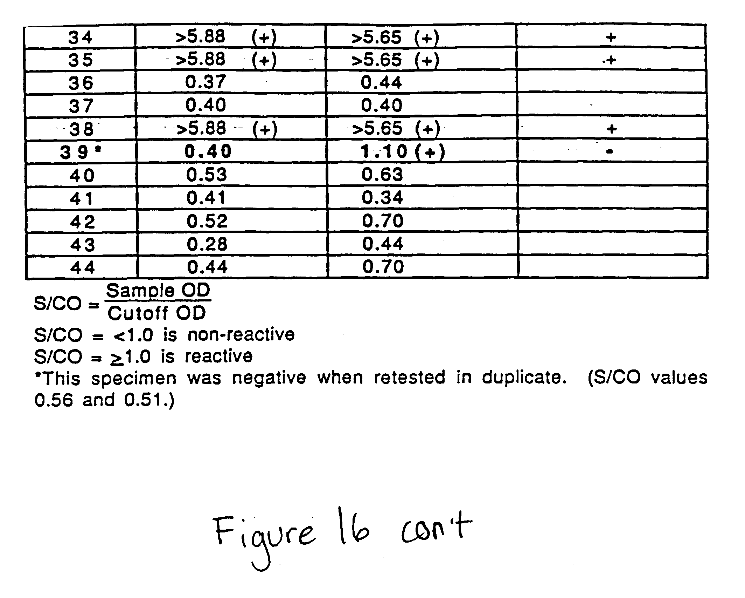

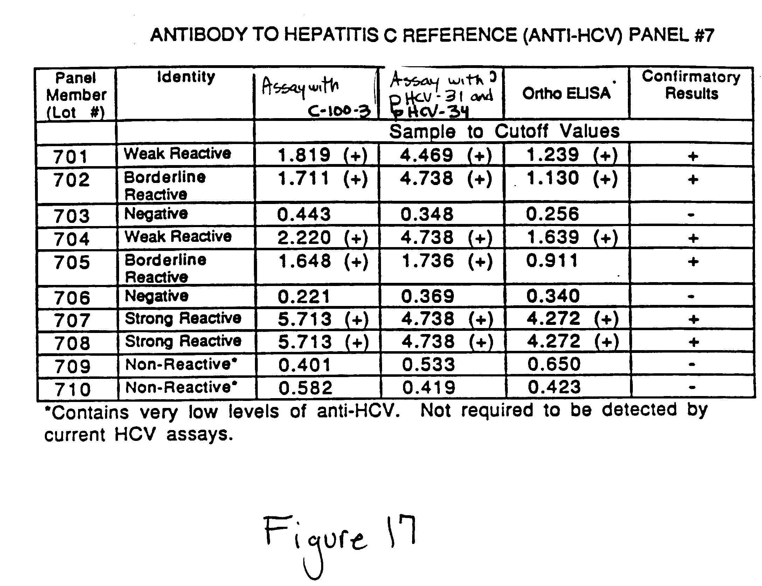

A reference panel for antibody to Hepatitis C was received from the Center for Biologics Evaluation and Research (CBER). This 10 member panel consists of eight reactive samples diluted in normal human sera negative for antibody to HCV and two sera that contain no detectable antibody to HCV. This panel was run on the Ortho first generation HCV EIA assay, the screening assay utilizing c100-3 and the screening assay utilizing pHCV-31 and pHCV-34. The assay results are presented in Figure 17.

The screening assay utilizing pHCV-31 and pHCV-34 detected all six of the HCV positive or borderline sample dilutions. The two non-reactive sample dilutions (709 and 710) appear to be diluted well beyond endpoint of antibody detectability for both screening assays. A marked increase was observed in the sample to cutoff values for three of the members on the screening assay utilizing pHCV-31 and pHCV-34 compared to the screening assay utilizing c100-3 or the Ortho first generation test. All repeatably reactive specimens were confirmed.

EXAMPLE 4. CONFIRMATORY ASSAY

The confirmatory assay provides a means for unequivocally identifying the presence of an antibody that is immunologically reactive with an HCV antigen. The confirmatory assay includes synthetic peptides or recombinant antigens representing major epitopes contained within the three distinct regions of the HCV genome, which are the same regions represented by the two recombinant antigens described in the screening assay. Recombinant proteins used in the confirmatory assay should have a heterologous source of antigen to that used in the primary screening assay (i.e. should not be an E.coli-derived recombinant antigen nor a recombinant antigen composed in part, of CKS sequences). Specimens repeatedly reactive in the primary screening assay are retested in the confirmatory assay. Aliquots containing identical amounts of specimen are contacted with a synthetic peptide or recombinant antigen individually coated onto a polystyrene bead. Seroreactivity for epitopes within the c100-3 region of the HCV genome are confirmed by use of the synthetic peptides sp67 and sp65. The synthetic peptide sp117 can also be used to confirm seroreactivity with the c100-3 region. Seroreactivity for HCV epitopes within the putative core region of HCV are confirmed by the use of the synthetic peptide sp75. In order to confirm seroreactivity for HCV epitopes within the 33c region of HCV, a recombinant antigen expressed as a chimeric protein with superoxide dismutase (SOD) in yeast is used. Finally, the antibody-antigen complex is detected.

The assay protocols were similar to those described in Example 3 above. The peptides are each individually coated onto polystyrene beads and used in an antibody capture format similar to that described for the screening assay. Ten microliters of specimen are added to the wells of a reaction tray along with 400 ul of a specimen diluent and a peptide coated bead. After one hour of incubation at 40°C, the beads are washed and 200 ul of conjugate (identical to that described in Example 3) is added to the wells of the reaction tray. After one hour of incubation at 40°C, the beads are washed, exposed to the OPD substrate for 30 minutes at room temperature and the reaction terminated by the addition of 1 N $H_2SO_4$. The absorbance is read at 492 nm. The cutoff value for the peptide assay is 4 times the mean of the negative control absorbance value.

1. Panels containing Specimens "At Risk" for HCV Infection.

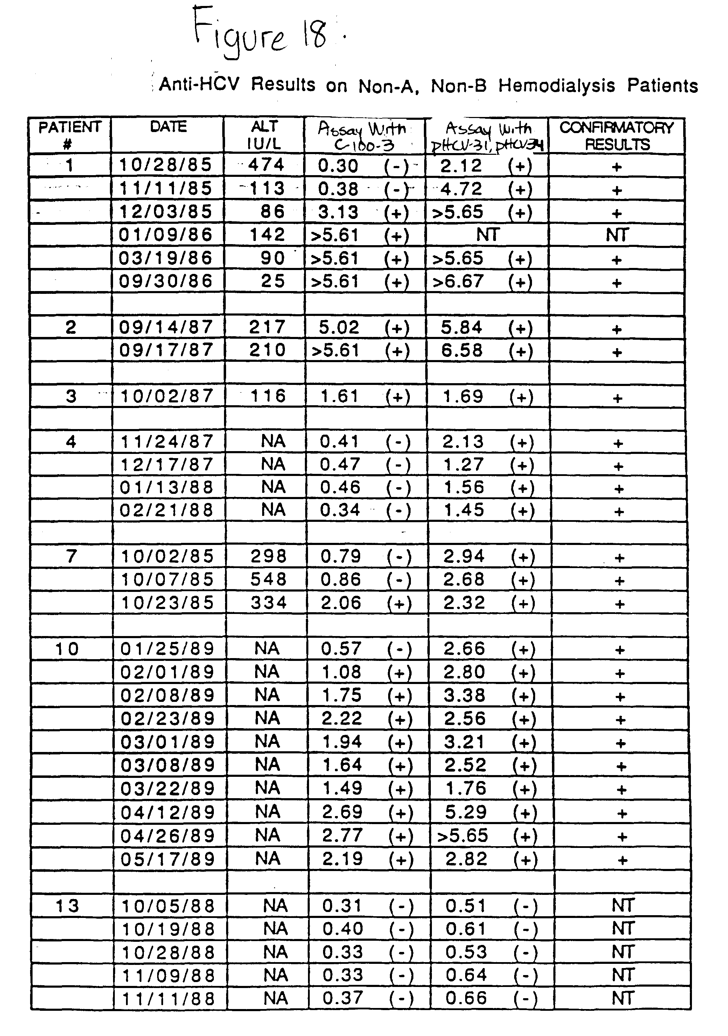

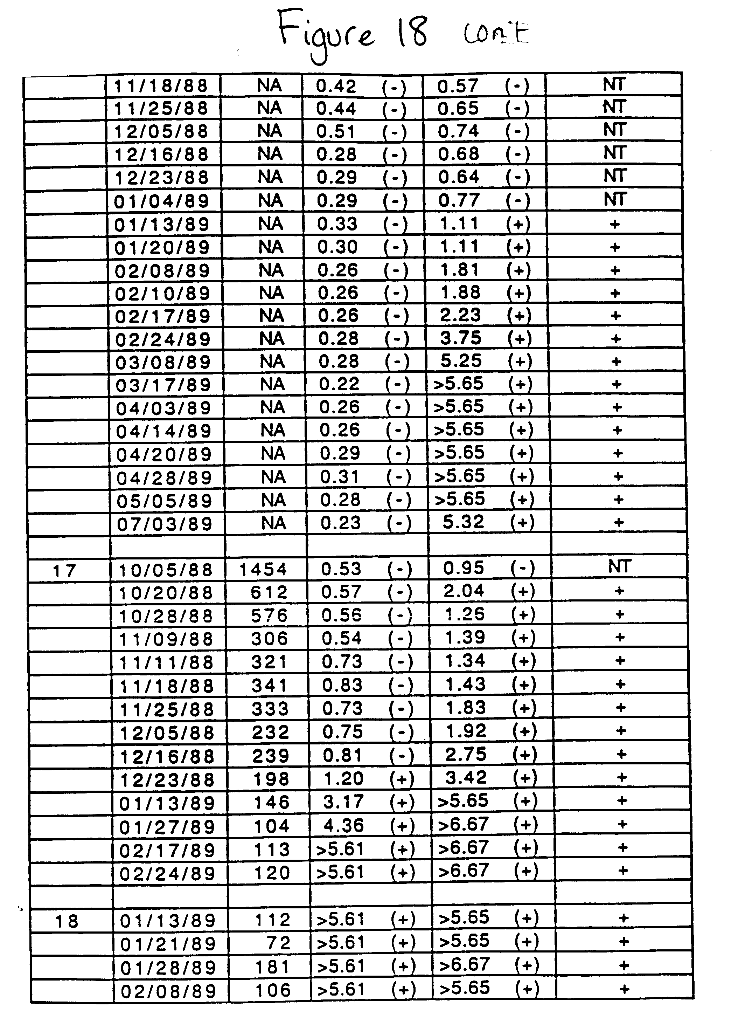

A group of 233 specimens representing 23 hemodialysis patients all with clinically diagnosed NANBH were supplied by Gary Gitnick, M.D. at the University of California, Los Angeles Center for the Health Sciences. These samples which were tested in by the screening assay utilizing c100-3 were subsequently tested in the screening assay which uses pHCV-31 and pHCV-34. A total of 7/23 patients (30.44%) were reactive in the c100-3 screening assay, with a total of 36 repeat reactive specimens. Ten of 23 patients (43.48%) were reactive by the screening assay utilizing pHCV-31 and pHCV-34, with a total of 70 repeatable reactives among the available specimens (Figure 18). Two specimens were unavailable for testing. All of the 36 repeatedly reactive specimens detected in the c100-3 screening assay were confirmed by synthetic peptide confirmatory assays. A total of 34 of these 36 were repeatedly reactive on HCV EIA utilizing pHCV-34 and pHCV-31: two specimens were not available for testing. Of the 36 specimens additionally detected by the screening assay utilizing pHCV-34 and pHCV-31, 9 were confirmed by the core peptide confirmatory assay (sp75) and 27 were confirmed by the SOD-33c confirmatory assay.

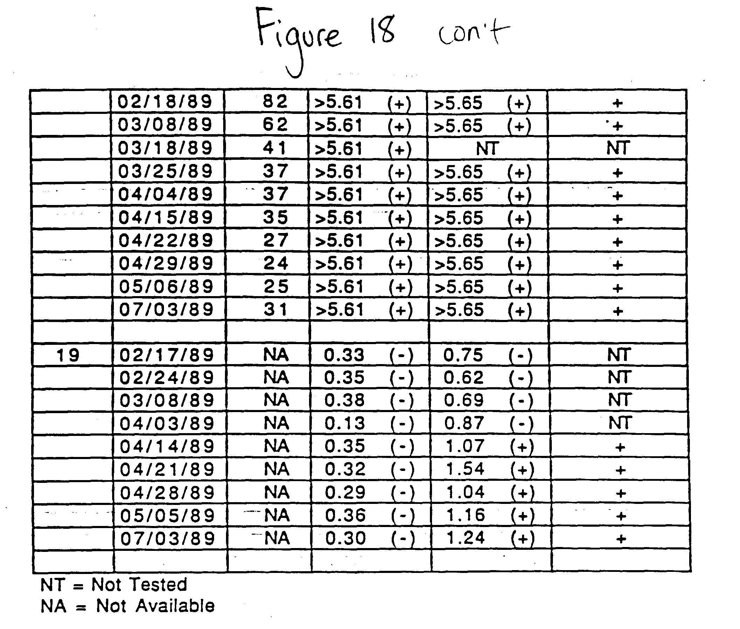

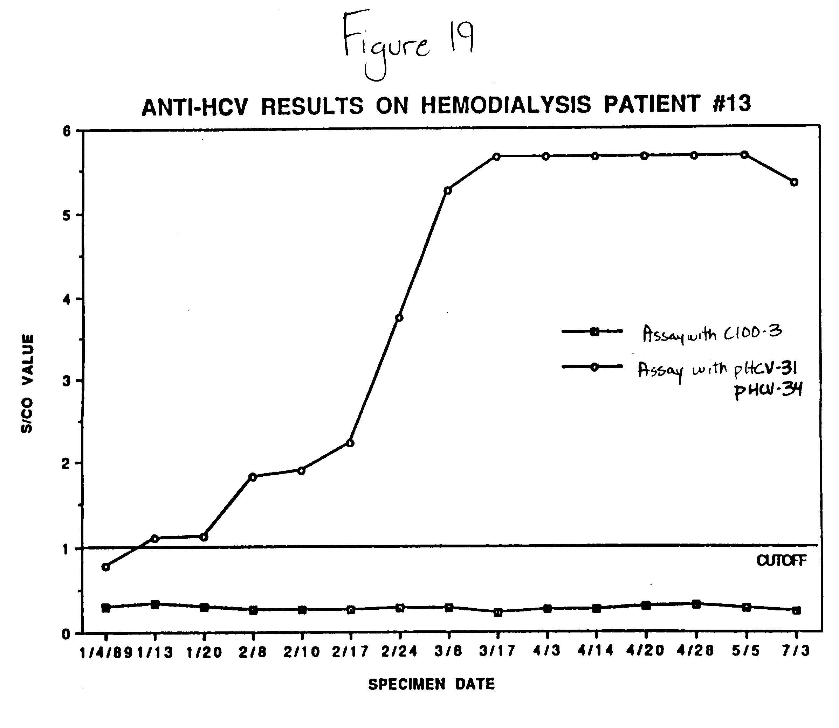

In summary these data indicate that detection of anti-HCV by the screening assay utilizing pHCV-31 and pHCV-34 may occur at an equivalent bleed date or as many as 9 months earlier, when compared to the c100-3 screening assay. Figure 19 depicts earlier detection by the screening assay utilizing pHCV-34 and pHCV-31 in a hemodialysis patient.

5. Acute/Chronic Non-A, Non-B Hepatitis

A population of specimens was identified from individuals diagnosed as having acute or chronic NANBH. Specimens from individuals with acute cases of NANBH were received from Gary Gitnick, M.D. at the University of California, Los Angeles Center for Health Sciences. The diagnosis of acute hepatitis was based on the presence of a cytolytic syndrome (ALT levels greater than 2X the upper normal limit) on at least 2 serum samples for a duration of less than 6 months with or without other biological abnormalities and clinical symptoms. All specimens were also negative for IgM antibodies to Hepatitis A Virus (HAV) and were negative for Hepatitis B surface Ag when tested with commercially available tests. Specimens from cases of chronic NANBH were obtained from two clinical sites. Individuals were diagnosed as having chronic NANBH based on the following criteria: persistently elevated ALT levels, liver biopsy results, and/or the absence of detectable HBsAg. Specimens with biopsy results were further categorized as either chronic active NANBH, chronic persistent NANBH, or chronic NANBH with cirrhosis.

These specimens were tested by both the c100-3 screening assay and the screening assay utilizing pHCV-34 and pHCV-31. The latter testing was performed in replicates of two by both the Quantum and PPC methods.

Community Acquired NANBH (Acute)

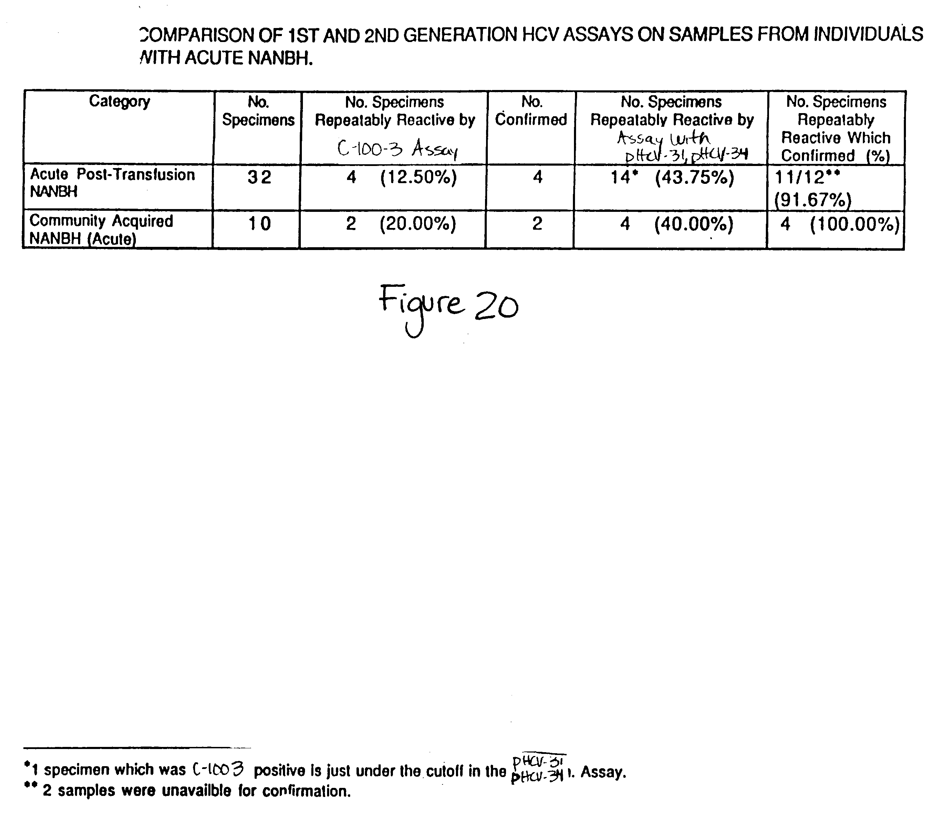

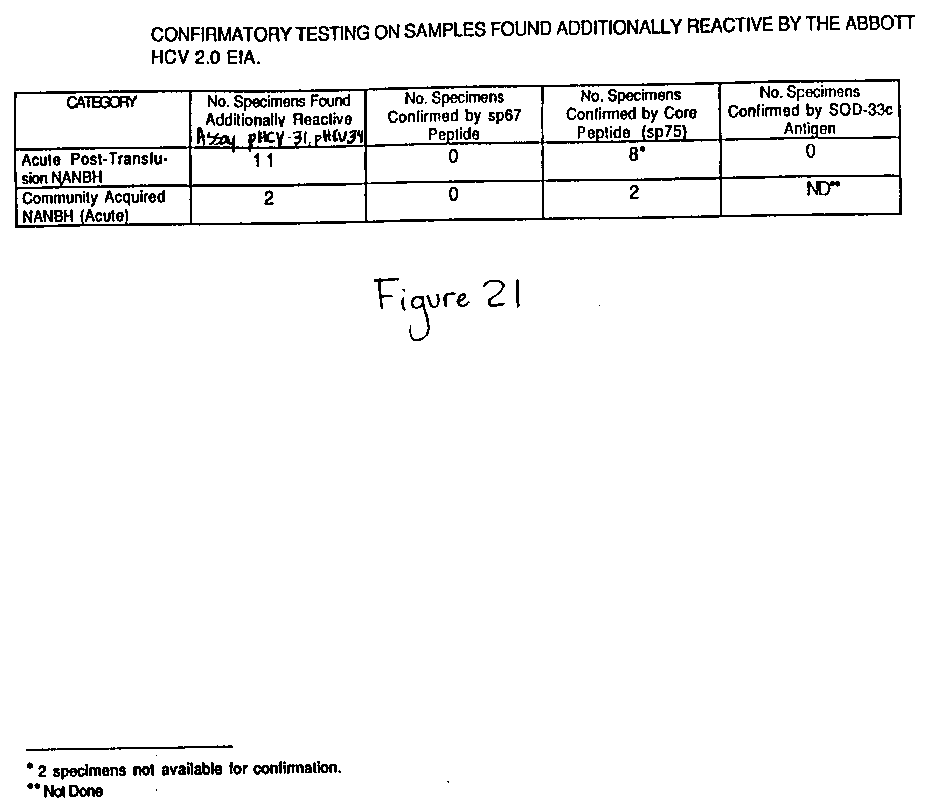

The c100-3 screening assay detected 2 of 10 specimens (20.00%) as repeatedly reactive, both of which were confirmed. The screening assay utilizing pHCV-34 and pHCV-31 detected both of these specimens plus and additional 2 specimens (Figure 20). These 2 specimens were confirmed by sp75 (see Figure 21).

Acute Post-Transfusion NANBH

The c100-3 assay detected 4 of 32 specimens (12.50%) as repeatedly reactive, all of which was confirmed. The screening assay utilizing pHCV-34 and pHCV-31 detected 3 out of these 4 specimens (75%) as reactive. The one sample that was missed had an S/CO of 0.95 by the latter screening test. This sample was confirmed by the sp67 peptide (Figure 20). In addition, the screening assay utilizing pHCV-34 and pHCV-31 detected 11 specimens not reactive in the c100-3 screening assay. Of the 9 specimens available for confirmation, 8 were confirmed by sp75 and 1 could not be confirmed but had an S/CO of 0.90 in the sp65 confirmatory test. (see Figure 21).

Chronic NANBH

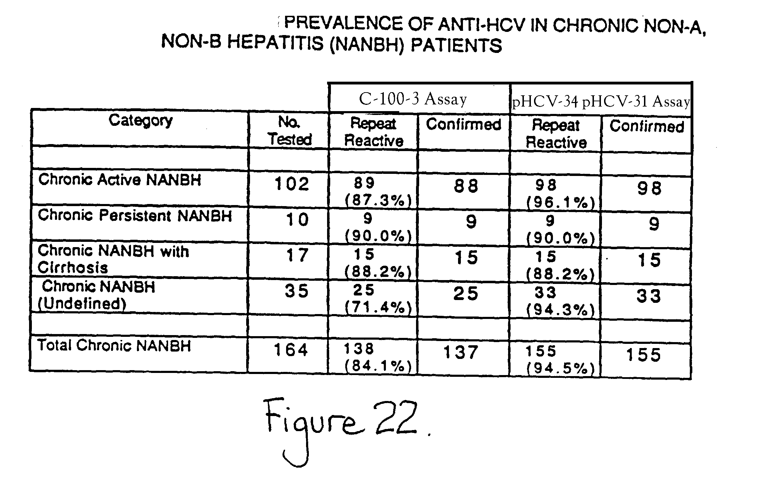

A summary of the results on these populations is shown in Figure 22. Overall, 155 of 164 (94.5%) chronic NANBH samples were detected by the screening test utilizing pHCV-31 and pHCV-34 using either Quantum or PPC. The 155 reactive samples were all confirmed in alternate assays using synthetic peptides based on sequences from either the c100, 33c or core regions of the HCV genome. In contrast, only 138 of 164 (84.1%) specimens were positive by the c100-3 assay. All but one of the 138 c100-3 samples were detected as positive by the screening assay utilizing pHCV-31 and pHCV-34. The one discordant specimen was not confirmed by either synthetic or neutralization assays. Conversely, there were 17 confirmed specimens which were positive only by the screening assay utilizing pHCV-34 and pHCV-31.

The results indicate that the screening assay utilizing pHCV-34 and pHCV-31 is more sensitive than the current test in detecting HCV positive individuals within chronically infected NANBH populations.

EXAMPLE 5. Competition ASSAY

The recombinant polypeptides containing antigenic HCV epitopes are useful for competition assays. To perform a neutralization assay, a recombinant polypeptide representing epitopes within the c100-3 region such as CKS-BCD (pHCV-23) is solubilized and mixed with a sample diluent to a final concentration of 0.5-50 ug/ml. Ten microliters of specimen or diluted specimen is added to a reaction well followed by 400 ul of the sample diluent containing the recombinant polypeptide and if desired, the mixture may be preincubated for about fifteen minutes to two hours. A bead coated with c100-3 antigen is then added to the reaction well and incubated for one hour at 40°C. After washing, 200 ul of a peroxidase labeled goat anti-human IgG in conjugate diluent is added and incubated for one hour at 40°C. After washing, OPD substrate is added and incubated at room temperature for thirty minutes. The reaction is terminated by the addition of 1 N sulfuric acid and the absorbance read at 492 nm.

Samples containing antibodies to the c100-3 antigen generate a reduced signal caused by the competitive binding of the peptides to these antibodies in solution. The percentage of competitive binding may be calculated by comparing the absorbance value of the sample in the presence of a recombinant polypeptide to the absorbance value of the sample assayed in the absence of a recombinant polypeptide at the same dilution.

EXAMPLE 6. IMMUNODOT ASSAY

The immunodot assay system uses a panel of purified recombinant polypeptides placed in an array on a nitrocellulose solid support. The prepared solid support is contacted with a sample and captures specific antibodies to HCV antigens. The captured antibodies are detected by a conjugate-specific reaction. Preferably, the conjugate specific reaction is quantified using a reflectance optics assembly within an instrument which has been described in U.S. Patent Applications Serial No. 07/227,408 filed August 2, 1988. The related U.S. Patent Applications Serial Nos. 07/227,272, 07/227,586 and 07/227,590 further describe specific methods and apparatus useful to perform an immunodot assay. The assay has also been described in U.S. Application Serial No. 07/532,489 filed June 6, 1990. Briefly, a nitrocellulose-base test cartridge is treated with multiple antigenic polypeptides. Each polypeptide is contained within a specific reaction zone on the test cartridge. After all the antigenic polypeptides have been placed on the nitrocellulose, excess binding sites on the nitrocellulose are blocked. The test cartridge is then contacted with a sample such that each antigenic polypeptide in each reaction zone will react if the sample contains the appropriate antibody. After reaction, the test cartridge is washed and any antigen-antibody reactions are identified using suitable well known reagents.

As described in the patent applications listed above, the entire process is amenable to automation. The specifications of these applications related to the method and apparatus for performing an immunodot assay are incorporated by reference herein.

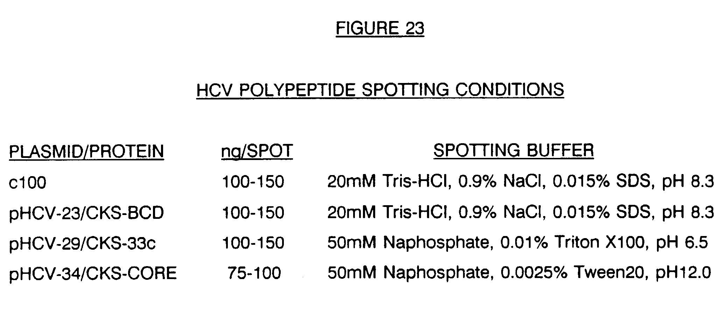

In a preferred immunodot assay, the recombinant polypeptides pHCV-23, pHCV-29, pHCV-34, and c100-3 were diluted in the preferred buffers, pH conditions, and spotting concentrations as summarized in Figure 23 and applied to a preassembled nitrocellulose test cartridge. After drying the cartridge overnight at room temperature 37°C, the non-specific binding capacity of the nitro-cellulose phase was blocked. The blocking solution contained 1% porcine gelatin, 1% casein enzymatic hydrolysate, 5% Tween-20, 0.1% sodium azide, 0.5 M sodium chloride and 20 mM Tris, pH 7.5.

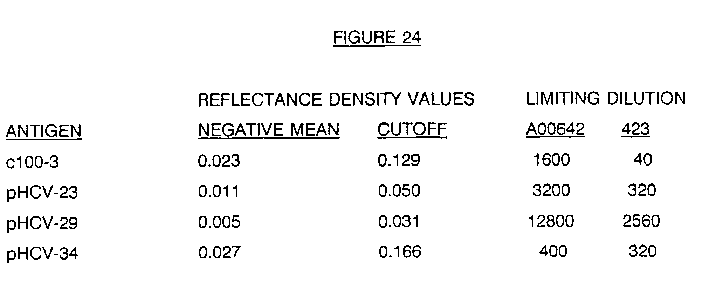

Forty normal donors were assayed by following the method described above. The mean reflectance density value then was determined for each of the recombinant proteins. A cutoff value was calculated as the negative mean plus six standard deviations. Test cartridges were incubated with samples A00642 and 423 (see Figure 24). Sample A00642 was from a convalescent non-A, non-B hepatitis patient, diluted in negative human plasma from 1:100 to 1:12800. The other sample, 423, was from a paid plasma donor which tested positive in an assay using a recombinant c100-3 polypeptide, diluted in negative human plasma from 1: 40 to 1:2560. After sample incubation, sequential incubations with a biotin-conjugated goat anti-human immunoglobulin-specific antibody, an alkaline phosphatase-conjugated rabbit anti-biotin specific antibody, and 5-bromo-4-chloro-3-indolyl phosphate produced a colored product at the site of the reaction. Sample to cutoff values (S/CO) were determined for all HCV recombinant proteins. Those S/CO values greater than or equal to 1.0 were considered reactive. The limiting dilution was defined as the lowest dilution at which the S/CO was greater than or equal to 1.0. As seen in Figure 24, each sample tested positive for all HCV recombinant proteins. The data demonstrate that reactivity for sample A00642 was greatest with pHCV-29, and decreased for the remaining antigens pHCV-23, c100-3, and pHCV-34. Sample 423 most strongly reacted with the recombinant proteins expressing pHCV-29 and pHCV-34, and to a lesser extent with pHCV-23 and c100-3.

## EXAMPLE 7 HCV CKS-NS5 EXPRESSION VECTORS

### A. Preparation of HCV CKS-NS5E

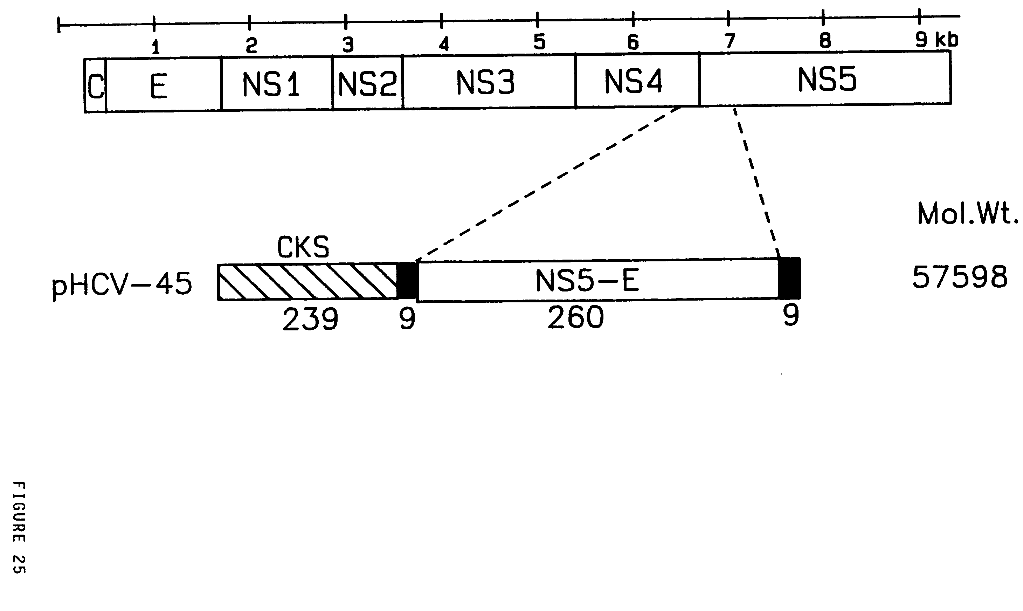

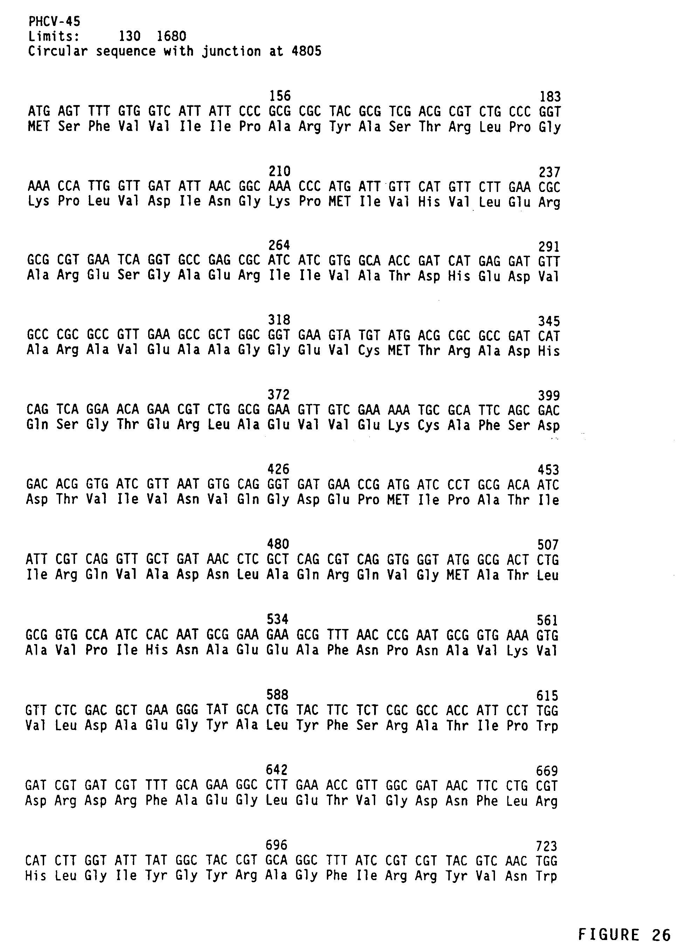

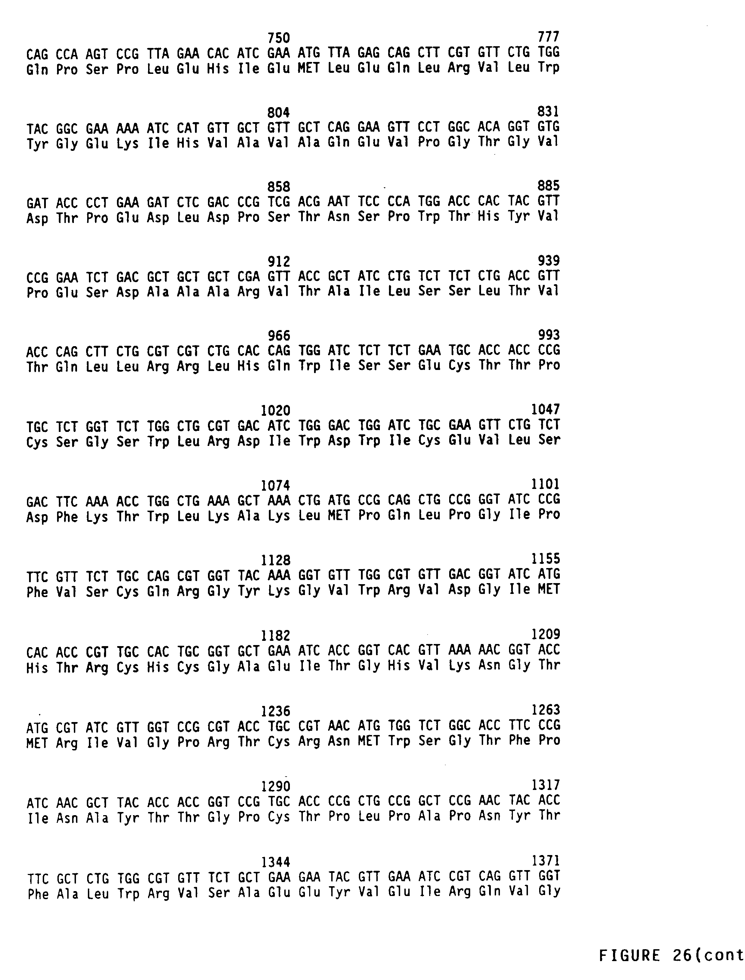

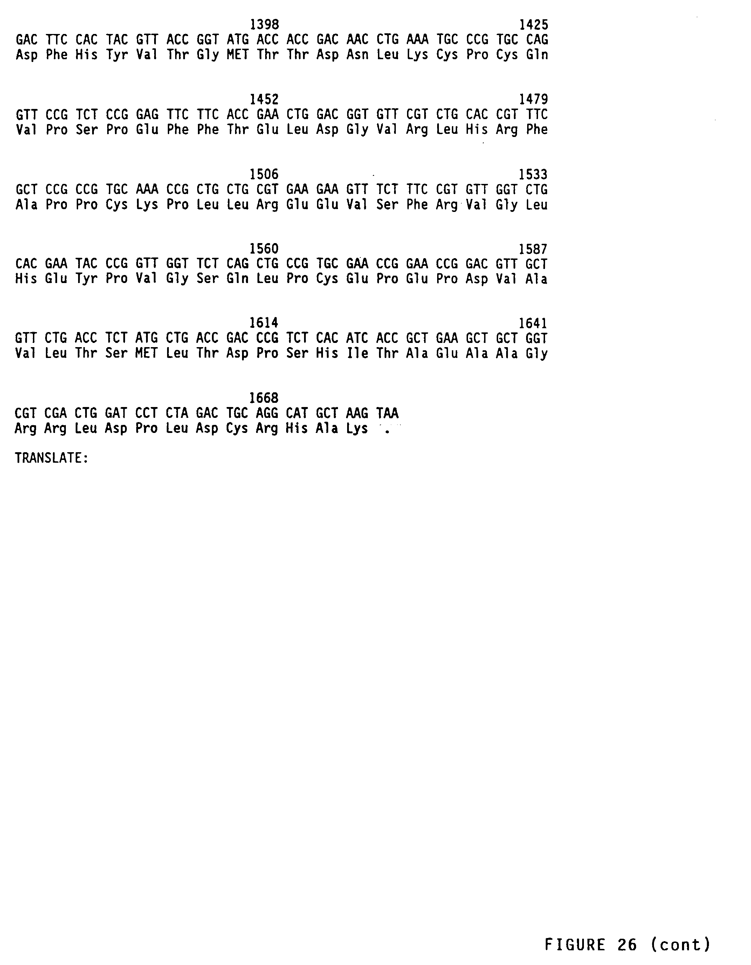

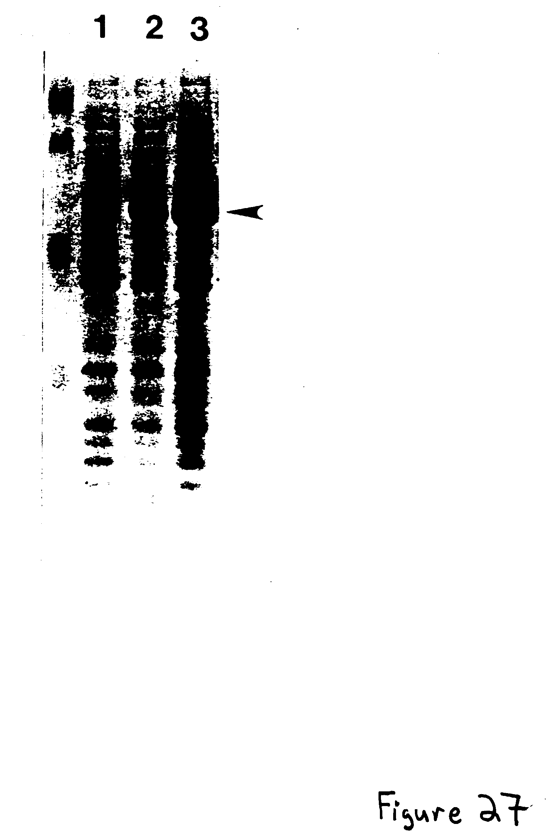

Eight individual oligonucleotides representing amino acids 1932-2191 of the HCV genome were ligated together and cloned as a 793 base pair EcoRI-BamHI fragment into the CKS fusion vector pJ0200. The resulting plasmid, designated pHCV-45, expresses the HCV CKS-NS5E antigen under control of the lac promoter. The HCV CKS-NS5E antigen consists of 239 amino acids of CKS, nine amino acids contributed by linker DNA sequences, and 260 amino acids from the HCV NS4/NS5 region (amino acids 1932-2191). Figure 25 presents a schematic representation of the recombinant antigen expressed by pHCV-45. Figure 26 presents the DNA and amino acid sequence of the HCV CKS-NS5E recombinant antigen produced by pHCV-45. Figure 27 presents the expression of pHCV-45 proteins in E.coli. Lane 1 contained the E.coli lysate containing pHCV-45 expressing the HCV CKS-NS5E antigen (amino acids 1932-2191) prior to induction and lanes 2 and 3 after 2 and 4 hours post induction, respectively. These results show that the pHCV-45 fusion protein has an apparent mobility corresponding to a molecular size of 55,000 daltons. This compares acceptably to the predicted molecular mass of 57,597 daltons.

### B. Preparation of HCV CKS-NS5F

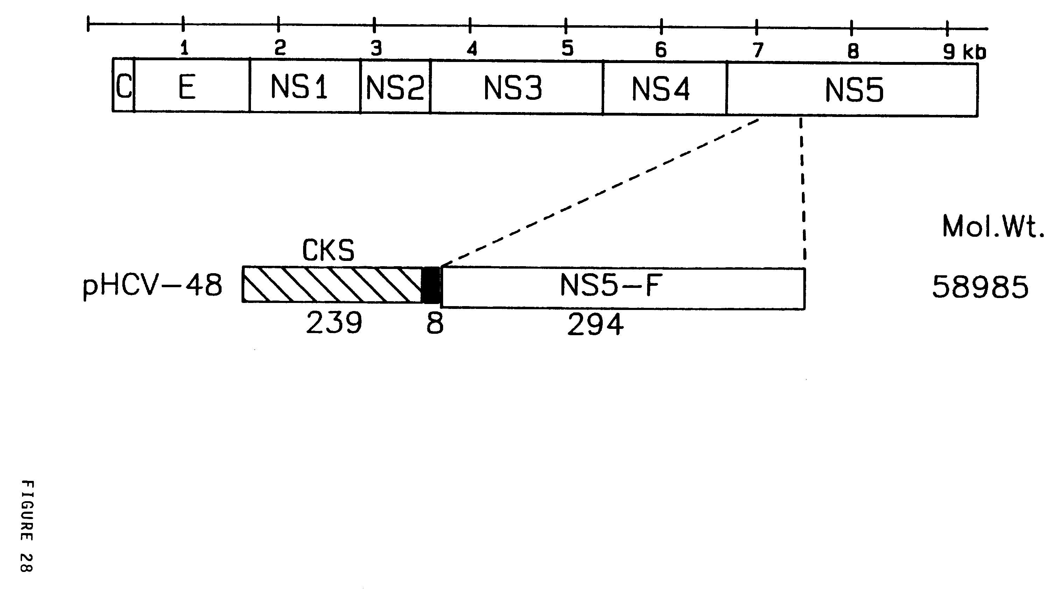

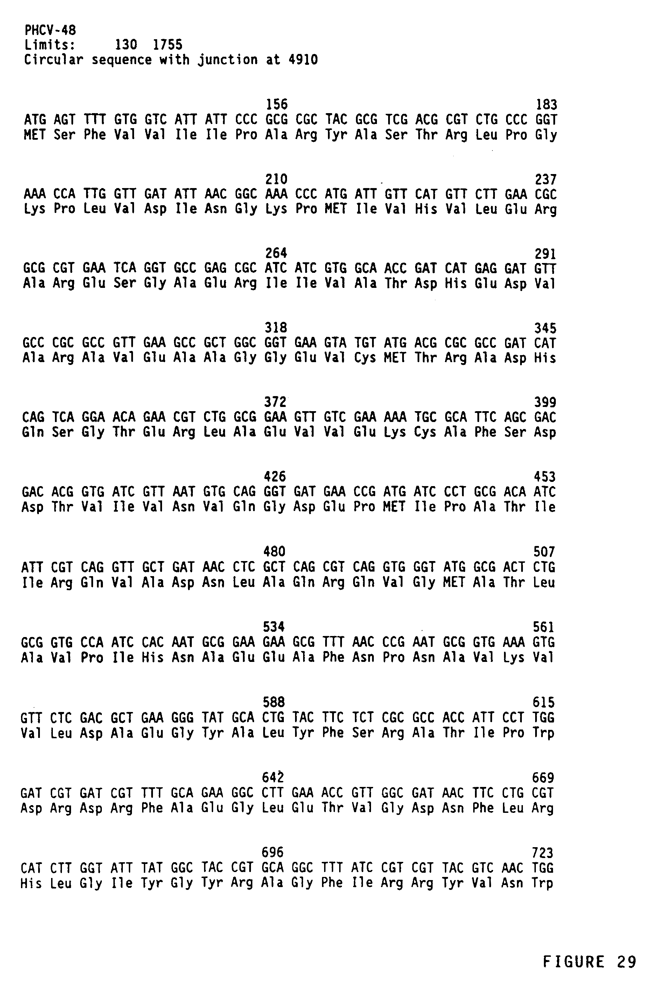

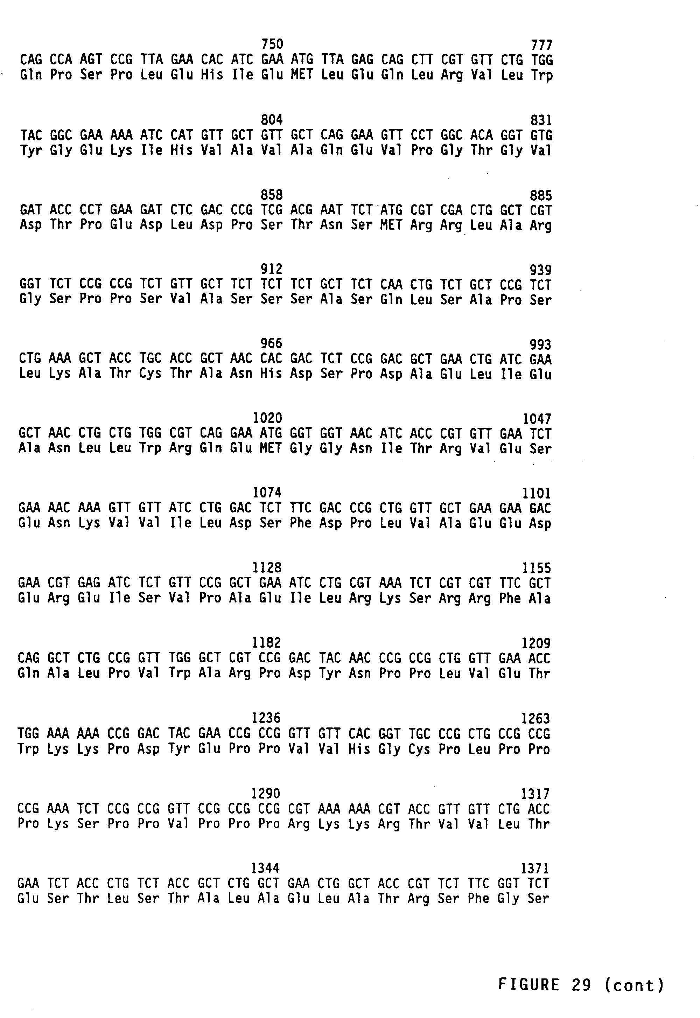

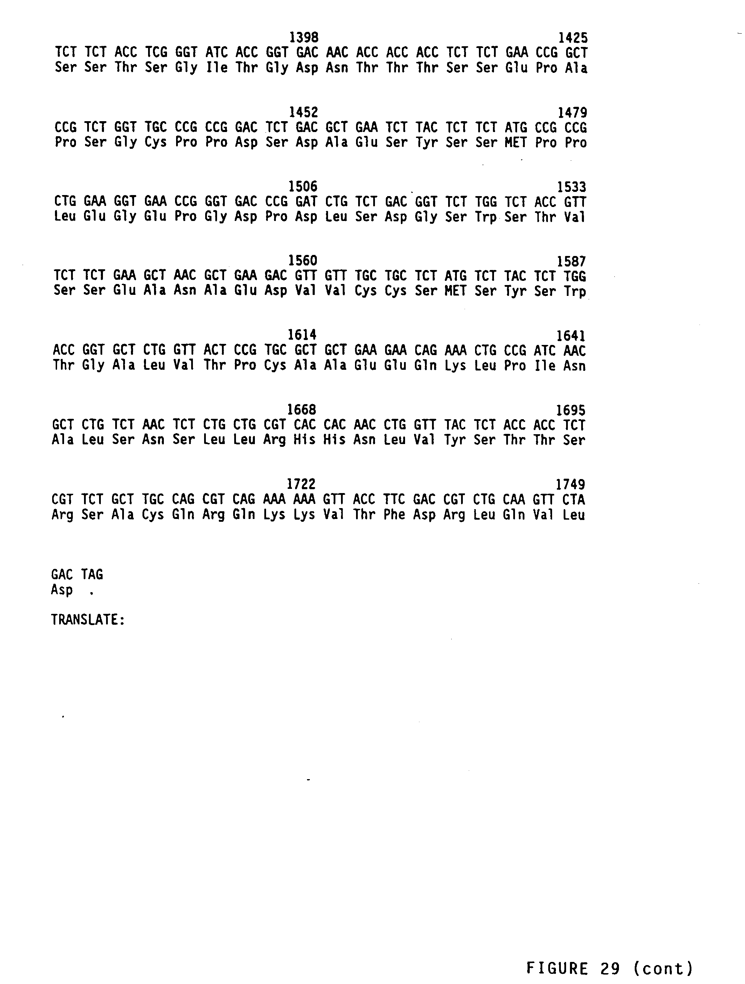

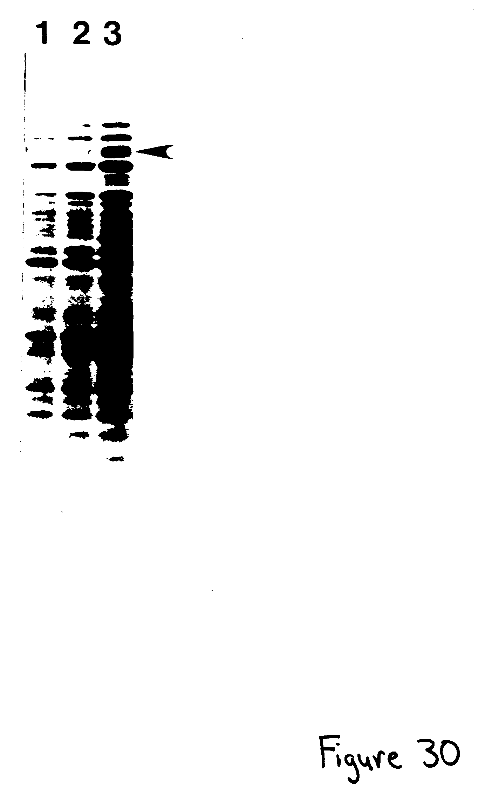

Eleven individual oligonucleotides representing amino acids 2188-2481 of the HCV genome were ligated together and cloned as a 895 base pair EcoRI-BamHI fragment into the CKS fusion vector pJ0200. The resulting plasmid, designated pHCV-48, expresses the HCV CKS-NS5F antigen under control of the lac promoter. The HCV CKS-NS5F antigen consists of 239 amino acids of CKS, eight amino acids contributed by linker DNA sequences, and 294 amino acids from the HCV NS5 region (amino acids 2188-2481). Figure 28 presents a schematic representation of the recombinant antigen expressed by pHCV-48. Figure 29 presents the DNA and amino acid sequence of the HCV CKS-NS5F recombinant antigen produced by pHCV-48. Figure 30 presents the expression of pHCV-48 proteins in E.coli. Lane 1 contained the E.coli lysate containing pHCV-48 expressing the HCV CKS-NS5F antigen (amino acids 2188-2481) prior to induction and lanes 2 and 3 after 2 and 4 hours post induction, respectively. These results show that the pHCV-48 fusion protein has an apparent mobility corresponding to a molecular size of 65,000 daltons. This compares acceptably to the predicted molecular mass of 58,985 daltons.

### C. Preparation of HCV CKS-NS5G

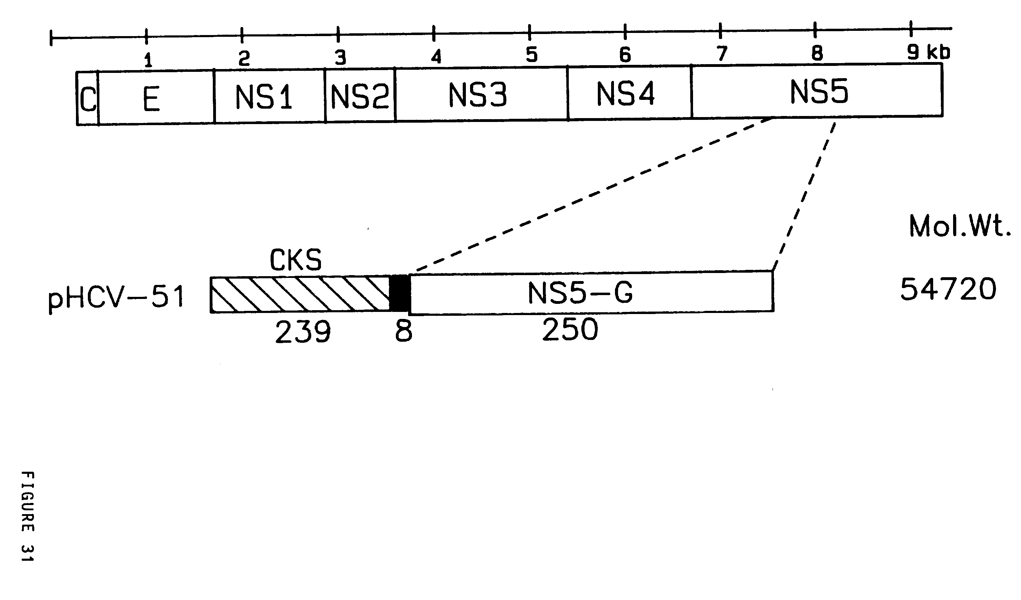

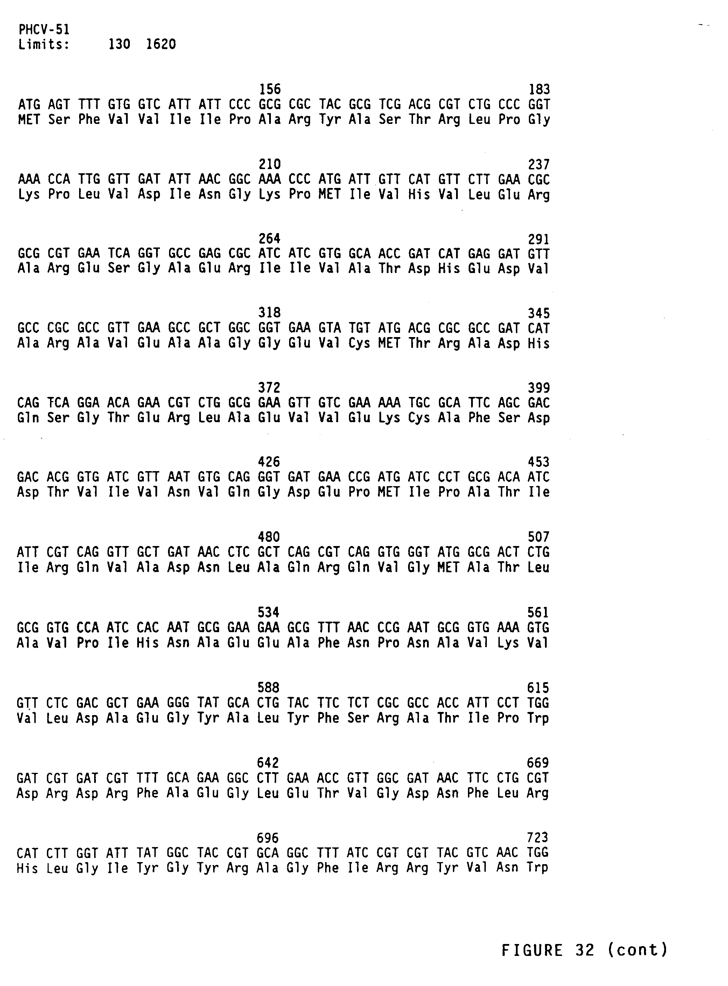

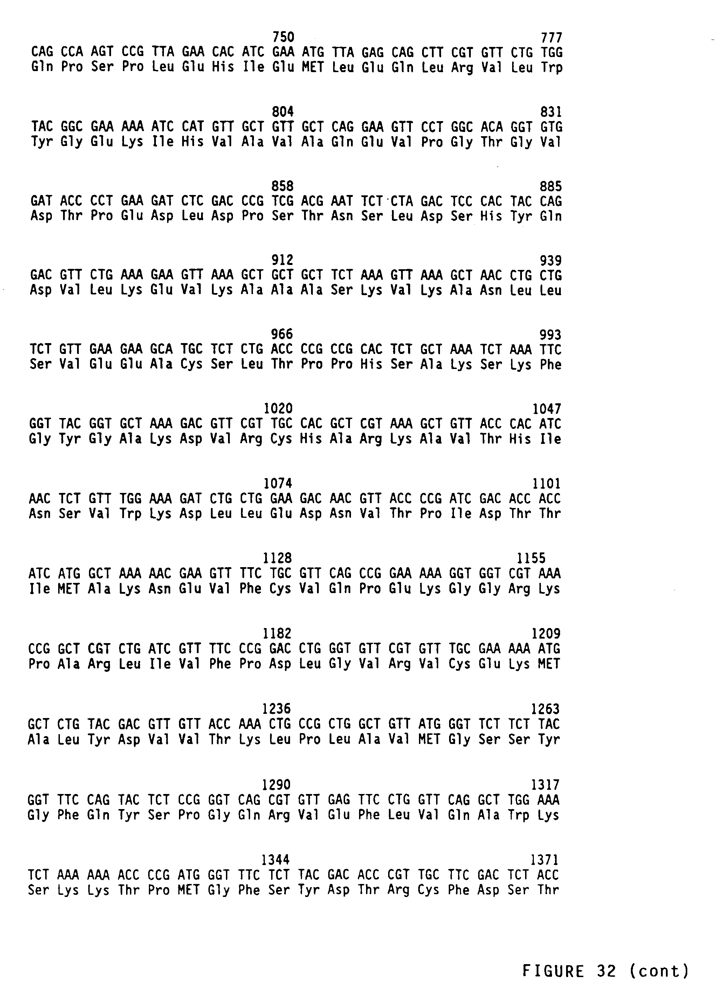

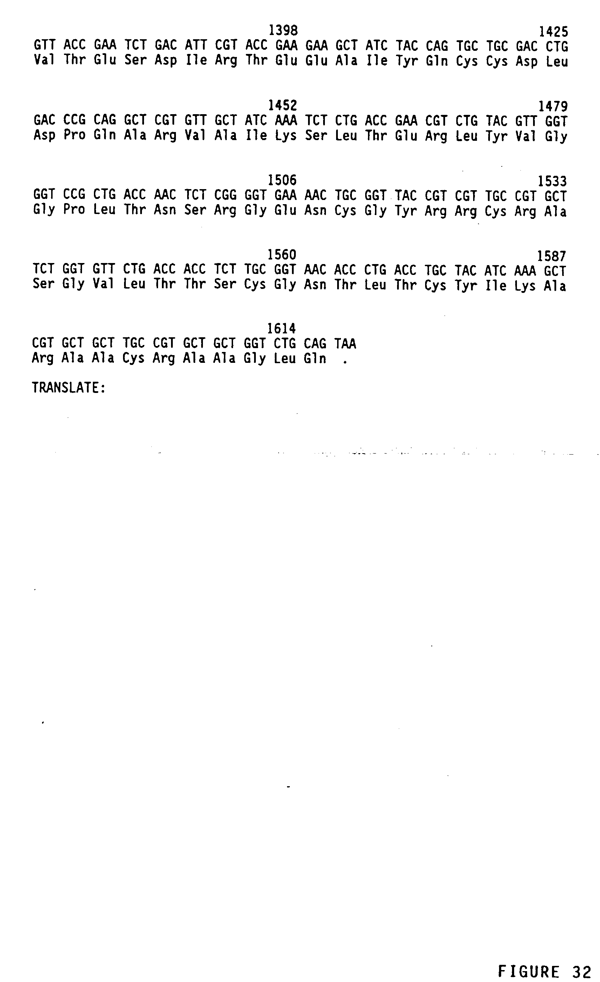

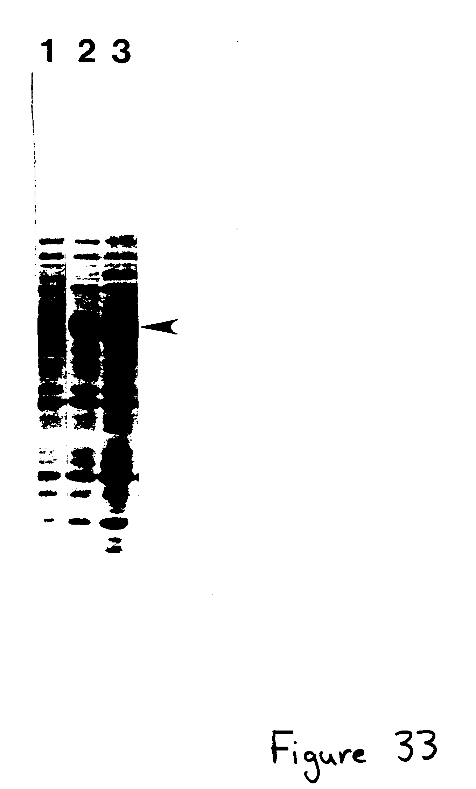

Seven individual oligonucleotides representing amino acids 2480-2729 of the HCV genome were ligated together and cloned as a 769 base pair EcoRI-BamHI fragment into the CKS fusion vector pJ0200. The resulting plasmid, designated pHCV-51, expresses the HCV CKS-NS5G antigen under control of the lac promoter. The HCV CKS-NS5G antigen consists of 239 amino acids of CKS, eight amino acids contributed by linker DNA sequences, and 250 amino acids from the HCV NS5 region (amino acids 2480-2729). Figure 31 presents a schematic representation of the recombinant antigen expressed by pHCV-51. Figure 32 presents the DNA and amino acid sequence of the HCV CKS-NS5G recombinant antigen produced by pHCV-51. Figure 33 presents the expression of pHCV-51 proteins in E.coli. Lane 1 contained the E.coli lysate containing pHCV-51 expressing the HCV CKS-NS5G antigen (amino acids 2480-2729) prior to induction and lanes 2 and 3 after 2 and 4 hours post induction, respectively. These results show that the pHCV-51 fusion protein has an apparent mobility corresponding to a molecular size of 55,000 daltons. This compares acceptably to the predicted molecular mass of 54,720 daltons.

D. Preparation of HCV CKS-NS5H

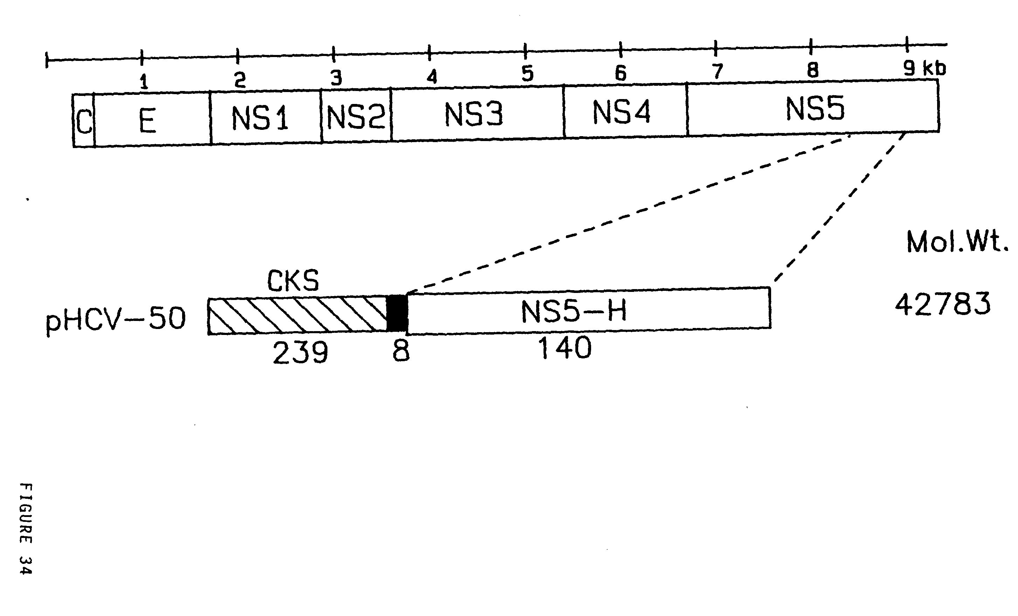

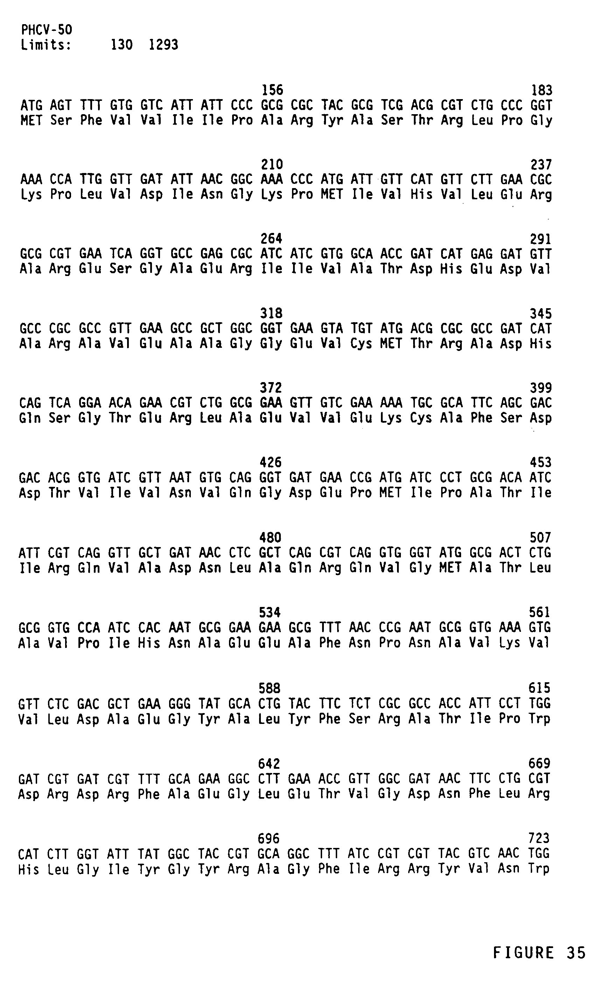

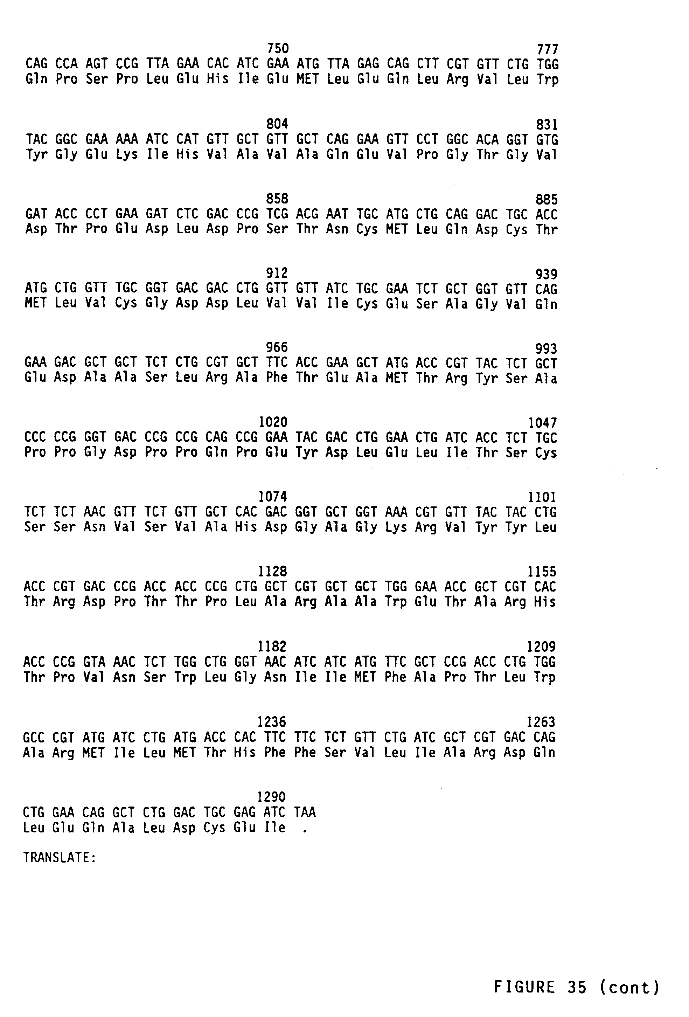

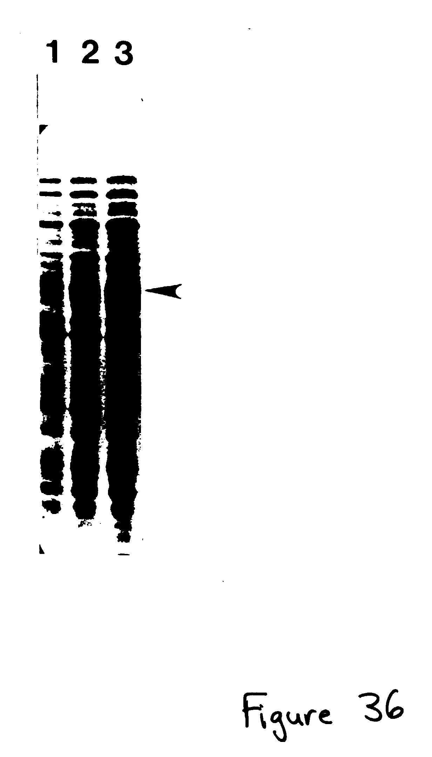

Six individual oligonucleotides representing amino acids 2728-2867 of the HCV genome were ligated together and cloned as a 439 base pair EcoRI-BamHI fragment into the CKS fusion vector pJ0200. The resulting plasmid, designated pHCV-50, expresses the HCV CKS-NS5H antigen under control of the lac promoter. The HCV CKS-NS5H antigen consists of 239 amino acids of CKS, eight amino acids contributed by linker DNA sequences, and 140 amino acids from the HCV NS5 region (amino acids 2728-2867). Figure 34 presents a schematic representation of the recombinant antigen expressed by pHCV-50. Figure 35 presents the DNA and amino acid sequence of the HCV CKS-NS5H recombinant antigen produced by pHCV-50. Figure 36 presents the expression of pHCV-50 proteins in E.coli. Lane 1 contained the E.coli lysate containing pHCV-50 expressing the HCV CKS-NS5H antigen (amino acids 2728-2867) prior to induction and lanes 2 and 3 after 2 and 4 hours post induction, respectively. These results show that the pHCV-50 fusion protein has an apparent mobility corresponding to a molecular size of 45,000 daltons. This compares acceptably to the predicted molecular mass of 42,783 daltons.

E. Preparation of HCV CKS-NS5I

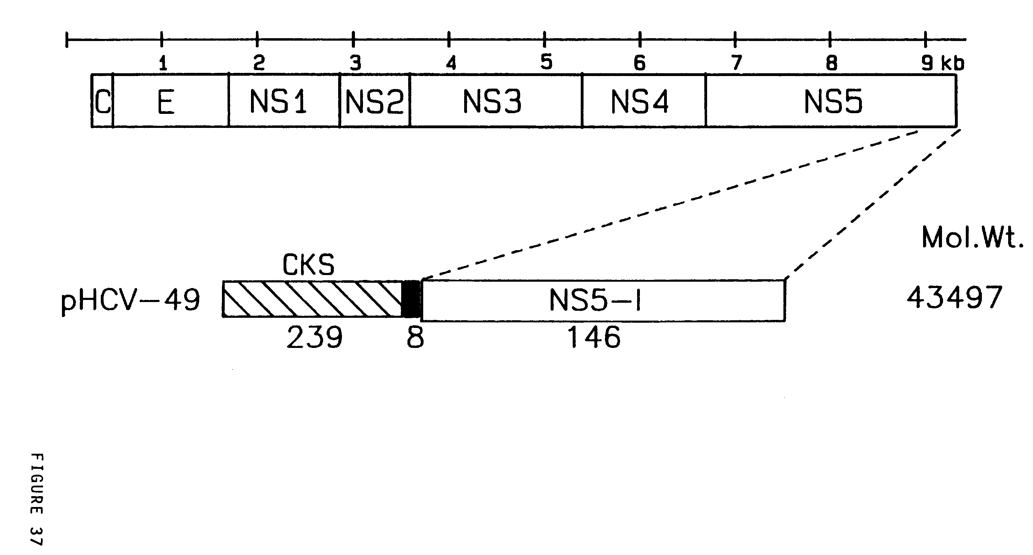

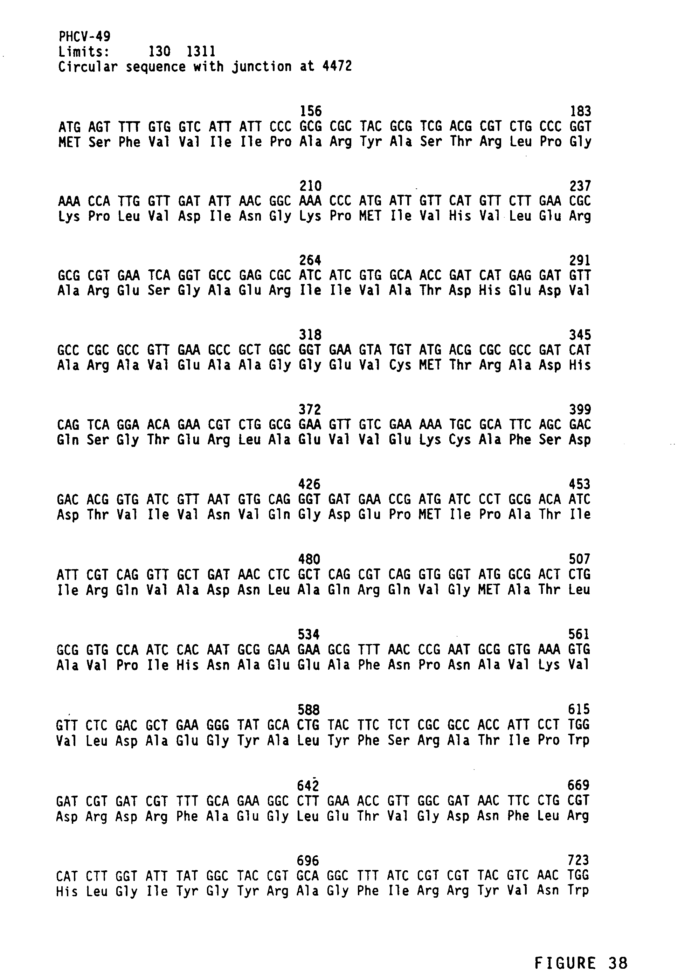

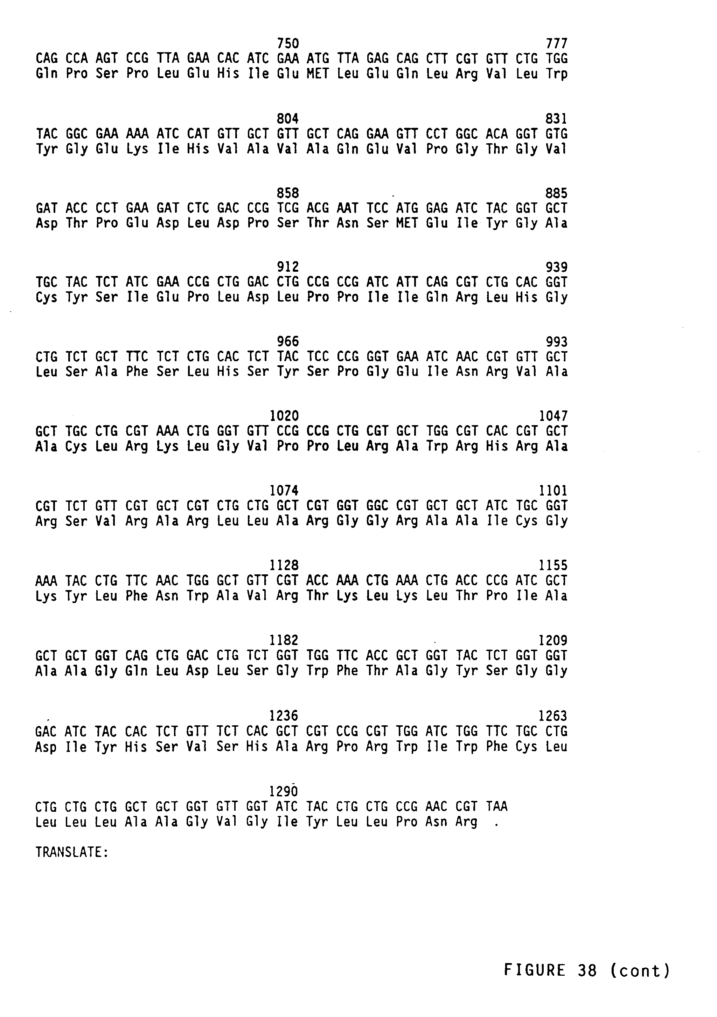

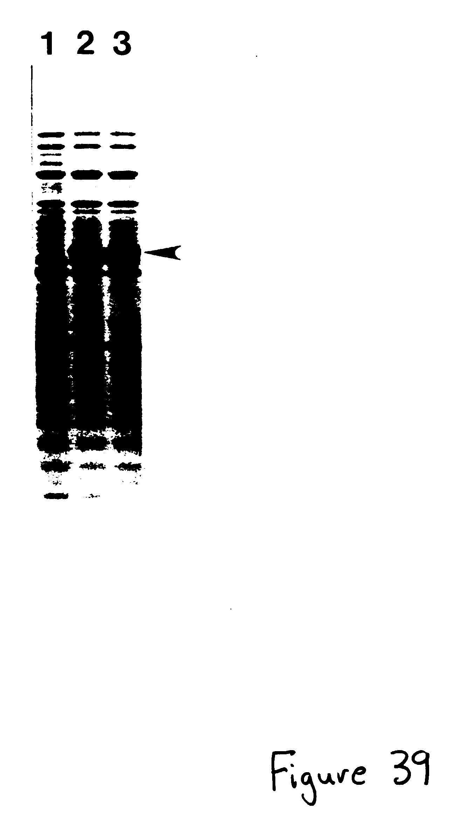

Six individual oligonucleotides representing amino acids 2866-3011 of the HCV genome were ligated together and cloned as a 460 base pair EcoRI-BamHI fragment into the CKS fusion vector pJ0200. The resulting plasmid, designated pHCV-49, expresses the HCV CKS-NS5I antigen under control of the lac promoter. The HCV CKS-NS5I antigen consists of 239 amino acids of CKS, eight amino acids contributed by linker DNA sequences, and 146 amino acids from the HCV NS5 region (amino acids 2866-3011). Figure 37 presents a schematic representation of the recombinant antigen expressed by pHCV-49. Figure 38 presents the DNA and amino acid sequence of the HCV CKS-NS5I recombinant antigen produced by pHCV-49. Figure 39 presents the expression of pHCV-49 proteins in E.coli. Lane 1 contained the E.coli lysate containing pHCV-49 expressing HCV CKS-NS5I antigen (amino acids 2866-3011) prior to induction and lanes 2 and 3 after 2 and 4 hours post induction, respectively. These results show that the pHCV-49 fusion protein has an apparent mobility corresponding to a molecular size of 42,000 daltons. This compares acceptably to the predicted molecular mass of 43,497 daltons.

F. Immunoblot of HCV CKS-NS5 Antigens

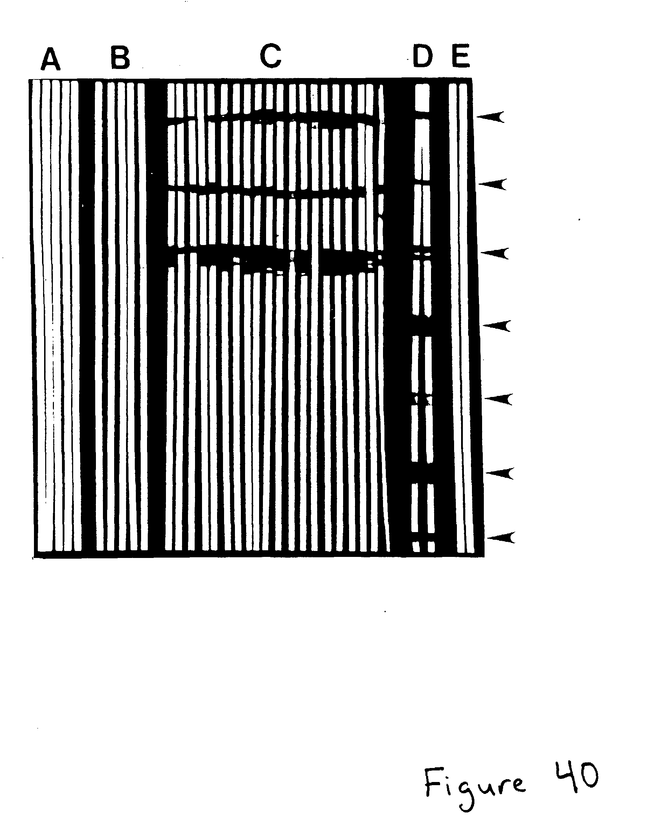

Induced E.coli lysates containing pHCV-23, pHCV-45, pHCV-48, pHCV-51, pHCV-50, or pHCV-49 were individually run on preparative SDS/PAGE gels to separate the various HCV CKS-NS5 or HCV CKS-BCD recombinant antigens assay from the majority of other E.coli proteins. Gel slices containing the separated individual HCV CKS-NS5 or HCV CKS-BCD recombinant antigens were then electropheretically transferred to nitrocellulose, and the nitrocellulose sheet cut into strips. Figure 40 presents the results of a Western Blot analysis of various serum or plasma samples using these nitrocellulose strips. The arrows on the right indicate the position of each HCV CKS-BCD or HCV CKS-NS5 recombinant antigen, from top to bottom pHCV-23 (HCV CKS-BCD), pHCV-45 (HCV CKS-NS5E), pHCV-48 (HCV CKS-NS5F), pHCV-51 (HCV CKS-NS5G), pHCV-50 (HCV CKS-NS5H), pHCV-49 (HCV CKS-NS5I), and pJO200 (CKS). Panel A contained five normal human plasma, panel B contained five normal human sera, panel C contained twenty human sera positive in the Abbott HCV EIA test, panel D contained two mouse sera directed against CKS, and panel E contained two normal mouse sera. Both the HCV CKS-NS5E antigen expressed by pHCV-45 and the HCV CKS-NS5F antigen expressed by pHCV-48 were immunoreactive when screened with human serum samples containing HCV antibodies.

EXAMPLE 8 HCV CKS-C100

A. Preparation of HCV CKS-C100 Vectors

Eighteen individual oligonucleotides representing amino acids 1569-1931 of the HCV genome were ligated together and cloned as four separate EcoRI-BamHI subfragments into the CKS fusion vector pJ0200. After subsequent DNA sequences confirmation, the four subfragments were digested with the appropriate restriction enzymes, gel purified, ligated together, and cloned as an 1102 base pair EcoRI-BamHI fragment in the CKS fusion vector pJ0200. The resulting plasmid, designated pHCV-24, expresses the HCV CKS-C100 antigen under control of the lac promoter. The HCV CKS-c100 antigen consists of 239 amino acids of CKS, eight amino acids contributed by linker DNA sequences, 363 amino acids from the HCV NS4 region (amino acids 1569-1931) and 10 additional amino acids contributed by linker DNA sequences. The HCV CKS-c100 antigen was expressed at very low levels by pHCV-24.

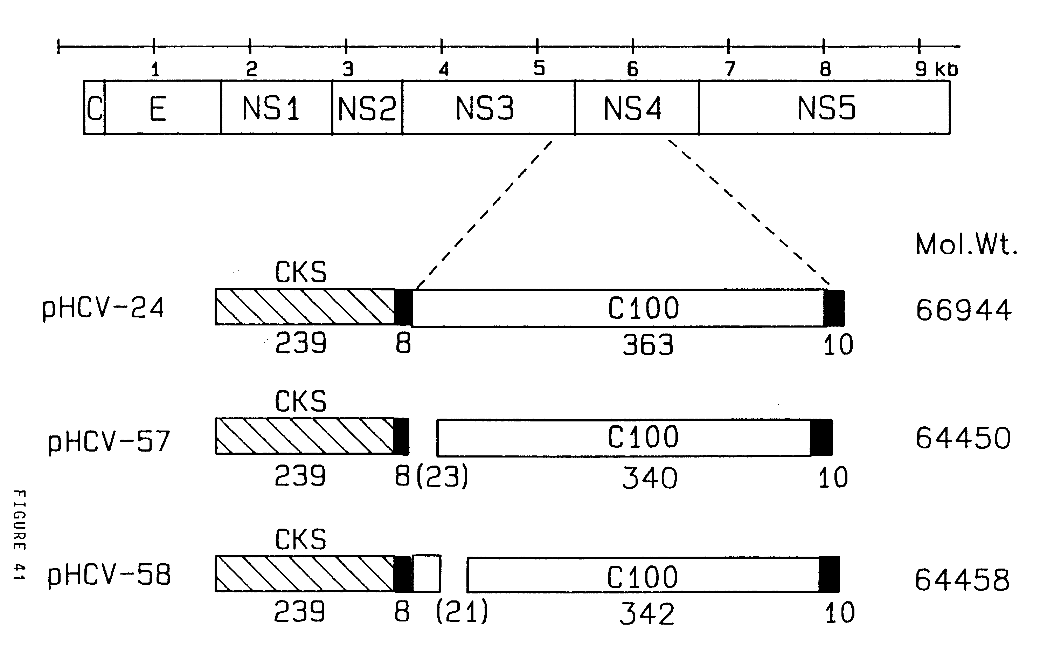

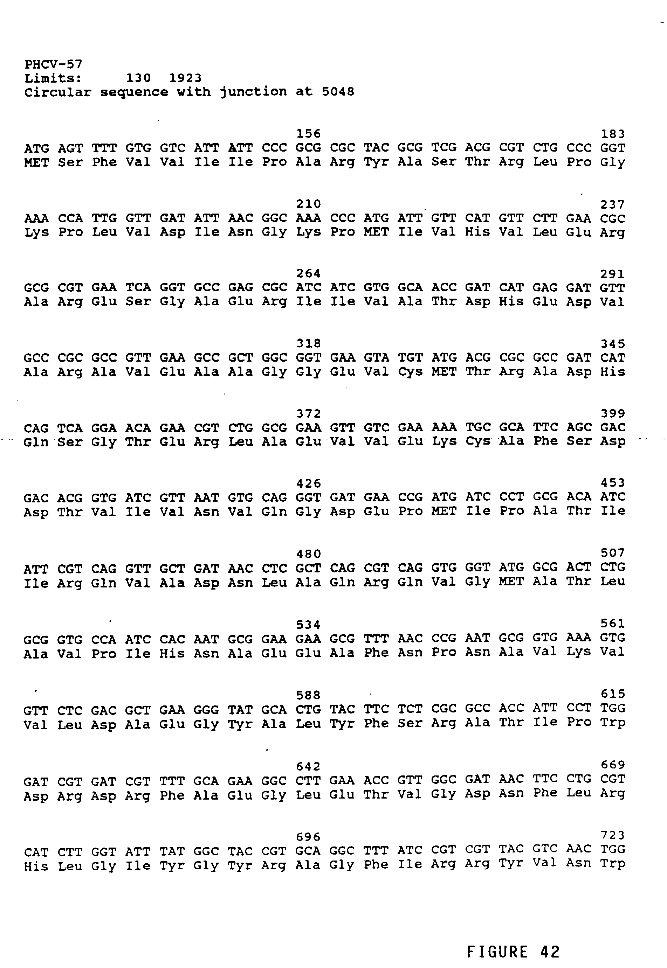

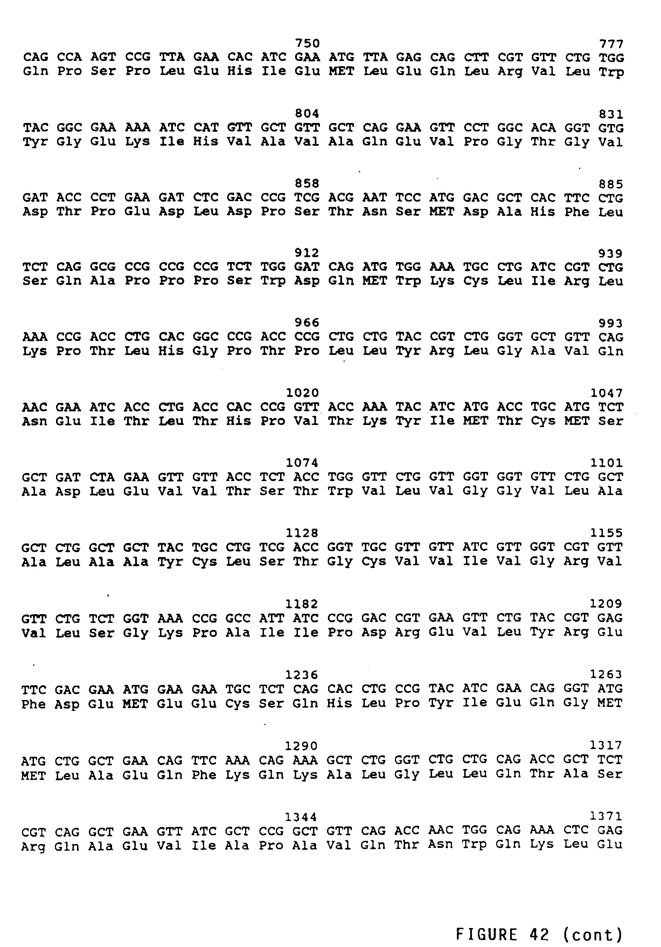

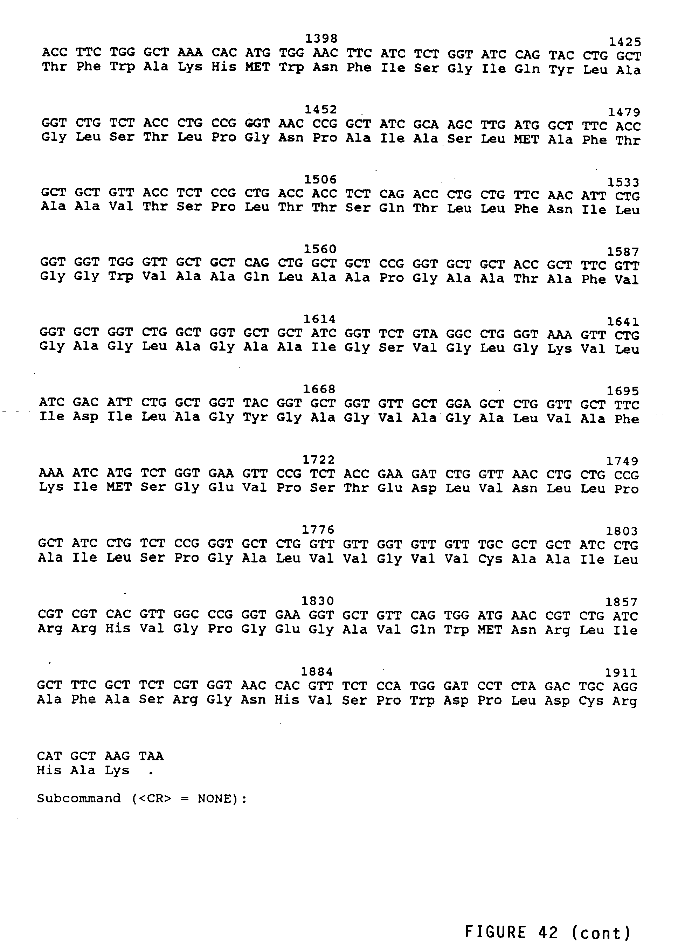

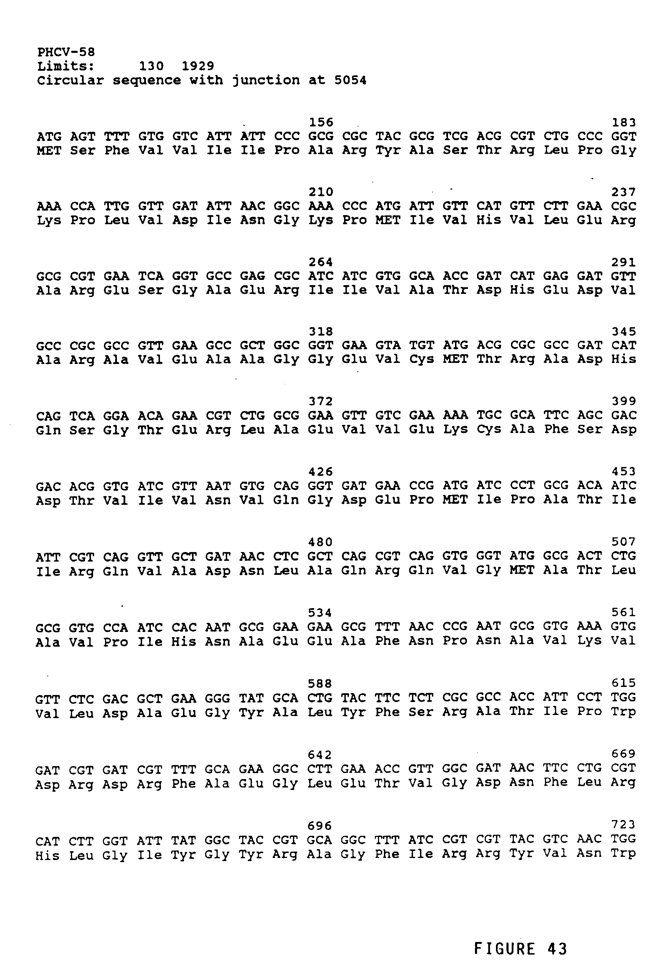

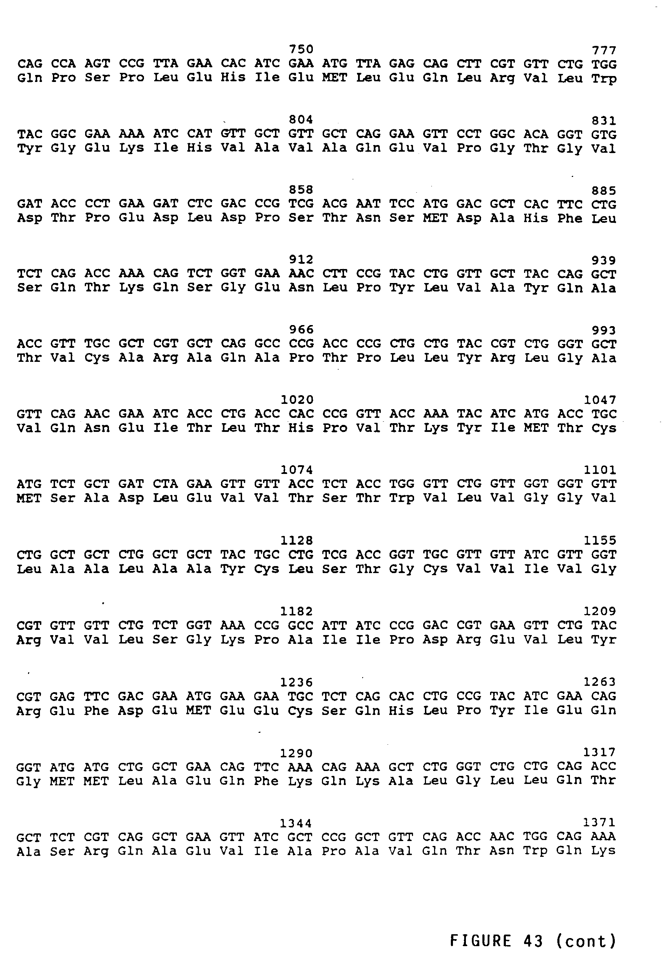

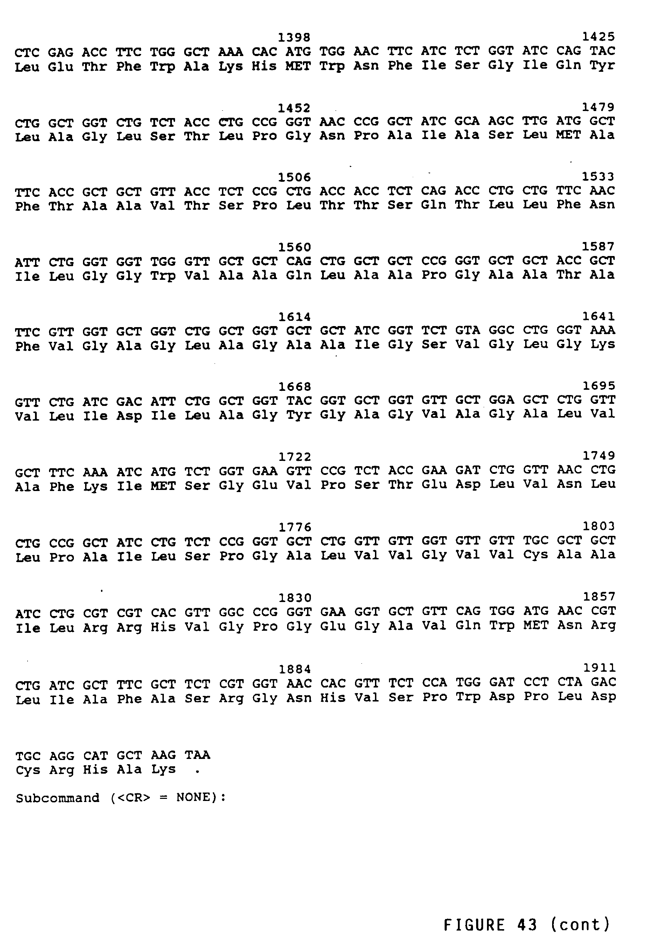

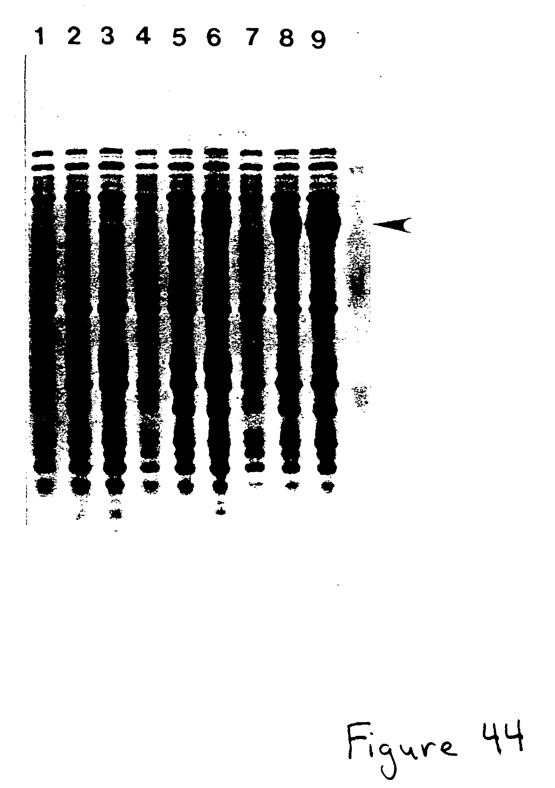

Poor expression levels of this HCV CKS-c100 recombinant antigen were overcome by constructing two additional clones containing deletions in the extreme amino terminal portion of the HCV c100 region. The first of these clones, designated pHCV-57, contains a 23 amino acid deletion (HCV amino acids 1575-1597) and was constructed by deleting a 69 base pair DdeI restriction fragment. The second of these clones, designated pHCV-58, contains a 21 amino acid deletion (HCV amino acids 1600-1620) and was constructed by deleting a 63 base pair NlaIV-HaeIII restriction fragment. Figure 41 presents a schematic representation of the recombinant antigens expressed by pHCV-24, pHCV-57, and pHCV-58. Figure 42 presents the DNA and amino acid sequence of the HCV-C100D1 recombinant antigen produced by pHCV-57. Figure 43 presents the DNA and amino acid sequence of the HCV-C100D2 recombinant antigen produced by pHCV-58. Figure 44 presents the expression of pHCV-24, pHCV-57, and pHCV-58 proteins in E.coli. Lane 1 contained the E.coli lysate containing pHCV-24 expressing the HCV CKS-c100 antigen (amino acids 1569-1931) prior to induction and lanes 2 and 3 after 2 and 4 hours post induction, respectively. Lane 4 contained the E.coli lysate containing pHCV-57 expressing the HCV-CKS-C100D1 antigen (amino acids 1569-1574 and 1598-1931) prior to induction and lanes 5 and 6 after 2 and 4 hours induction, respectively. Lane 7 contained the E.coli lysate containing pHCV-58 expressing the HCV CKS-C100D2 antigen (amino acids 1569-1599 and 1621-1931) prior to induction, and lanes 8 and 9 after 2 and 4 hours induction, respectively. These results show that both the pHCV-57 and pHCV-58 fusion proteins express at significantly higher levels than the pHCV-24 fusion protein and that both the pHCV-57 and pHCV-58 fusion proteins have an apparent mobility corresponding to a molecular size of 65,000 daltons. This compares acceptably to the predicted molecular mass of 64,450 daltons for pHCV-57 and 64,458 daltons for pHCV-58.

## EXAMPLE 9 HCV PCR DERIVED EXPRESSION VECTORS

### A. Preparation of HCV DNA Fragments

RNA was extracted from the serum of various chimpanzees or humans infected with HCV by first subjecting the samples to digestion with Proteinase K and SDS for 1 hour at 37° centigrade followed by numerous phenol:chloroform extractions. The RNA was then concentrated by several ethanol precipitations and resuspended in water. RNA samples were then reverse transcribed according to supplier's instructions using a specific primer. A second primer was then added and PCR amplification was performed according to supplier's instructions. An aliquot of this PCR reaction was then subjected to an additional round of PCR using nested primers located internal to the first set of primers. In general, these primers also contained restriction endonuclease recognition sequences to be used for subsequent cloning. An aliquot of this second round nested PCR reaction was then subjected to agarose gel electrophoresis and Southern blot analysis to confirm the specificity of the PCR reaction. The remainder of the PCR reaction was then digested with the appropriate restriction enzymes, the HCV DNA fragment of interest gel purified, and ligated to an appropriate cloning vector. This ligation was then transformed into E.coli and single colonies were isolated and plasmid DNA prepared for DNA sequences analysis. The DNA sequences was then evaluated to confirm that the specific HCV coding region of interest was intact. HCV DNA fragments obtained in this manner were then cloned into appropriate vectors for expression analysis.

### B. Preparation of HCV CKS-NS3

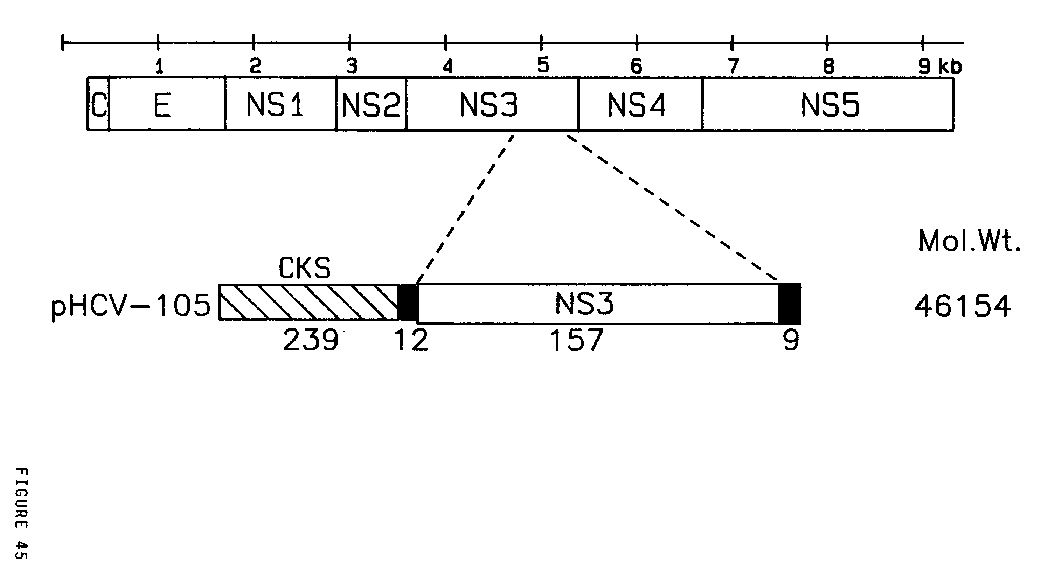

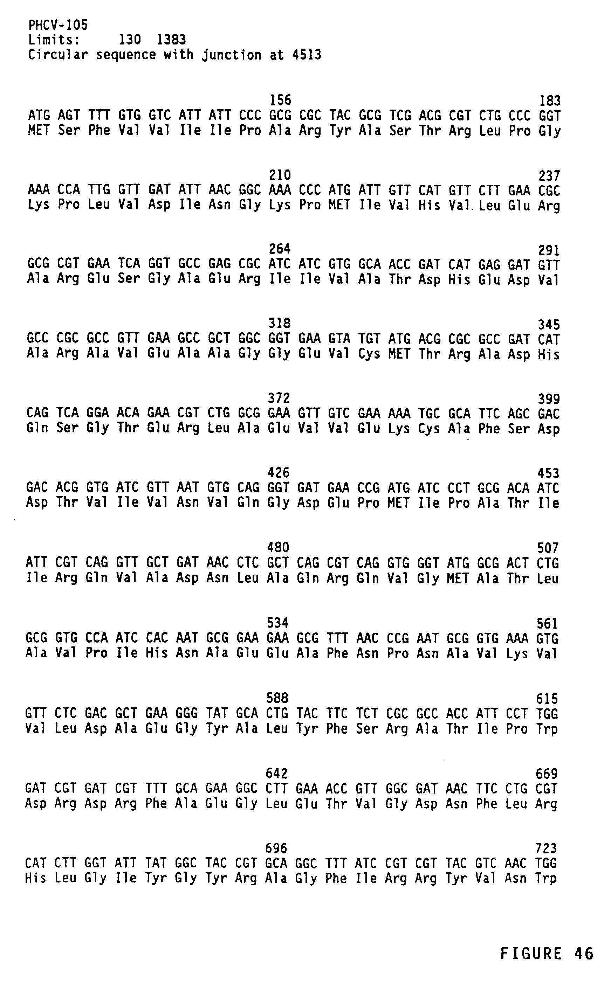

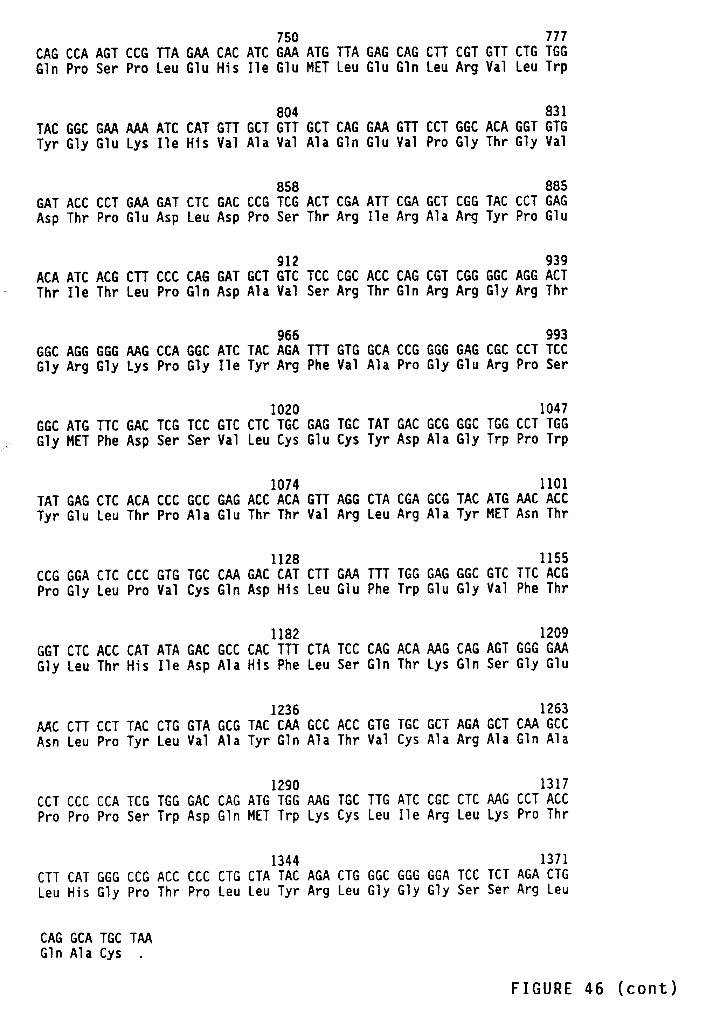

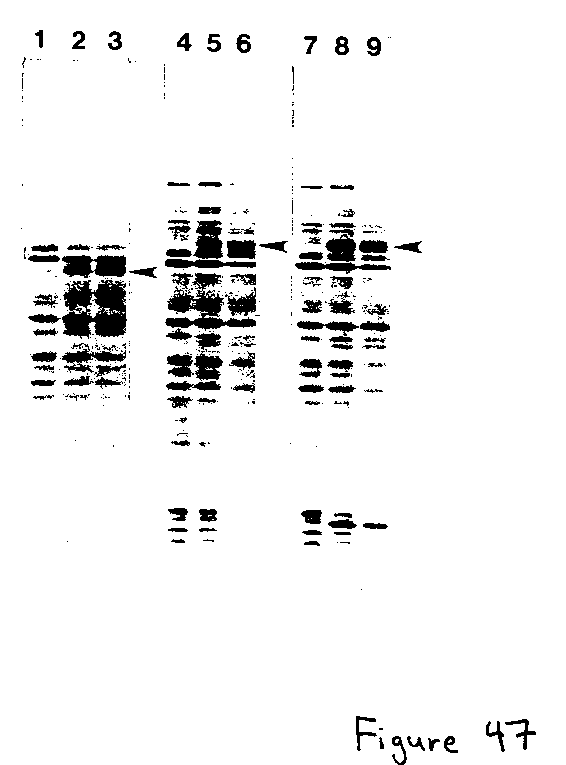

Using the methods detailed above, a 474 base pair DNA fragment from the putative NS3 region of HCV was generated by PCR. This fragment represents HCV amino acids #1473-1629 and was cloned into the CKS expression vector pJ0201 by blunt-end ligation. The resulting clone, designated pHCV-105, expresses the HCV CKS-NS3 antigen under control of the lac promoter. The HCV CKS-NS3 antigen consists of 239 amino acids of CKS, 12 amino acids contributed by linker DNA sequences, 157 amino acids from the HCV NS3 region (amino acids 1473-1629), and 9 additional amino acids contributed by linker DNA sequences. Figure 45 presents a schematic representation of the pHCV-105 antigen. Figure 46 presents the DNA and amino acid sequence of the HCV CKS-NS3 recombinant antigen produced by pHCV-105. Figure 47 presents the expression of pHCV-105 proteins in E.coli. Lane 1 contained the E.coli lysate containing pHCV-105 expressing the HCV CKS-NS3 antigen (amino acids 1472-1629) prior to induction and lanes 2 and 3 after 2 and 4 hours induction, respectively. These results show that the pHCV-105 fusion protein has an apparent mobility corresponding to a molecular mass of 43,000 daltons. This compares acceptably to the predicted molecular mass of 46,454 daltons.

### C. Preparation of HCV CKS-5'ENV

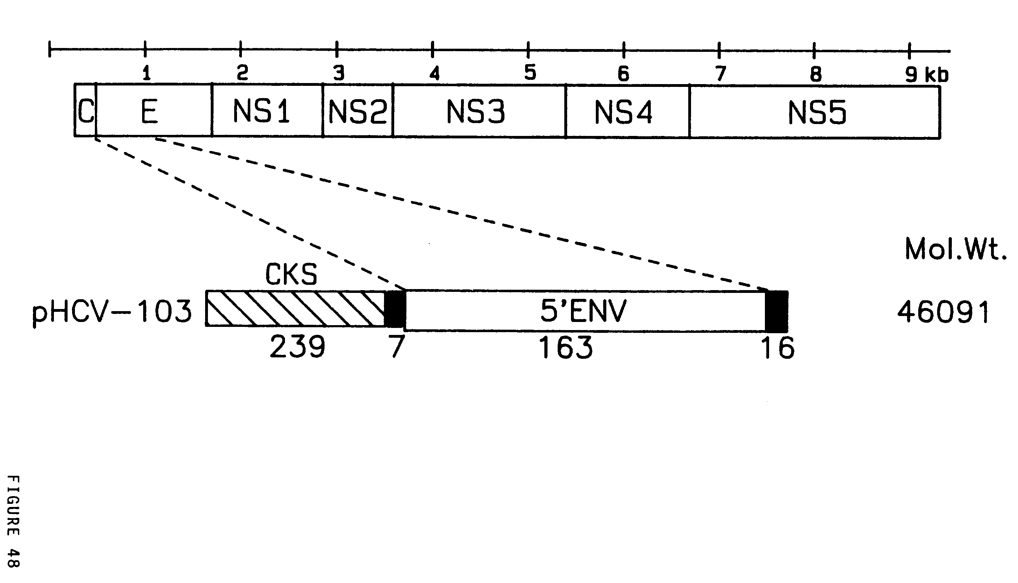

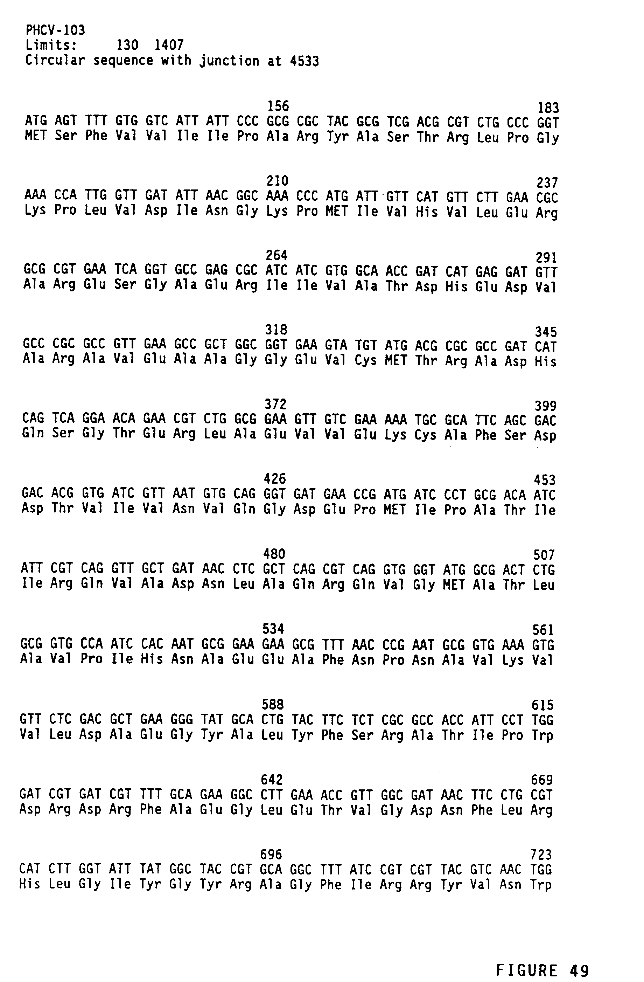

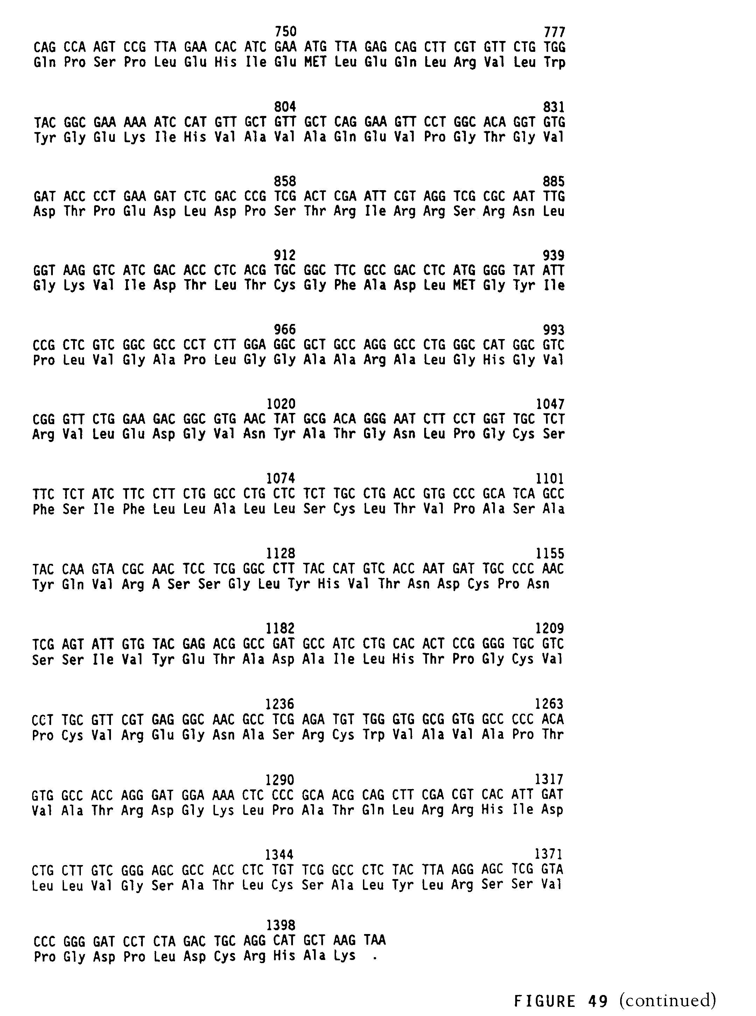

Using the methods detailed above, a 489 base pair DNA fragment from the putative envelope region of HCV was generated by PCR. This fragment represents the HCV amino acids 114-276 and was cloned into the CKS expression vector pJ0202 using EcoRI-BamHI restriction sites. The resulting clone, designated pHCV-103, expresses the HCV CKS-5'ENV antigen under control of the lac promoter. The HCV CKS-5'ENV antigen consists of 239 amino acids of CKS, 7 amino acids contributed by linker DNA sequences, 163 amino acids from the HCV envelope region (amino acids 114-276), and 16 additional amino acids contributed by linker DNA sequences. Figure 48 presents a schematic representation of the pHCV-103 antigen. Figure 49 presents the DNA and amino acid sequence of the HCV CKS-5'ENV recombinant antigen produced by pHCV-103. Figure 47 presents the expression of pHCV-103 proteins in E.coli. Lane 1 contained the E.coli lysate containing pHCV-103 expressing the HCV CKS-5'ENV antigen (amino acids 114-276) prior to induction and lanes 5 and 6 after 2 and 4 hours induction, respectively. These results show that the pHCV-103 fusion protein has an apparent mobility corresponding to a molecular mass of 47,000 daltons. This compares acceptably to the predicted molecular mass of 46,091 daltons.

D. Preparation of HCV CKS-3'ENV

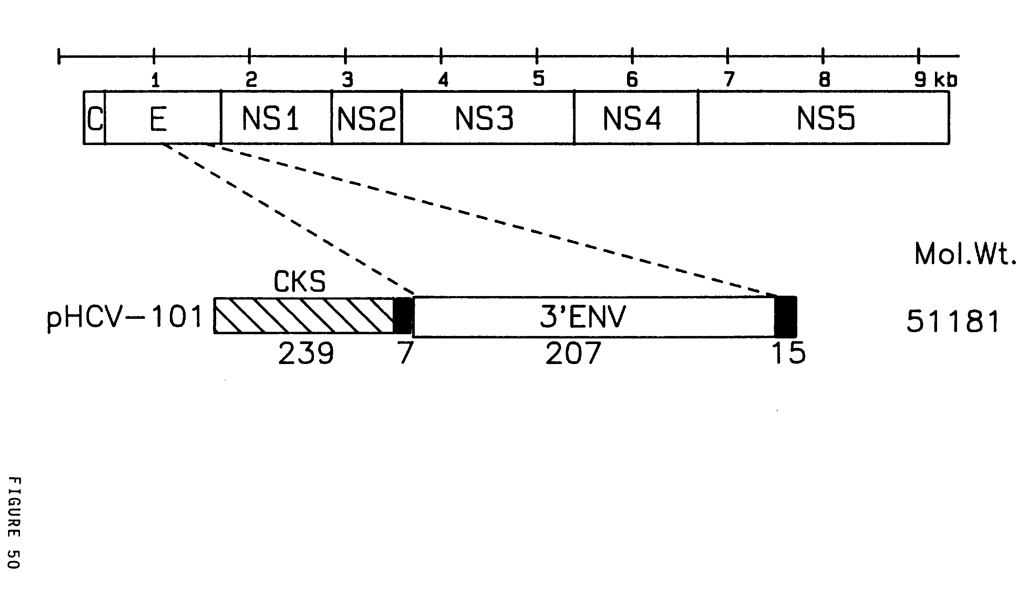

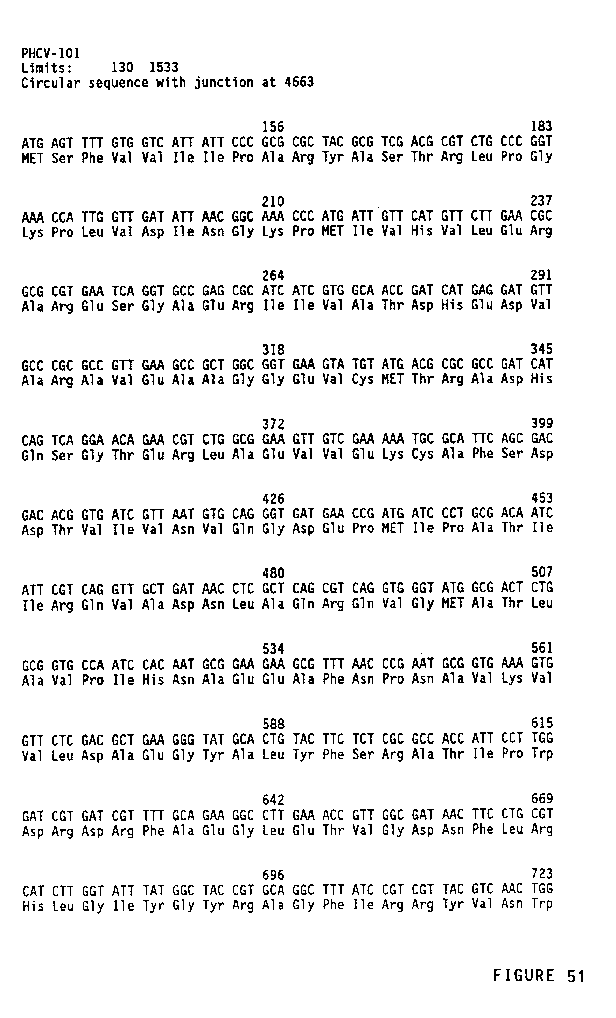

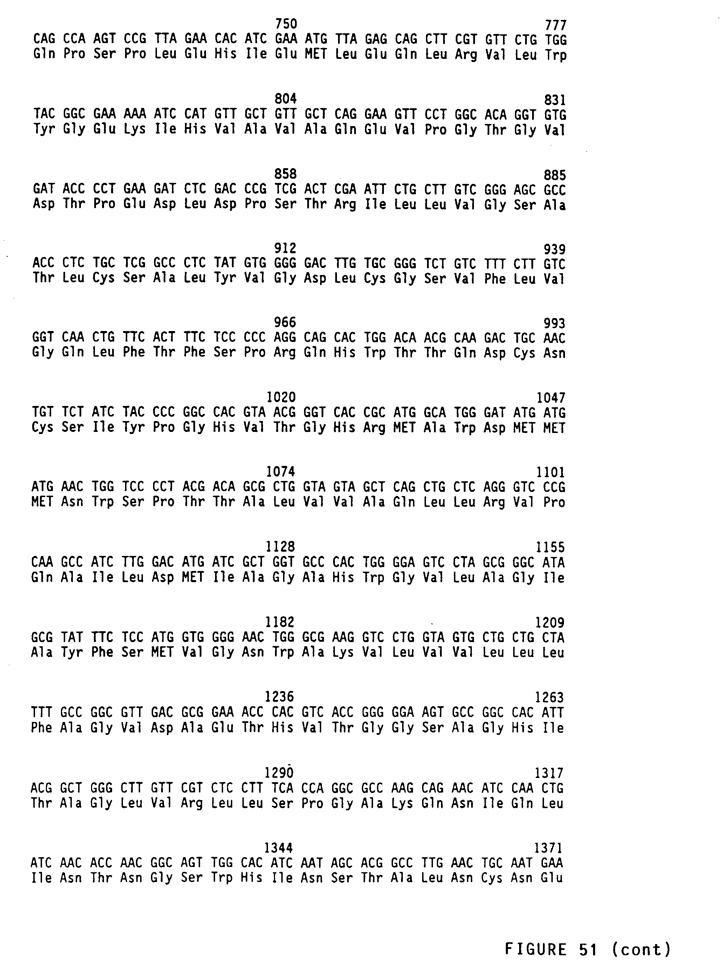

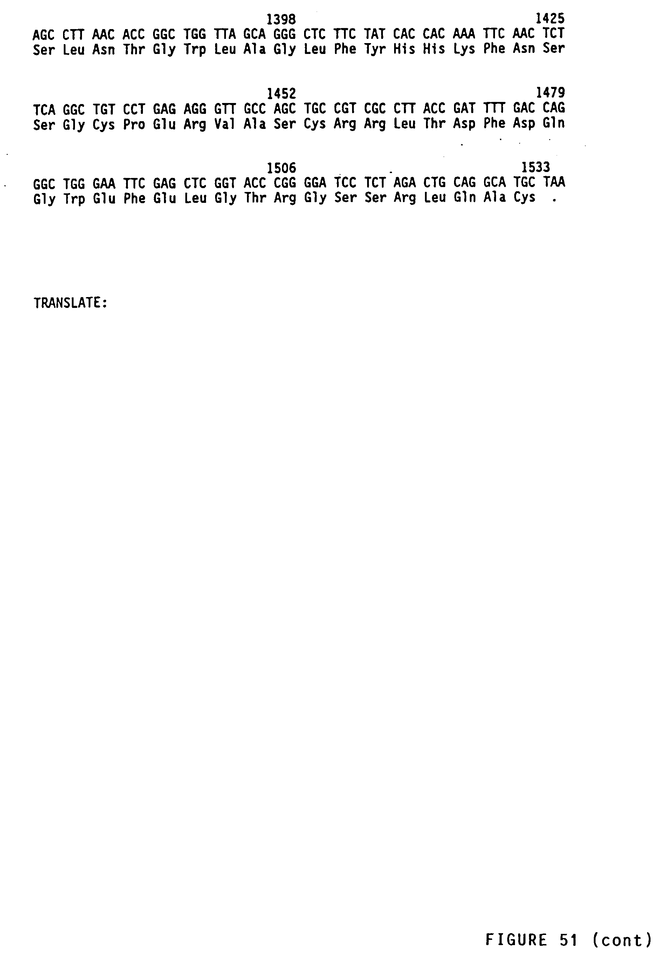

Using the methods detailed above, a 621 base pair DNA fragment form the putative envelope region of HCV was generated by PCR. This fragment represents HCV amino acids 263-469 and was cloned into the CKS expression vector pJ0202 using EcoRI restriction sites. The resulting clone, designated pHCV-101, expresses the HCV CKS-3'ENV antigen under control of the lac promoter. The HCV CKS-3'ENV antigen consists of 239 amino acids of CKS, 7 amino acids contributed by linker DNA sequences, 207 amino acids from the HCV envelope region (amino acids 263-469), and 15 additional amino acids contributed by linker DNA sequences. Figure 50 presents a schematic representation of the pHCV-101 antigen. Figure 51 presents the DNA and amino acid sequence of the HCV CKS-3'ENV recombinant antigen produced by pHCV-101. Figure 47 presents the expression of pHCV-101 proteins in E.coli Lane 7 contained the E.coli lysate containing pHCV-101 expressing the HCV CKS-3'ENV antigen (amino acids 263-469) prior to induction and lanes 8 and 9 after 2 and 4 hours induction, respectively. These resulting show that the pHCV-101 fusion protein has an apparent mobility corresponding to a molecular mass of 47,000 daltons. This compares acceptably to the predicted molecular mass of 51,181 daltons.

E. Preparation of HCV CKS-NS2

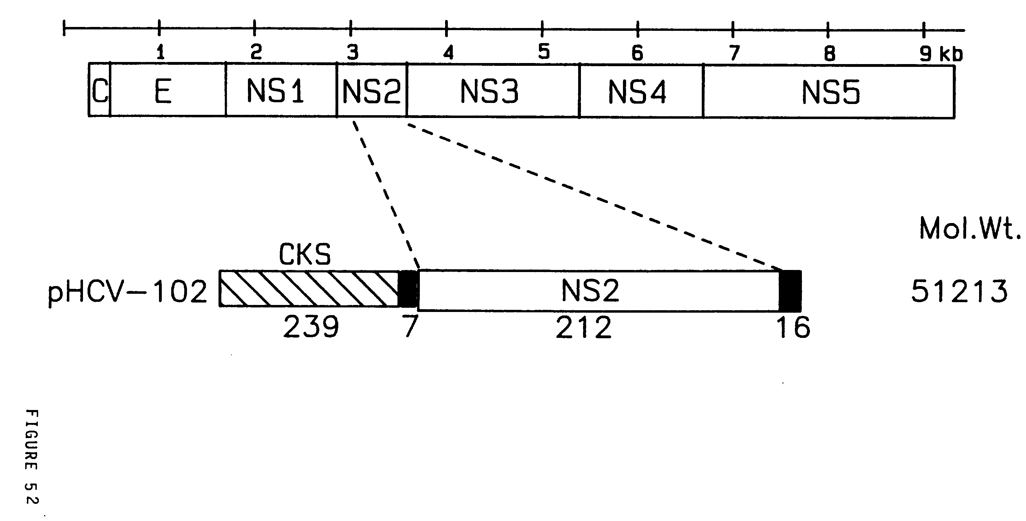

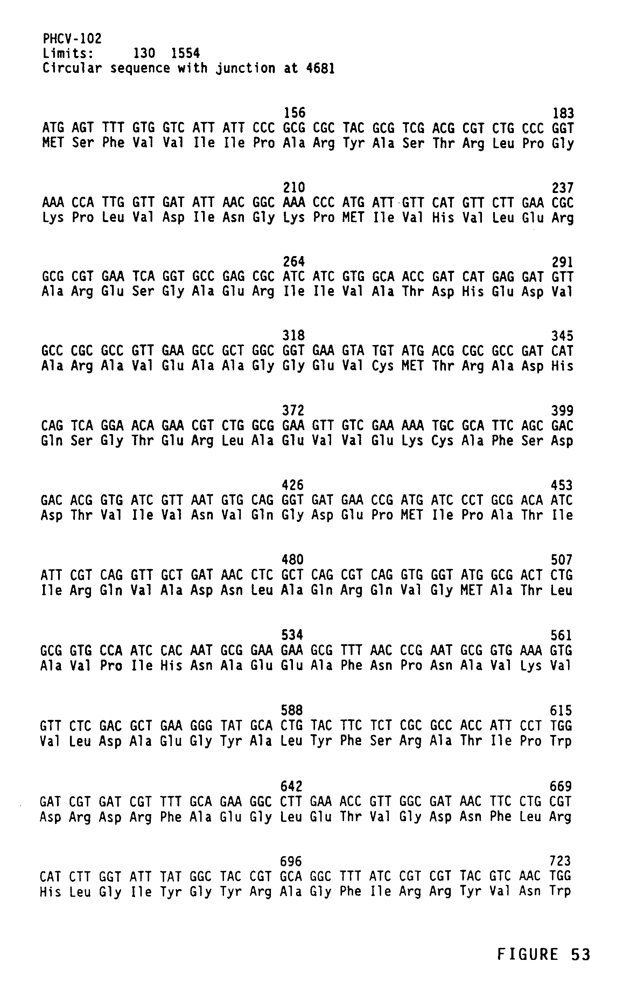

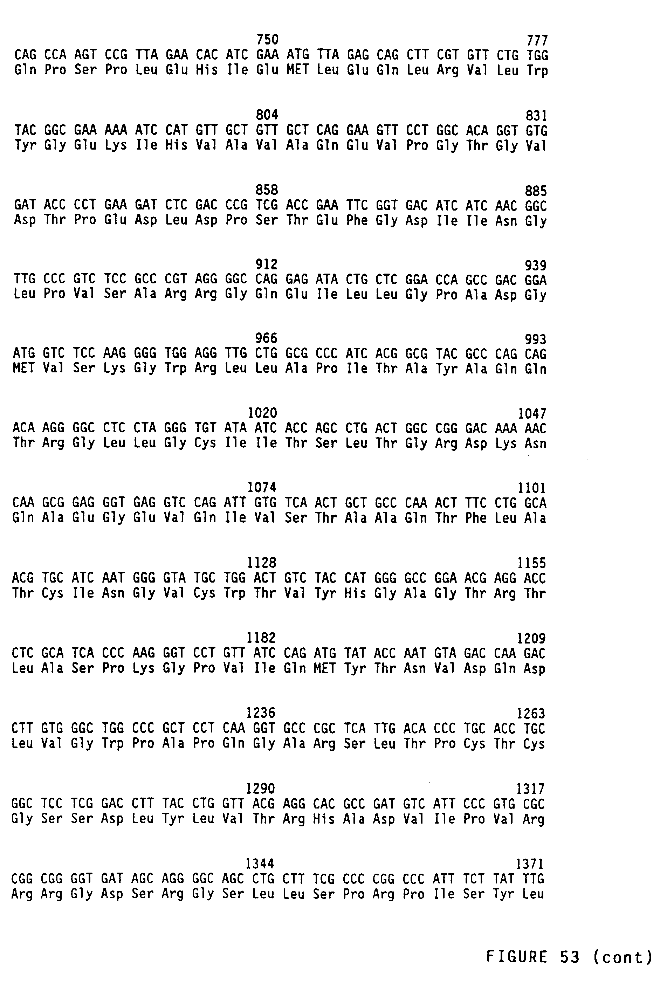

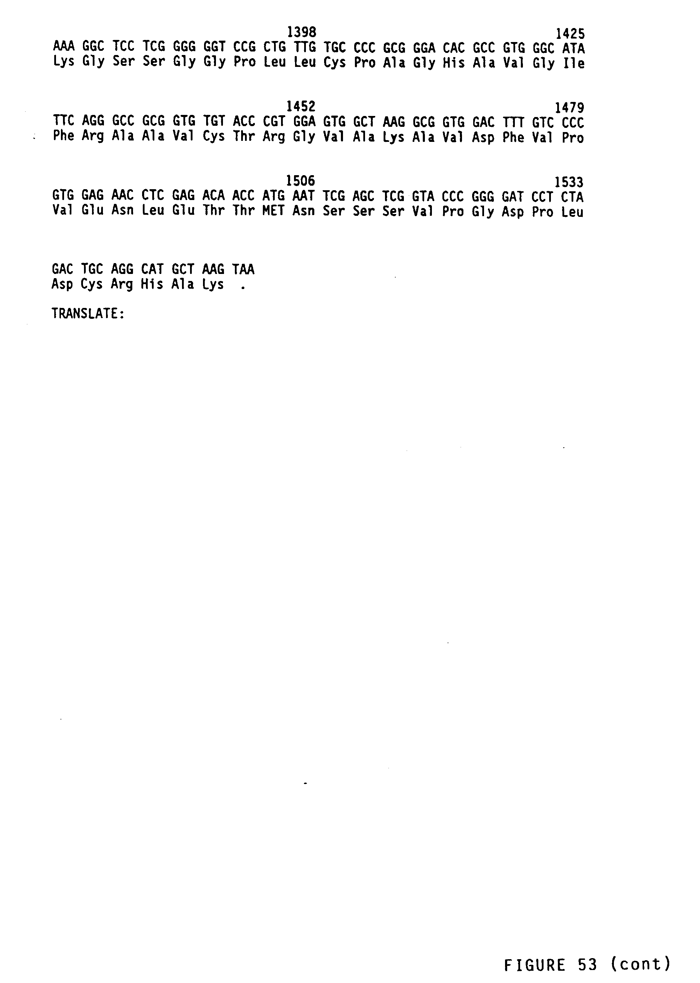

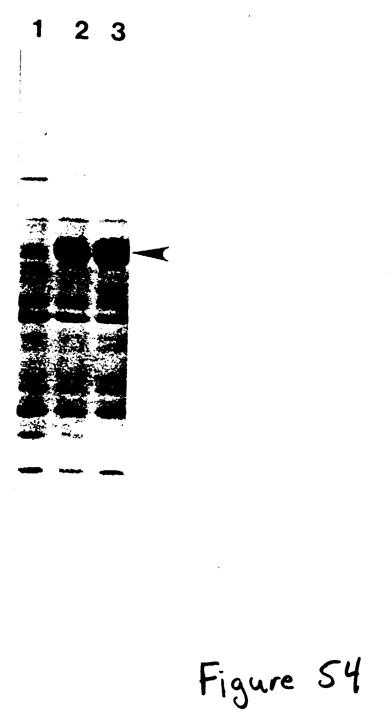

Using the methods detailed above, a 636 base pair DNA fragment from the putative NS2 region of HCV was generated by PCR. This fragment represents the HCV amino acids 994-1205 and was cloned into the CKS expression vector pJ0201 using EcoRI restriction sites. The resulting clone, designated pHCV-102, expresses the HCV CKS-NS2 antigen under control of the lac promoter. The HCV CKS-NS2 antigen consists of 239 amino acids of CKS, 7 amino acids contributed by linker DNA sequences, 212 amino acids from the HCV NS2 region (amino acids 994-1205), and 16 additional amino acids contributed by linker DNA sequences. Figure 52 presents a schematic representation of the pHCV-102 antigen. Figure 53 presents the DNA and amino acid sequence of the HCV CKS-NS2 recombinant antigen produced by pHCV-102. Figure 54 presents the expression of pHCV-102 proteins in E.coli. Lane 1 contained the E.coli lysate containing pHCV-102 expressing the HCV CKS-NS2 antigen (amino acids 994-1205) prior to induction and lanes 2 and 3 after 2 and 4 hours induction, respectively. These results show that the pHCV-102 fusion protein has an apparent mobility corresponding to a molecular mass of 53,000 daltons. This compares acceptably to the predicted molecular mass of 51,213 daltons.

F. Preparation of HCV CKS-NS1

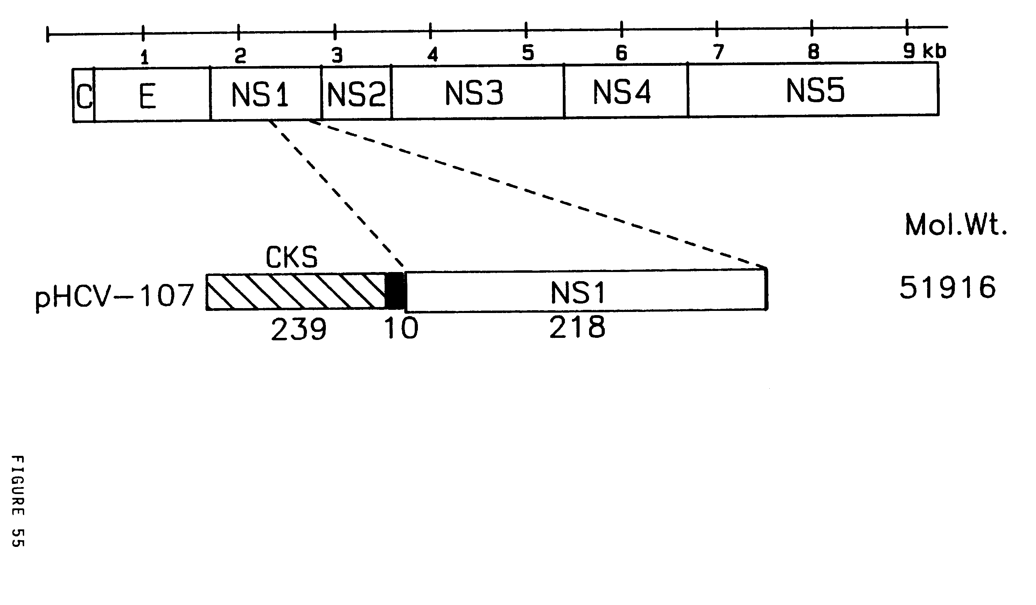

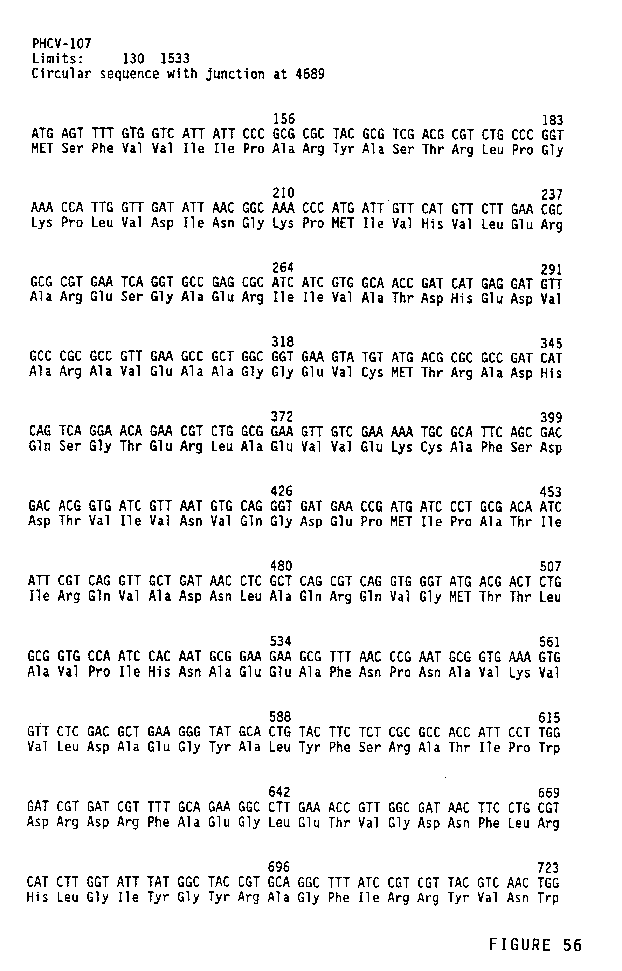

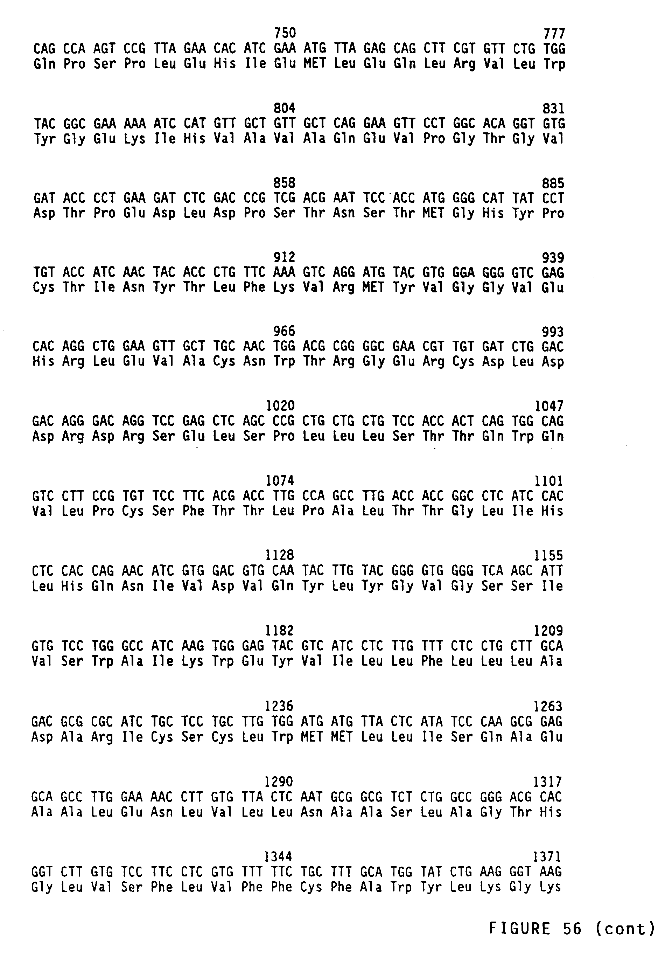

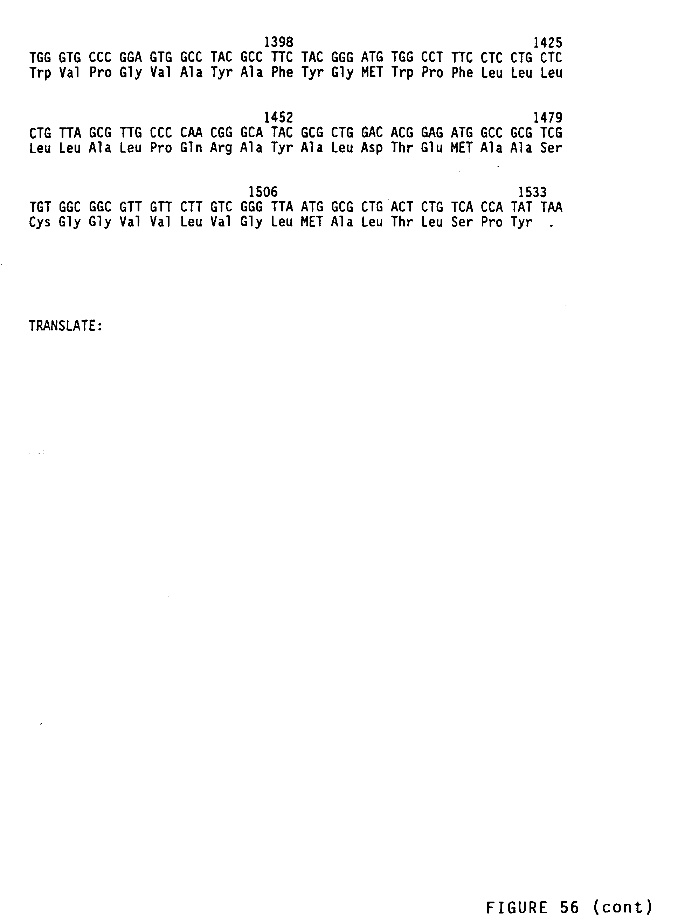

Using the methods detailed above, a 654 base pair DNA fragment from the putative NS1 region of HCV was generated by PCR. This fragment represents HCV amino acids 617-834 and was cloned into the CKS expression vector pJ0200 using EcoRI-BamHI restriction sites. The resulting clone, designated pHCV-107, expresses the HCV CKS-NS1 antigen under control of the lac promoter. The HCV CKS-NS1 antigen consists of 239 amino acids of CKS, 10 amino acids contributed by linker DNA sequences, and 218 amino acids from the HCV NS1 region (amino acids 617-834). Figure 55 presents a schematic representation of the pHCV-107 antigen. Figure 56 presents the DNA and amino acid sequence of the HCV CKS-NS1 recombinant antigen produced by pHCV-107.

G. Preparation of HCV CKS-ENV

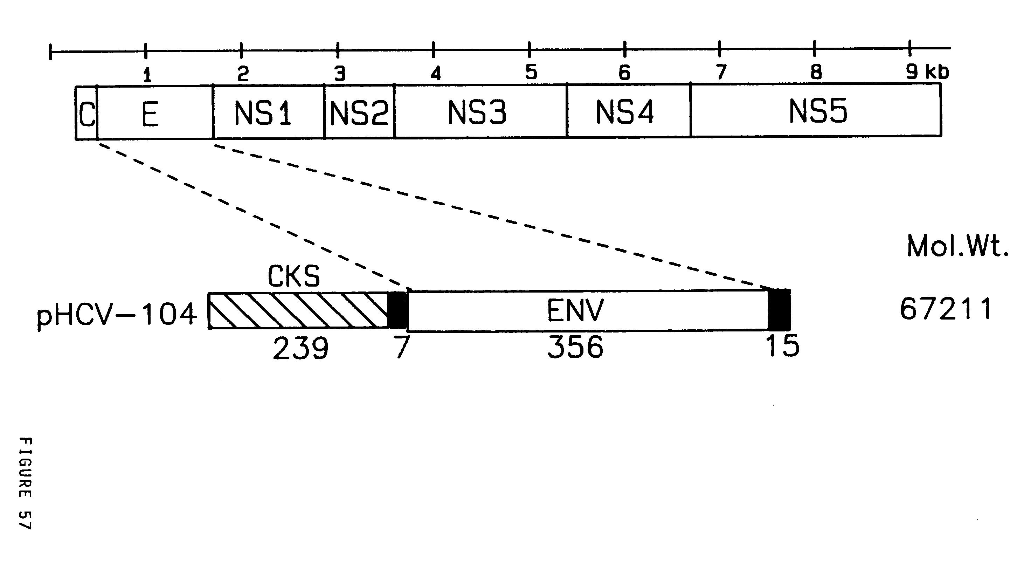

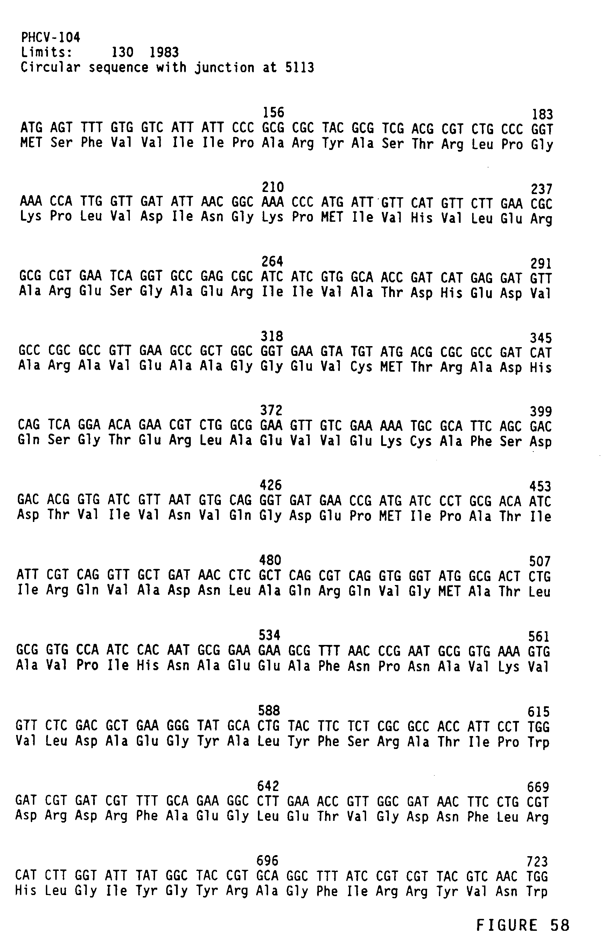

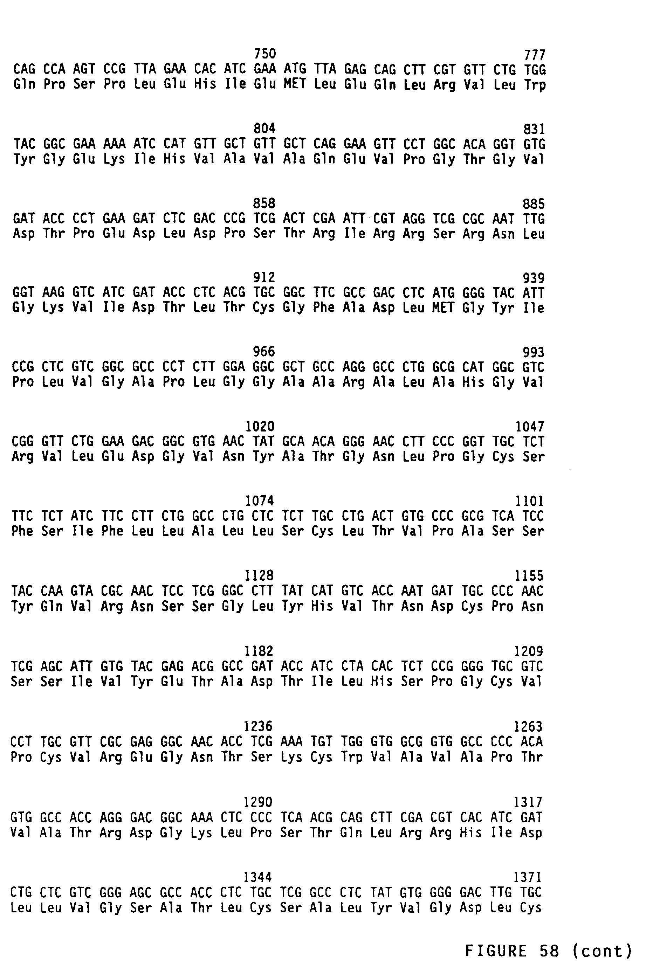

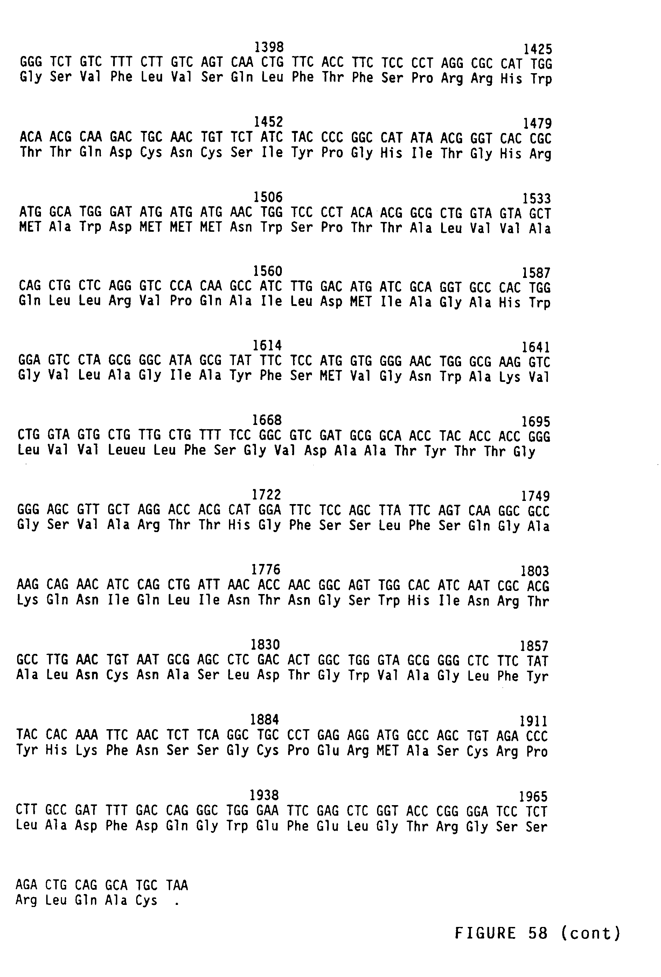

Using the methods detailed above, a 1068 base pair DNA fragment from the putative envelope region of HCV was generated by PCR. This fragment represents HCV amino acids #114-469 and was cloned into the CKS expression vector pJ0202 using EcoRI restriction sites. The resulting clone, designated pHCV-104, expresses the HCV CKS-ENV antigen under control of the lac promoter. The HCV CKS-ENV antigen consists of 239 amino acids of CKS, 7 amino acids contributed by linker DNA sequences, 356 amino acids from the HCV envelope region (amino acids 114-469), and 15 additional amino acids contributed by linker DNA sequences. Figure 57 presents a schematic representation of the pHCV-104 antigen. Figure 58 presents the DNA and amino acid sequence of the HCV CKS-ENV recombinant antigen produced by pHCV-104.

The recombinant antigens, either alone or in combination, can be used in the assay formats provided herein and exemplified in the Examples. It also is contemplated that these recombinant antigens can be used to develop specific inhibitors of viral replication and used for therapeutic purposes, such as for vaccines. Other applications and modifications of the use of these antigens and the specific embodiments of this inventions as set forth herein, will be apparent to those skilled in the art. Accordingly, the invention is intended to be limited only in accordance with the appended claims.

## Claims

1. An assay for identifying the presence of an antibody immunologically reactive with an HCV antigen in a fluid sample comprising contacting the sample with

   (a) at least two recombinant proteins wherein one recombinant protein is a fusion protein of the polypeptide encoded by the E. coli CMP-KDO synthetase gene (CKS) and a polypeptide representing the HCV genome from both amino acids 1192-1457 and 1676-1931 as defined in Figures 1, 11 and 12 and the second recombinant protein is a fusion protein of the polypeptide encoded by the E. coli CMP-KDO synthetase gene (CKS) and a polypeptide representing the HCV genome from amino acids 1-150 as defined in Figures 1,5 and 6, or
   (b) at least two polypeptides wherein one polypeptide represents two noncontiguous coding regions of the HCV genome from amino acids 1192-1457 and 1676-1931 and the second polypeptide represents the HCV genome from amino acids 1-150, the first and second polypeptides being obtainable by recombinant or synthetic methodologies other than through expression as fusions with a polypeptide encoded by the CKS gene,

   under conditions suitable for complexing the antibody with the polypeptide, and detecting the antibody-polypeptide complex.

2. An assay for identifying the presence of an antibody immunologically reactive with an HCV antigen in a fluid sample comprising contacting the sample with

   (a) at least three recombinant proteins wherein one recombinant protein is a fusion protein of the polypeptide encoded by the E. coli CMP-KDO synthetase gene (CKS) and a polypeptide representing the HCV genome from amino acids 1192-1457 obtainable by the plasmid of Figure 9 and obtainable by the strain deposited under ATCC accession no. 68381, the second recombinant protein is a fusion protein of the polypeptide encoded by the E. coli CMP-KDO synthetase gene (CKS) and a polypeptide representing the HCV genome from amino acids 1676-1931 obtainable by the plasmid of Figure 8 and obtainable by the strain deposited under ATCC accession no. 68380 and the third recombinant protein is a fusion protein of the polypeptide encoded by the E. coli CMP-KDO synthetase gene (CKS) and a polypeptide representing the HCV genome from amino acids 1-150 as defined in Figures 1, 5 and 6, or
   (b) at least three polypeptides wherein one polypeptide represents the HCV genome from amino acids 1192-1457, the second polypeptide represents the HCV genome from amino acids 1676-1931 and the third polypeptide represents the HCV genome from amino acids 1-150, the first, second and third polypeptides being obtainable by recombinant or synthetic methodologies other than through expression as fusions with a polypeptide encoded by the CKS gene,

   under conditions suitable for complexing the antibody with the polypeptide, and detecting the antibody-polypeptide complex.

3. A confirmatory assay for identifying the presence of an antibody in a fluid sample immunologically reactive with an HCV antigen wherein the sample is used to prepare first and second immunologically equivalent aliquots comprising the steps of

   (a) contacting the first aliquot with

      (i) at least two recombinant proteins wherein one recombinant protein is a fusion protein of the polypeptide encoded by the E. coli CMP-KDO synthetase gene (CKS) and a polypeptide representing the HCV genome from both amino acids 1192-1457 and 1676-1931 as defined in Figures 1, 11 and 12 and the second recombinant protein is a fusion protein of the polypeptide encoded by the E. coli CMP-KDO synthetase gene (CKS) and a polypeptide representing the HCV genome from amino acids 1-150 as defined in Figures 1,5 and 6, or
      (ii) at least two polypeptides wherein one polypeptide represents two noncontiguous coding regions of the HCV genome from amino acids 1192-1457 and 1676-1931 and the second polypeptide represents the HCV genome from amino acids 1-150, the first and second polypeptides being obtainable by recombinant or synthetic methodologies other than through expression as fusions with a polypeptide encoded by the CKS gene,

   under conditions suitable for complexing the antibody with the polypeptide and wherein the first antibody-antigen complex is detected, and

(b) contacting the second aliquot with a polypeptide selected from the group consisting of

synthetic polypeptides representing amino acids 1866-1930, 1684-1750, 1-75 or 1689-1805 of the HCV sequence,
a chimeric protein of superoxide dismutase and the HCV 33 region of Figure 1,
a polypeptide representing the HCV genome from amino acids 1676-1931, said polypeptide being obtainable by recombinant or synthetic methodologies other than through expression as fusions with a polypeptide encoded by the CKS gene,
a polypeptide representing the HCV genome from amino acids 1192-1457, said polypeptide being obtainable by recombinant or synthetic methodologies other than through expression as fusions with a polypeptide encoded by the CKS gene,
a polypeptide representing two noncontiguous coding regions of the HCV genome from amino acids 1192-1457 and 1676-1931, said polypeptide being obtainable by recombinant or synthetic methodologies other than through expression as fusions with a polypeptide encoded by the CKS gene,
a polypeptide representing the HCV genome from amino acids 1-150, said polypeptide being obtainable by recombinant or synthetic methodologies other than through expression as fusions with a polypeptide encoded by the CKS gene,
a polypeptide representing the HCV genome from amino acids 1569-1931 containing a 23 amino acid deletion of HCV amino acids 1575-1597, said polypeptide being obtainable by recombinant or synthetic methodologies other than through expression as fusions with a polypeptide encoded by the CKS gene,
a polypeptide representing the HCV genome from amino acids 1569-1931 containing a 21 amino acid deletion of HCV amino acids 1600-1620, said polypeptide being obtainable by recombinant or synthetic methodologies other than through expression as fusions with a polypeptide encoded by the CKS gene,
under conditions suitable to form a second antibody-antigen complex; and detecting the second antibody-antigen complex; wherein the polypeptides selected in the first aliquot are not the same as the polypeptides selected in the second aliquot.

**4.** A confirmatory assay for identifying the presence of an antibody in a fluid sample immunologically reactive with an HCV antigen wherein the sample is used to prepare first and second immunologically equivalent aliquots comprising the steps of

(a) contacting the first aliquot with

(i) at least three recombinant proteins wherein one recombinant protein is a fusion protein of the polypeptide encoded by the E. coli CMP-KDO synthetase gene (CKS) and a polypeptide representing the HCV genome from amino acids 1192-1457 capable of being encoded by the plasmid of Figure 9 and obtainable by the strain deposited under ATCC accession no. 68381, the second recombinant protein is a fusion protein of the polypeptide encoded by the E. coli CMP-KDO synthetase gene (CKS) and a polypeptide representing the HCV genome from amino acids 1676-1931 obtainable by the plasmid of Figure 8 and obtainable by the strain deposited under ATCC accession no. 68380 and the third recombinant protein is a fusion protein of the polypeptide encoded by the E. coli CMP-KDO synthetase gene (CKS) and a polypeptide representing the HCV genome from amino acids 1-150 as defined in Figures 1,5 and 6, or
(ii) at least three polypeptides wherein one polypeptide represents the HCV genome from amino acids 1192-1457, the second polypeptide represents the HCV genome from amino acids 1676-1931 and the third polypeptide represents the HCV genome from amino acids 1-150, the first, second and third polypeptides being obtainable by recombinant or synthetic methodologies other than through expression as fusions with a polypeptide encoded by the CKS gene,

under conditions suitable for complexing the antibody with the polypeptide and wherein the first antibody-antigen complex is detected, and

(b) contacting the second aliquot with a polypeptide selected from the group consisting of

synthetic polypeptides representing amino acids 1866-1930, 1684-1750, 1-75 or 1689-1805 of the HCV sequence,

a chimeric protein of superoxide dismutase and the HCV 33 region of Figure 1,
a polypeptide representing the HCV genome from amino acids 1676-1931, said polypeptide being obtainable by recombinant or synthetic methodologies other than through expression as fusions with a polypeptide encoded by the CKS gene,
a polypeptide representing the HCV genome from amino acids 1192-1457, said polypeptide being obtainable by recombinant or synthetic methodologies other than through expression as fusions with a polypeptide encoded by the CKS gene,
a polypeptide representing two noncontiguous coding regions of the HCV genome from amino acids 1192-1457 and 1676-1931, said polypeptide being obtainable by recombinant or synthetic methodologies other than through expression as fusions with a polypeptide encoded by the CKS gene,
a polypeptide representing the HCV genome from amino acids 1-150, said polypeptide being obtainable by recombinant or synthetic methodologies other than through expression as fusions with a polypeptide encoded by the CKS gene,
a polypeptide representing the HCV genome from amino acids 1569-1931 containing a 23 amino acid deletion of HCV amino acids 1575-1597, said polypeptide being obtainable by recombinant or synthetic methodologies other than through expression as fusions with a polypeptide encoded by the CKS gene,
a polypeptide representing the HCV genome from amino acids 1569-1931 containing a 21 amino acid deletion of HCV amino acids 1600-1620, said polypeptide being obtainable by recombinant or synthetic methodologies other than through expression as fusions with a polypeptide encoded by the CKS gene,
under conditions suitable to form a second antibody-antigen complex; and detecting the second antibody-antigen complex; wherein the polypeptides selected in the first aliquot are not the same as the polypeptides selected in the second aliquot.

**5.** A confirmatory assay according to Claim 3 or 4 wherein in step (b) the second aliquot may be furthermore contacted with one or more polypeptides selected from the group consisting of the polypeptides representing the HCV genome from amino acids 1932-2191, 2188-2481, 2866-3011, 2728-2867, 2480-2729, 263-469, 994-1205, 114-276, 114-469, 1472-1629, 617-834, said polypeptide being obtainable by recombinant or synthetic methodologies other than through expression as fusions with a polypeptide encoded by the CKS gene.

**6.** An immunoassay kit comprising:

(a) at least two recombinant proteins wherein one recombinant protein is a fusion protein of the polypeptide encoded by the E. coli CMP-KDO synthetase gene (CKS) and a polypeptide representing the HCV genome from both amino acids 1192-1457 and 1676-1931 as defined in Figures 1, 11 and 12 and the second recombinant protein is a fusion protein of the polypeptide encoded by the E. coli CMP-KDO synthetase gene (CKS) and a polypeptide representing the HCV genome from amino acids 1-150 as defined in Figures 1,5 and 6, or
(b) at least two polypeptides wherein one polypeptide represents two noncontiguous coding regions of the HCV genome from amino acids 1192-1457 and 1676-1931 and the second polypeptide represents the HCV genome from amino acids 1-150, the first and second polypeptides being obtainable by recombinant or synthetic methodologies other than through expression as fusions with a polypeptide encoded by the CKS gene;

one or more sample preparation reagents; and one or more detection and signal producing reagents.

**7.** An immunoassay kit comprising:

(a) at least three recombinant proteins wherein one recombinant protein is a fusion protein of the polypeptide encoded by the E. coli CMP-KDO synthetase gene (CKS) and a polypeptide representing the HCV genome from amino acids 1192-1457 obtainable by the plasmid of Figure 9 and obtainable by the strain deposited under ATCC accession no. 68381, the second recombinant protein is a fusion protein of the polypeptide encoded by the E. coli CMP-KDO synthetase gene (CKS) and a polypeptide representing the HCV genome from amino acids 1676-1931 obtainable by the plasmid of Figure 8 and obtainable by the strain deposited under ATCC accession no. 68380 and the third recombinant protein is a fusion protein of the polypeptide encoded by the E. coli CMP-KDO synthetase gene (CKS) and a polypeptide representing the HCV genome from amino acids 1-150 as defined in Figures 1,5 and 6, or
(b) at least three polypeptides wherein one polypeptide represents the HCV genome from amino acids 1192-1457, the second polypeptide represents the HCV genome from amino acids 1676-1931 and the third polypeptide represents the HCV genome from amino acids 1-150, the first, second and third polypeptides being obtainable by recombinant or synthetic methodologies other than through expression as fusions with a polypeptide encoded by the CKS gene;

one or more sample preparation reagents; and one or more detection and signal producing reagents.

**Patentansprüche**

1. Assay zur Identifizierung der Anwesenheit eines Antikörpers, der gegenüber einem HCV-Antigen in einer flüssigen Probe immunologisch reaktiv ist, wobei der Assay das In-Kontakt-Bringen der Probe mit folgendem umfaßt:

   (a) mindestens zwei rekombinanten Proteinen, wobei ein rekombinantes Protein ein Fusionsprotein des Polypeptids ist, für das das E.coli CMP-KDO Synthetasegen (CKS) codiert und eines Polypeptids, das das HCV-Genom von sowohl den Aminosäuren 1192-1457 als auch 1676-1931, wie in den Figuren 1, 11 und 12 definiert, darstellt und wobei das zweite rekombinante Protein ein Fusionsprotein aus dem Polypeptid ist, für das das E.coli CMP-KDO Synthetasegen (CKS) codiert, und einem Polypeptid, das das HCV Genom von den Aminosäuren 1-150, wie in den Figuren 1, 5 und 6 definiert, darstellt, oder
   (b) mindestens zwei Polypeptiden, wobei ein Polypeptid zwei nicht benachbarte codierende Bereiche des HCV-Genoms von den Aminosäuren 1192-1457 und 1676-1931 darstellt und das zweite Polypeptid das HCV-Genom von den Aminosäuren 1-150 darstellt, wobei das erste und zweite Polypeptid durch rekombinante oder synthetische Methodologien außer der Expression als Fusionen mit einem Polypeptid, für das das CKS-Gen codiert, erhältlich sind,

   unter Bedingungen, die zur Komplexbildung des Antikörpers mit dem Polypeptid, und zum Nachweis des Antikörper-Polypeptidkomplexes geeignet sind.

2. Assay zur Identifizierung der Anwesenheit eines Antikörpers, der gegenüber einem HCV-Antigen in einer flüssigen Probe immunologisch reaktiv ist, wobei der Assay das In-Kontakt-Bringen der Probe mit folgendem umfaßt:

   (a) mindestens drei rekombinanten Proteinen, wobei ein rekombinantes Protein ein Fusionsprotein des Polypeptids ist, für das das CMP-KDO Synthetase Gen (CKS) codiert, und eines Polypeptids, das das HCV-Genom von den Aminosäuren 1192-1457 darstellt, und das aus dem Plasmid nach Figur 9 und aus dem Stamm erhältlich ist, der unter der ATCC Zugangsnummer 68381 hinterlegt wurde, und wobei das zweite rekombinante Protein ein Fusionsprotein des Polypeptids ist, für das das E.coli CMP-KDO Synthetasegen (CKS) codiert, und eines Polypeptids, das das HCV-Genom von den Aminosäuren 1676-1931 darstellt, und das aus dem Plasmid nach Figur 8 und aus dem Stamm erhältlich ist, der unter der ATCC Zugangsnummer 68380 hinterlegt wurde, und wobei das dritte rekombinante Protein ein Fusionsprotein des Polypeptids ist, für das das E.coli CMP-KDO Synthetasegen (CKS) codiert, und eines Polypeptids, das das HCV-Genom von den Aminosäuren 1-150 darstellt, wie in den Figuren 1, 5 und 6 definiert, oder
   (b) mindestens drei Polypeptiden, wobei ein Polypeptid das HCV-Genom von den Aminosäuren 1192-1457 darstellt, das zweite Polypeptid das HCV-Genom von den Aminosäuren 1676-1931 darstellt, und das dritte Polypeptid das HCV-Genom von den Aminosäuren 1-150 darstellt, und wobei das erste, zweite und dritte Polypeptid durch rekombinante oder synthetische Methodologien außer der Expression als Fusionen mit einem Polypeptid erhältlich sind, für das das CKS-Gen codiert,

   unter Bedingungen, die zur Komplexbildung des Antikörpers mit dem Polypeptid und zum Nachweis des Antikörper-Polypeptidkomplexes geeignet sind.

3. Ein Bestätigungsassay zum Identifizieren der Anwesenheit eines Antikörpers in einer flüssigen Probe, wobei der Antikörper gegenüber einem HCV-Antigen immunologisch reaktiv ist, wobei die Probe verwendet wird, um einen ersten und zweiten immunologisch gleichwertigen aliquoten Anteil herzustellen, wobei der Bestätigungsassay folgende Schritte umfaßt:

   (a) In-Kontakt-Bringen des ersten aliquoten Anteils mit

      (i) mindestens zwei rekombinanten Proteinen, wobei ein rekombinantes Protein ein Fusionsprotein des Polypeptids ist, für das das E.coli CMP-KDO Synthetasegen (CKS) codiert und eines Polypeptids, das das HCV-Genom von den beiden Aminosäuren 1192-1457 und 1676-1931, wie in den Figuren 1, 11 und 12 definiert, darstellt, und wobei das zweite rekombinante Protein ein Fusionsprotein des Polypeptids ist für das das E.coli CMP-KDO Synthetasegen (CKS) codiert, und eines Polypeptids, das das HCV-Genom von den Aminosäuren 1-150 darstellt, wie in den Figuren 1, 5 und 6 definiert, oder

(ii) mindestens zwei Polypeptiden, wobei ein Polypeptid zwei nicht benachbarte codierende Bereiche des HCV-Genoms von den Aminosäuren 1192-1457 und 1676-1931 darstellt, und wobei das zweite Polypeptid das HCV-Genom von den Aminosäuren von 1-150 darstellt, wobei das erste und zweite Polypeptid durch rekombinante oder synthetische Methodologien außer der Expression als Fusionen mit einem Polypeptid erhältlich sind, für das das CKS-Gen codiert,

unter Bedingungen, die zur Komplexbildung des Antikörpers mit dem Polypeptid geeignet sind, und wobei der erste Antikörper-Antigenkomplex nachgewiesen wird, und

(b) In-Kontakt-Bringen des zweiten aliquoten Anteils mit einem Polypeptid, das aus der Gruppe gewählt ist, die aus folgendem besteht:

synthetischen Polypeptiden, welche die Aminosäuren 1866-1930, 1684-1750, 1-75 oder 1689-1805 der HCV-Sequenz darstellen,

einem Schimärenprotein aus Peroxiddismutase und dem HCV 33 Bereich von Figur 1,

einem Polypeptid, das das HCV-Genom von den Aminosäuren 1676-1931 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von den Aminosäuren 1192-1457 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das zwei nicht benachbarte codierende Bereiche des HCV-Genoms der Aminosäuren 1192-1457 und 1676-1931 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von Aminosäure 1-150 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von Aminosäuren 1569-1931 darstellt, das eine 23-Aminosäuren-Deletion der HCV Aminosäuren 1575-1597 enthält, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von den Aminosäuren 1569-1931 darstellt, das eine 21-Aminosäuren-Deletion der HCV-Aminosäuren 1600-1620 enthält, wobei das Polypeptid durch rekombinante oder synthetische Methodologien außer als durch Expression als Fusionen mit einem Polypeptid erhältlich ist, für das das CKS-Gen codiert,

unter Bedingungen, die geeignet sind, einen zweiten Antikörper-Antigenkomplex zu bilden; und Nachweis des zweiten Antikörper-Antigenkomplexes; wobei die Polypeptide, die beim ersten aliquoten Anteil gewählt werden, nicht die gleichen sind, wie die Polypeptide, die beim zweiten aliquoten Anteil gewählt werden.

**4.** Ein Bestätigungsassay zur Identifizierung der Anwesenheit eines Antikörpers in einer flüssigen Probe, der gegenüber einem HCV Antigen immunologisch reaktiv ist, wobei die Probe verwendet wird, um einen ersten und zweiten immunologisch gleichwertigen aliquoten Anteil herzustellen, wobei der Bestätigungsassay folgende Schritte umfaßt:

(a) das In-Kontakt-Bringen des ersten aliquoten Anteils mit

(i) mindestens drei rekombinanten Proteinen, wobei ein rekombinantes Protein ein Fusionprotein des Polypeptids ist, für das das E.coli CMP-KDO Synthetasegen (CKS) codiert, und eines Polypeptids, das das HCV-Genom von den Aminosäuren 1192-1457 darstellt, das von dem Plasmid nach Figur 9 codiert werden kann, und das aus dem Stamm erhältlich ist, der unter der ATCC Zugangsnummer 68381 hinterlegt worden ist, wobei das zweite rekombinante Protein ein Fusionsprotein des Polypeptids ist, für das das E.coli CMP-KDO Synthetasegen (CKS) codiert, und eines Polypeptids, das das HCV-Genom von den Aminosäuren 1676-1931 darstellt, das aus dem Plasmid nach Figur 8 erhältlich ist, und das aus dem Stamm erhältlich ist, der unter der ATCC Zugangsnummer 68380 hinterlegt worden ist, und wobei das dritte rekombinante Protein ein Fusionsprotein aus dem Polypeptid ist, für das das E.coli CMP-KDO Synthetasegen (CKS) codiert und einem Polypeptid, das das HCV-Genom von den Aminosäuren 1-150 darstellt, wie in den Figuren 1, 5 und 6 definiert, oder

(ii) mindestens drei Polypeptiden, wobei ein Polypeptid das HCV-Genom von den Aminosäuren 1192-1457 darstellt, wobei das zweite Polypeptid das HCV-Genom von den Aminosäuren 1676-1931 darstellt, und wobei das dritte Polypeptid das HCV-Genom von den Aminosäuren 1-150 darstellt, wobei das erste, zweite und dritte Polypeptid durch rekombinante oder synthetische Methodologien außer als durch Expression als Fusionen mit einem Polypeptid erhältlich sind, für das das CKS-Gen codiert,

unter Bedingungen, die zur Komplexbildung des Antikörpers mit dem Polypeptid geeignet sind, und wobei der erste Antikörper-Antigenkomplex nachgewiesen wird, und

(b) In-Kontakt-Bringen des zweiten aliquoten Anteils mit einem Polypeptid, das aus der Gruppe gewählt ist, die aus folgendem besteht:

synthetischen Polypeptiden, die die Aminosäuren 1866-1930, 1684-1750, 1-75 oder 1689-1805 der HCV Sequenz darstellen,

einem Schimären-Protein von Peroxiddismutase und der HCV 33 Region von Figur 1,

einem Polypeptid, das das HCV-Genom von den Aminosäuren 1676-1931 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von den Aminosäuren 1192-1457 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das zwei nicht benachbarte codierende Regionen des HCV-Genoms von den Aminosäuren 1192-1457 und 1676-1931 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von den Aminosäuren 1-150 darstellt, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von den Aminosäuren 1569-1931 darstellt, das eine 23-Aminosäuren-Deletion der HCV-Aminosäuren 1575-1597 enthält, wobei das Polypeptid durch rekombinante oder synthetische Methodologien außer als durch Expression als Fusionen mit einem Polypeptid erhältlich ist, für das das CKS-Gen codiert,

einem Polypeptid, das das HCV-Genom von den Aminosäuren 1569-1931 darstellt, das eine 21-Aminosäuren-Deletion der HCV-Aminosäuren 1600-1620 enthält, wobei das Polypeptid durch rekombinante oder synthetische Methodologien außer als durch Expression als Fusionen mit einem Polypeptid erhältlich ist, für das das CKS-Gen codiert,

unter Bedingungen, die zur Ausbildung eines zweiten Antikörper-Antigenkomplexes geeignet sind;

und Nachweis des zweiten Antikörper-Antigenkomplexes, wobei die Polypeptide, die im ersten aliquoten Anteil gewählt werden, nicht die gleichen sind, wie die Polypeptide, die in dem zweiten aliquoten Anteil gewählt werden.

**5.** Bestätigungsassay nach Anspruch 3 oder 4, wobei in Schritt (b) der zweite aliquote Anteil weiter mit einem oder mehreren Polypeptiden in Kontakt gebracht werden kann, die aus der Gruppe gewählt sind, die aus den Polypeptiden besteht, die das HCV-Genom von den Aminosäuren 1932-2191, 2188-2481, 2866-3011, 2728-2867, 2480-2729, 263-469, 994-1205, 114-276, 114-469, 1472-1629, 617-834, wobei das Polypeptid durch rekombinante oder synthetische Methodologien außer als durch Expression als Fusionen mit einem Polypeptid erhalten wird, für das das CKS-Gen codiert.

6. Ein Immunoassaykit, das folgendes umfaßt:

(a) mindestens zwei rekombinante Proteine, wobei ein Protein ein Fusionsprotein aus dem Polypeptid, für das das E.coli CMP-KDO Synthetasegen (CKS) codiert und aus einem Polypeptid ist, das das HCV-Genom von den beiden Aminosäuren 1192-1457 und 1676-1931 darstellt, wie in den Figuren 1, 11 und 12 definiert, und wobei das zweite rekombinante Protein ein Fusionsprotein aus dem Polypeptid ist, für das das E.coli CMP-KDO Synthetasegen (CKS) codiert, und aus einem Polypeptid, das das HCV-Genom von den Aminosäuren 1-150 darstellt, wie definiert in den Figuren 1, 5 und 6 definiert, oder
(b) mindestens zwei Polypeptiden, wobei ein Polypeptid zwei nicht benachbarte codierende Bereiche des HCV-Genoms von den Aminosäuren von 1192-1457 und von 1676-1931 darstellt, und wobei das zweite Polypeptid das HCV-Genom von den Aminosäuren 1-150 darstellt, wobei das erste und zweite Polypeptid durch rekombinante oder synthetische Methodologien außer als durch Expression als Fusionen mit einem Polypeptid erhältlich sind, für das das CKS-Gen codiert;

eines odere mehrere Proben-Vorbereitungsreagenzien;
und eines oder mehrere Nachweis- und signalerzeugende Reagenzien.

7. Immunoassaykit, der folgendes umfaßt:

(a) mindestens drei rekombinante Proteine, wobei ein rekombinantes Protein ein Fusionsprotein aus dem Polypeptid ist, für das das E. coli CMP-KDO Synthetasegen (CKS) codiert, und aus einem Polypeptid, das das HCV-Genom von den Aminosäuren 1192-1457 darstellt, das aus dem Plasmid nach Figur 9 und aus dem Stamm erhältlich ist, der unter der ATCC Zugangshr. 68301 hinterlegt worden ist, wobei das zweite rekombinante Protein ein Fusionsprotein aus dem Polypeptid, für das das E.coli CMP-KDO Synthetasegen (CKS) codiert, und aus einem Polypeptid ist, das das HCV-Genom von den Aminosäuren 1676-1931 darstellt, das aus dem Plasmid nach Figur 8 und aus dem Stamm erhältlich ist, der unter der ATCC Zugangsnr. 68380 hinterlegt worden ist, und wobei das dritte rekombinante Protein ein Fusionsprotein aus dem Polypeptid ist, für das das E.coli CMP-KDO Synthetasegen (CKS) codiert und aus einem Polypeptid ist, das das HCV-Genom von den Aminosäuren 1-150 darstellt, wie in den Figuren 1, 5 und 6 definiert, oder
(b) mindestens drei Polypeptide, wobei ein Polypeptid das HCV-Genom von den Aminosäuren 1192-1457 darstellt, das zweite Polypeptid das HCV-Genom von den Aminosäuren 1676-1931 darstellt, und das dritte Polypeptid das HCV-Genom von den Aminosäuren von 1-150 darstellt, wobei das erste, zweite und dritte Polypeptid durch rekombinante oder synthetische Methodologien außer als durch Expression als Fusionen mit einem Polypeptid erhältlich sind, für die das CKS-Gen codiert;

eines oder mehrere Proben-Vorbereitungsreagenzien;
und eines oder mehrere Nachweis- und signalerzeugende Reagenzien.

**Revendications**

1. Essai de détermination de la présence d'un anticorps immunologiquement réactif avec un antigène anti-HCV dans un échantillon de fluide, consistant à mettre l'échantillon en contact avec

(a) au moins deux protéines recombinantes dont l'une est une protéine de fusion du polypeptide codé par le gène de la CMP-KDO synthétase (CKS) de E. coli et d'un polypeptide représentant le génome du HCV à la fois des acides aminés 1192 à 1457 et 1676 à 1931 tels que définis sur les figures 1, 11 et 12, et l'autre protéine recombinante est une protéine de fusion du polypeptide codé par le gène de la CMP-KDO synthétase (CKS) de E. coli et d'un polypeptide représentant le génome du HCV des acides aminés 1 à 150 tels que définis sur les figures 1, 5 et 6 ; ou bien
(b) au moins deux polypeptides dont l'un représente deux régions codantes non contiguës du génome du HCV des acides aminés 1192 à 1457 et 1676 à 1931, et le deuxième polypeptide représente le génome du HCV des acides aminés 1 à 150, les premier et deuxième polypeptides pouvant être obtenus par des méthodes de recombinaison ou de synthèse autrement que par une expression sous forme de fusions avec un polypeptide codé par le gène CKS ;

dans des conditions appropriées pour complexer l'anticorps avec le polypeptide, et à détecter le complexe anticorps/polypeptide.

2. Essai de détermination de la présence d'un anticorps immunologiquement réactif avec un antigène anti-HCV dans un échantillon de fluide, consistant à mettre l'échantillon en contact avec

(a) au moins trois protéines recombinantes dont l'une est une protéine de fusion du polypeptide codé par le gène de la CMP-KDO synthétase (CKS) de E. coli et d'un polypeptide représentant le génome du HCV des acides aminés 1192 à 1457 et pouvant être obtenu par le plasmide de la figure 9 et obtenu par la souche déposée sous le numéro d'entrée ATCC 68381, la deuxième protéine recombinante est une protéine de fusion du polypeptide codée par le gène de la CMP-KDO synthétase (CKS) de E. coli et d'un polypeptide représentant le génome du HCV des acides aminés 1676 à 1931 et pouvant être obtenu par le plasmide de la figure 8 et obtenu par la souche déposée sous le numéro d'entrée ATCC 68380, et la troisième protéine recombinante est une protéine de fusion du polypeptide codé par le gène de la CMP-KDO synthétase (CKS) de E. coli et d'un polypeptide représentant le génome du HCV des acides aminés 1 à 150 tels que définis sur les figures 1, 5 et 6 ; ou bien
(b) au moins trois polypeptides, dont l'un représente le génome du HCV des acides aminés 1192 à 1457, le deuxième polypeptide représente le génome du HCV des acides aminés 1676 à 1931 et le troisième polypeptide représente le génome du HCV des acides aminés 1 à 150, les premier, deuxième et troisième polypeptides pouvant être obtenus par des méthodes de recombinaison ou de synthèse autrement que par une expression sous forme de fusions avec un polypeptide codé par le gène CKS ;

dans des conditions appropriées pour complexer l'anticorps avec le polypeptide, et à détecter le complexe anticorps/polypeptide.

**3.** Essai de confirmation pour déterminer la présence d'un anticorps dans un échantillon de fluide immunologiquement réactif avec un antigène anti-HCV, dans lequel l'échantillon est utilisé pour préparer des première et deuxième aliquotes immunologiquement équivalentes, comprenant les étapes consistant à :

(a) mettre la première aliquote en contact avec

(i) au moins deux protéines recombinantes dont l'une est une protéine de fusion du polypeptide codé par le gène de la CMP-KDO synthétase (CKS) de E. coli et d'un polypeptide représentant le génome du HCV à la fois des acides aminés 1192 à 1457 et 1676 à 1931 tels que définis sur les figures 1, 11 et 12, et l'autre protéine recombinante est une protéine de fusion du polypeptide codé par le gène de la CMP-KDO synthétase (CKS) de E. coli et d'un polypeptide représentant le génome du HCV des acides aminés 1 à 150 tels que définis sur les figures 1, 5 et 6 ; ou bien
(ii) au moins deux polypeptides dont l'un représente deux régions codantes non contiguës du génome du HCV des acides aminés 1192 à 1457 et 1676 à 1931, et le deuxième polypeptide représente le génome du HCV des acides aminés 1 à 150, les premier et deuxième polypeptides pouvant être obtenus par des méthodes de recombinaison ou de synthèse autrement que par une expression sous forme de fusions avec un polypeptide codé par le gène CKS ;

dans des conditions appropriées pour complexer l'anticorps avec le polypeptide et dans lesquelles le premier complexe anticorps/antigène est détecté ; et
(b) mettre la deuxième aliquote en contact avec un polypeptide choisi dans le groupe comprenant :

des polypeptides de synthèse représentant les acides aminés 1866 à 1930, 1684 à 1750, 1 à 75 ou 1689 à 1805 de la séquence du HCV,
une protéine chimérique de la superoxyde dismutase et de la région HCV 33 de la figure 1,
un polypeptide représentant le génome du HCV des acides aminés 1676 à 1931, ledit polypeptide pouvant être obtenu par des méthodes de recombinaison ou de synthèse autrement que par une expression sous forme de fusions avec un polypeptide codé par le gène CKS,
un polypeptide représentant le génome du HCV des acides aminés 1192 à 1457, ledit polypeptide pouvant être obtenu par des méthodes de recombinaison ou de synthèse autrement que par une expression sous forme de fusions avec un polypeptide codé par le gène CKS,
un polypeptide représentant deux régions codantes non contiguës du génome du HCV des acides aminés 1192 à 1457 et 1676 à 1931, ledit polypeptide pouvant être obtenu par des méthodes de recombinaison ou de synthèse autrement que par une expression sous forme de fusions avec un polypeptide codé par le gène CKS,
un polypeptide représentant le génome du HCV des acides aminés 1 à 150, ledit polypeptide pouvant être obtenu par des méthodes de recombinaison ou de synthèse autrement que par une expression sous forme de fusions avec un polypeptide codé par le gène CKS,

un polypeptide représentant le génome du HCV des acides aminés 1569 à 1931 contenant une suppression de 23 acides aminés, les acides aminés 1575 à 1597 du HCV, ledit polypeptide pouvant être obtenu par des méthodes de recombinaison ou de synthèse autrement que par une expression sous forme de fusions avec un polypeptide codé par le gène CKS,

un polypeptide représentant le génome du HCV des acides aminés 1569 à 1931 contenant une suppression de 21 acides aminés, les acides aminés 1600 à 1620 du HCV, ledit polypeptide pouvant être obtenu par des méthodes de recombinaison ou de synthèse autrement que par une expression sous forme de fusions avec un polypeptide codé par le gène CKS ;

dans des conditions appropriées pour former un deuxième complexe anticorps/antigène ; et à détecter le deuxième complexe anticorps/antigène ; dans lequel les polypeptides sélectionnés dans la première aliquote ne sont pas les mêmes que les polypeptides sélectionnés dans la deuxième aliquote.

**4.** Essai de confirmation pour déterminer la présence d'un anticorps dans un échantillon de fluide immunologiquement réactif avec un antigène anti-HCV, dans lequel l'échantillon est utilisé pour préparer des première et deuxième aliquotes immunologiquement équivalentes, comprenant les étapes consistant à :

(a) mettre la première aliquote en contact avec

(i) au moins trois protéines recombinantes dont l'une est une protéine de fusion du polypeptide codé par le gène de la CMP-KDO synthétase (CKS) de E. coli et d'un polypeptide représentant le génome du HCV des acides aminés 1192 à 1457 et pouvant être obtenu par le plasmide de la figure 9 et obtenu par la souche déposée sous le numéro d'entrée ATCC 68381, la deuxième protéine recombinante st une protéine de fusion du polypeptide codé par le gène de la CMP-KDO synthétase (CKS) de E. coli et d'un polypeptide représentant le génome du HCV des acides aminés 1676 à 1931 et pouvant être obtenu par le plasmide de la figure 8 et obtenu par la souche déposée sous le numéro d'entrée ATCC 68380, et la troisième protéine recombinante est une protéine de fusion du polypeptide codé par le gène de la CMP-KDO synthétase (CKS) de E. coli et d'un polypeptide représentant le génome du HCV des acides aminés 1 à 150 tels que définis sur les figures 1, 5 et 6 ; ou bien

(ii) au moins trois polypeptides, dont l'un représente le génome du HCV des acides aminés 1192 à 1457, le deuxième polypeptide représente le génome du HCV des acides aminés 1676 à 1931 et le troisième polypeptide représente le génome du HCV des acides aminés 1 à 150, les premier, deuxième et troisième polypeptides pouvant être obtenus par des méthodes de recombinaison ou de synthèse autrement que par une expression sous forme de fusions avec un polypeptide codé par le gène CKS ;

dans des conditions appropriées pour complexer l'anticorps avec le polypeptide et dans lesquelles le premier complexe anticorps/antigène est détecté ; et

(b) mettre la deuxième aliquote en contact avec un polypeptide choisi dans le groupe comprenant :

des polypeptides de synthèse représentant les acides aminés 1866 à 1930, 1684 à 1750, 1 à 75 ou 1689 à 1805 de la séquence du HCV,

une protéine chimérique de la superoxyde dismutase et de la région HCV 33 de la figure 1,

un polypeptide représentant le génome du HCV des acides aminés 1676 à 1931, ledit polypeptide pouvant être obtenu par des méthodes de recombinaison ou de synthèse autrement que par une expression sous forme de fusions avec un polypeptide codé par le gène CKS,

un polypeptide représentant le génome du HCV des acides aminés 1192 à 1457, ledit polypeptide pouvant être obtenu par des méthodes de recombinaison ou de synthèse autrement que par une expression sous forme de fusions avec un polypeptide codé par le gène CKS,

un polypeptide représentant deux régions codantes non contiguës du génome du HCV des acides aminés 1192 à 1457 et 1676 à 1931, ledit polypeptide pouvant être obtenu par des méthodes de recombinaison ou de synthèse autrement que par une expression sous forme de fusions avec un polypeptide codé par le gène CKS,
un polypeptide représentant le génome du HCV des acides aminés 1 à 150, ledit polypeptide pouvant être obtenu par des méthodes de recombinaison ou de synthèse autrement que par une expression sous forme de fusions avec un polypeptide codé par le gène CKS,
un polypeptide représentant le génome du HCV des acides aminés 1569 à 1931 contenant une suppression de 23 acides aminés, les acides aminés 1575 à 1597 du HCV, ledit polypeptide pouvant être obtenu par des méthodes de recombinaison ou de synthèse autrement que par une expression sous forme de fusions avec un polypeptide codé par le gène CKS,
un polypeptide représentant le génome du HCV des acides aminés 1569 à 1931 contenant une suppression de 21 acides aminés, les acides aminés 1600 à 1620 du HCV, ledit polypeptide pouvant être obtenu par des méthodes de recombinaison ou de synthèse autrement que par une expression sous forme de fusions avec un polypeptide codé par le gène CKS ;
dans des conditions appropriées pour former un deuxième complexe anticorps/antigène ; et à détecter le deuxième complexe anticorps/antigène ; dans lequel les polypeptides sélectionnés dans la première aliquotes ne sont pas les mêmes que les polypeptides sélectionnés dans la deuxième aliquote.

**5.** Essai de confirmation selon la revendication 3 ou la revendication 4, dans lequel, à l'étape (b), la deuxième aliquote peut être en plus mise en contact avec un ou plusieurs polypeptides choisis dans le groupe comprenant les polypeptides représentant les génome HCV des acides aminés 1932 à 2191, 2188 à 2481, 2866 à 3011, 2728 à 2867, 2480 à 2729, 263 à 469, 994 à 1205, 114 à 276, 114 à 469, 1472 à 1629, 617 à 834, ledit polypeptide pouvant être obtenu par des méthodes de recombinaison ou de synthèse autrement que par une expression sous forme de fusions avec un polypeptide codé par le gène CKS.

**6.** Trousse d'immunodosage comprenant :

(a) au moins deux protéines recombinantes dont l'une est une protéine de fusion du polypeptide codé par le gène de la CMP-KDO synthétase (CKS) de E. coli et d'un polypeptide représentant le génome du HCV à la fois des acides aminés 1192 à 1457 et 1676 à 1931 tels que définis sur les figures 1, 11 et 12, et l'autre protéine recombinante est une protéine de fusion du polypeptide codé par le gène de la CMP-KDO synthétase (CKS) de E. coli et d'un polypeptide représentant le génome du HCV des acides aminés 1 à 150 tels que définis sur les figures 1, 5 et 6 ; ou bien
(b) au moins deux polypeptides dont l'un représente deux régions codantes non contiguës du génome du HCV des acides aminés 1192 à 1457 et 1676 à 1931, et le deuxième polypeptide représente le génome du HCV des acides aminés 1 à 150, les premier et deuxième polypeptide pouvant être obtenus par des méthodes de recombinaison ou de synthèse autrement que par une expression sous forme de fusions avec un polypeptide codé par le gène CKS ;

un ou plusieurs réactifs de préparation des échantillons ;
un ou plusieurs réactifs de détection et de génération de signal.

**7.** Trousse d'immunodosage comprenant :

(a) au moins trois protéines recombinantes dont l'une est une protéine de fusion du polypeptide codé par le gène de la CMP-KDO synthétase (CKS) de E. coli et d'un polypeptide représentant le génome du HCV des acides aminés 1192 à 1457 et pouvant être obtenu par le plasmide de la figure 9 et obtenu par la souche déposée sous le numéro d'entrée ATCC 68381, la deuxième protéine recombinante st une protéine de fusion du polypeptide codé par le gène de la CMP-KDO synthétase (CKS) de E. coli et d'un polypeptide représentant le génome du HCV des acides aminés 1676 à 1931 et pouvant être obtenu par le plasmide de la figure 8 et obtenu par la souche déposée sous le numéro d'entrée ATCC 68380, et la troisième protéine recombinante est une protéine de fusion du polypeptide codé par le gène de la CMP-KDO synthétase (CKS) de E. coli et d'un polypeptide représentant le génome du HCV des acides aminés 1 à 150 tels que définis sur les figures 1, 5 et 6 ; ou bien
(b) au moins trois polypeptides, dont l'un représente le génome du HCV des acides aminés 1192 à 1457, le deuxième polypeptide représente le génome du HCV des acides aminés 1676 à 1931 et le troisième polypeptide représente le génome du HCV des acides aminés 1 à 150, les premier, deuxième et troisième polypeptides pouvant être obtenus par des méthodes de recombinaison ou de synthèse autrement que par une expression sous forme de fusions avec un polypeptide codé par le gène CKS ;

- un ou plusieurs réactifs de préparation des échantillons ;
- un ou plusieurs réactifs de détection et de génération de signal.

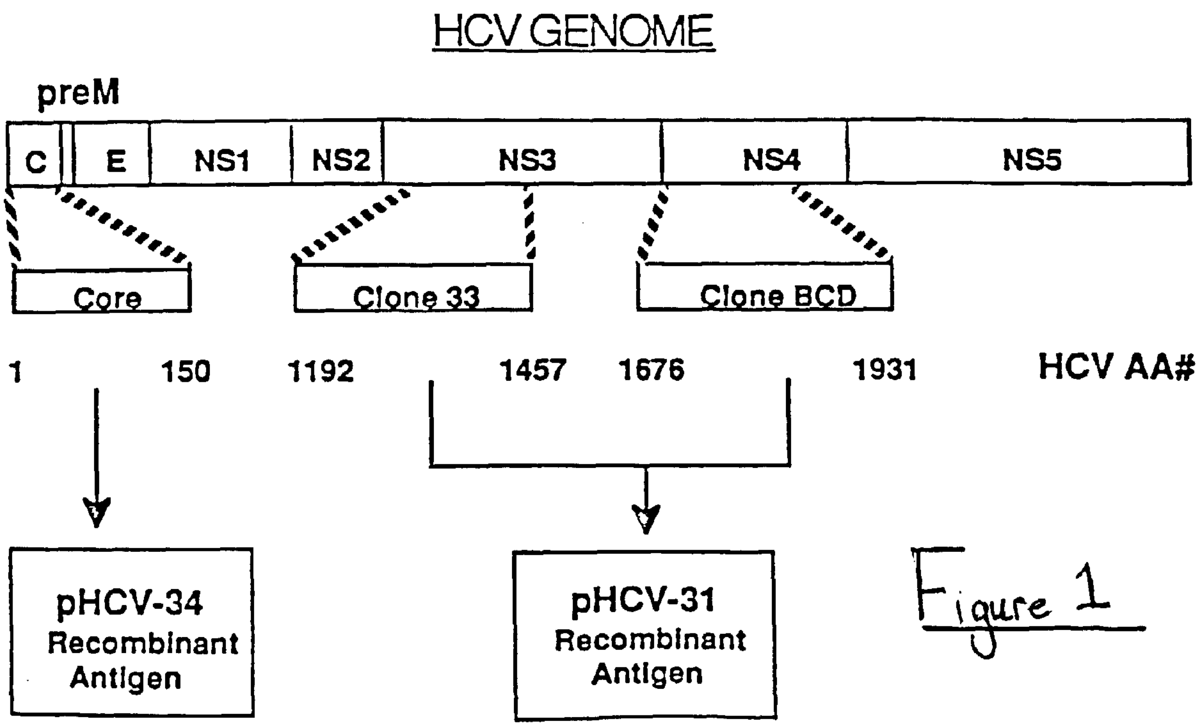
HCV GENOME
preM
C
E
NS1
NS2
NS3
NS4
NS5
Core
Clone 33
Clone BCD
1
150
1192
1457
1676
1931
HCV AA#
pHCV-34
Recombinant
Antigen
pHCV-31
Recombinant
Antigen

Figure 1

SEROLOGIC PROFILE OF CHIMPANZEES INOCULATED WITH HEPATITIS C VIRUS

| | | | ALT (mlU/ml) ELEVATION* (DPI) | | | | DETECTION OF SEROCONVERSION TO HCV PROTEINS | | |
|---|---|---|---|---|---|---|---|---|---|
| ID # | NAME | Pre ** (range) | First | Peak | Duration | Maximum value | C100-3 (DPI) | pHCV-31 pHCV-34 (DPI) | DAYS DIFFERENT |
| CH 427 | COLONEL | 29 - 53 | 56 | 75 | 24 | 280 | 77 | 56 | 21 |
| CH 479 | JR | 14 - 20 | 91 | 91 | 7 | 156 | 133 | 98 | 35 |
| CH 477 | KIST | 17 - 31 | 30 | 35 | 12 | 107 | 70 | 70 | 0 |
| CH 335 | LEO | 16 - 20 | 38 | 46 | 21 | 295 | 59 | 38 | 21 |
| CH 120 | LOLITA | 15 - 28 | 33 | 65 | 39 | 435 | 65 | 100 | 35 |
| CH 21 | MELILOT | 12 - 30 | 68 | 75 | 14 | 190 | 82 | 66 | 16 |
| CH 379 | PAN | 19 - 27 | 49 | 68 | 28 | 250 | 119 | 98 | 21 |

* twice the upper limit of normal values

** eleven preinoculation samples per animal

Figure 2.

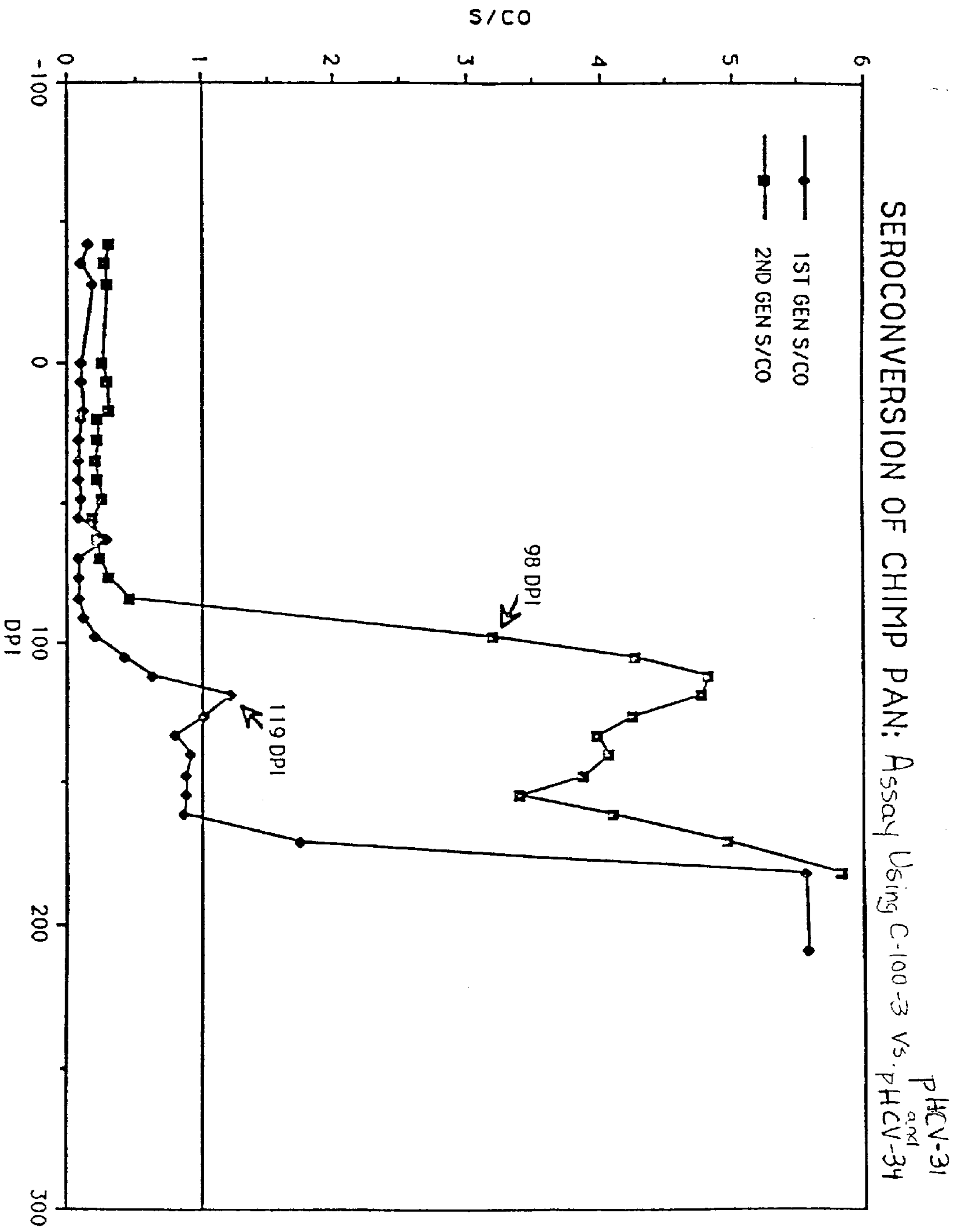
SEROCONVERSION OF CHIMP PAN: Assay Using C-100-3 vs. pHCV-31 and pHCV-34
1ST GEN S/CO
2ND GEN S/CO
98 DPI
119 DPI
S/CO
DPI
0
1
2
3
4
5
6
-100
0
100
200
300

Figure 3.

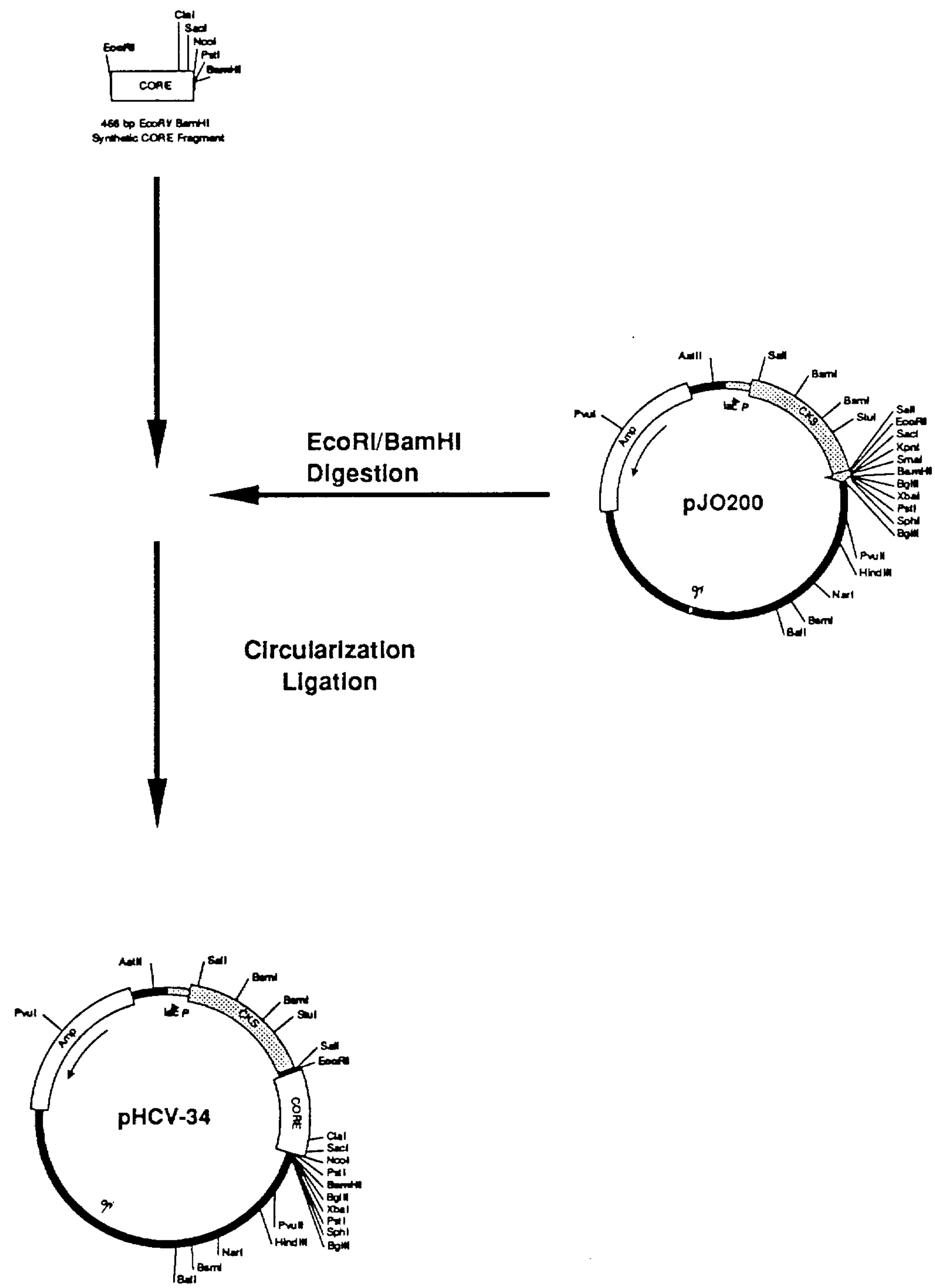


Figure 4 Construction of Plasmid pHCV-34.

# Figure 5

Complete DNA sequence of pHCV-34. The predicted amino acid sequence of the structural gene is included with the DNA sequence.

```
        10         20         30         40         50         60         70
GAATTAATTC CCATTAATGT GAGTTAGCTC ACTCATTAGG CACCCCAGGC TTTACACTTT ATGTTCCGGC

        80         90        100        110        120       129
                                                                >
TCGTATTTTG TGTGGAATTG TGAGCGGATA ACAATTGGGC ATCCAGTAAG GAGGTTTAA ATG
                                                                MET

    138         147         156         165         174         183
AGT TTT GTG GTC ATT ATT CCC GCG CGC TAC GCG TCG ACG CGT CTG CCC GGT AAA
Ser Phe Val Val Ile Ile Pro Ala Arg Tyr Ala Ser Thr Arg Leu Pro Gly Lys

    192         201         210         219         228         237
CCA TTG GTT GAT ATT AAC GGC AAA CCC ATG ATT GTT CAT GTT CTT GAA CGC GCG
Pro Leu Val Asp Ile Asn Gly Lys Pro MET Ile Val His Val Leu Glu Arg Ala

    246         255         264         273         282         291
CGT GAA TCA GGT GCC GAG CGC ATC ATC GTG GCA ACC GAT CAT GAG GAT GTT GCC
Arg Glu Ser Gly Ala Glu Arg Ile Ile Val Ala Thr Asp His Glu Asp Val Ala

    300         309         318         327         336         345
CGC GCC GTT GAA GCC GCT GGC GGT GAA GTA TGT ATG ACG CGC GCC GAT CAT CAG
Arg Ala Val Glu Ala Ala Gly Gly Glu Val Cys MET Thr Arg Ala Asp His Gln

    354         363         372         381         390         399
TCA GGA ACA GAA CGT CTG GCG GAA GTT GTC GAA AAA TGC GCA TTC AGC GAC GAC
Ser Gly Thr Glu Arg Leu Ala Glu Val Val Glu Lys Cys Ala Phe Ser Asp Asp

    408         417         426         435         444         453
ACG GTG ATC GTT AAT GTG CAG GGT GAT GAA CCG ATG ATC CCT GCG ACA ATC ATT
Thr Val Ile Val Asn Val Gln Gly Asp Glu Pro MET Ile Pro Ala Thr Ile Ile

    462         471         480         489         498         507
CGT CAG GTT GCT GAT AAC CTC GCT CAG CGT CAG GTG GGT ATG GCG ACT CTG GCG
Arg Gln Val Ala Asp Asn Leu Ala Gln Arg Gln Val Gly MET Ala Thr Leu Ala

    516         525         534         543         552         561
GTG CCA ATC CAC AAT GCG GAA GAA GCG TTT AAC CCG AAT GCG GTG AAA GTG GTT
Val Pro Ile His Asn Ala Glu Glu Ala Phe Asn Pro Asn Ala Val Lys Val Val

    570         579         588         597         606         615
CTC GAC GCT GAA GGG TAT GCA CTG TAC TTC TCT CGC GCC ACC ATT CCT TGG GAT
Leu Asp Ala Glu Gly Tyr Ala Leu Tyr Phe Ser Arg Ala Thr Ile Pro Trp Asp
```

Figure 5 con't

624 633 642 651 660 669

CGT GAT CGT TTT GCA GAA GGC CTT GAA ACC GTT GGC GAT AAC TTC CTG CGT CAT
Arg Asp Arg Phe Ala Glu Gly Leu Glu Thr Val Gly Asp Asn Phe Leu Arg His

678 687 696 705 714 723

CTT GGT ATT TAT GGC TAC CGT GCA GGC TTT ATC CGT CGT TAC GTC AAC TGG CAG
Leu Gly Ile Tyr Gly Tyr Arg Ala Gly Phe Ile Arg Arg Tyr Val Asn Trp Gln

732 741 750 759 768 777

CCA AGT CCG TTA GAA CAC ATC GAA ATG TTA GAG CAG CTT CGT GTT CTG TGG TAC
Pro Ser Pro Leu Glu His Ile Glu MET Leu Glu Gln Leu Arg Val Leu Trp Tyr

786 795 804 813 822 831

GGC GAA AAA ATC CAT GTT GCT GTT GCT CAG GAA GTT CCT GGC ACA GGT GTG GAT
Gly Glu Lys Ile His Val Ala Val Ala Gln Glu Val Pro Gly Thr Gly Val Asp

840 849 858 867 876 885

ACC CCT GAA GAT CTC GAC CCG TCG ACG AAT TCC ATG TCT ACC AAC CCG AAA CCG
Thr Pro Glu Asp Leu Asp Pro Ser Thr Asn Ser MET Ser Thr Asn Pro Lys Pro

894 903 912 921 930 939

CAG AAA AAA AAC AAA CGT AAC ACC AAC CGT CGT CCG CAG GAC GTT AAA TTC CCG
Gln Lys Lys Asn Lys Arg Asn Thr Asn Arg Arg Pro Gln Asp Val Lys Phe Pro

948 957 966 975 984 993

GGT GGT GGT CAG ATC GTT GGT GGT GTT TAC CTG CTG CCG CGT CGT GGT CCG CGT
Gly Gly Gly Gln Ile Val Gly Gly Val Tyr Leu Leu Pro Arg Arg Gly Pro Arg

1002 1011 1020 1029 1038 1047

CTG GGT GTT CGT GCT ACG CGT AAA ACC TCT GAA CGT TCT CAG CCG CGT GGG CGT
Leu Gly Val Arg Ala Thr Arg Lys Thr Ser Glu Arg Ser Gln Pro Arg Gly Arg

1056 1065 1074 1083 1092 1101

CGT CAG CCG ATC CCG AAA GCT CGT CGT CCG GAA GGT CGT ACC TGG GCT CAG CCG
Arg Gln Pro Ile Pro Lys Ala Arg Arg Pro Glu Gly Arg Thr Trp Ala Gln Pro

1110 1119 1128 1137 1146 1155

GGT TAC CCG TGG CCG CTG TAC GGT AAC GAA GGT TGC GGT TGG GCT GGT TGG CTG
Gly Tyr Pro Trp Pro Leu Tyr Gly Asn Glu Gly Cys Gly Trp Ala Gly Trp Leu

1164 1173 1182 1191 1200 1209

CTG TCT CCG CGT GGA TCT CGT CCG TCT TGG GGT CCG ACC GAC CCG CGT CGT CGT
Leu Ser Pro Arg Gly Ser Arg Pro Ser Trp Gly Pro Thr Asp Pro Arg Arg Arg

Figure 5 con't

```
  1218      1227      1236      1245      1254      1263
TCT CGT AAC CTT GGT AAA GTT ATC GAT ACC CTG ACC TGC GGT TTC GCT GAC CTG
Ser Arg Asn Leu Gly Lys Val Ile Asp Thr Leu Thr Cys Gly Phe Ala Asp Leu

  1272      1281      1290      1299      1308      1317
ATG GGT TAC ATA CCG CTG GTT GGA GCT CCG CTG GGT GGT GCT GCT CGT GCT TAA
MET Gly Tyr Ile Pro Leu Val Gly Ala Pro Leu Gly Gly Ala Ala Arg Ala  .

      1330       1340       1350       1360       1370       1380       1390
CCCATGGATC CTCTAGACTG CAGGCATGCT AAGTAAGTAG ATCTTGAGCG CGTTCGCGCT GAAATGCGCT

      1400       1410       1420       1430       1440       1450       1460
AATTTCACTT CACGACACTT CAGCCAATTT TGGGAGGAGT GTCGTACCGT TACGATTTTC CTCAATTTTT

      1470       1480       1490       1500       1510       1520       1530
CTTTTCAACA ATTGATCTCA TTCAGGTGAC ATCTTTTATA TTGGCGCTCA TTATGAAAGC AGTAGCTTTT

      1540       1550       1560       1570       1580       1590       1600
ATGAGGGTAA TCTGAATGGA ACAGCTGCGT GCCGAATTAA GCCATTTACT GGGCGAAAAA CTCAGTCGTA

      1610       1620       1630       1640       1650       1660       1670
TTGAGTGCGT CAATGAAAAA GCGGATACGG CGTTGTGGGC TTTGTATGAC AGCCAGGGAA ACCCAATGCC

      1680       1690       1700       1710       1720       1730       1740
GTTAATGGCA AGAAGCTTAG CCCGCCTAAT GAGCGGGCTT TTTTTTCGAC GCGAGGCTGG ATGGCCTTCC

      1750       1760       1770       1780       1790       1800       1810
CCATTATGAT TCTTCTCGCT TCCGGCGGCA TCGGGATGCC CGCGTTGCAG GCCATGCTGT CCAGGCAGGT

      1820       1830       1840       1850       1860       1870       1880
AGATGACGAC CATCAGGGAC AGCTTCAAGG ATCGCTCGCG GCTCTTACCA GCCTAACTTC GATCACTGGA

      1890       1900       1910       1920       1930       1940       1950
CCGCTGATCG TCACGGCGAT TTATGCCGCC TCGGCGAGCA CATGGAACGG GTTGGCATGG ATTGTAGGCG

      1960       1970       1980       1990       2000       2010       2020

CCGCCCTATA CCTTGTCTGC CTCCCCGCGT TGCGTCGCGG TGCATGGAGC CGGGCCACCT CGACCTGAAT
      2030       2040       2050       2060       2070       2080       2090

GGAAGCCGGC GGCACCTCGC TAACGGATTC ACCACTCCAA GAATTGGAGC CAATCAATTC TTGCGGAGAA
      2100       2110       2120       2130       2140       2150       2160

CTGTGAATGC GCAAACCAAC CCTTGGCAGA ACATATCCAT CGCGTCCGCC ATCTCCAGCA GCCGCACGCG

      2170       2180       2190       2200       2210       2220       2230
GCGCATCTCG GGCAGCGTTG GGTCCTGGCC ACGGGTGCGC ATGATCGTGC TCCTGTCGTT GAGGACCCGG

      2240       2250       2260       2270       2280       2290       2300
CTAGGCTGGC GGGGTTGCCT TACTGGTTAG CAGAATGAAT CACCGATACG CGAGCGAACG TGAAGCGACT
```

Figure 5 con't

2310 2320 2330 2340 2350 2360 2370
GCTGCTGCAA AACGTCTGCG ACCTGAGCAA CAACATGAAT GGTCTTCGGT TTCCGTGTTT CGTAAAGTCT

2380 2390 2400 2410 2420 2430 2440
GGAAACGCGG AAGTCAGCGC CCTGCACCAT TATGTTCCGG ATCTGCATCG CAGGATGCTG CTGGCTACCC

2450 2460 2470 2480 2490 2500 2510
TGTGGAACAC CTACATCTGT ATTAACGAAG CGCTTCTTCC GCTTCCTCGC TCACTGACTC GCTGCGCTCG

2520 2530 2540 2550 2560 2570 2580
GTCGTTCGGC TGCGGCGAGC GGTATCAGCT CACTCAAAGG CGGTAATACG GTTATCCACA GAATCAGGGG

2590 2600 2610 2620 2630 2640 2650
ATAACGCAGG AAAGAACATG TGAGCAAAAG GCCAGCAAAA GGCCAGGAAC CGTAAAAAGG CCGCGTTGCT

2660 2670 2680 2690 2700 2710 2720
GGCGTTTTTC CATAGGCTCC GCCCCCCTGA CGAGCATCAC AAAAATCGAC GCTCAAGTCA GAGGTGGCGA

2730 2740 2750 2760 2770 2780 2790
AACCCGACAG GACTATAAAG ATACCAGGCG TTTCCCCCTG GAAGCTCCCT CGTGCGCTCT CCTGTTCCGA

2800 2810 2820 2830 2840 2850 2860
CCCTGCCGCT TACCGGATAC CTGTCCGCCT TTCTCCCTTC GGGAAGCGTG GCGCTTTCTC AATGCTCACG

2870 2880 2890 2900 2910 2920 2930
CTGTAGGTAT CTCAGTTCGG TGTAGGTCGT TCGCTCCAAG CTGGGCTGTG TGCACGAACC CCCCGTTCAG

2940 2950 2960 2970 2980 2990 3000
CCCGACCGCT GCGCCTTATC CGGTAACTAT CGTCTTGAGT CCAACCCGGT AAGACACGAC TTATCGCCAC

3010 3020 3030 3040 3050 3060 3070
TGGCAGCAGC CACTGGTAAC AGGATTAGCA GAGCGAGGTA TGTAGGCGGT GCTACAGAGT TCTTGAAGTG

3080 3090 3100 3110 3120 3130 3140
GTGGCCTAAC TACGGCTACA CTAGAAGGAC AGTATTTGGT ATCTGCGCTC TGCTGAAGCC AGTTACCTTC

3150 3160 3170 3180 3190 3200 3210
GGAAAAAGAG TTGGTAGCTC TTGATCCGGC AAACAAACCA CCGCTGGTAG CGGTGGTTTT TTTGTTTGCA

3220 3230 3240 3250 3260 3270 3280
AGCAGCAGAT TACGCGCAGA AAAAAAGGAT CTCAAGAAGA TCCTTTGATC TTTTCTACGG GGTCTGACGC

3290 3300 3310 3320 3330 3340 3350
TCAGTGGAAC GAAAACTCAC GTTAAGGGAT TTTGGTCATG AGATTATCAA AAAGGATCTT CACCTAGATC

3360 3370 3380 3390 3400 3410 3420
CTTTTAAATT AAAAATGAAG TTTTAAATCA ATCTAAAGTA TATATGAGTA AACTTGGTCT GACAGTTACC

3430 3440 3450 3460 3470 3480 3490
AATGCTTAAT CAGTGAGGCA CCTATCTCAG CGATCTGTCT ATTTCGTTCA TCCATAGTTG CCTGACTCCC

3500 3510 3520 3530 3540 3550 3560
CGTCGTGTAG ATAACTACGA TACGGGAGGG CTTACCATCT GGCCCCAGTG CTGCAATGAT ACCGCGAGAC

3570 3580 3590 3600 3610 3620 3630
CCACGCTCAC CGGCTCCAGA TTTATCAGCA ATAAACCAGC CAGCCGGAAG GGCCGAGCGC AGAAGTGGTC

Figure 5 con't

3640 3650 3660 3670 3680 3690 3700
CTGCAACTTT ATCCGCCTCC ATCCAGTCTA TTAATTGTTG CCGGGAAGCT AGAGTAAGTA GTTCGCCAGT

3710 3720 3730 3740 3750 3760 3770
TAATAGTTTG CGCAACGTTG TTGCCATTGC TACAGGCATC GTGGTGTCAC GCTCGTCGTT TGGTATGGCT

3780 3790 3800 3810 3820 3830 3840
TCATTCAGCT CCGGTTCCCA ACGATCAAGG CGAGTTACAT GATCCCCCAT GTTGTGCAAA AAAGCGGTTA

3850 3860 3870 3880 3890 3900 3910
GCTCCTTCGG TCCTCCGATC GTTGTCAGAA GTAAGTTGGC CGCAGTGTTA TCACTCATGG TTATGGCAGC

3920 3930 3940 3950 3960 3970 3980
ACTGCATAAT TCTCTTACTG TCATGCCATC CGTAAGATGC TTTTCTGTGA CTGGTGAGTA CTCAACCAAG

3990 4000 4010 4020 4030 4040 4050
TCATTCTGAG AATAGTGTAT GCGGCGACCG AGTTGCTCTT GCCCGGCGTC AACACGGGAT AATACCGCGC

4060 4070 4080 4090 4100 4110 4120
CACATAGCAG AACTTTAAAA GTGCTCATCA TTGGAAAACG TTCTTCGGGG CGAAAACTCT CAAGGATCTT

4130 4140 4150 4160 4170 4180 4190
ACCGCTGTTG AGATCCAGTT CGATGTAACC CACTCGTGCA CCCAACTGAT CTTCAGCATC TTTTACTTTC

4200 4210 4220 4230 4240 4250 4260
ACCAGCGTTT CTGGGTGAGC AAAAACAGGA AGGCAAAATG CCGCAAAAAA GGGAATAAGG GCGACACGGA

4270 4280 4290 4300 4310 4320 4330
AATGTTGAAT ACTCATACTC TTCCTTTTTC AATATTATTG AAGCATTTAT CAGGGTTATT GTCTCATGAG

4340 4350 4360 4370 4380 4390 4400
CGGATACATA TTTGAATGTA TTTAGAAAAA TAAACAAATA GGGGTTCCGC GCACATTTCC CCGAAAAGTG

4410 4420 4430 4440 4450 4460 4470
CCACCTGACG TCTAAGAAAC CATTATTATC ATGACATTAA CCTATAAAAA TAGGCGTATC ACGAGGCCCT

4480
TTCGTCTTCA A

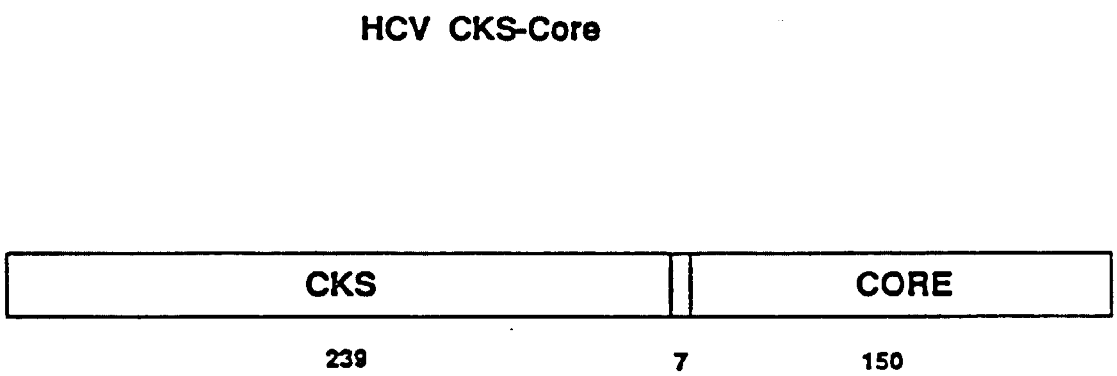


Figure 6. Recombinant Protein Encoded by pHCV-34.

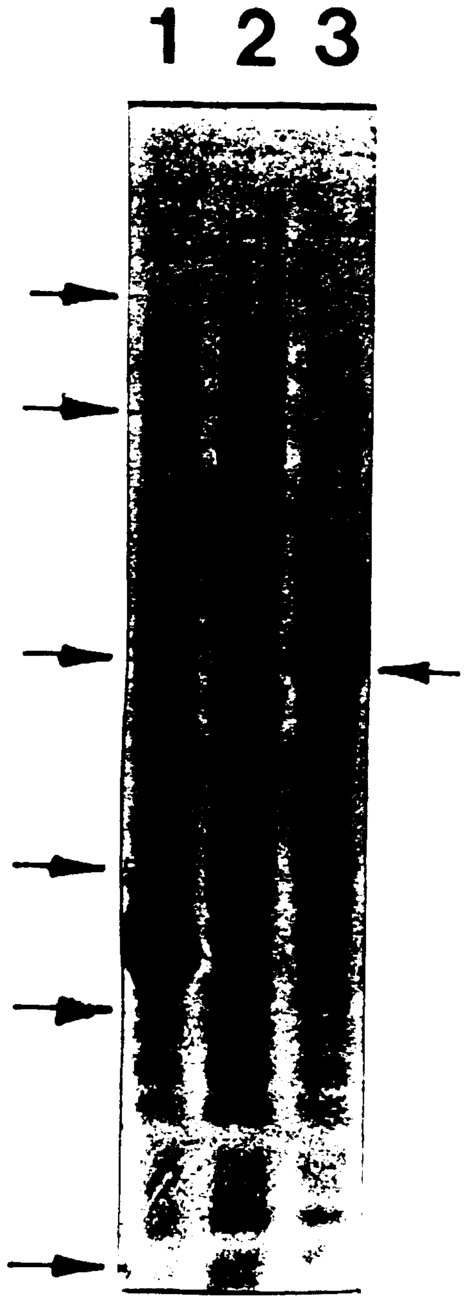


Figure 7.

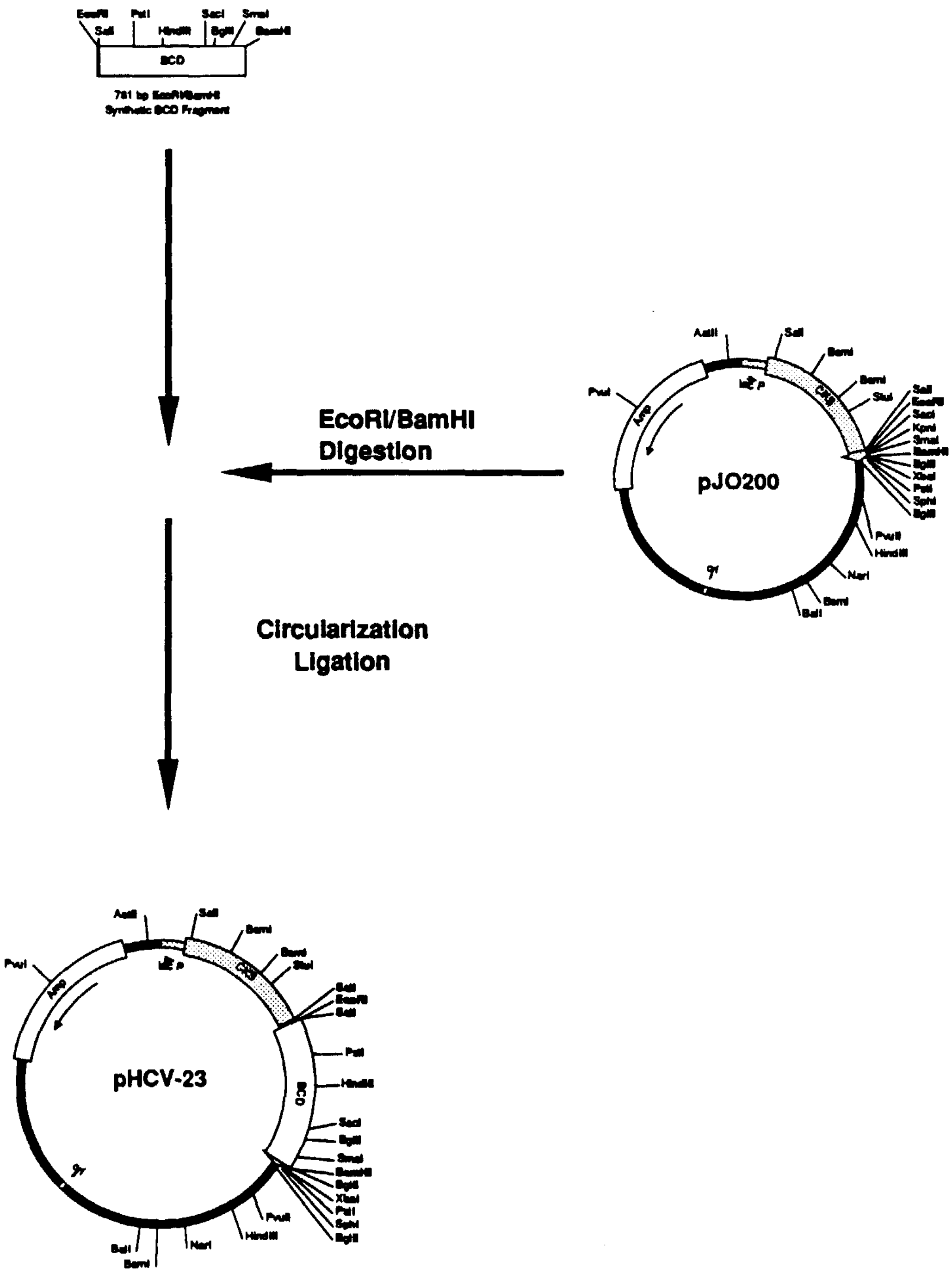


Figure 8 Construction of Plasmid pHCV-23.

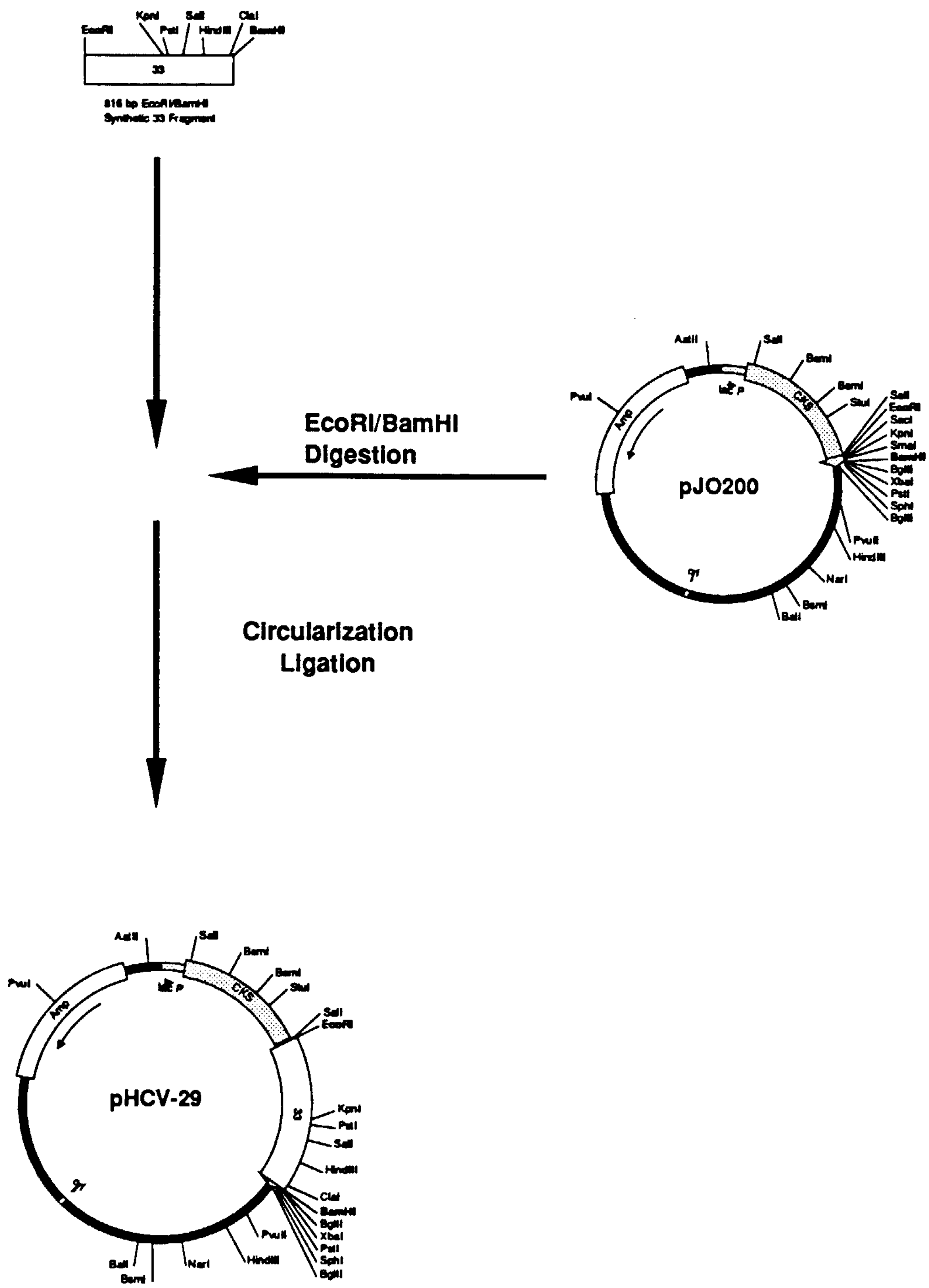


Figure 9 Construction of Plasmid pHCV-29.

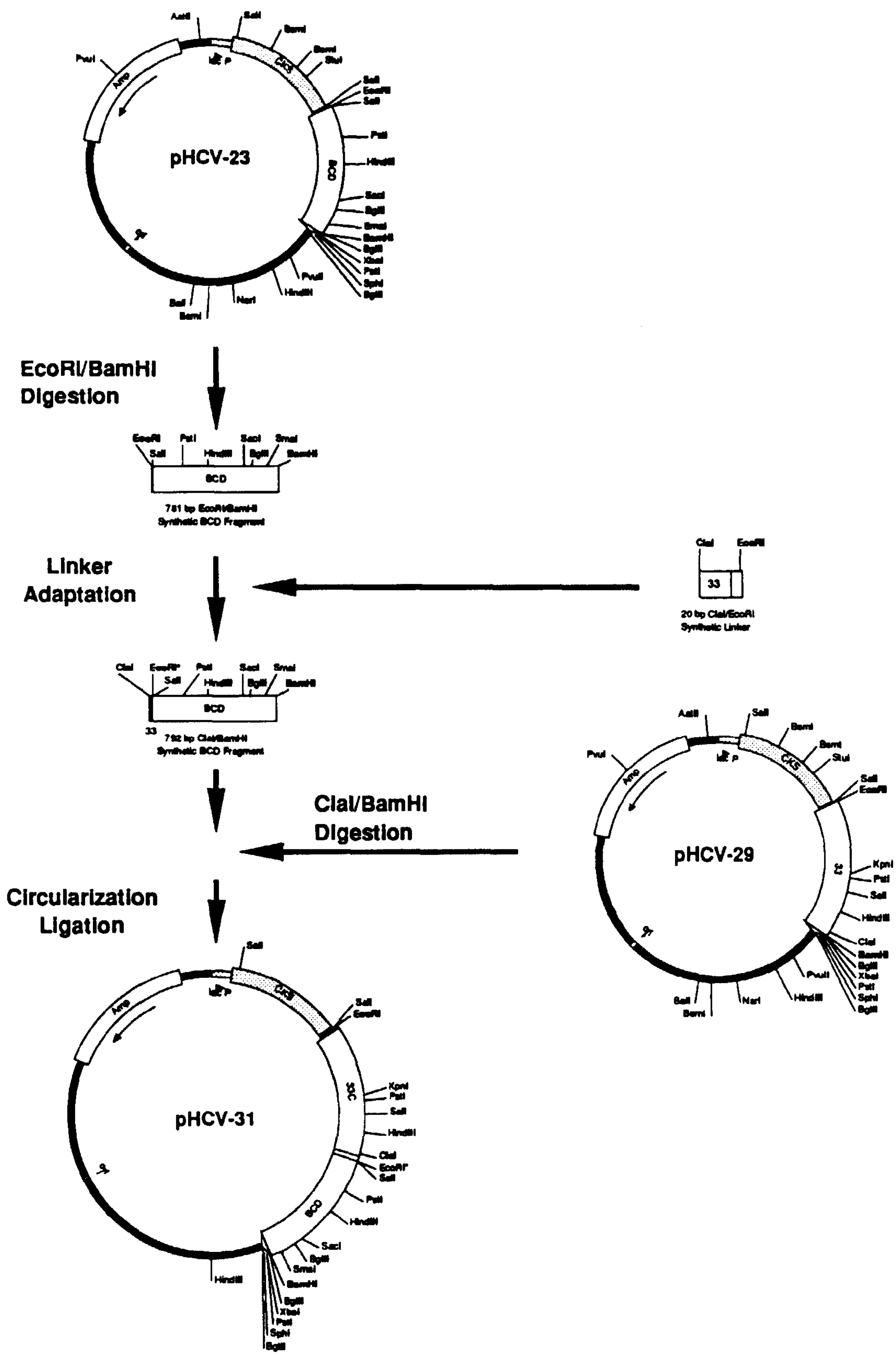


Figure 10 Construction of Plasmid pHCV-31.

# Figure 11

Complete DNA sequence of pHCV-31. The predicted amino acid sequence of the structural gene is included with the DNA sequence.

```
         10         20         30         40         50         60         70
GAATTAATTC CCATTAATGT GAGTTAGCTC ACTCATTAGG CACCCCAGGC TTTACACTTT ATGTTCCGGC

         80         90        100        110        120        129
                                                                   >
TCGTATTTTG TGTGGAATTG TGAGCGGATA ACAATTGGGC ATCCAGTAAG GAGGTTTAA ATG
                                                                 MET

    138         147         156         165         174         183

AGT TTT GTG GTC ATT ATT CCC GCG CGC TAC GCG TCG ACG CGT CTG CCC GGT AAA
Ser Phe Val Val Ile Ile Pro Ala Arg Tyr Ala Ser Thr Arg Leu Pro Gly Lys

    192         201         210         219         228         237

CCA TTG GTT GAT ATT AAC GGC AAA CCC ATG ATT GTT CAT GTT CTT GAA CGC GCG
Pro Leu Val Asp Ile Asn Gly Lys Pro MET Ile Val His Val Leu Glu Arg Ala

    246         255         264         273         282         291

CGT GAA TCA GGT GCC GAG CGC ATC ATC GTG GCA ACC GAT CAT GAG GAT GTT GCC
Arg Glu Ser Gly Ala Glu Arg Ile Ile Val Ala Thr Asp His Glu Asp Val Ala

    300         309         318         327         336         345

CGC GCC GTT GAA GCC GCT GGC GGT GAA GTA TGT ATG ACG CGC GCC GAT CAT CAG
Arg Ala Val Glu Ala Ala Gly Gly Glu Val Cys MET Thr Arg Ala Asp His Gln

    354         363         372         381         390         399

TCA GGA ACA GAA CGT CTG GCG GAA GTT GTC GAA AAA TGC GCA TTC AGC GAC GAC
Ser Gly Thr Glu Arg Leu Ala Glu Val Val Glu Lys Cys Ala Phe Ser Asp Asp

    408         417         426         435         444         453

ACG GTG ATC GTT AAT GTG CAG GGT GAT GAA CCG ATG ATC CCT GCG ACA ATC ATT
Thr Val Ile Val Asn Val Gln Gly Asp Glu Pro MET Ile Pro Ala Thr Ile Ile

    462         471         480         489         498         507

CGT CAG GTT GCT GAT AAC CTC GCT CAG CGT CAG GTG GGT ATG GCG ACT CTG GCG
Arg Gln Val Ala Asp Asn Leu Ala Gln Arg Gln Val Gly MET Ala Thr Leu Ala

    516         525         534         543         552         561

GTG CCA ATC CAC AAT GCG GAA GAA GCG TTT AAC CCG AAT GCG GTG AAA GTG GTT
Val Pro Ile His Asn Ala Glu Glu Ala Phe Asn Pro Asn Ala Val Lys Val Val
```

Figure 11. con't

```
     570         579         588         597         606         615
CTC GAC GCT GAA GGG TAT GCA CTG TAC TTC TCT CGC GCC ACC ATT CCT TGG GAT
Leu Asp Ala Glu Gly Tyr Ala Leu Tyr Phe Ser Arg Ala Thr Ile Pro Trp Asp

     624         633         642         651         660         669
CGT GAT CGT TTT GCA GAA GGC CTT GAA ACC GTT GGC GAT AAC TTC CTG CGT CAT
Arg Asp Arg Phe Ala Glu Gly Leu Glu Thr Val Gly Asp Asn Phe Leu Arg His

     678         687         696         705         714         723
CTT GGT ATT TAT GGC TAC CGT GCA GGC TTT ATC CGT CGT TAC GTC AAC TGG CAG
Leu Gly Ile Tyr Gly Tyr Arg Ala Gly Phe Ile Arg Arg Tyr Val Asn Trp Gln

     732         741         750         759         768         777
CCA AGT CCG TTA GAA CAC ATC GAA ATG TTA GAG CAG CTT CGT GTT CTG TGG TAC
Pro Ser Pro Leu Glu His Ile Glu MET Leu Glu Gln Leu Arg Val Leu Trp Tyr

     786         795         804         813         822         831
GGC GAA AAA ATC CAT GTT GCT GTT GCT CAG GAA GTT CCT GGC ACA GGT GTG GAT
Gly Glu Lys Ile His Val Ala Val Ala Gln Glu Val Pro Gly Thr Gly Val Asp

     840         849         858         867         876         885
ACC CCT GAA GAT CTC GAC CCG TCG ACG AAT TCC ATG GCT GTT GAC TTT ATC CCG
Thr Pro Glu Asp Leu Asp Pro Ser Thr Asn Ser MET Ala Val Asp Phe Ile Pro

     894         903         912         921         930         939
GTT GAA AAT CTC GAG ACT ACT ATG CGT TCT CCG GTT TTC ACT GAC AAC TCT TCT
Val Glu Asn Leu Glu Thr Thr MET Arg Ser Pro Val Phe Thr Asp Asn Ser Ser

     948         957         966         975         984         993
CCG CCG GTT GTT CCG CAG TCT TTC CAG GTT GCT CAC CTG CAT GCT CCG ACT GGT
Pro Pro Val Val Pro Gln Ser Phe Gln Val Ala His Leu His Ala Pro Thr Gly

    1002        1011        1020        1029        1038        1047
TCT GGT AAA TCT ACT AAA GTT CCA GCT GCT TAC GCT GCT CAG GGT TAC AAA GTT
Ser Gly Lys Ser Thr Lys Val Pro Ala Ala Tyr Ala Ala Gln Gly Tyr Lys Val

    1056        1065        1074        1083        1092        1101
CTG GTT CTG AAC CCG TCT GTT GCT GCT ACT CTG GGT TTC GGC GCC TAC ATG TCT
Leu Val Leu Asn Pro Ser Val Ala Ala Thr Leu Gly Phe Gly Ala Tyr MET Ser

    1110        1119        1128        1137        1146        1155
AAA GCT CAC GGT ATC GAC CCG AAC ATT CGT ACT GGT GTA CGT ACT ATC ACT ACT
Lys Ala His Gly Ile Asp Pro Asn Ile Arg Thr Gly Val Arg Thr Ile Thr Thr
```

Figure 11 con't

```
  1164      1173      1182      1191      1200      1209
GGT TCT CCG ATC ACT TAC TCT ACT TAC GGT AAA TTC CTG GCT GAC GGT GGT TGC
Gly Ser Pro Ile Thr Tyr Ser Thr Tyr Gly Lys Phe Leu Ala Asp Gly Gly Cys

  1218      1227      1236      1245      1254      1263
TCT GGT GGT GCT TAC GAT ATC ATC ATC TGC GAC GAA TGC CAC TCT ACT GAC GCT
Ser Gly Gly Ala Tyr Asp Ile Ile Ile Cys Asp Glu Cys His Ser Thr Asp Ala

  1272      1281      1290      1299      1308      1317
ACT TCT ATC CTG GGT ATC GGT ACC GTT CTG GAC CAG GCT GAA ACT GCA GGT GCT
Thr Ser Ile Leu Gly Ile Gly Thr Val Leu Asp Gln Ala Glu Thr Ala Gly Ala

  1326      1335      1344      1353      1362      1371
CGT CTG GTT GTT CTG GCT ACT GCT ACT CCG CCG GGT TCT GTT ACT GTT CCG CAC
Arg Leu Val Val Leu Ala Thr Ala Thr Pro Pro Gly Ser Val Thr Val Pro His

  1380      1389      1398      1407      1416      1425
CCG AAC ATC GAA GAA GTT GCT CTG TCG ACT ACT GGT GAA ATC CCG TTC TAC GGT
Pro Asn Ile Glu Glu Val Ala Leu Ser Thr Thr Gly Glu Ile Pro Phe Tyr Gly

  1434      1443      1452      1461      1470      1479
AAA GCT ATC CCG CTC GAG GTT ATC AAA GGT GGT CGT CAC CTG ATT TTC TGC CAC
Lys Ala Ile Pro Leu Glu Val Ile Lys Gly Gly Arg His Leu Ile Phe Cys His

  1488      1497      1506      1515      1524      1533
TCT AAA AAA AAA TGC GAC GAA CTG GCT GCT AAG CTT GTT GCT CTG GGT ATC AAC
Ser Lys Lys Lys Cys Asp Glu Leu Ala Ala Lys Leu Val Ala Leu Gly Ile Asn

  1542      1551      1560      1569      1578      1587
GCT GTT GCT TAC TAC CGT GGT CTG GAC GTT TCT GTT ATC CCG ACT TCT GGT GAC
Ala Val Ala Tyr Tyr Arg Gly Leu Asp Val Ser Val Ile Pro Thr Ser Gly Asp

  1596      1605      1614      1623      1632      1641
GTT GTT GTT GTG GCC ACT GAC GCT CTG ATG ACT GGT TAC ACT GGT GAC TTC GAC
Val Val Val Val Ala Thr Asp Ala Leu MET Thr Gly Tyr Thr Gly Asp Phe Asp

  1650      1659      1668      1677      1686      1695
TCT GTT ATC GAT TGC AAC ACT TGC AAT TCG TCG ACC GGT TGC GTT GTT ATC GTT
Ser Val Ile Asp Cys Asn Thr Cys Asn Ser Ser Thr Gly Cys Val Val Ile Val

  1704      1713      1722      1731      1740      1749
GGT CGT GTT GTT CTG TCT GGT AAA CCG GCC ATT ATC CCG GAC CGT GAA GTT CTG
Gly Arg Val Val Leu Ser Gly Lys Pro Ala Ile Ile Pro Asp Arg Glu Val Leu
```

Figure 11 con't

| 1758 | | | 1767 | | | 1776 | | | 1785 | | | 1794 | | | 1803 | | |
|---|---|---|---|---|---|---|---|---|---|---|---|---|---|---|---|---|---|
| TAC | CGT | GAG | TTC | GAC | GAA | ATG | GAA | GAA | TGC | TCT | CAG | CAC | CTG | CCG | TAC | ATC | GAA |
| Tyr | Arg | Glu | Phe | Asp | Glu | MET | Glu | Glu | Cys | Ser | Gln | His | Leu | Pro | Tyr | Ile | Glu |
| 1812 | | | 1821 | | | 1830 | | | 1839 | | | 1848 | | | 1857 | | |
| CAG | GGT | ATG | ATG | CTG | GCT | GAA | CAG | TTC | AAA | CAG | AAA | GCT | CTG | GGT | CTG | CTG | CAG |
| Gln | Gly | MET | MET | Leu | Ala | Glu | Gln | Phe | Lys | Gln | Lys | Ala | Leu | Gly | Leu | Leu | Gln |
| 1866 | | | 1875 | | | 1884 | | | 1893 | | | 1902 | | | 1911 | | |
| ACC | GCT | TCT | CGT | CAG | GCT | GAA | GTT | ATC | GCT | CCG | GCT | GTT | CAG | ACC | AAC | TGG | CAG |
| Thr | Ala | Ser | Arg | Gln | Ala | Glu | Val | Ile | Ala | Pro | Ala | Val | Gln | Thr | Asn | Trp | Gln |
| 1920 | | | 1929 | | | 1938 | | | 1947 | | | 1956 | | | 1965 | | |
| AAA | CTC | GAG | ACC | TTC | TGG | GCT | AAA | CAC | ATG | TGG | AAC | TTC | ATC | TCT | GGT | ATC | CAG |
| Lys | Leu | Glu | Thr | Phe | Trp | Ala | Lys | His | MET | Trp | Asn | Phe | Ile | Ser | Gly | Ile | Gln |
| 1974 | | | 1983 | | | 1992 | | | 2001 | | | 2010 | | | 2019 | | |
| TAC | CTG | GCT | GGT | CTG | TCT | ACC | CTG | CCG | GGT | AAC | CCG | GCT | ATC | GCA | AGC | TTG | ATG |
| Tyr | Leu | Ala | Gly | Leu | Ser | Thr | Leu | Pro | Gly | Asn | Pro | Ala | Ile | Ala | Ser | Leu | MET |
| 2028 | | | 2037 | | | 2046 | | | 2055 | | | 2064 | | | 2073 | | |
| GCT | TTC | ACC | GCT | GCT | GTT | ACC | TCT | CCG | CTG | ACC | ACC | TCT | CAG | ACC | CTG | CTG | TTC |
| Ala | Phe | Thr | Ala | Ala | Val | Thr | Ser | Pro | Leu | Thr | Thr | Ser | Gln | Thr | Leu | Leu | Phe |
| 2082 | | | 2091 | | | 2100 | | | 2109 | | | 2118 | | | 2127 | | |
| AAC | ATT | CTG | GGT | GGT | TGG | GTT | GCT | GCT | CAG | CTG | GCT | GCT | CCG | GGT | GCT | GCT | ACC |
| Asn | Ile | Leu | Gly | Gly | Trp | Val | Ala | Ala | Gln | Leu | Ala | Ala | Pro | Gly | Ala | Ala | Thr |
| 2136 | | | 2145 | | | 2154 | | | 2163 | | | 2172 | | | 2181 | | |
| GCT | TTC | GTT | GGT | GCT | GGT | CTG | GCT | GGT | GCT | GCT | ATC | GGT | TCT | GTA | GGC | CTG | GGT |
| Ala | Phe | Val | Gly | Ala | Gly | Leu | Ala | Gly | Ala | Ala | Ile | Gly | Ser | Val | Gly | Leu | Gly |
| 2190 | | | 2199 | | | 2208 | | | 2217 | | | 2226 | | | 2235 | | |
| AAA | GTT | CTG | ATC | GAC | ATT | CTG | GCT | GGT | TAC | GGT | GCT | GGT | GTT | GCT | GGA | GCT | CTG |
| Lys | Val | Leu | Ile | Asp | Ile | Leu | Ala | Gly | Tyr | Gly | Ala | Gly | Val | Ala | Gly | Ala | Leu |
| 2244 | | | 2253 | | | 2262 | | | 2271 | | | 2280 | | | 2289 | | |
| GTT | GCT | TTC | AAA | ATC | ATG | TCT | GGT | GAA | GTT | CCG | TCT | ACC | GAA | GAT | CTG | GTT | AAC |
| Val | Ala | Phe | Lys | Ile | MET | Ser | Gly | Glu | Val | Pro | Ser | Thr | Glu | Asp | Leu | Val | Asn |
| 2298 | | | 2307 | | | 2316 | | | 2325 | | | 2334 | | | 2343 | | |
| CTG | CTG | CCG | GCT | ATC | CTG | TCT | CCG | GGT | GCT | CTG | GTT | GTT | GGT | GTT | GTT | TGC | GCT |
| Leu | Leu | Pro | Ala | Ile | Leu | Ser | Pro | Gly | Ala | Leu | Val | Val | Gly | Val | Val | Cys | Ala |

Figure 11 con't

2352 2361 2370 2379 2388 2397
GCT ATC CTG CGT CGT CAC GTT GGC CCG GGT GAA GGT GCT GTT CAG TGG ATG AAC
Ala Ile Leu Arg Arg His Val Gly Pro Gly Glu Gly Ala Val Gln Trp MET Asn

2406 2415 2424 2433 2442 2451
CGT CTG ATC GCT TTC GCT TCT CGT GGT AAC CAC GTT TCT CCA TGG GAT CCT CTA
Arg Leu Ile Ala Phe Ala Ser Arg Gly Asn His Val Ser Pro Trp Asp Pro Leu

2460 2469 2485 2495 2505 2515
>
GAC TGC AGG CAT GCT AAG TAA GTAGATCTTG AGCGCGTTCG CGCTGAAATG CGCTAATTTC
Asp Cys Arg His Ala Lys

2525 2535 2545 2555 2565 2575 2585
ACTTCACGAC ACTTCAGCCA ATTTTGGGAG GAGTGTCGTA CCGTTACGAT TTTCCTCAAT TTTTCTTTTC

2595 2605 2615 2625 2635 2645 2655
AACAATTGAT CTCATTCAGG TGACATCTTT TATATTGGCG CTCATTATGA AAGCAGTAGC TTTTATGAGG

2665 2675 2685 2695 2705 2715 2725
GTAATCTGAA TGGAACAGCT GCGTGCCGAA TTAAGCCATT TACTGGGCGA AAAACTCAGT CGTATTGAGT

2735 2745 2755 2765 2775 2785 2795
GCGTCAATGA AAAAGCGGAT ACGGCGTTGT GGGCTTTGTA TGACAGCCAG GGAAACCCAA TGCCGTTAAT

2805 2815 2825 2835 2845 2855 2865
GGCAAGAAGC TTAGCCCGCC TAATGAGCGG GCTTTTTTTT CGACGCGAGG CTGGATGGCC TTCCCCATTA

2875 2885 2895 2905 2915 2925 2935
TGATTCTTCT CGCTTCCGGC GGCATCGGGA TGCCCGCGTT GCAGGCCATG CTGTCCAGGC AGGTAGATGA

2945 2955 2965 2975 2985 2995 3005
CGACCATCAG GGACAGCTTC AAGGATCGCT CGCGGCTCTT ACCAGCCTAA CTTCGATCAC TGGACCGCTG

3015 3025 3035 3045 3055 3065 3075
ATCGTCACGG CGATTTATGC CGCCTCGGCG AGCACATGGA ACGGGTTGGC ATGGATTGTA GGCGCCGCCC

3085 3095 3105 3115 3125 3135 3145
TATACCTTGT CTGCCTCCCC GCGTTGCGTC GCGGTGCATG GAGCCGGGCC ACCTCGACCT GAATGGAAGC

3155 3165 3175 3185 3195 3205 3215
CGGCGGCACC TCGCTAACGG ATTCACCACT CCAAGAATTG GAGCCAATCA ATTCTTGCGG AGAACTGTGA

3225 3235 3245 3255 3265 3275 3285
ATGCGCAAAC CAACCCTTGG CAGAACATAT CCATCGCGTC CGCCATCTCC AGCAGCCGCA CGCGGCGCAT

3295 3305 3315 3325 3335 3345 3355
CTCGGGCAGC GTTGGGTCCT GGCCACGGGT GCGCATGATC GTGCTCCTGT CGTTGAGGAC CCGGCTAGGC

3365 3375 3385 3395 3405 3415 3425
TGGCGGGGTT GCCTTACTGG TTAGCAGAAT GAATCACCGA TACGCGAGCG AACGTGAAGC GACTGCTGCT

3435 3445 3455 3465 3475 3485 3495
GCAAAACGTC TGCGACCTGA GCAACAACAT GAATGGTCTT CGGTTTCCGT GTTTCGTAAA GTCTGGAAAC

Figure 11 con't

| 3505 | 3515 | 3525 | 3535 | 3545 | 3555 | 3565 |
|---|---|---|---|---|---|---|
| GCGGAAGTCA | GCGCCCTGCA | CCATTATGTT | CCGGATCTGC | ATCGCAGGAT | GCTGCTGGCT | ACCCTGTGGA |
| 3575 | 3585 | 3595 | 3605 | 3615 | 3625 | 3635 |
| ACACCTACAT | CTGTATTAAC | GAAGCGCTTC | TTCCGCTTCC | TCGCTCACTG | ACTCGCTGCG | CTCGGTCGTT |
| 3645 | 3655 | 3665 | 3675 | 3685 | 3695 | 3705 |
| CGGCTGCGGC | GAGCGGTATC | AGCTCACTCA | AAGGCGGTAA | TACGGTTATC | CACAGAATCA | GGGGATAACG |
| 3715 | 3725 | 3735 | 3745 | 3755 | 3765 | 3775 |
| CAGGAAAGAA | CATGTGAGCA | AAAGGCCAGC | AAAAGGCCAG | GAACCGTAAA | AAGGCCGCGT | TGCTGGCGTT |
| 3785 | 3795 | 3805 | 3815 | 3825 | 3835 | 3845 |
| TTTCCATAGG | CTCCGCCCCC | CTGACGAGCA | TCACAAAAAT | CGACGCTCAA | GTCAGAGGTG | GCGAAACCCG |
| 3855 | 3865 | 3875 | 3885 | 3895 | 3905 | 3915 |
| ACAGGACTAT | AAAGATACCA | GGCGTTTCCC | CCTGGAAGCT | CCCTCGTGCG | CTCTCCTGTT | CCGACCCTGC |
| 3925 | 3935 | 3945 | 3955 | 3965 | 3975 | 3985 |
| CGCTTACCGG | ATACCTGTCC | GCCTTTCTCC | CTTCGGGAAG | CGTGGCGCTT | TCTCAATGCT | CACGCTGTAG |
| 3995 | 4005 | 4015 | 4025 | 4035 | 4045 | 4055 |
| GTATCTCAGT | TCGGTGTAGG | TCGTTCGCTC | CAAGCTGGGC | TGTGTGCACG | AACCCCCCGT | TCAGCCCGAC |
| 4065 | 4075 | 4085 | 4095 | 4105 | 4115 | 4125 |
| CGCTGCGCCT | TATCCGGTAA | CTATCGTCTT | GAGTCCAACC | CGGTAAGACA | CGACTTATCG | CCACTGGCAG |
| 4135 | 4145 | 4155 | 4165 | 4175 | 4185 | 4195 |
| CAGCCACTGG | TAACAGGATT | AGCAGAGCGA | GGTATGTAGG | CGGTGCTACA | GAGTTCTTGA | AGTGGTGGCC |
| 4205 | 4215 | 4225 | 4235 | 4245 | 4255 | 4265 |
| TAACTACGGC | TACACTAGAA | GGACAGTATT | TGGTATCTGC | GCTCTGCTGA | AGCCAGTTAC | CTTCGGAAAA |
| 4275 | 4285 | 4295 | 4305 | 4315 | 4325 | 4335 |
| AGAGTTGGTA | GCTCTTGATC | CGGCAAACAA | ACCACCGCTG | GTAGCGGTGG | TTTTTTTGTT | TGCAAGCAGC |
| 4345 | 4355 | 4365 | 4375 | 4385 | 4395 | 4405 |
| AGATTACGCG | CAGAAAAAAA | GGATCTCAAG | AAGATCCTTT | GATCTTTTCT | ACGGGGTCTG | ACGCTCAGTG |
| 4415 | 4425 | 4435 | 4445 | 4455 | 4465 | 4475 |
| GAACGAAAAC | TCACGTTAAG | GGATTTTGGT | CATGAGATTA | TCAAAAAGGA | TCTTCACCTA | GATCCTTTTA |
| 4485 | 4495 | 4505 | 4515 | 4525 | 4535 | 4545 |
| AATTAAAAAT | GAAGTTTTAA | ATCAATCTAA | AGTATATATG | AGTAAACTTG | GTCTGACAGT | TACCAATGCT |
| 4555 | 4565 | 4575 | 4585 | 4595 | 4605 | 4615 |
| TAATCAGTGA | GGCACCTATC | TCAGCGATCT | GTCTATTTCG | TTCATCCATA | GTTGCCTGAC | TCCCCGTCGT |
| 4625 | 4635 | 4645 | 4655 | 4665 | 4675 | 4685 |
| GTAGATAACT | ACGATACGGG | AGGGCTTACC | ATCTGGCCCC | AGTGCTGCAA | TGATACCGCG | AGACCCACGC |
| 4695 | 4705 | 4715 | 4725 | 4735 | 4745 | 4755 |
| TCACCGGCTC | CAGATTTATC | AGCAATAAAC | CAGCCAGCCG | GAAGGGCCGA | GCGCAGAAGT | GGTCCTGCAA |
| 4765 | 4775 | 4785 | 4795 | 4805 | 4815 | 4825 |
| CTTTATCCGC | CTCCATCCAG | TCTATTAATT | GTTGCCGGGA | AGCTAGAGTA | AGTAGTTCGC | CAGTTAATAG |

Figure 11 con't

```
      4835       4845       4855       4865       4875       4885       4895
TTTGCGCAAC GTTGTTGCCA TTGCTACAGG CATCGTGGTG TCACGCTCGT CGTTTGGTAT GGCTTCATTC

      4905       4915       4925       4935       4945       4955       4965
AGCTCCGGTT CCCAACGATC AAGGCGAGTT ACATGATCCC CCATGTTGTG CAAAAAAGCG GTTAGCTCCT

      4975       4985       4995       5005       5015       5025       5035
TCGGTCCTCC GATCGTTGTC AGAAGTAAGT TGGCCGCAGT GTTATCACTC ATGGTTATGG CAGCACTGCA

      5045       5055       5065       5075       5085       5095       5105
TAATTCTCTT ACTGTCATGC CATCCGTAAG ATGCTTTTCT GTGACTGGTG AGTACTCAAC CAAGTCATTC

      5115       5125       5135       5145       5155       5165       5175
TGAGAATAGT GTATGCGGCG ACCGAGTTGC TCTTGCCCGG CGTCAACACG GGATAATACC GCGCCACATA

      5185       5195       5205       5215       5225       5235       5245
GCAGAACTTT AAAAGTGCTC ATCATTGGAA AACGTTCTTC GGGGCGAAAA CTCTCAAGGA TCTTACCGCT

      5255       5265       5275       5285       5295       5305       5315
GTTGAGATCC AGTTCGATGT AACCCACTCG TGCACCCAAC TGATCTTCAG CATCTTTTAC TTTCACCAGC

      5325       5335       5345       5355       5365       5375       5385
GTTTCTGGGT GAGCAAAAAC AGGAAGGCAA AATGCCGCAA AAAAGGGAAT AAGGGCGACA CGGAAATGTT

      5395       5405       5415       5425       5435       5445       5455
GAATACTCAT ACTCTTCCTT TTTCAATATT ATTGAAGCAT TTATCAGGGT TATTGTCTCA TGAGCGGATA

      5465       5475       5485       5495       5505       5515       5525
CATATTTGAA TGTATTTAGA AAAATAAACA AATAGGGGTT CCGCGCACAT TTCCCCGAAA AGTGCCACCT

      5535       5545       5555       5565       5575       5585       5595
GACGTCTAAG AAACCATTAT TATCATGACA TTAACCTATA AAAATAGGCG TATCACGAGG CCCTTTCGTC

TTCAA
```

HCV CKS-33-BCD

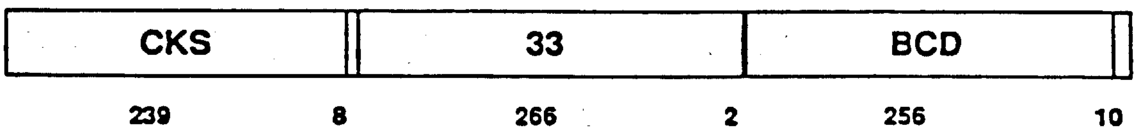


Recombinant Protein encoded by pHCV-31.

Figure 12.

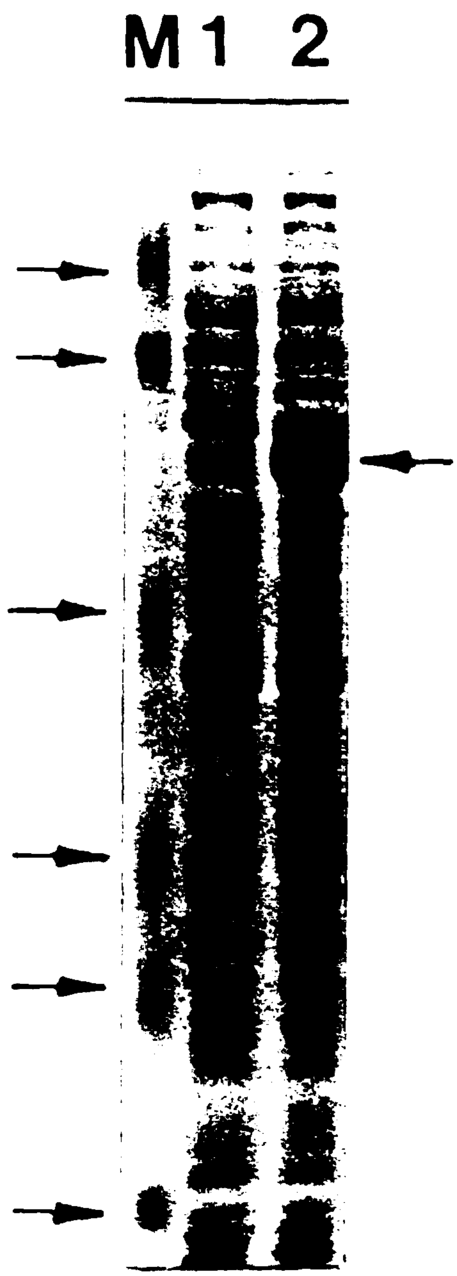


Figure 13

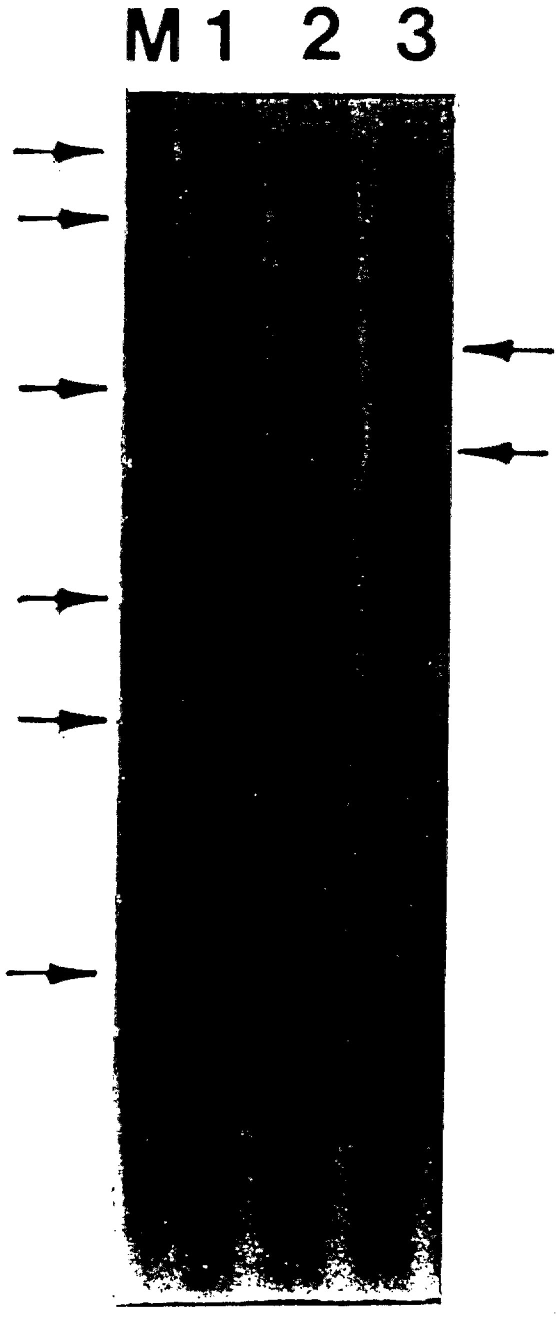


Figure 14.

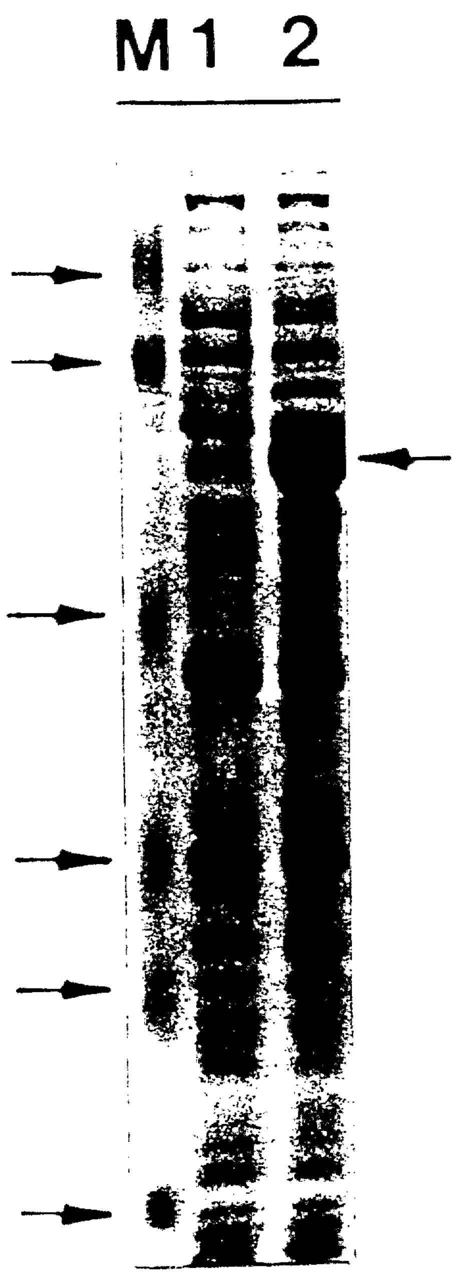


Figure 13

NANB Panel II (H. Alter, NIH)

| SAMPLE | Assay with C100-3<br>MANUAL S/CO | Assay with pHCV-31 pHCV-34<br>MANUAL S/CO | CONFIRMATORY RESULTS |
|---|---|---|---|
| 1 | >5.88 (+) | >5.65 (+) | + |
| 2 | 0.63 | 0.54 | |
| 3 | >5.88 (+) | >5.65 (+) | + |
| 4 | >5.88 (+) | >5.65 (+) | + |
| 5 | 0.43 | 0.46 | |
| 6 | >5.88 (+) | >5.65 (+) | + |
| 7 | 0.46 | 0.61 | |
| 8 | 0.41 | 0.70 | |
| 9 | 1.87 (+) | 1.83 (+) | + |
| 10 | 0.35 | 4.88 (+) | + |
| 11 | 0.48 | 0.49 | |
| 12 | 0.32 | 0.50 | |
| 13 | 0.48 | 0.83 | |
| 14 | 0.37 | 0.37 | |
| 15 | >5.88 (+) | >5.65 (+) | + |
| 16 | >5.88 (+) | >5.65 (+) | + |
| 17 | 0.34 | 0.44 | |
| 18 | 3.01 (+) | 2.33 (+) | + |
| 19 | 0.74 | 0.72 | |
| 20 | 0.53 | 0.76 | |
| 21 | >5.88 (+) | >5.65 (+) | + |
| 22 | 0.24 | 0.30 | |
| 23 | >5.88 (+) | >5.65 (+) | + |
| 24 | 0.69 | 0.84 | |
| 25 | 0.50 | 0.75 | |
| 26 | 3.41 (+) | 2.38 (+) | + |
| 27 | 0.62 | 0.82 | |
| 28 | 0.61 | 0.53 | |
| 29 | 0.34 | 4.94 (+) | + |
| 30 | 1.58 (+) | 1.85 (+) | + |
| 31 | 0.32 | 0.52 | |
| 32 | >5.88 (+) | >5.65 (+) | + |
| 33 | 0.45 | 0.58 | |

* Confirmatory testing was done with sp117, a synthetic peptide of 117 amino acids from within the immunodominant region of c100-3.

Figure 16

| | | | |
|---|---|---|---|
| 34 | >5.88 (+) | >5.65 (+) | + |
| 35 | >5.88 (+) | >5.65 (+) | + |
| 36 | 0.37 | 0.44 | |
| 37 | 0.40 | 0.40 | |
| 38 | >5.88 (+) | >5.65 (+) | + |
| 39* | 0.40 | 1.10 (+) | - |
| 40 | 0.53 | 0.63 | |
| 41 | 0.41 | 0.34 | |
| 42 | 0.52 | 0.70 | |
| 43 | 0.28 | 0.44 | |
| 44 | 0.44 | 0.70 | |

$$S/CO = \frac{\text{Sample OD}}{\text{Cutoff OD}}$$

S/CO = <1.0 is non-reactive

S/CO = ≥1.0 is reactive

*This specimen was negative when retested in duplicate. (S/CO values 0.56 and 0.51.)

Figure 16 con't

ANTIBODY TO HEPATITIS C REFERENCE (ANTI-HCV) PANEL #7

| Panel Member (Lot #) | Identity | Assay with C-100-3 | Assay with pHCV-31 and pHCV-34 | Ortho ELISA* | Confirmatory Results |
|---|---|---|---|---|---|
| | | Sample to Cutoff Values | | | |
| 701 | Weak Reactive | 1.819 (+) | 4.469 (+) | 1.239 (+) | + |
| 702 | Borderline Reactive | 1.711 (+) | 4.738 (+) | 1.130 (+) | + |
| 703 | Negative | 0.443 | 0.348 | 0.256 | - |
| 704 | Weak Reactive | 2.220 (+) | 4.738 (+) | 1.639 (+) | + |
| 705 | Borderline Reactive | 1.648 (+) | 1.736 (+) | 0.911 | + |
| 706 | Negative | 0.221 | 0.369 | 0.340 | - |
| 707 | Strong Reactive | 5.713 (+) | 4.738 (+) | 4.272 (+) | + |
| 708 | Strong Reactive | 5.713 (+) | 4.738 (+) | 4.272 (+) | + |
| 709 | Non-Reactive* | 0.401 | 0.533 | 0.650 | - |
| 710 | Non-Reactive* | 0.582 | 0.419 | 0.423 | - |

*Contains very low levels of anti-HCV. Not required to be detected by current HCV assays.

Figure 17

Figure 18.

Anti-HCV Results on Non-A, Non-B Hemodialysis Patients

| PATIENT # | DATE | ALT IU/L | Assay With C-100-3 | | Assay With pHCV-31, pHCV-34 | | CONFIRMATORY RESULTS |
|---|---|---|---|---|---|---|---|
| 1 | 10/28/85 | 474 | 0.30 | (-) | 2.12 | (+) | + |
| | 11/11/85 | 113 | 0.38 | (-) | 4.72 | (+) | + |
| | 12/03/85 | 86 | 3.13 | (+) | >5.65 | (+) | + |
| | 01/09/86 | 142 | >5.61 | (+) | NT | | NT |
| | 03/19/86 | 90 | >5.61 | (+) | >5.65 | (+) | + |
| | 09/30/86 | 25 | >5.61 | (+) | >6.67 | (+) | + |
| | | | | | | | |
| 2 | 09/14/87 | 217 | 5.02 | (+) | 5.84 | (+) | + |
| | 09/17/87 | 210 | >5.61 | (+) | 6.58 | (+) | + |
| | | | | | | | |
| 3 | 10/02/87 | 116 | 1.61 | (+) | 1.69 | (+) | + |
| | | | | | | | |
| 4 | 11/24/87 | NA | 0.41 | (-) | 2.13 | (+) | + |
| | 12/17/87 | NA | 0.47 | (-) | 1.27 | (+) | + |
| | 01/13/88 | NA | 0.46 | (-) | 1.56 | (+) | + |
| | 02/21/88 | NA | 0.34 | (-) | 1.45 | (+) | + |
| | | | | | | | |
| 7 | 10/02/85 | 298 | 0.79 | (-) | 2.94 | (+) | + |
| | 10/07/85 | 548 | 0.86 | (-) | 2.68 | (+) | + |
| | 10/23/85 | 334 | 2.06 | (+) | 2.32 | (+) | + |
| | | | | | | | |
| 10 | 01/25/89 | NA | 0.57 | (-) | 2.66 | (+) | + |
| | 02/01/89 | NA | 1.08 | (+) | 2.80 | (+) | + |
| | 02/08/89 | NA | 1.75 | (+) | 3.38 | (+) | + |
| | 02/23/89 | NA | 2.22 | (+) | 2.56 | (+) | + |
| | 03/01/89 | NA | 1.94 | (+) | 3.21 | (+) | + |
| | 03/08/89 | NA | 1.64 | (+) | 2.52 | (+) | + |
| | 03/22/89 | NA | 1.49 | (+) | 1.76 | (+) | + |
| | 04/12/89 | NA | 2.69 | (+) | 5.29 | (+) | + |
| | 04/26/89 | NA | 2.77 | (+) | >5.65 | (+) | + |
| | 05/17/89 | NA | 2.19 | (+) | 2.82 | (+) | + |
| | | | | | | | |
| 13 | 10/05/88 | NA | 0.31 | (-) | 0.51 | (-) | NT |
| | 10/19/88 | NA | 0.40 | (-) | 0.61 | (-) | NT |
| | 10/28/88 | NA | 0.33 | (-) | 0.53 | (-) | NT |
| | 11/09/88 | NA | 0.33 | (-) | 0.64 | (-) | NT |
| | 11/11/88 | NA | 0.37 | (-) | 0.66 | (-) | NT |

Figure 18 con't

| | | | | | | | |
|---|---|---|---|---|---|---|---|
| | 11/18/88 | NA | 0.42 | (-) | 0.57 | (-) | NT |
| | 11/25/88 | NA | 0.44 | (-) | 0.65 | (-) | NT |
| | 12/05/88 | NA | 0.51 | (-) | 0.74 | (-) | NT |
| | 12/16/88 | NA | 0.28 | (-) | 0.68 | (-) | NT |
| | 12/23/88 | NA | 0.29 | (-) | 0.64 | (-) | NT |
| | 01/04/89 | NA | 0.29 | (-) | 0.77 | (-) | NT |
| | 01/13/89 | NA | 0.33 | (-) | 1.11 | (+) | + |
| | 01/20/89 | NA | 0.30 | (-) | 1.11 | (+) | + |
| | 02/08/89 | NA | 0.26 | (-) | 1.81 | (+) | + |
| | 02/10/89 | NA | 0.26 | (-) | 1.88 | (+) | + |
| | 02/17/89 | NA | 0.26 | (-) | 2.23 | (+) | + |
| | 02/24/89 | NA | 0.28 | (-) | 3.75 | (+) | + |
| | 03/08/89 | NA | 0.28 | (-) | 5.25 | (+) | + |
| | 03/17/89 | NA | 0.22 | (-) | >5.65 | (+) | + |
| | 04/03/89 | NA | 0.26 | (-) | >5.65 | (+) | + |
| | 04/14/89 | NA | 0.26 | (-) | >5.65 | (+) | + |
| | 04/20/89 | NA | 0.29 | (-) | >5.65 | (+) | + |
| | 04/28/89 | NA | 0.31 | (-) | >5.65 | (+) | + |
| | 05/05/89 | NA | 0.28 | (-) | >5.65 | (+) | + |
| | 07/03/89 | NA | 0.23 | (-) | 5.32 | (+) | + |
| | | | | | | | |
| 17 | 10/05/88 | 1454 | 0.53 | (-) | 0.95 | (-) | NT |
| | 10/20/88 | 612 | 0.57 | (-) | 2.04 | (+) | + |
| | 10/28/88 | 576 | 0.56 | (-) | 1.26 | (+) | + |
| | 11/09/88 | 306 | 0.54 | (-) | 1.39 | (+) | + |
| | 11/11/88 | 321 | 0.73 | (-) | 1.34 | (+) | + |
| | 11/18/88 | 341 | 0.83 | (-) | 1.43 | (+) | + |
| | 11/25/88 | 333 | 0.73 | (-) | 1.83 | (+) | + |
| | 12/05/88 | 232 | 0.75 | (-) | 1.92 | (+) | + |
| | 12/16/88 | 239 | 0.81 | (-) | 2.75 | (+) | + |
| | 12/23/88 | 198 | 1.20 | (+) | 3.42 | (+) | + |
| | 01/13/89 | 146 | 3.17 | (+) | >5.65 | (+) | + |
| | 01/27/89 | 104 | 4.36 | (+) | >6.67 | (+) | + |
| | 02/17/89 | 113 | >5.61 | (+) | >6.67 | (+) | + |
| | 02/24/89 | 120 | >5.61 | (+) | >6.67 | (+) | + |
| | | | | | | | |
| 18 | 01/13/89 | 112 | >5.61 | (+) | >5.65 | (+) | + |
| | 01/21/89 | 72 | >5.61 | (+) | >5.65 | (+) | + |
| | 01/28/89 | 181 | >5.61 | (+) | >6.67 | (+) | + |
| | 02/08/89 | 106 | >5.61 | (+) | >5.65 | (+) | + |

Figure 18 con't

| | | | | | |
|---|---|---|---|---|---|
| | 02/18/89 | 82 | >5.61 (+) | >5.65 (+) | + |
| | 03/08/89 | 62 | >5.61 (+) | >5.65 (+) | + |
| | 03/18/89 | 41 | >5.61 (+) | NT | NT |
| | 03/25/89 | 37 | >5.61 (+) | >5.65 (+) | + |
| | 04/04/89 | 37 | >5.61 (+) | >5.65 (+) | + |
| | 04/15/89 | 35 | >5.61 (+) | >5.65 (+) | + |
| | 04/22/89 | 27 | >5.61 (+) | >5.65 (+) | + |
| | 04/29/89 | 24 | >5.61 (+) | >5.65 (+) | + |
| | 05/06/89 | 25 | >5.61 (+) | >5.65 (+) | + |
| | 07/03/89 | 31 | >5.61 (+) | >5.65 (+) | + |
| | | | | | |
| 19 | 02/17/89 | NA | 0.33 (-) | 0.75 (-) | NT |
| | 02/24/89 | NA | 0.35 (-) | 0.62 (-) | NT |
| | 03/08/89 | NA | 0.38 (-) | 0.69 (-) | NT |
| | 04/03/89 | NA | 0.13 (-) | 0.87 (-) | NT |
| | 04/14/89 | NA | 0.35 (-) | 1.07 (+) | + |
| | 04/21/89 | NA | 0.32 (-) | 1.54 (+) | + |
| | 04/28/89 | NA | 0.29 (-) | 1.04 (+) | + |
| | 05/05/89 | NA | 0.36 (-) | 1.16 (+) | + |
| | 07/03/89 | NA | 0.30 (-) | 1.24 (+) | + |
| | | | | | |

NT = Not Tested
NA = Not Available

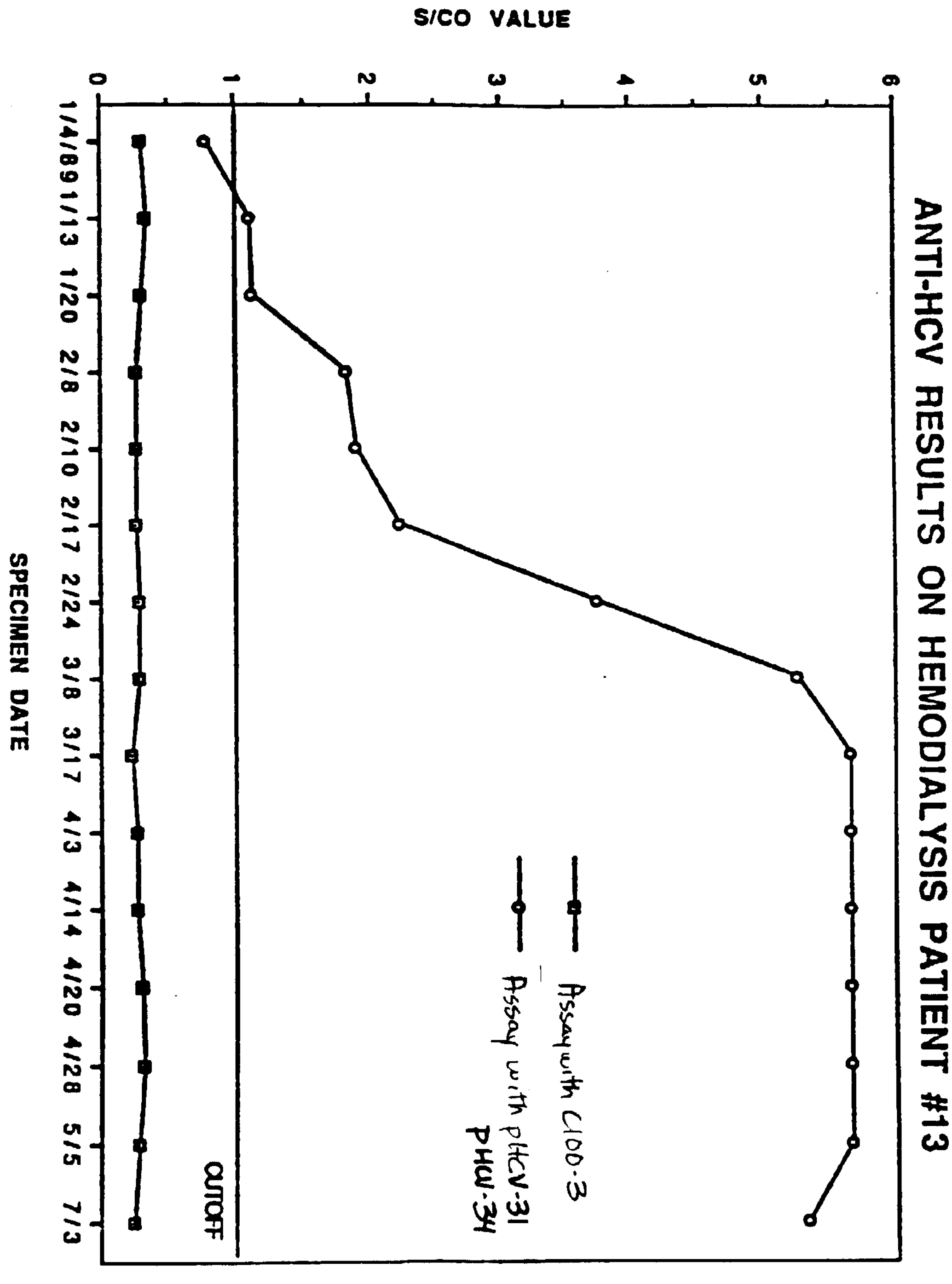
ANTI-HCV RESULTS ON HEMODIALYSIS PATIENT #13
S/CO VALUE
0
1
2
3
4
5
6
1/4/89
1/13
1/20
2/8
2/10
2/17
2/24
3/8
3/17
4/3
4/14
4/20
4/28
5/5
7/3
SPECIMEN DATE
CUTOFF
Assay with C100-3
Assay with pHCV-31
PHCV-34

Figure 19

COMPARISON OF 1ST AND 2ND GENERATION HCV ASSAYS ON SAMPLES FROM INDIVIDUALS WITH ACUTE NANBH.

| Category | No. Specimens | No. Specimens Repeatably Reactive by C-100-3 Assay | No. Confirmed | No. Specimens Repeatably Reactive by Assay with pHCV-31, pHCV-34 | No. Specimens Repeatably Reactive Which Confirmed (%) |
|---|---|---|---|---|---|
| Acute Post-Transfusion NANBH | 32 | 4 (12.50%) | 4 | 14* (43.75%) | 11/12** (91.67%) |
| Community Acquired NANBH (Acute) | 10 | 2 (20.00%) | 2 | 4 (40.00%) | 4 (100.00%) |

Figure 20

*1 specimen which was C-100-3 positive is just under the cutoff in the pHCV-31 pHCV-34. Assay.
** 2 samples were unavailble for confirmation.

CONFIRMATORY TESTING ON SAMPLES FOUND ADDITIONALLY REACTIVE BY THE ABBOTT HCV 2.0 EIA.

| CATEGORY | No. Specimens Found Additionally Reactive Assay pHCV-31, pHCV34 | No. Specimens Confirmed by sp67 Peptide | No. Specimens Confirmed by Core Peptide (sp75) | No. Specimens Confirmed by SOD-33c Antigen |
|---|---|---|---|---|
| Acute Post-Transfusion NANBH | 11 | 0 | 8* | 0 |
| Community Acquired NANBH (Acute) | 2 | 0 | 2 | ND** |

Figure 21

* 2 specimens not available for confirmation.
** Not Done

PREVALENCE OF ANTI-HCV IN CHRONIC NON-A, NON-B HEPATITIS (NANBH) PATIENTS

| Category | No. Tested | C-100-3 Assay Repeat Reactive | C-100-3 Assay Confirmed | pHCV-34 pHCV-31 Assay Repeat Reactive | pHCV-34 pHCV-31 Assay Confirmed |
|---|---|---|---|---|---|
| | | | | | |
| Chronic Active NANBH | 102 | 89 (87.3%) | 88 | 98 (96.1%) | 98 |
| Chronic Persistent NANBH | 10 | 9 (90.0%) | 9 | 9 (90.0%) | 9 |
| Chronic NANBH with Cirrhosis | 17 | 15 (88.2%) | 15 | 15 (88.2%) | 15 |
| Chronic NANBH (Undefined) | 35 | 25 (71.4%) | 25 | 33 (94.3%) | 33 |
| | | | | | |
| Total Chronic NANBH | 164 | 138 (84.1%) | 137 | 155 (94.5%) | 155 |

Figure 22.

FIGURE 23

HCV POLYPEPTIDE SPOTTING CONDITIONS

| PLASMID/PROTEIN | ng/SPOT | SPOTTING BUFFER |
|---|---|---|
| c100 | 100-150 | 20mM Tris-HCl, 0.9% NaCl, 0.015% SDS, pH 8.3 |
| pHCV-23/CKS-BCD | 100-150 | 20mM Tris-HCl, 0.9% NaCl, 0.015% SDS, pH 8.3 |
| pHCV-29/CKS-33c | 100-150 | 50mM Naphosphate, 0.01% Triton X100, pH 6.5 |
| pHCV-34/CKS-CORE | 75-100 | 50mM Naphosphate, 0.0025% Tween20, pH12.0 |

FIGURE 24

| | REFLECTANCE DENSITY VALUES | | LIMITING DILUTION | |
|---|---|---|---|---|
| ANTIGEN | NEGATIVE MEAN | CUTOFF | A00642 | 423 |
| c100-3 | 0.023 | 0.129 | 1600 | 40 |
| pHCV-23 | 0.011 | 0.050 | 3200 | 320 |
| pHCV-29 | 0.005 | 0.031 | 12800 | 2560 |
| pHCV-34 | 0.027 | 0.166 | 400 | 320 |

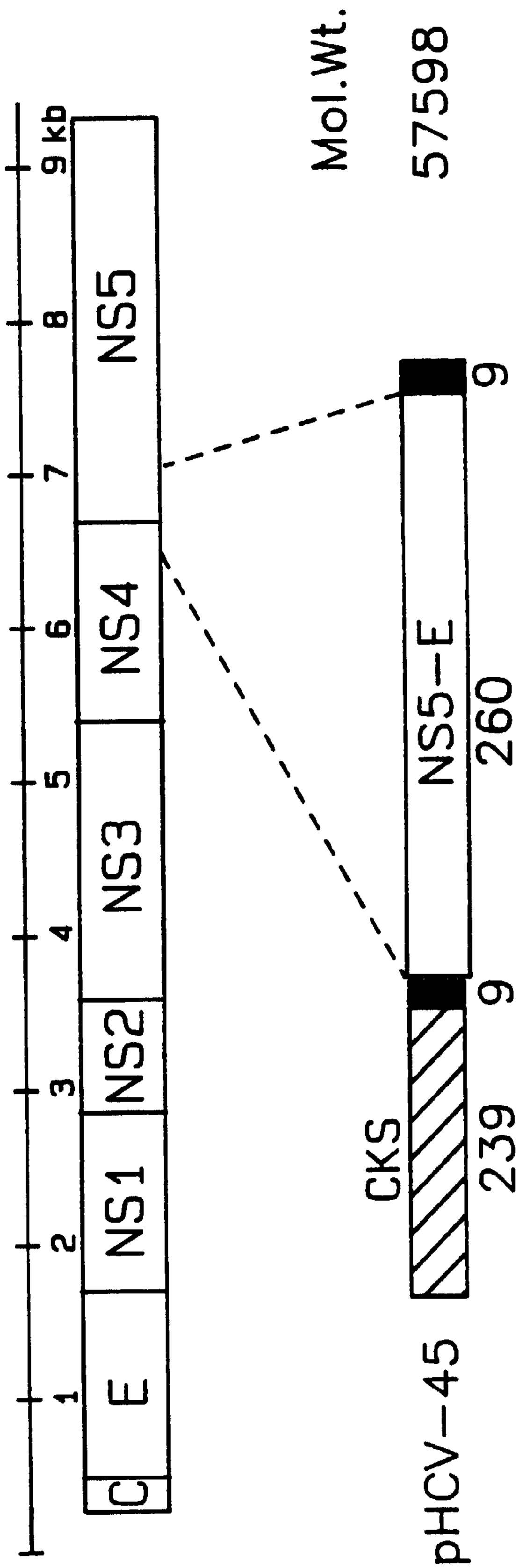
Mol.Wt.
57598
9 kb
8
7
6
5
4
3
2
1
NS5
NS4
NS3
NS2
NS1
E
C
NS5—E
9
260
9
CKS
239
pHCV—45

FIGURE 25

PHCV-45
Limits: 130 1680
Circular sequence with junction at 4805

```
                                156                             183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                             237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                             291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                             345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                             399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                             453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                             507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                             561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                             615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                             669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                             723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 26

```
                                750                                777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                804                                831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                858                                885
GAT ACC CCT GAA GAT CTC GAC CCG TCG ACG AAT TCC CCA TGG ACC CAC TAC GTT
Asp Thr Pro Glu Asp Leu Asp Pro Ser Thr Asn Ser Pro Trp Thr His Tyr Val

                                912                                939
CCG GAA TCT GAC GCT GCT GCT CGA GTT ACC GCT ATC CTG TCT TCT CTG ACC GTT
Pro Glu Ser Asp Ala Ala Ala Arg Val Thr Ala Ile Leu Ser Ser Leu Thr Val

                                966                                993
ACC CAG CTT CTG CGT CGT CTG CAC CAG TGG ATC TCT TCT GAA TGC ACC ACC CCG
Thr Gln Leu Leu Arg Arg Leu His Gln Trp Ile Ser Ser Glu Cys Thr Thr Pro

                               1020                               1047
TGC TCT GGT TCT TGG CTG CGT GAC ATC TGG GAC TGG ATC TGC GAA GTT CTG TCT
Cys Ser Gly Ser Trp Leu Arg Asp Ile Trp Asp Trp Ile Cys Glu Val Leu Ser

                               1074                               1101
GAC TTC AAA ACC TGG CTG AAA GCT AAA CTG ATG CCG CAG CTG CCG GGT ATC CCG
Asp Phe Lys Thr Trp Leu Lys Ala Lys Leu MET Pro Gln Leu Pro Gly Ile Pro

                               1128                               1155
TTC GTT TCT TGC CAG CGT GGT TAC AAA GGT GTT TGG CGT GTT GAC GGT ATC ATG
Phe Val Ser Cys Gln Arg Gly Tyr Lys Gly Val Trp Arg Val Asp Gly Ile MET

                               1182                               1209
CAC ACC CGT TGC CAC TGC GGT GCT GAA ATC ACC GGT CAC GTT AAA AAC GGT ACC
His Thr Arg Cys His Cys Gly Ala Glu Ile Thr Gly His Val Lys Asn Gly Thr

                               1236                               1263
ATG CGT ATC GTT GGT CCG CGT ACC TGC CGT AAC ATG TGG TCT GGC ACC TTC CCG
MET Arg Ile Val Gly Pro Arg Thr Cys Arg Asn MET Trp Ser Gly Thr Phe Pro

                               1290                               1317
ATC AAC GCT TAC ACC ACC GGT CCG TGC ACC CCG CTG CCG GCT CCG AAC TAC ACC
Ile Asn Ala Tyr Thr Thr Gly Pro Cys Thr Pro Leu Pro Ala Pro Asn Tyr Thr

                               1344                               1371
TTC GCT CTG TGG CGT GTT TCT GCT GAA GAA TAC GTT GAA ATC CGT CAG GTT GGT
Phe Ala Leu Trp Arg Val Ser Ala Glu Glu Tyr Val Glu Ile Arg Gln Val Gly
```

FIGURE 26(cont

```
                                            1398                                 1425
GAC TTC CAC TAC GTT ACC GGT ATG ACC ACC GAC AAC CTG AAA TGC CCG TGC CAG
Asp Phe His Tyr Val Thr Gly MET Thr Thr Asp Asn Leu Lys Cys Pro Cys Gln

                                            1452                                 1479
GTT CCG TCT CCG GAG TTC TTC ACC GAA CTG GAC GGT GTT CGT CTG CAC CGT TTC
Val Pro Ser Pro Glu Phe Phe Thr Glu Leu Asp Gly Val Arg Leu His Arg Phe

                                            1506                                 1533
GCT CCG CCG TGC AAA CCG CTG CTG CGT GAA GAA GTT TCT TTC CGT GTT GGT CTG
Ala Pro Pro Cys Lys Pro Leu Leu Arg Glu Glu Val Ser Phe Arg Val Gly Leu

                                            1560                                 1587
CAC GAA TAC CCG GTT GGT TCT CAG CTG CCG TGC GAA CCG GAA CCG GAC GTT GCT
His Glu Tyr Pro Val Gly Ser Gln Leu Pro Cys Glu Pro Glu Pro Asp Val Ala

                                            1614                                 1641
GTT CTG ACC TCT ATG CTG ACC GAC CCG TCT CAC ATC ACC GCT GAA GCT GCT GGT
Val Leu Thr Ser MET Leu Thr Asp Pro Ser His Ile Thr Ala Glu Ala Ala Gly

                                            1668
CGT CGA CTG GAT CCT CTA GAC TGC AGG CAT GCT AAG TAA
Arg Arg Leu Asp Pro Leu Asp Cys Arg His Ala Lys  .
```

TRANSLATE:

FIGURE 26 (cont)

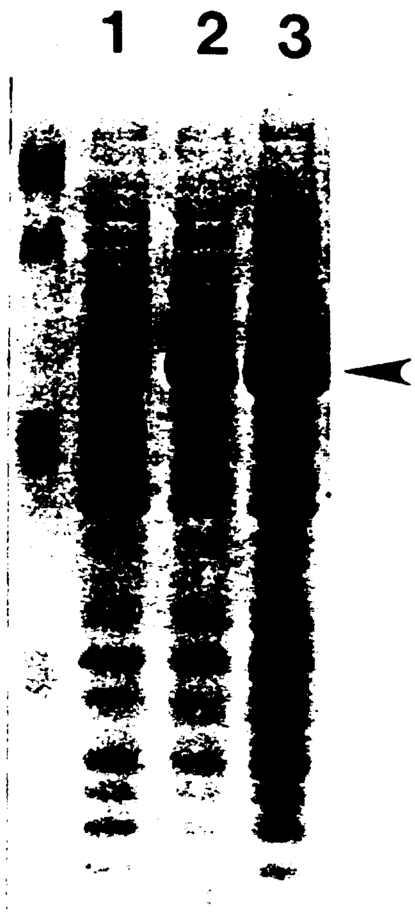


Figure 27

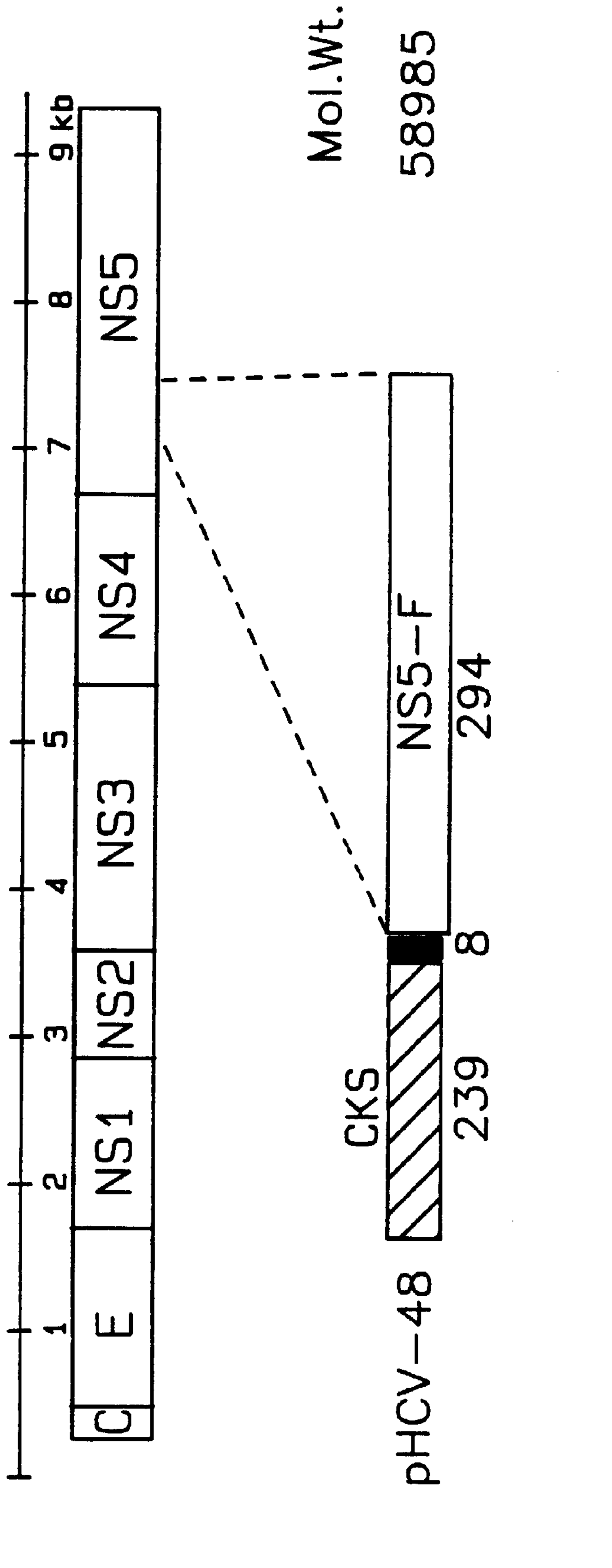
1
2
3
4
5
6
7
8
9 kb
C
E
NS1
NS2
NS3
NS4
NS5
Mol.Wt.
58985
NS5-F
294
8
CKS
239
pHCV-48

FIGURE 28

PHCV-48
Limits: 130 1755
Circular sequence with junction at 4910

```
                                156                                  183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                  237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                  291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                  345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                  399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                  453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                  507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                                  561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                  615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                  669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                  723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 29

```
                                          750                                777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                          804                                831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                          858                                885
GAT ACC CCT GAA GAT CTC GAC CCG TCG ACG AAT TCT ATG CGT CGA CTG GCT CGT
Asp Thr Pro Glu Asp Leu Asp Pro Ser Thr Asn Ser MET Arg Arg Leu Ala Arg

                                          912                                939
GGT TCT CCG CCG TCT GTT GCT TCT TCT TCT GCT TCT CAA CTG TCT GCT CCG TCT
Gly Ser Pro Pro Ser Val Ala Ser Ser Ser Ala Ser Gln Leu Ser Ala Pro Ser

                                          966                                993
CTG AAA GCT ACC TGC ACC GCT AAC CAC GAC TCT CCG GAC GCT GAA CTG ATC GAA
Leu Lys Ala Thr Cys Thr Ala Asn His Asp Ser Pro Asp Ala Glu Leu Ile Glu

                                          1020                               1047
GCT AAC CTG CTG TGG CGT CAG GAA ATG GGT GGT AAC ATC ACC CGT GTT GAA TCT
Ala Asn Leu Leu Trp Arg Gln Glu MET Gly Gly Asn Ile Thr Arg Val Glu Ser

                                          1074                               1101
GAA AAC AAA GTT GTT ATC CTG GAC TCT TTC GAC CCG CTG GTT GCT GAA GAA GAC
Glu Asn Lys Val Val Ile Leu Asp Ser Phe Asp Pro Leu Val Ala Glu Glu Asp

                                          1128                               1155
GAA CGT GAG ATC TCT GTT CCG GCT GAA ATC CTG CGT AAA TCT CGT CGT TTC GCT
Glu Arg Glu Ile Ser Val Pro Ala Glu Ile Leu Arg Lys Ser Arg Arg Phe Ala

                                          1182                               1209
CAG GCT CTG CCG GTT TGG GCT CGT CCG GAC TAC AAC CCG CCG CTG GTT GAA ACC
Gln Ala Leu Pro Val Trp Ala Arg Pro Asp Tyr Asn Pro Pro Leu Val Glu Thr

                                          1236                               1263
TGG AAA AAA CCG GAC TAC GAA CCG CCG GTT GTT CAC GGT TGC CCG CTG CCG CCG
Trp Lys Lys Pro Asp Tyr Glu Pro Pro Val Val His Gly Cys Pro Leu Pro Pro

                                          1290                               1317
CCG AAA TCT CCG CCG GTT CCG CCG CCG CGT AAA AAA CGT ACC GTT GTT CTG ACC
Pro Lys Ser Pro Pro Val Pro Pro Pro Arg Lys Lys Arg Thr Val Val Leu Thr

                                          1344                               1371
GAA TCT ACC CTG TCT ACC GCT CTG GCT GAA CTG GCT ACC CGT TCT TTC GGT TCT
Glu Ser Thr Leu Ser Thr Ala Leu Ala Glu Leu Ala Thr Arg Ser Phe Gly Ser
```

FIGURE 29 (cont)

```
                                1398                                  1425
TCT TCT ACC TCG GGT ATC ACC GGT GAC AAC ACC ACC ACC TCT TCT GAA CCG GCT
Ser Ser Thr Ser Gly Ile Thr Gly Asp Asn Thr Thr Thr Ser Ser Glu Pro Ala

                                1452                                  1479
CCG TCT GGT TGC CCG CCG GAC TCT GAC GCT GAA TCT TAC TCT TCT ATG CCG CCG
Pro Ser Gly Cys Pro Pro Asp Ser Asp Ala Glu Ser Tyr Ser Ser MET Pro Pro

                                1506                                  1533
CTG GAA GGT GAA CCG GGT GAC CCG GAT CTG TCT GAC GGT TCT TGG TCT ACC GTT
Leu Glu Gly Glu Pro Gly Asp Pro Asp Leu Ser Asp Gly Ser Trp Ser Thr Val

                                1560                                  1587
TCT TCT GAA GCT AAC GCT GAA GAC GTT GTT TGC TGC TCT ATG TCT TAC TCT TGG
Ser Ser Glu Ala Asn Ala Glu Asp Val Val Cys Cys Ser MET Ser Tyr Ser Trp

                                1614                                  1641
ACC GGT GCT CTG GTT ACT CCG TGC GCT GCT GAA GAA CAG AAA CTG CCG ATC AAC
Thr Gly Ala Leu Val Thr Pro Cys Ala Ala Glu Glu Gln Lys Leu Pro Ile Asn

                                1668                                  1695
GCT CTG TCT AAC TCT CTG CTG CGT CAC CAC AAC CTG GTT TAC TCT ACC ACC TCT
Ala Leu Ser Asn Ser Leu Leu Arg His His Asn Leu Val Tyr Ser Thr Thr Ser

                                1722                                  1749
CGT TCT GCT TGC CAG CGT CAG AAA AAA GTT ACC TTC GAC CGT CTG CAA GTT CTA
Arg Ser Ala Cys Gln Arg Gln Lys Lys Val Thr Phe Asp Arg Leu Gln Val Leu

GAC TAG
Asp  .
```

TRANSLATE:

FIGURE 29 (cont)

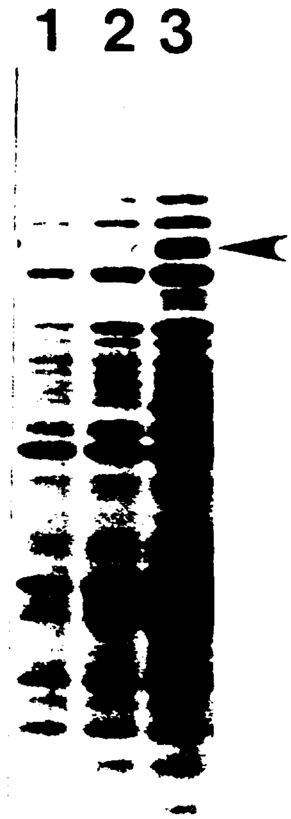


Figure 30

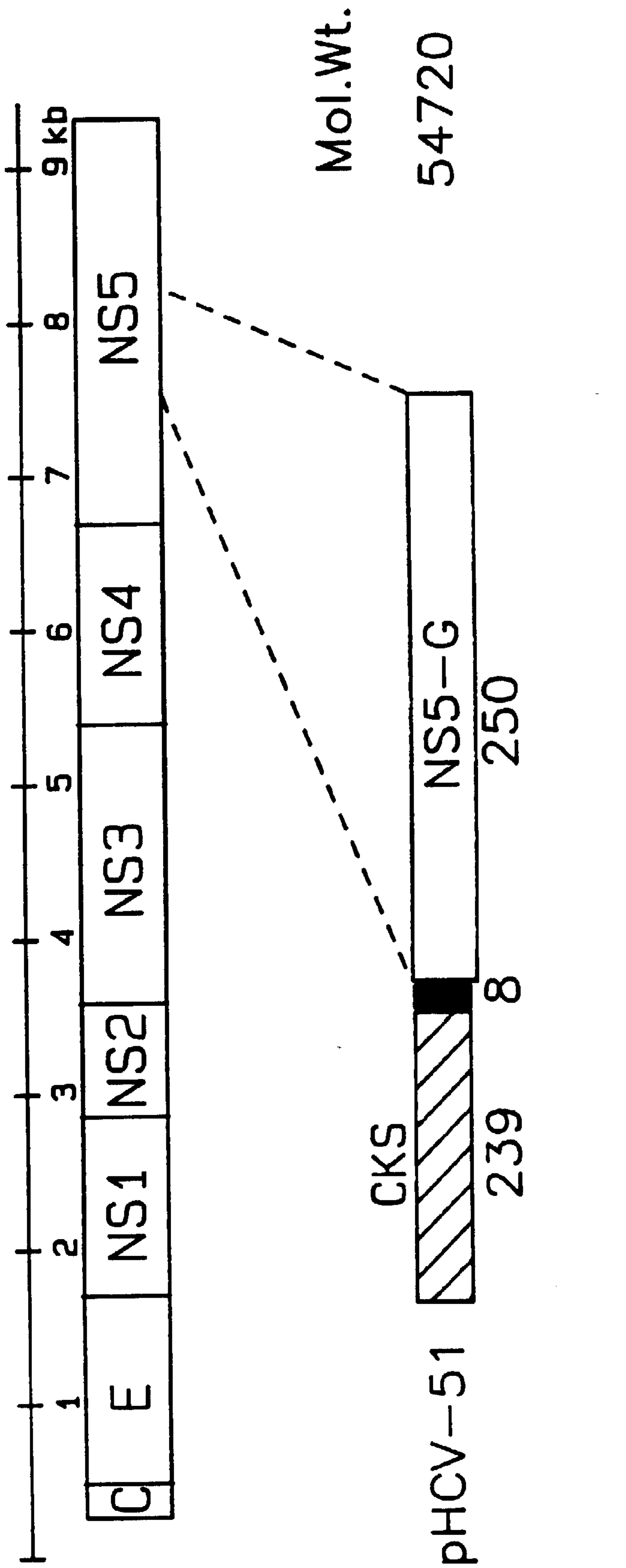
Mol.Wt.
54720
9 kb
8
7
6
5
4
3
2
1
C
E
NS1
NS2
NS3
NS4
NS5
NS5–G
250
8
CKS
239
pHCV–51

FIGURE 31

PHCV-51
Limits: 130 1620

```
                                156                                183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                                561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 32 (cont)

```
                                        750                             777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                        804                             831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                        858                             885
GAT ACC CCT GAA GAT CTC GAC CCG TCG ACG AAT TCT CTA GAC TCC CAC TAC CAG
Asp Thr Pro Glu Asp Leu Asp Pro Ser Thr Asn Ser Leu Asp Ser His Tyr Gln

                                        912                             939
GAC GTT CTG AAA GAA GTT AAA GCT GCT GCT TCT AAA GTT AAA GCT AAC CTG CTG
Asp Val Leu Lys Glu Val Lys Ala Ala Ala Ser Lys Val Lys Ala Asn Leu Leu

                                        966                             993
TCT GTT GAA GAA GCA TGC TCT CTG ACC CCG CCG CAC TCT GCT AAA TCT AAA TTC
Ser Val Glu Glu Ala Cys Ser Leu Thr Pro Pro His Ser Ala Lys Ser Lys Phe

                                       1020                            1047
GGT TAC GGT GCT AAA GAC GTT CGT TGC CAC GCT CGT AAA GCT GTT ACC CAC ATC
Gly Tyr Gly Ala Lys Asp Val Arg Cys His Ala Arg Lys Ala Val Thr His Ile

                                       1074                            1101
AAC TCT GTT TGG AAA GAT CTG CTG GAA GAC AAC GTT ACC CCG ATC GAC ACC ACC
Asn Ser Val Trp Lys Asp Leu Leu Glu Asp Asn Val Thr Pro Ile Asp Thr Thr

                                       1128                            1155
ATC ATG GCT AAA AAC GAA GTT TTC TGC GTT CAG CCG GAA AAA GGT GGT CGT AAA
Ile MET Ala Lys Asn Glu Val Phe Cys Val Gln Pro Glu Lys Gly Gly Arg Lys

                                       1182                            1209
CCG GCT CGT CTG ATC GTT TTC CCG GAC CTG GGT GTT CGT GTT TGC GAA AAA ATG
Pro Ala Arg Leu Ile Val Phe Pro Asp Leu Gly Val Arg Val Cys Glu Lys MET

                                       1236                            1263
GCT CTG TAC GAC GTT GTT ACC AAA CTG CCG CTG GCT GTT ATG GGT TCT TCT TAC
Ala Leu Tyr Asp Val Val Thr Lys Leu Pro Leu Ala Val MET Gly Ser Ser Tyr

                                       1290                            1317
GGT TTC CAG TAC TCT CCG GGT CAG CGT GTT GAG TTC CTG GTT CAG GCT TGG AAA
Gly Phe Gln Tyr Ser Pro Gly Gln Arg Val Glu Phe Leu Val Gln Ala Trp Lys

                                       1344                            1371
TCT AAA AAA ACC CCG ATG GGT TTC TCT TAC GAC ACC CGT TGC TTC GAC TCT ACC
Ser Lys Lys Thr Pro MET Gly Phe Ser Tyr Asp Thr Arg Cys Phe Asp Ser Thr
```

FIGURE 32 (cont)

```
                                        1398                                      1425
GTT ACC GAA TCT GAC ATT CGT ACC GAA GAA GCT ATC TAC CAG TGC TGC GAC CTG
Val Thr Glu Ser Asp Ile Arg Thr Glu Glu Ala Ile Tyr Gln Cys Cys Asp Leu

                                        1452                                      1479
GAC CCG CAG GCT CGT GTT GCT ATC AAA TCT CTG ACC GAA CGT CTG TAC GTT GGT
Asp Pro Gln Ala Arg Val Ala Ile Lys Ser Leu Thr Glu Arg Leu Tyr Val Gly

                                        1506                                      1533
GGT CCG CTG ACC AAC TCT CGG GGT GAA AAC TGC GGT TAC CGT CGT TGC CGT GCT
Gly Pro Leu Thr Asn Ser Arg Gly Glu Asn Cys Gly Tyr Arg Arg Cys Arg Ala

                                        1560                                      1587
TCT GGT GTT CTG ACC ACC TCT TGC GGT AAC ACC CTG ACC TGC TAC ATC AAA GCT
Ser Gly Val Leu Thr Thr Ser Cys Gly Asn Thr Leu Thr Cys Tyr Ile Lys Ala

                                        1614
CGT GCT GCT TGC CGT GCT GCT GGT CTG CAG TAA
Arg Ala Ala Cys Arg Ala Ala Gly Leu Gln  .
```

TRANSLATE:

FIGURE 32

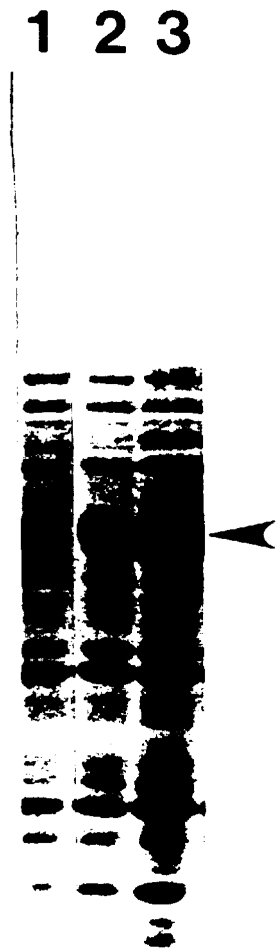


Figure 33

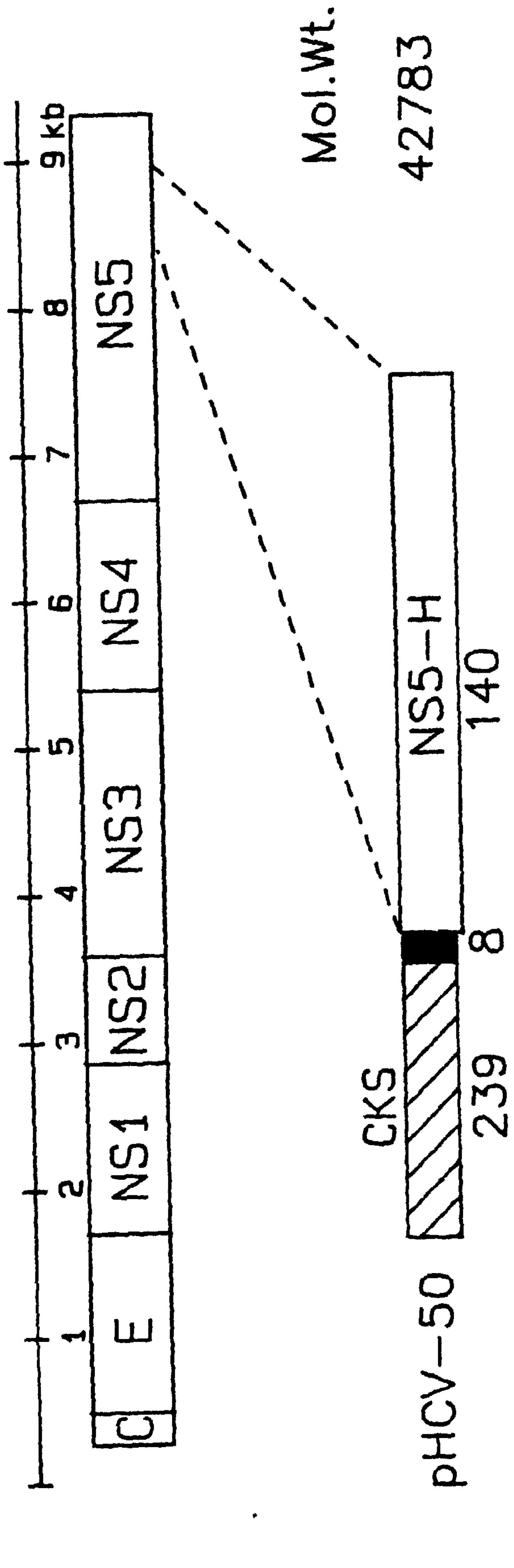
Mol.Wt.
42783
9 kb
8
7
6
5
4
3
2
1
C
E
NS1
NS2
NS3
NS4
NS5
NS5–H
140
8
CKS
239
pHCV–50

FIGURE 34

PHCV-50
Limits: 130 1293

```
                                156                                183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                                561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 35

```
                                            750                          777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                            804                          831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                            858                          885
GAT ACC CCT GAA GAT CTC GAC CCG TCG ACG AAT TGC ATG CTG CAG GAC TGC ACC
Asp Thr Pro Glu Asp Leu Asp Pro Ser Thr Asn Cys MET Leu Gln Asp Cys Thr

                                            912                          939
ATG CTG GTT TGC GGT GAC GAC CTG GTT GTT ATC TGC GAA TCT GCT GGT GTT CAG
MET Leu Val Cys Gly Asp Asp Leu Val Val Ile Cys Glu Ser Ala Gly Val Gln

                                            966                          993
GAA GAC GCT GCT TCT CTG CGT GCT TTC ACC GAA GCT ATG ACC CGT TAC TCT GCT
Glu Asp Ala Ala Ser Leu Arg Ala Phe Thr Glu Ala MET Thr Arg Tyr Ser Ala

                                           1020                         1047
CCC CCG GGT GAC CCG CCG CAG CCG GAA TAC GAC CTG GAA CTG ATC ACC TCT TGC
Pro Pro Gly Asp Pro Pro Gln Pro Glu Tyr Asp Leu Glu Leu Ile Thr Ser Cys

                                           1074                         1101
TCT TCT AAC GTT TCT GTT GCT CAC GAC GGT GCT GGT AAA CGT GTT TAC TAC CTG
Ser Ser Asn Val Ser Val Ala His Asp Gly Ala Gly Lys Arg Val Tyr Tyr Leu

                                           1128                         1155
ACC CGT GAC CCG ACC ACC CCG CTG GCT CGT GCT GCT TGG GAA ACC GCT CGT CAC
Thr Arg Asp Pro Thr Thr Pro Leu Ala Arg Ala Ala Trp Glu Thr Ala Arg His

                                           1182                         1209
ACC CCG GTA AAC TCT TGG CTG GGT AAC ATC ATC ATG TTC GCT CCG ACC CTG TGG
Thr Pro Val Asn Ser Trp Leu Gly Asn Ile Ile MET Phe Ala Pro Thr Leu Trp

                                           1236                         1263
GCC CGT ATG ATC CTG ATG ACC CAC TTC TTC TCT GTT CTG ATC GCT CGT GAC CAG
Ala Arg MET Ile Leu MET Thr His Phe Phe Ser Val Leu Ile Ala Arg Asp Gln

                                           1290
CTG GAA CAG GCT CTG GAC TGC GAG ATC TAA
Leu Glu Gln Ala Leu Asp Cys Glu Ile  .
```

TRANSLATE:

FIGURE 35 (cont)

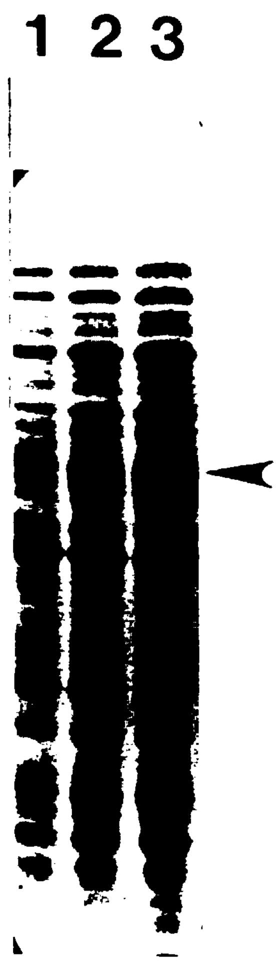


Figure 36

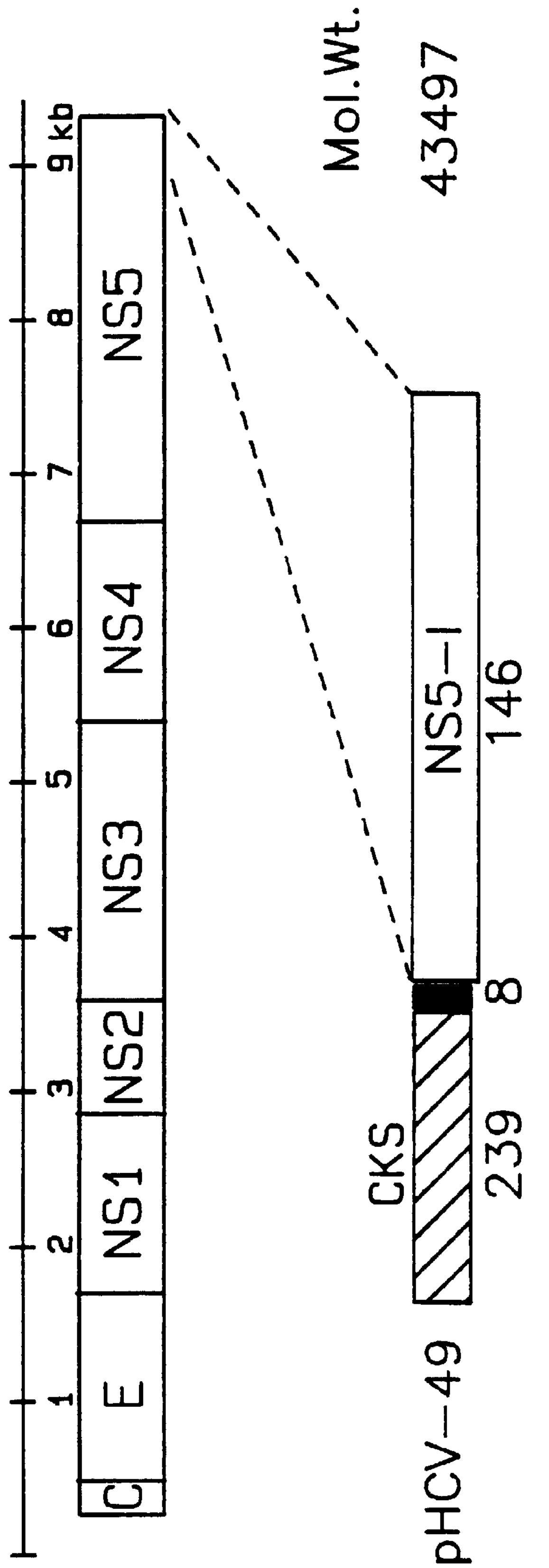

1
2
3
4
5
6
7
8
9 kb
C
E
NS1
NS2
NS3
NS4
NS5
Mol.Wt.
43497
pHCV–49
CKS
239
8
NS5–I
146


FIGURE 37

PHCV-49
Limits: 130 1311
Circular sequence with junction at 4472

```
                                156                                 183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                 237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                 291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                 345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                 399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                 453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                 507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                                 561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                 615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                 669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                 723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 38

```
                                    750                                 777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                    804                                 831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                    858                                 885
GAT ACC CCT GAA GAT CTC GAC CCG TCG ACG AAT TCC ATG GAG ATC TAC GGT GCT
Asp Thr Pro Glu Asp Leu Asp Pro Ser Thr Asn Ser MET Glu Ile Tyr Gly Ala

                                    912                                 939
TGC TAC TCT ATC GAA CCG CTG GAC CTG CCG CCG ATC ATT CAG CGT CTG CAC GGT
Cys Tyr Ser Ile Glu Pro Leu Asp Leu Pro Pro Ile Ile Gln Arg Leu His Gly

                                    966                                 993
CTG TCT GCT TTC TCT CTG CAC TCT TAC TCC CCG GGT GAA ATC AAC CGT GTT GCT
Leu Ser Ala Phe Ser Leu His Ser Tyr Ser Pro Gly Glu Ile Asn Arg Val Ala

                                   1020                                1047
GCT TGC CTG CGT AAA CTG GGT GTT CCG CCG CTG CGT GCT TGG CGT CAC CGT GCT
Ala Cys Leu Arg Lys Leu Gly Val Pro Pro Leu Arg Ala Trp Arg His Arg Ala

                                   1074                                1101
CGT TCT GTT CGT GCT CGT CTG CTG GCT CGT GGT GGC CGT GCT GCT ATC TGC GGT
Arg Ser Val Arg Ala Arg Leu Leu Ala Arg Gly Gly Arg Ala Ala Ile Cys Gly

                                   1128                                1155
AAA TAC CTG TTC AAC TGG GCT GTT CGT ACC AAA CTG AAA CTG ACC CCG ATC GCT
Lys Tyr Leu Phe Asn Trp Ala Val Arg Thr Lys Leu Lys Leu Thr Pro Ile Ala

                                   1182                                1209
GCT GCT GGT CAG CTG GAC CTG TCT GGT TGG TTC ACC GCT GGT TAC TCT GGT GGT
Ala Ala Gly Gln Leu Asp Leu Ser Gly Trp Phe Thr Ala Gly Tyr Ser Gly Gly

                                   1236                                1263
GAC ATC TAC CAC TCT GTT TCT CAC GCT CGT CCG CGT TGG ATC TGG TTC TGC CTG
Asp Ile Tyr His Ser Val Ser His Ala Arg Pro Arg Trp Ile Trp Phe Cys Leu

                                   1290
CTG CTG CTG GCT GCT GGT GTT GGT ATC TAC CTG CTG CCG AAC CGT TAA
Leu Leu Leu Ala Ala Gly Val Gly Ile Tyr Leu Leu Pro Asn Arg  .
```

TRANSLATE:

FIGURE 38 (cont)

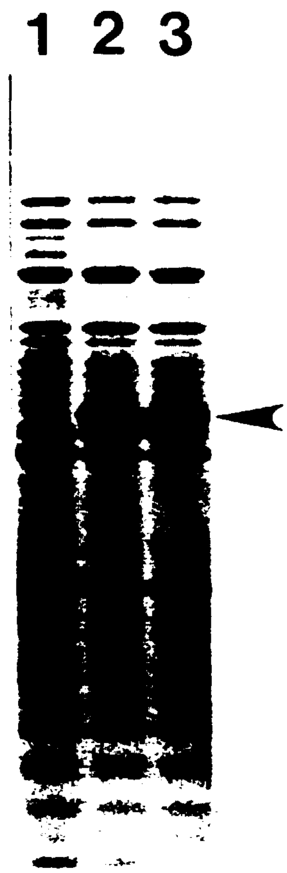


Figure 39

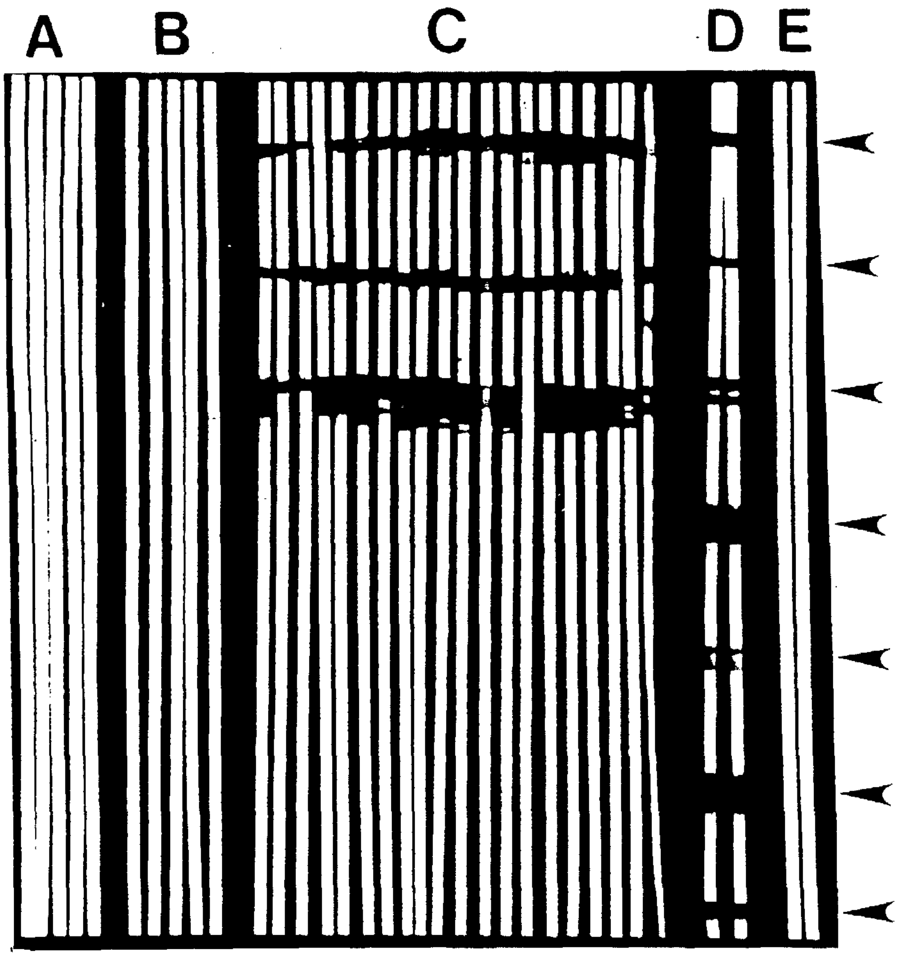


Figure 40

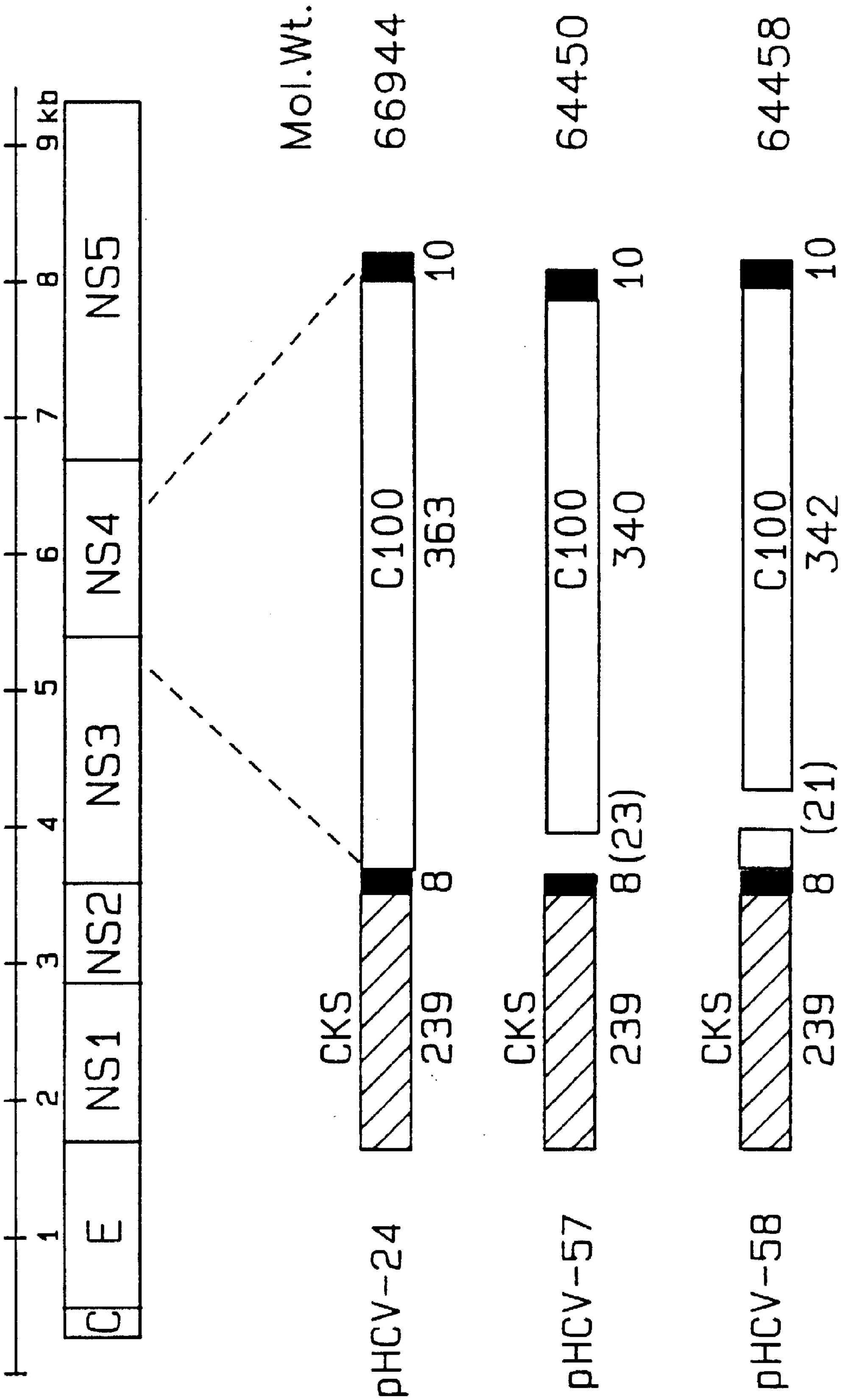
1
2
3
4
5
6
7
8
9 kb
C
E
NS1
NS2
NS3
NS4
NS5
Mol.Wt.
pHCV-24
CKS
239
8
C100
363
10
66944
pHCV-57
CKS
239
8(23)
C100
340
10
64450
pHCV-58
CKS
239
8
(21)
C100
342
10
64458

FIGURE 41

PHCV-57
Limits: 130 1923
Circular sequence with junction at 5048

```
                                        156                                183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                        210                                237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                        264                                291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                        318                                345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                        372                                399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                        426                                453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                        480                                507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                        534                                561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                        588                                615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                        642                                669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                        696                                723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 42

```
                                                750                                                777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                                804                                                831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                                858                                                885
GAT ACC CCT GAA GAT CTC GAC CCG TCG ACG AAT TCC ATG GAC GCT CAC TTC CTG
Asp Thr Pro Glu Asp Leu Asp Pro Ser Thr Asn Ser MET Asp Ala His Phe Leu

                                                912                                                939
TCT CAG GCG CCG CCG CCG TCT TGG GAT CAG ATG TGG AAA TGC CTG ATC CGT CTG
Ser Gln Ala Pro Pro Pro Ser Trp Asp Gln MET Trp Lys Cys Leu Ile Arg Leu

                                                966                                                993
AAA CCG ACC CTG CAC GGC CCG ACC CCG CTG CTG TAC CGT CTG GGT GCT GTT CAG
Lys Pro Thr Leu His Gly Pro Thr Pro Leu Leu Tyr Arg Leu Gly Ala Val Gln

                                               1020                                               1047
AAC GAA ATC ACC CTG ACC CAC CCG GTT ACC AAA TAC ATC ATG ACC TGC ATG TCT
Asn Glu Ile Thr Leu Thr His Pro Val Thr Lys Tyr Ile MET Thr Cys MET Ser

                                               1074                                               1101
GCT GAT CTA GAA GTT GTT ACC TCT ACC TGG GTT CTG GTT GGT GGT GTT CTG GCT
Ala Asp Leu Glu Val Val Thr Ser Thr Trp Val Leu Val Gly Gly Val Leu Ala

                                               1128                                               1155
GCT CTG GCT GCT TAC TGC CTG TCG ACC GGT TGC GTT GTT ATC GTT GGT CGT GTT
Ala Leu Ala Ala Tyr Cys Leu Ser Thr Gly Cys Val Val Ile Val Gly Arg Val

                                               1182                                               1209
GTT CTG TCT GGT AAA CCG GCC ATT ATC CCG GAC CGT GAA GTT CTG TAC CGT GAG
Val Leu Ser Gly Lys Pro Ala Ile Ile Pro Asp Arg Glu Val Leu Tyr Arg Glu

                                               1236                                               1263
TTC GAC GAA ATG GAA GAA TGC TCT CAG CAC CTG CCG TAC ATC GAA CAG GGT ATG
Phe Asp Glu MET Glu Glu Cys Ser Gln His Leu Pro Tyr Ile Glu Gln Gly MET

                                               1290                                               1317
ATG CTG GCT GAA CAG TTC AAA CAG AAA GCT CTG GGT CTG CTG CAG ACC GCT TCT
MET Leu Ala Glu Gln Phe Lys Gln Lys Ala Leu Gly Leu Leu Gln Thr Ala Ser

                                               1344                                               1371
CGT CAG GCT GAA GTT ATC GCT CCG GCT GTT CAG ACC AAC TGG CAG AAA CTC GAG
Arg Gln Ala Glu Val Ile Ala Pro Ala Val Gln Thr Asn Trp Gln Lys Leu Glu
```

FIGURE 42 (cont)

```
                                  1398                                 1425
ACC TTC TGG GCT AAA CAC ATG TGG AAC TTC ATC TCT GGT ATC CAG TAC CTG GCT
Thr Phe Trp Ala Lys His MET Trp Asn Phe Ile Ser Gly Ile Gln Tyr Leu Ala

                                  1452                                 1479
GGT CTG TCT ACC CTG CCG GGT AAC CCG GCT ATC GCA AGC TTG ATG GCT TTC ACC
Gly Leu Ser Thr Leu Pro Gly Asn Pro Ala Ile Ala Ser Leu MET Ala Phe Thr

                                  1506                                 1533
GCT GCT GTT ACC TCT CCG CTG ACC ACC TCT CAG ACC CTG CTG TTC AAC ATT CTG
Ala Ala Val Thr Ser Pro Leu Thr Thr Ser Gln Thr Leu Leu Phe Asn Ile Leu

                                  1560                                 1587
GGT GGT TGG GTT GCT GCT CAG CTG GCT GCT CCG GGT GCT GCT ACC GCT TTC GTT
Gly Gly Trp Val Ala Ala Gln Leu Ala Ala Pro Gly Ala Ala Thr Ala Phe Val

                                  1614                                 1641
GGT GCT GGT CTG GCT GGT GCT GCT ATC GGT TCT GTA GGC CTG GGT AAA GTT CTG
Gly Ala Gly Leu Ala Gly Ala Ala Ile Gly Ser Val Gly Leu Gly Lys Val Leu

                                  1668                                 1695
ATC GAC ATT CTG GCT GGT TAC GGT GCT GGT GTT GCT GGA GCT CTG GTT GCT TTC
Ile Asp Ile Leu Ala Gly Tyr Gly Ala Gly Val Ala Gly Ala Leu Val Ala Phe

                                  1722                                 1749
AAA ATC ATG TCT GGT GAA GTT CCG TCT ACC GAA GAT CTG GTT AAC CTG CTG CCG
Lys Ile MET Ser Gly Glu Val Pro Ser Thr Glu Asp Leu Val Asn Leu Leu Pro

                                  1776                                 1803
GCT ATC CTG TCT CCG GGT GCT CTG GTT GTT GGT GTT GTT TGC GCT GCT ATC CTG
Ala Ile Leu Ser Pro Gly Ala Leu Val Val Gly Val Val Cys Ala Ala Ile Leu

                                  1830                                 1857
CGT CGT CAC GTT GGC CCG GGT GAA GGT GCT GTT CAG TGG ATG AAC CGT CTG ATC
Arg Arg His Val Gly Pro Gly Glu Gly Ala Val Gln Trp MET Asn Arg Leu Ile

                                  1884                                 1911
GCT TTC GCT TCT CGT GGT AAC CAC GTT TCT CCA TGG GAT CCT CTA GAC TGC AGG
Ala Phe Ala Ser Arg Gly Asn His Val Ser Pro Trp Asp Pro Leu Asp Cys Arg

CAT GCT AAG TAA
His Ala Lys  .

Subcommand (<CR> = NONE):
```

FIGURE 42 (cont)

PHCV-58
Limits: 130 1929
Circular sequence with junction at 5054

```
                                        156                                         183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                        210                                         237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                        264                                         291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                        318                                         345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                        372                                         399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                        426                                         453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                        480                                         507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                        534                                         561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                        588                                         615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                        642                                         669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                        696                                         723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 43

```
                                        750                                777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                        804                                831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                        858                                885
GAT ACC CCT GAA GAT CTC GAC CCG TCG ACG AAT TCC ATG GAC GCT CAC TTC CTG
Asp Thr Pro Glu Asp Leu Asp Pro Ser Thr Asn Ser MET Asp Ala His Phe Leu

                                        912                                939
TCT CAG ACC AAA CAG TCT GGT GAA AAC CTT CCG TAC CTG GTT GCT TAC CAG GCT
Ser Gln Thr Lys Gln Ser Gly Glu Asn Leu Pro Tyr Leu Val Ala Tyr Gln Ala

                                        966                                993
ACC GTT TGC GCT CGT GCT CAG GCC CCG ACC CCG CTG CTG TAC CGT CTG GGT GCT
Thr Val Cys Ala Arg Ala Gln Ala Pro Thr Pro Leu Leu Tyr Arg Leu Gly Ala

                                       1020                               1047
GTT CAG AAC GAA ATC ACC CTG ACC CAC CCG GTT ACC AAA TAC ATC ATG ACC TGC
Val Gln Asn Glu Ile Thr Leu Thr His Pro Val Thr Lys Tyr Ile MET Thr Cys

                                       1074                               1101
ATG TCT GCT GAT CTA GAA GTT GTT ACC TCT ACC TGG GTT CTG GTT GGT GGT GTT
MET Ser Ala Asp Leu Glu Val Val Thr Ser Thr Trp Val Leu Val Gly Gly Val

                                       1128                               1155
CTG GCT GCT CTG GCT GCT TAC TGC CTG TCG ACC GGT TGC GTT GTT ATC GTT GGT
Leu Ala Ala Leu Ala Ala Tyr Cys Leu Ser Thr Gly Cys Val Val Ile Val Gly

                                       1182                               1209
CGT GTT GTT CTG TCT GGT AAA CCG GCC ATT ATC CCG GAC CGT GAA GTT CTG TAC
Arg Val Val Leu Ser Gly Lys Pro Ala Ile Ile Pro Asp Arg Glu Val Leu Tyr

                                       1236                               1263
CGT GAG TTC GAC GAA ATG GAA GAA TGC TCT CAG CAC CTG CCG TAC ATC GAA CAG
Arg Glu Phe Asp Glu MET Glu Glu Cys Ser Gln His Leu Pro Tyr Ile Glu Gln

                                       1290                               1317
GGT ATG ATG CTG GCT GAA CAG TTC AAA CAG AAA GCT CTG GGT CTG CTG CAG ACC
Gly MET MET Leu Ala Glu Gln Phe Lys Gln Lys Ala Leu Gly Leu Leu Gln Thr

                                       1344                               1371
GCT TCT CGT CAG GCT GAA GTT ATC GCT CCG GCT GTT CAG ACC AAC TGG CAG AAA
Ala Ser Arg Gln Ala Glu Val Ile Ala Pro Ala Val Gln Thr Asn Trp Gln Lys
```

FIGURE 43 (cont)

```
                                        1398                                         1425
CTC GAG ACC TTC TGG GCT AAA CAC ATG TGG AAC TTC ATC TCT GGT ATC CAG TAC
Leu Glu Thr Phe Trp Ala Lys His MET Trp Asn Phe Ile Ser Gly Ile Gln Tyr

                                        1452                                         1479
CTG GCT GGT CTG TCT ACC CTG CCG GGT AAC CCG GCT ATC GCA AGC TTG ATG GCT
Leu Ala Gly Leu Ser Thr Leu Pro Gly Asn Pro Ala Ile Ala Ser Leu MET Ala

                                        1506                                         1533
TTC ACC GCT GCT GTT ACC TCT CCG CTG ACC ACC TCT CAG ACC CTG CTG TTC AAC
Phe Thr Ala Ala Val Thr Ser Pro Leu Thr Thr Ser Gln Thr Leu Leu Phe Asn

                                        1560                                         1587
ATT CTG GGT GGT TGG GTT GCT GCT CAG CTG GCT GCT CCG GGT GCT GCT ACC GCT
Ile Leu Gly Gly Trp Val Ala Ala Gln Leu Ala Ala Pro Gly Ala Ala Thr Ala

                                        1614                                         1641
TTC GTT GGT GCT GGT CTG GCT GGT GCT GCT ATC GGT TCT GTA GGC CTG GGT AAA
Phe Val Gly Ala Gly Leu Ala Gly Ala Ala Ile Gly Ser Val Gly Leu Gly Lys

                                        1668                                         1695
GTT CTG ATC GAC ATT CTG GCT GGT TAC GGT GCT GGT GTT GCT GGA GCT CTG GTT
Val Leu Ile Asp Ile Leu Ala Gly Tyr Gly Ala Gly Val Ala Gly Ala Leu Val

                                        1722                                         1749
GCT TTC AAA ATC ATG TCT GGT GAA GTT CCG TCT ACC GAA GAT CTG GTT AAC CTG
Ala Phe Lys Ile MET Ser Gly Glu Val Pro Ser Thr Glu Asp Leu Val Asn Leu

                                        1776                                         1803
CTG CCG GCT ATC CTG TCT CCG GGT GCT CTG GTT GTT GGT GTT GTT TGC GCT GCT
Leu Pro Ala Ile Leu Ser Pro Gly Ala Leu Val Val Gly Val Val Cys Ala Ala

                                        1830                                         1857
ATC CTG CGT CGT CAC GTT GGC CCG GGT GAA GGT GCT GTT CAG TGG ATG AAC CGT
Ile Leu Arg Arg His Val Gly Pro Gly Glu Gly Ala Val Gln Trp MET Asn Arg

                                        1884                                         1911
CTG ATC GCT TTC GCT TCT CGT GGT AAC CAC GTT TCT CCA TGG GAT CCT CTA GAC
Leu Ile Ala Phe Ala Ser Arg Gly Asn His Val Ser Pro Trp Asp Pro Leu Asp


TGC AGG CAT GCT AAG TAA
Cys Arg His Ala Lys  .

Subcommand (<CR> = NONE):
```

FIGURE 43 (cont)

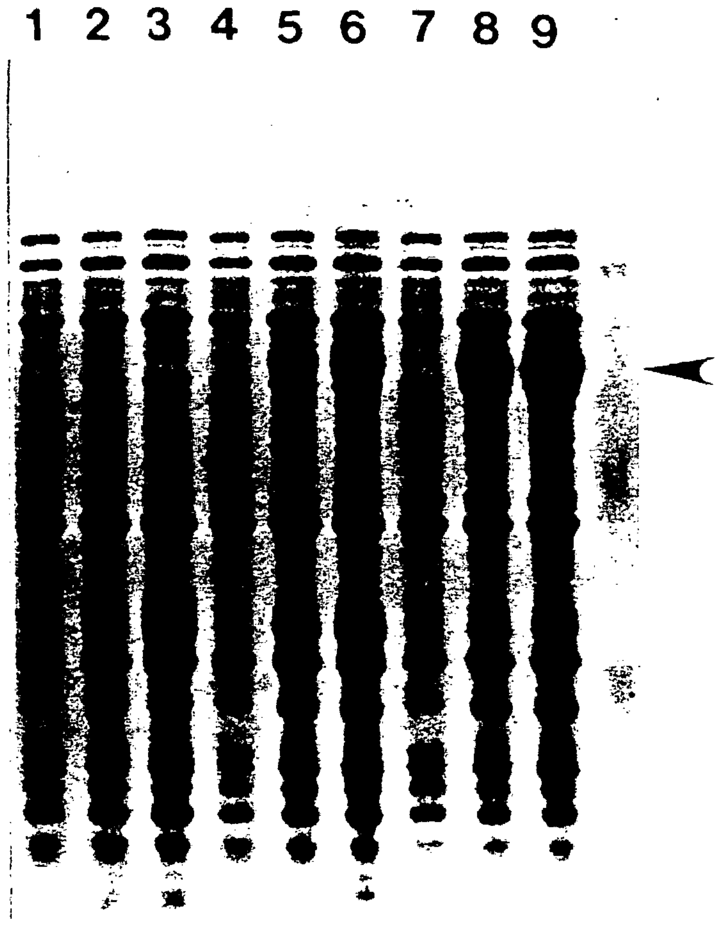


Figure 44

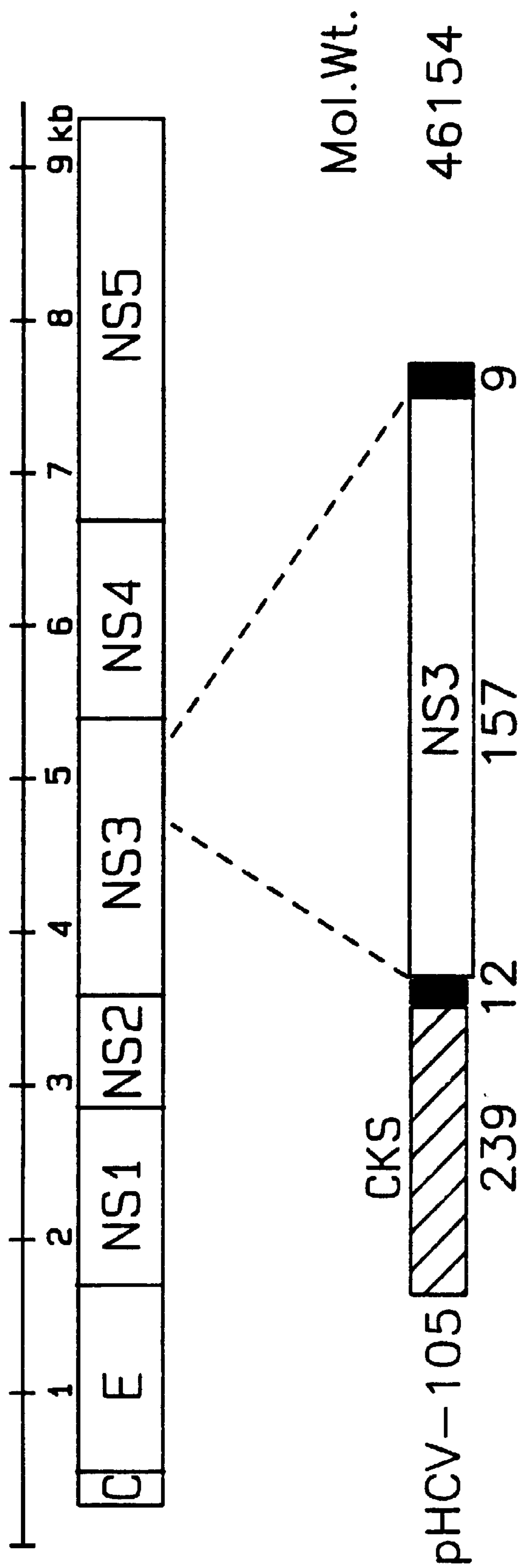
9 kb
8
7
6
5
4
3
2
1
C
E
NS1
NS2
NS3
NS4
NS5
Mol.Wt.
46154
pHCV-105
CKS
239
12
NS3
157
9

FIGURE 45

PHCV-105
Limits: 130 1383
Circular sequence with junction at 4513

```
                                156                                  183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                  237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                  291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                  345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                  399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                  453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                  507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                                  561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                  615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                  669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                  723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 46

```
                                     750                         777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                     804                         831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                     858                         885
GAT ACC CCT GAA GAT CTC GAC CCG TCG ACT CGA ATT CGA GCT CGG TAC CCT GAG
Asp Thr Pro Glu Asp Leu Asp Pro Ser Thr Arg Ile Arg Ala Arg Tyr Pro Glu

                                     912                         939
ACA ATC ACG CTT CCC CAG GAT GCT GTC TCC CGC ACC CAG CGT CGG GGC AGG ACT
Thr Ile Thr Leu Pro Gln Asp Ala Val Ser Arg Thr Gln Arg Arg Gly Arg Thr

                                     966                         993
GGC AGG GGG AAG CCA GGC ATC TAC AGA TTT GTG GCA CCG GGG GAG CGC CCT TCC
Gly Arg Gly Lys Pro Gly Ile Tyr Arg Phe Val Ala Pro Gly Glu Arg Pro Ser

                                    1020                        1047
GGC ATG TTC GAC TCG TCC GTC CTC TGC GAG TGC TAT GAC GCG GGC TGG CCT TGG
Gly MET Phe Asp Ser Ser Val Leu Cys Glu Cys Tyr Asp Ala Gly Trp Pro Trp

                                    1074                        1101
TAT GAG CTC ACA CCC GCC GAG ACC ACA GTT AGG CTA CGA GCG TAC ATG AAC ACC
Tyr Glu Leu Thr Pro Ala Glu Thr Thr Val Arg Leu Arg Ala Tyr MET Asn Thr

                                    1128                        1155
CCG GGA CTC CCC GTG TGC CAA GAC CAT CTT GAA TTT TGG GAG GGC GTC TTC ACG
Pro Gly Leu Pro Val Cys Gln Asp His Leu Glu Phe Trp Glu Gly Val Phe Thr

                                    1182                        1209
GGT CTC ACC CAT ATA GAC GCC CAC TTT CTA TCC CAG ACA AAG CAG AGT GGG GAA
Gly Leu Thr His Ile Asp Ala His Phe Leu Ser Gln Thr Lys Gln Ser Gly Glu

                                    1236                        1263
AAC CTT CCT TAC CTG GTA GCG TAC CAA GCC ACC GTG TGC GCT AGA GCT CAA GCC
Asn Leu Pro Tyr Leu Val Ala Tyr Gln Ala Thr Val Cys Ala Arg Ala Gln Ala

                                    1290                        1317
CCT CCC CCA TCG TGG GAC CAG ATG TGG AAG TGC TTG ATC CGC CTC AAG CCT ACC
Pro Pro Pro Ser Trp Asp Gln MET Trp Lys Cys Leu Ile Arg Leu Lys Pro Thr

                                    1344                        1371
CTT CAT GGG CCG ACC CCC CTG CTA TAC AGA CTG GGC GGG GGA TCC TCT AGA CTG
Leu His Gly Pro Thr Pro Leu Leu Tyr Arg Leu Gly Gly Gly Ser Ser Arg Leu

CAG GCA TGC TAA
Gln Ala Cys  .
```

FIGURE 46 (cont)

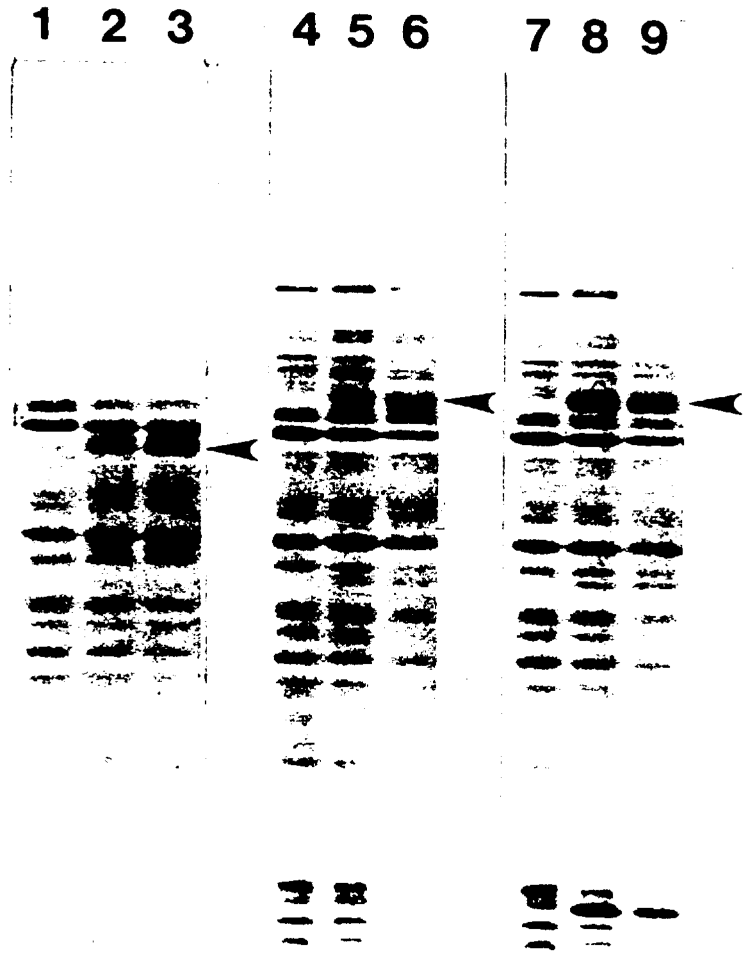


Figure 47

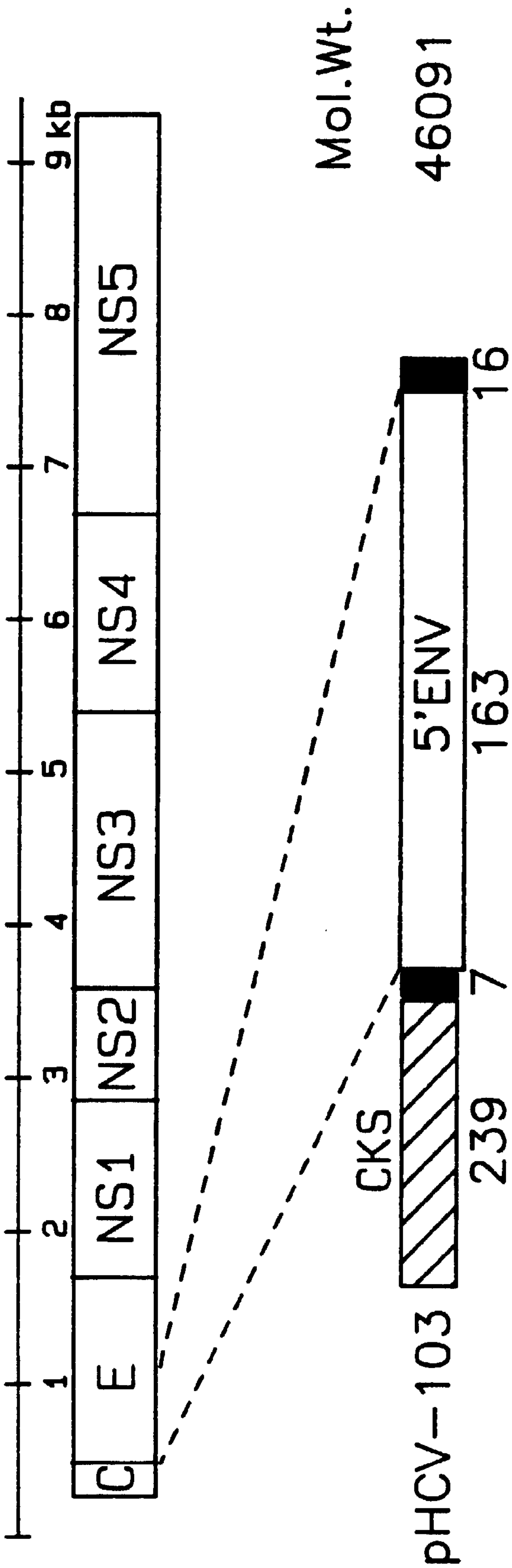
1
2
3
4
5
6
7
8
9 kb
C
E
NS1
NS2
NS3
NS4
NS5
Mol.Wt.
46091
pHCV-103
CKS
239
7
5'ENV
163
16

FIGURE 48

PHCV-103
Limits: 130 1407
Circular sequence with junction at 4533

```
                                156                                  183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                  237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                  291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                  345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                  399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                  453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                  507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                                  561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                  615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                  669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                  723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 49

```
                                  750                                  777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                  804                                  831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                  858                                  885
GAT ACC CCT GAA GAT CTC GAC CCG TCG ACT CGA ATT CGT AGG TCG CGC AAT TTG
Asp Thr Pro Glu Asp Leu Asp Pro Ser Thr Arg Ile Arg Arg Ser Arg Asn Leu

                                  912                                  939
GGT AAG GTC ATC GAC ACC CTC ACG TGC GGC TTC GCC GAC CTC ATG GGG TAT ATT
Gly Lys Val Ile Asp Thr Leu Thr Cys Gly Phe Ala Asp Leu MET Gly Tyr Ile

                                  966                                  993
CCG CTC GTC GGC GCC CCT CTT GGA GGC GCT GCC AGG GCC CTG GGC CAT GGC GTC
Pro Leu Val Gly Ala Pro Leu Gly Gly Ala Ala Arg Ala Leu Gly His Gly Val

                                 1020                                 1047
CGG GTT CTG GAA GAC GGC GTG AAC TAT GCG ACA GGG AAT CTT CCT GGT TGC TCT
Arg Val Leu Glu Asp Gly Val Asn Tyr Ala Thr Gly Asn Leu Pro Gly Cys Ser

                                 1074                                 1101
TTC TCT ATC TTC CTT CTG GCC CTG CTC TCT TGC CTG ACC GTG CCC GCA TCA GCC
Phe Ser Ile Phe Leu Leu Ala Leu Leu Ser Cys Leu Thr Val Pro Ala Ser Ala

                                 1128                                 1155
TAC CAA GTA CGC AAC TCC TCG GGC CTT TAC CAT GTC ACC AAT GAT TGC CCC AAC
Tyr Gln Val Arg A Ser Ser Gly Leu Tyr His Val Thr Asn Asp Cys Pro Asn

                                 1182                                 1209
TCG AGT ATT GTG TAC GAG ACG GCC GAT GCC ATC CTG CAC ACT CCG GGG TGC GTC
Ser Ser Ile Val Tyr Glu Thr Ala Asp Ala Ile Leu His Thr Pro Gly Cys Val

                                 1236                                 1263
CCT TGC GTT CGT GAG GGC AAC GCC TCG AGA TGT TGG GTG GCG GTG GCC CCC ACA
Pro Cys Val Arg Glu Gly Asn Ala Ser Arg Cys Trp Val Ala Val Ala Pro Thr

                                 1290                                 1317
GTG GCC ACC AGG GAT GGA AAA CTC CCC GCA ACG CAG CTT CGA CGT CAC ATT GAT
Val Ala Thr Arg Asp Gly Lys Leu Pro Ala Thr Gln Leu Arg Arg His Ile Asp

                                 1344                                 1371
CTG CTT GTC GGG AGC GCC ACC CTC TGT TCG GCC CTC TAC TTA AGG AGC TCG GTA
Leu Leu Val Gly Ser Ala Thr Leu Cys Ser Ala Leu Tyr Leu Arg Ser Ser Val

                                 1398
CCC GGG GAT CCT CTA GAC TGC AGG CAT GCT AAG TAA
Pro Gly Asp Pro Leu Asp Cys Arg His Ala Lys  .
```

FIGURE 49 (continued)

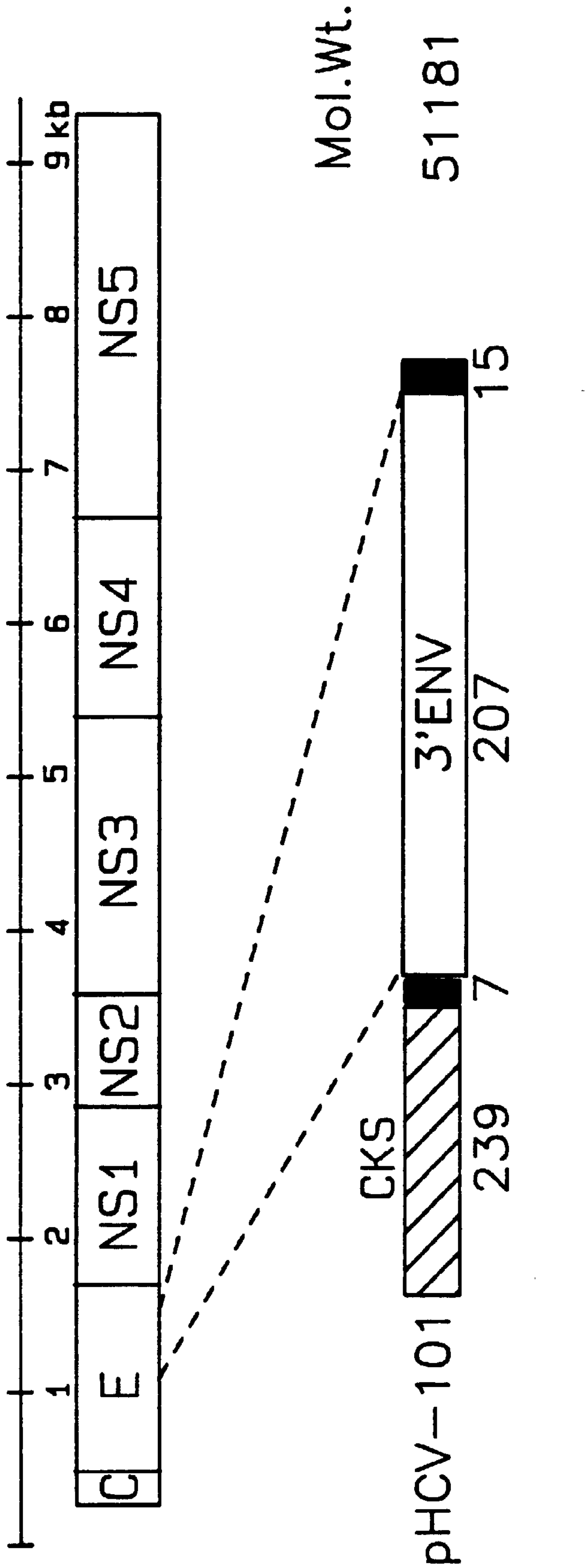
Mol.Wt.
51181
9 kb
8
7
6
5
4
3
2
1
C
E
NS1
NS2
NS3
NS4
NS5
15
3'ENV
207
7
CKS
239
pHCV—101

FIGURE 50

PHCV-101
Limits: 130 1533
Circular sequence with junction at 4663

```
                                    156                                183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                    210                                237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                    264                                291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                    318                                345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                    372                                399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                    426                                453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                    480                                507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                    534                                561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                    588                                615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                    642                                669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                    696                                723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 51

```
                                        750                                777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                        804                                831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                        858                                885
GAT ACC CCT GAA GAT CTC GAC CCG TCG ACT CGA ATT CTG CTT GTC GGG AGC GCC
Asp Thr Pro Glu Asp Leu Asp Pro Ser Thr Arg Ile Leu Leu Val Gly Ser Ala

                                        912                                939
ACC CTC TGC TCG GCC CTC TAT GTG GGG GAC TTG TGC GGG TCT GTC TTT CTT GTC
Thr Leu Cys Ser Ala Leu Tyr Val Gly Asp Leu Cys Gly Ser Val Phe Leu Val

                                        966                                993
GGT CAA CTG TTC ACT TTC TCC CCC AGG CAG CAC TGG ACA ACG CAA GAC TGC AAC
Gly Gln Leu Phe Thr Phe Ser Pro Arg Gln His Trp Thr Thr Gln Asp Cys Asn

                                       1020                               1047
TGT TCT ATC TAC CCC GGC CAC GTA ACG GGT CAC CGC ATG GCA TGG GAT ATG ATG
Cys Ser Ile Tyr Pro Gly His Val Thr Gly His Arg MET Ala Trp Asp MET MET

                                       1074                               1101
ATG AAC TGG TCC CCT ACG ACA GCG CTG GTA GTA GCT CAG CTG CTC AGG GTC CCG
MET Asn Trp Ser Pro Thr Thr Ala Leu Val Val Ala Gln Leu Leu Arg Val Pro

                                       1128                               1155
CAA GCC ATC TTG GAC ATG ATC GCT GGT GCC CAC TGG GGA GTC CTA GCG GGC ATA
Gln Ala Ile Leu Asp MET Ile Ala Gly Ala His Trp Gly Val Leu Ala Gly Ile

                                       1182                               1209
GCG TAT TTC TCC ATG GTG GGG AAC TGG GCG AAG GTC CTG GTA GTG CTG CTG CTA
Ala Tyr Phe Ser MET Val Gly Asn Trp Ala Lys Val Leu Val Val Leu Leu Leu

                                       1236                               1263
TTT GCC GGC GTT GAC GCG GAA ACC CAC GTC ACC GGG GGA AGT GCC GGC CAC ATT
Phe Ala Gly Val Asp Ala Glu Thr His Val Thr Gly Gly Ser Ala Gly His Ile

                                       1290                               1317
ACG GCT GGG CTT GTT CGT CTC CTT TCA CCA GGC GCC AAG CAG AAC ATC CAA CTG
Thr Ala Gly Leu Val Arg Leu Leu Ser Pro Gly Ala Lys Gln Asn Ile Gln Leu

                                       1344                               1371
ATC AAC ACC AAC GGC AGT TGG CAC ATC AAT AGC ACG GCC TTG AAC TGC AAT GAA
Ile Asn Thr Asn Gly Ser Trp His Ile Asn Ser Thr Ala Leu Asn Cys Asn Glu
```

FIGURE 51 (cont)

```
                                        1398                                         1425
AGC CTT AAC ACC GGC TGG TTA GCA GGG CTC TTC TAT CAC CAC AAA TTC AAC TCT
Ser Leu Asn Thr Gly Trp Leu Ala Gly Leu Phe Tyr His His Lys Phe Asn Ser

                                        1452                                         1479
TCA GGC TGT CCT GAG AGG GTT GCC AGC TGC CGT CGC CTT ACC GAT TTT GAC CAG
Ser Gly Cys Pro Glu Arg Val Ala Ser Cys Arg Arg Leu Thr Asp Phe Asp Gln

                                        1506                                    1533
GGC TGG GAA TTC GAG CTC GGT ACC CGG GGA TCC TCT AGA CTG CAG GCA TGC TAA
Gly Trp Glu Phe Glu Leu Gly Thr Arg Gly Ser Ser Arg Leu Gln Ala Cys  .
```

TRANSLATE:

FIGURE 51 (cont)

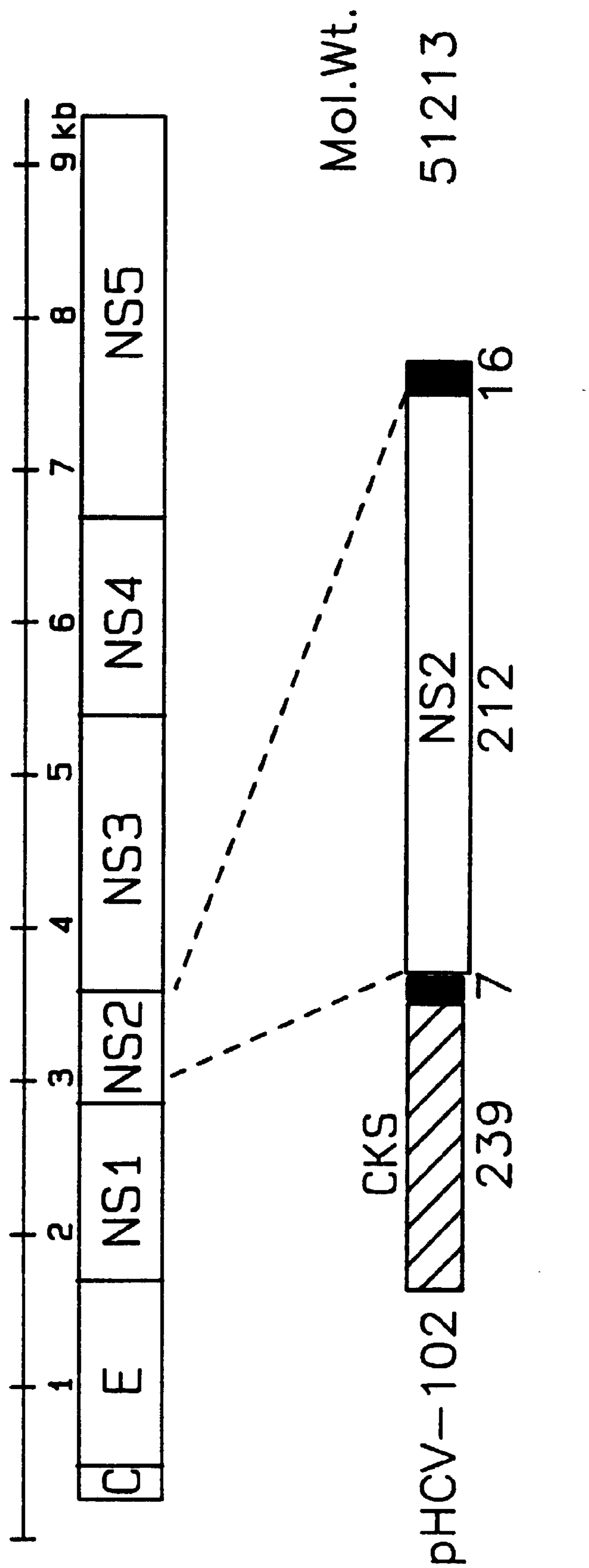
Mol.Wt.
51213
1
2
3
4
5
6
7
8
9 kb
C
E
NS1
NS2
NS3
NS4
NS5
pHCV—102
CKS
239
7
NS2
212
16

FIGURE 52

PHCV-102
Limits: 130 1554
Circular sequence with junction at 4681

```
                                156                                183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                534                                561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 53

```
                                750                                   777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                804                                   831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                858                                   885
GAT ACC CCT GAA GAT CTC GAC CCG TCG ACC GAA TTC GGT GAC ATC ATC AAC GGC
Asp Thr Pro Glu Asp Leu Asp Pro Ser Thr Glu Phe Gly Asp Ile Ile Asn Gly

                                912                                   939
TTG CCC GTC TCC GCC CGT AGG GGC CAG GAG ATA CTG CTC GGA CCA GCC GAC GGA
Leu Pro Val Ser Ala Arg Arg Gly Gln Glu Ile Leu Leu Gly Pro Ala Asp Gly

                                966                                   993
ATG GTC TCC AAG GGG TGG AGG TTG CTG GCG CCC ATC ACG GCG TAC GCC CAG CAG
MET Val Ser Lys Gly Trp Arg Leu Leu Ala Pro Ile Thr Ala Tyr Ala Gln Gln

                               1020                                  1047
ACA AGG GGC CTC CTA GGG TGT ATA ATC ACC AGC CTG ACT GGC CGG GAC AAA AAC
Thr Arg Gly Leu Leu Gly Cys Ile Ile Thr Ser Leu Thr Gly Arg Asp Lys Asn

                               1074                                  1101
CAA GCG GAG GGT GAG GTC CAG ATT GTG TCA ACT GCT GCC CAA ACT TTC CTG GCA
Gln Ala Glu Gly Glu Val Gln Ile Val Ser Thr Ala Ala Gln Thr Phe Leu Ala

                               1128                                  1155
ACG TGC ATC AAT GGG GTA TGC TGG ACT GTC TAC CAT GGG GCC GGA ACG AGG ACC
Thr Cys Ile Asn Gly Val Cys Trp Thr Val Tyr His Gly Ala Gly Thr Arg Thr

                               1182                                  1209
CTC GCA TCA CCC AAG GGT CCT GTT ATC CAG ATG TAT ACC AAT GTA GAC CAA GAC
Leu Ala Ser Pro Lys Gly Pro Val Ile Gln MET Tyr Thr Asn Val Asp Gln Asp

                               1236                                  1263
CTT GTG GGC TGG CCC GCT CCT CAA GGT GCC CGC TCA TTG ACA CCC TGC ACC TGC
Leu Val Gly Trp Pro Ala Pro Gln Gly Ala Arg Ser Leu Thr Pro Cys Thr Cys

                               1290                                  1317
GGC TCC TCG GAC CTT TAC CTG GTT ACG AGG CAC GCC GAT GTC ATT CCC GTG CGC
Gly Ser Ser Asp Leu Tyr Leu Val Thr Arg His Ala Asp Val Ile Pro Val Arg

                               1344                                  1371
CGG CGG GGT GAT AGC AGG GGC AGC CTG CTT TCG CCC CGG CCC ATT TCT TAT TTG
Arg Arg Gly Asp Ser Arg Gly Ser Leu Leu Ser Pro Arg Pro Ile Ser Tyr Leu
```

FIGURE 53 (cont)

```
                              1398                                               1425
AAA GGC TCC TCG GGG GGT CCG CTG TTG TGC CCC GCG GGA CAC GCC GTG GGC ATA
Lys Gly Ser Ser Gly Gly Pro Leu Leu Cys Pro Ala Gly His Ala Val Gly Ile

                              1452                                               1479
TTC AGG GCC GCG GTG TGT ACC CGT GGA GTG GCT AAG GCG GTG GAC TTT GTC CCC
Phe Arg Ala Ala Val Cys Thr Arg Gly Val Ala Lys Ala Val Asp Phe Val Pro

                              1506                                               1533
GTG GAG AAC CTC GAG ACA ACC ATG AAT TCG AGC TCG GTA CCC GGG GAT CCT CTA
Val Glu Asn Leu Glu Thr Thr MET Asn Ser Ser Ser Val Pro Gly Asp Pro Leu

GAC TGC AGG CAT GCT AAG TAA
Asp Cys Arg His Ala Lys  .

TRANSLATE:
```

FIGURE 53 (cont)

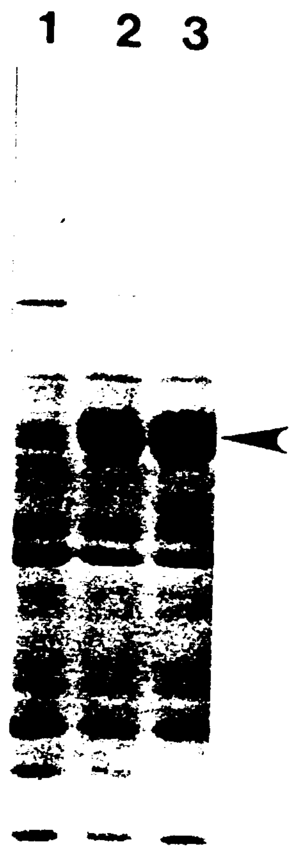


Figure 54

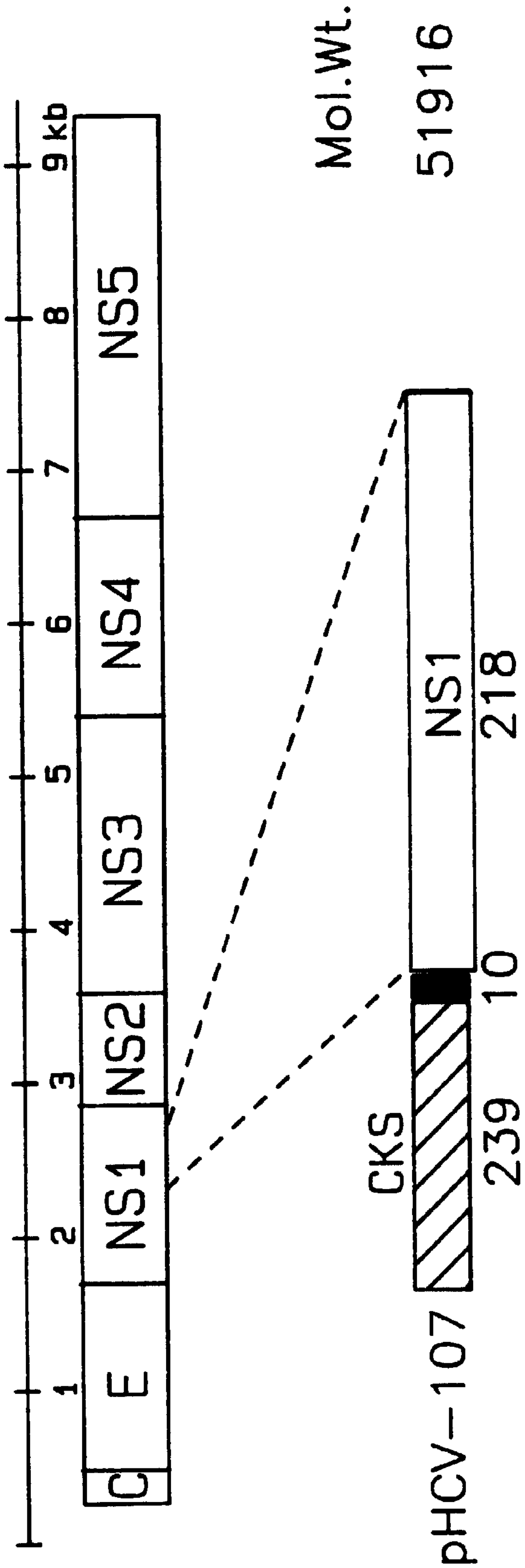
Mol.Wt.
51916
9 kb
8
7
6
5
4
3
2
1
C
E
NS1
NS2
NS3
NS4
NS5
NS1
218
10
CKS
239
pHCV−107

FIGURE 55

PHCV-107
Limits: 130 1533
Circular sequence with junction at 4689

```
                                156                                183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                210                                237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                264                                291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                318                                345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                372                                399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                426                                453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                480                                507
ATT CGT CAG GTT GCT GAT AAC CTC GCT CAG CGT CAG GTG GGT ATG ACG ACT CTG
Ile Arg Gln Val Ala Asp Asn Leu Ala Gln Arg Gln Val Gly MET Thr Thr Leu

                                534                                561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                588                                615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                642                                669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                696                                723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 56

```
                                750                                777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                804                                831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                858                                885
GAT ACC CCT GAA GAT CTC GAC CCG TCG ACG AAT TCC ACC ATG GGG CAT TAT CCT
Asp Thr Pro Glu Asp Leu Asp Pro Ser Thr Asn Ser Thr MET Gly His Tyr Pro

                                912                                939
TGT ACC ATC AAC TAC ACC CTG TTC AAA GTC AGG ATG TAC GTG GGA GGG GTC GAG
Cys Thr Ile Asn Tyr Thr Leu Phe Lys Val Arg MET Tyr Val Gly Gly Val Glu

                                966                                993
CAC AGG CTG GAA GTT GCT TGC AAC TGG ACG CGG GGC GAA CGT TGT GAT CTG GAC
His Arg Leu Glu Val Ala Cys Asn Trp Thr Arg Gly Glu Arg Cys Asp Leu Asp

                               1020                               1047
GAC AGG GAC AGG TCC GAG CTC AGC CCG CTG CTG CTG TCC ACC ACT CAG TGG CAG
Asp Arg Asp Arg Ser Glu Leu Ser Pro Leu Leu Leu Ser Thr Thr Gln Trp Gln

                               1074                               1101
GTC CTT CCG TGT TCC TTC ACG ACC TTG CCA GCC TTG ACC ACC GGC CTC ATC CAC
Val Leu Pro Cys Ser Phe Thr Thr Leu Pro Ala Leu Thr Thr Gly Leu Ile His

                               1128                               1155
CTC CAC CAG AAC ATC GTG GAC GTG CAA TAC TTG TAC GGG GTG GGG TCA AGC ATT
Leu His Gln Asn Ile Val Asp Val Gln Tyr Leu Tyr Gly Val Gly Ser Ser Ile

                               1182                               1209
GTG TCC TGG GCC ATC AAG TGG GAG TAC GTC ATC CTC TTG TTT CTC CTG CTT GCA
Val Ser Trp Ala Ile Lys Trp Glu Tyr Val Ile Leu Leu Phe Leu Leu Leu Ala

                               1236                               1263
GAC GCG CGC ATC TGC TCC TGC TTG TGG ATG ATG TTA CTC ATA TCC CAA GCG GAG
Asp Ala Arg Ile Cys Ser Cys Leu Trp MET MET Leu Leu Ile Ser Gln Ala Glu

                               1290                               1317
GCA GCC TTG GAA AAC CTT GTG TTA CTC AAT GCG GCG TCT CTG GCC GGG ACG CAC
Ala Ala Leu Glu Asn Leu Val Leu Leu Asn Ala Ala Ser Leu Ala Gly Thr His

                               1344                               1371
GGT CTT GTG TCC TTC CTC GTG TTT TTC TGC TTT GCA TGG TAT CTG AAG GGT AAG
Gly Leu Val Ser Phe Leu Val Phe Phe Cys Phe Ala Trp Tyr Leu Lys Gly Lys
```

FIGURE 56 (cont)

```
                              1398                                        1425
TGG GTG CCC GGA GTG GCC TAC GCC TTC TAC GGG ATG TGG CCT TTC CTC CTG CTC
Trp Val Pro Gly Val Ala Tyr Ala Phe Tyr Gly MET Trp Pro Phe Leu Leu Leu

                              1452                                        1479
CTG TTA GCG TTG CCC CAA CGG GCA TAC GCG CTG GAC ACG GAG ATG GCC GCG TCG
Leu Leu Ala Leu Pro Gln Arg Ala Tyr Ala Leu Asp Thr Glu MET Ala Ala Ser

                          1506                                        1533
TGT GGC GGC GTT GTT CTT GTC GGG TTA ATG GCG CTG ACT CTG TCA CCA TAT TAA
Cys Gly Gly Val Val Leu Val Gly Leu MET Ala Leu Thr Leu Ser Pro Tyr  .
```

TRANSLATE:

FIGURE 56 (cont)

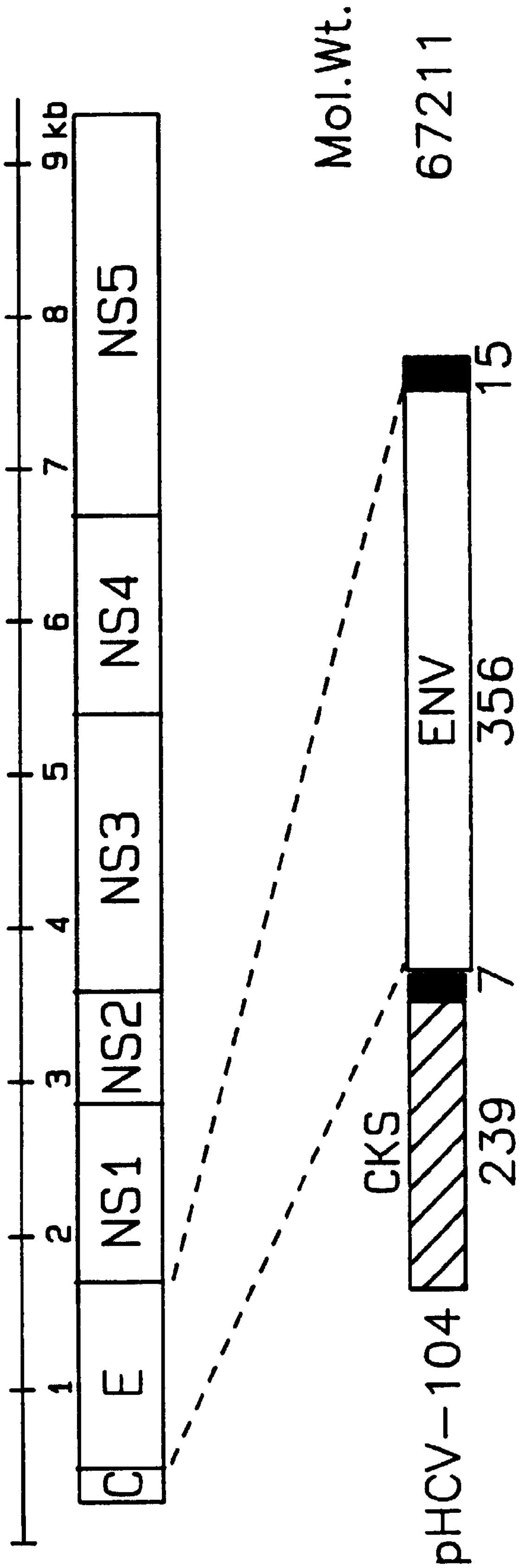
Mol.Wt.
67211
pHCV-104
CKS
239
7
ENV
356
15
C
E
NS1
NS2
NS3
NS4
NS5
1
2
3
4
5
6
7
8
9 kb

FIGURE 57

PHCV-104
Limits: 130 1983
Circular sequence with junction at 5113

```
                                        156                                  183
ATG AGT TTT GTG GTC ATT ATT CCC GCG CGC TAC GCG TCG ACG CGT CTG CCC GGT
MET Ser Phe Val Val Ile Ile Pro Ala Arg Tyr Ala Ser Thr Arg Leu Pro Gly

                                        210                                  237
AAA CCA TTG GTT GAT ATT AAC GGC AAA CCC ATG ATT GTT CAT GTT CTT GAA CGC
Lys Pro Leu Val Asp Ile Asn Gly Lys Pro MET Ile Val His Val Leu Glu Arg

                                        264                                  291
GCG CGT GAA TCA GGT GCC GAG CGC ATC ATC GTG GCA ACC GAT CAT GAG GAT GTT
Ala Arg Glu Ser Gly Ala Glu Arg Ile Ile Val Ala Thr Asp His Glu Asp Val

                                        318                                  345
GCC CGC GCC GTT GAA GCC GCT GGC GGT GAA GTA TGT ATG ACG CGC GCC GAT CAT
Ala Arg Ala Val Glu Ala Ala Gly Gly Glu Val Cys MET Thr Arg Ala Asp His

                                        372                                  399
CAG TCA GGA ACA GAA CGT CTG GCG GAA GTT GTC GAA AAA TGC GCA TTC AGC GAC
Gln Ser Gly Thr Glu Arg Leu Ala Glu Val Val Glu Lys Cys Ala Phe Ser Asp

                                        426                                  453
GAC ACG GTG ATC GTT AAT GTG CAG GGT GAT GAA CCG ATG ATC CCT GCG ACA ATC
Asp Thr Val Ile Val Asn Val Gln Gly Asp Glu Pro MET Ile Pro Ala Thr Ile

                                        480                                  507
ATT CGT CAG GTT GCT GAT AAC CTC GCT CAG CGT CAG GTG GGT ATG GCG ACT CTG
Ile Arg Gln Val Ala Asp Asn Leu Ala Gln Arg Gln Val Gly MET Ala Thr Leu

                                        534                                  561
GCG GTG CCA ATC CAC AAT GCG GAA GAA GCG TTT AAC CCG AAT GCG GTG AAA GTG
Ala Val Pro Ile His Asn Ala Glu Glu Ala Phe Asn Pro Asn Ala Val Lys Val

                                        588                                  615
GTT CTC GAC GCT GAA GGG TAT GCA CTG TAC TTC TCT CGC GCC ACC ATT CCT TGG
Val Leu Asp Ala Glu Gly Tyr Ala Leu Tyr Phe Ser Arg Ala Thr Ile Pro Trp

                                        642                                  669
GAT CGT GAT CGT TTT GCA GAA GGC CTT GAA ACC GTT GGC GAT AAC TTC CTG CGT
Asp Arg Asp Arg Phe Ala Glu Gly Leu Glu Thr Val Gly Asp Asn Phe Leu Arg

                                        696                                  723
CAT CTT GGT ATT TAT GGC TAC CGT GCA GGC TTT ATC CGT CGT TAC GTC AAC TGG
His Leu Gly Ile Tyr Gly Tyr Arg Ala Gly Phe Ile Arg Arg Tyr Val Asn Trp
```

FIGURE 58

```
                                        750                                  777
CAG CCA AGT CCG TTA GAA CAC ATC GAA ATG TTA GAG CAG CTT CGT GTT CTG TGG
Gln Pro Ser Pro Leu Glu His Ile Glu MET Leu Glu Gln Leu Arg Val Leu Trp

                                        804                                  831
TAC GGC GAA AAA ATC CAT GTT GCT GTT GCT CAG GAA GTT CCT GGC ACA GGT GTG
Tyr Gly Glu Lys Ile His Val Ala Val Ala Gln Glu Val Pro Gly Thr Gly Val

                                        858                                  885
GAT ACC CCT GAA GAT CTC GAC CCG TCG ACT CGA ATT CGT AGG TCG CGC AAT TTG
Asp Thr Pro Glu Asp Leu Asp Pro Ser Thr Arg Ile Arg Arg Ser Arg Asn Leu

                                        912                                  939
GGT AAG GTC ATC GAT ACC CTC ACG TGC GGC TTC GCC GAC CTC ATG GGG TAC ATT
Gly Lys Val Ile Asp Thr Leu Thr Cys Gly Phe Ala Asp Leu MET Gly Tyr Ile

                                        966                                  993
CCG CTC GTC GGC GCC CCT CTT GGA GGC GCT GCC AGG GCC CTG GCG CAT GGC GTC
Pro Leu Val Gly Ala Pro Leu Gly Gly Ala Ala Arg Ala Leu Ala His Gly Val

                                       1020                                 1047
CGG GTT CTG GAA GAC GGC GTG AAC TAT GCA ACA GGG AAC CTT CCC GGT TGC TCT
Arg Val Leu Glu Asp Gly Val Asn Tyr Ala Thr Gly Asn Leu Pro Gly Cys Ser

                                       1074                                 1101
TTC TCT ATC TTC CTT CTG GCC CTG CTC TCT TGC CTG ACT GTG CCC GCG TCA TCC
Phe Ser Ile Phe Leu Leu Ala Leu Leu Ser Cys Leu Thr Val Pro Ala Ser Ser

                                       1128                                 1155
TAC CAA GTA CGC AAC TCC TCG GGC CTT TAT CAT GTC ACC AAT GAT TGC CCC AAC
Tyr Gln Val Arg Asn Ser Ser Gly Leu Tyr His Val Thr Asn Asp Cys Pro Asn

                                       1182                                 1209
TCG AGC ATT GTG TAC GAG ACG GCC GAT ACC ATC CTA CAC TCT CCG GGG TGC GTC
Ser Ser Ile Val Tyr Glu Thr Ala Asp Thr Ile Leu His Ser Pro Gly Cys Val

                                       1236                                 1263
CCT TGC GTT CGC GAG GGC AAC ACC TCG AAA TGT TGG GTG GCG GTG GCC CCC ACA
Pro Cys Val Arg Glu Gly Asn Thr Ser Lys Cys Trp Val Ala Val Ala Pro Thr

                                       1290                                 1317
GTG GCC ACC AGG GAC GGC AAA CTC CCC TCA ACG CAG CTT CGA CGT CAC ATC GAT
Val Ala Thr Arg Asp Gly Lys Leu Pro Ser Thr Gln Leu Arg Arg His Ile Asp

                                       1344                                 1371
CTG CTC GTC GGG AGC GCC ACC CTC TGC TCG GCC CTC TAT GTG GGG GAC TTG TGC
Leu Leu Val Gly Ser Ala Thr Leu Cys Ser Ala Leu Tyr Val Gly Asp Leu Cys
```

FIGURE 58 (cont)

```
                                         1398                                          1425
GGG TCT GTC TTT CTT GTC AGT CAA CTG TTC ACC TTC TCC CCT AGG CGC CAT TGG
Gly Ser Val Phe Leu Val Ser Gln Leu Phe Thr Phe Ser Pro Arg Arg His Trp

                                         1452                                          1479
ACA ACG CAA GAC TGC AAC TGT TCT ATC TAC CCC GGC CAT ATA ACG GGT CAC CGC
Thr Thr Gln Asp Cys Asn Cys Ser Ile Tyr Pro Gly His Ile Thr Gly His Arg

                                         1506                                          1533
ATG GCA TGG GAT ATG ATG ATG AAC TGG TCC CCT ACA ACG GCG CTG GTA GTA GCT
MET Ala Trp Asp MET MET MET Asn Trp Ser Pro Thr Thr Ala Leu Val Val Ala

                                         1560                                          1587
CAG CTG CTC AGG GTC CCA CAA GCC ATC TTG GAC ATG ATC GCA GGT GCC CAC TGG
Gln Leu Leu Arg Val Pro Gln Ala Ile Leu Asp MET Ile Ala Gly Ala His Trp

                                         1614                                          1641
GGA GTC CTA GCG GGC ATA GCG TAT TTC TCC ATG GTG GGG AAC TGG GCG AAG GTC
Gly Val Leu Ala Gly Ile Ala Tyr Phe Ser MET Val Gly Asn Trp Ala Lys Val

                                         1668                                          1695
CTG GTA GTG CTG TTG CTG TTT TCC GGC GTC GAT GCG GCA ACC TAC ACC ACC GGG
Leu Val Val Leueu Leu Phe Ser Gly Val Asp Ala Ala Thr Tyr Thr Thr Gly

                                         1722                                          1749
GGG AGC GTT GCT AGG ACC ACG CAT GGA TTC TCC AGC TTA TTC AGT CAA GGC GCC
Gly Ser Val Ala Arg Thr Thr His Gly Phe Ser Ser Leu Phe Ser Gln Gly Ala

                                         1776                                          1803
AAG CAG AAC ATC CAG CTG ATT AAC ACC AAC GGC AGT TGG CAC ATC AAT CGC ACG
Lys Gln Asn Ile Gln Leu Ile Asn Thr Asn Gly Ser Trp His Ile Asn Arg Thr

                                         1830                                          1857
GCC TTG AAC TGT AAT GCG AGC CTC GAC ACT GGC TGG GTA GCG GGG CTC TTC TAT
Ala Leu Asn Cys Asn Ala Ser Leu Asp Thr Gly Trp Val Ala Gly Leu Phe Tyr

                                         1884                                          1911
TAC CAC AAA TTC AAC TCT TCA GGC TGC CCT GAG AGG ATG GCC AGC TGT AGA CCC
Tyr His Lys Phe Asn Ser Ser Gly Cys Pro Glu Arg MET Ala Ser Cys Arg Pro

                                         1938                                          1965
CTT GCC GAT TTT GAC CAG GGC TGG GAA TTC GAG CTC GGT ACC CGG GGA TCC TCT
Leu Ala Asp Phe Asp Gln Gly Trp Glu Phe Glu Leu Gly Thr Arg Gly Ser Ser

AGA CTG CAG GCA TGC TAA
Arg Leu Gln Ala Cys  .
```

FIGURE 58 (cont)